(19) Europäisches Patentamt
European Patent Office
Office européen des brevets

(11) EP 4 553 076 A1

(12) **EUROPEAN PATENT APPLICATION**
published in accordance with Art. 153(4) EPC

(43) Date of publication:
**14.05.2025 Bulletin 2025/20**

(21) Application number: **23834855.1**

(22) Date of filing: **04.07.2023**

(51) International Patent Classification (IPC):
***C07D 487/04*** (2006.01) ***C07D 519/00*** (2006.01)
***A61K 31/497*** (2006.01) ***A61K 31/5377*** (2006.01)
***A61K 31/4188*** (2006.01) ***A61K 31/454*** (2006.01)
***A61K 31/501*** (2006.01) ***A61K 31/437*** (2006.01)
***A61K 31/496*** (2006.01) ***A61P 19/02*** (2006.01)
***A61P 31/06*** (2006.01) ***A61P 37/00*** (2006.01)
***A61P 35/00*** (2006.01) ***A61P 17/04*** (2006.01)
***A61P 17/10*** (2006.01) ***A61P 17/14*** (2006.01)
***A61P 17/00*** (2006.01) ***A61P 25/28*** (2006.01)
***A61P 27/02*** (2006.01) ***A61P 3/10*** (2006.01)

(52) Cooperative Patent Classification (CPC):
**A61K 31/4188; A61K 31/437; A61K 31/454; A61K 31/496; A61K 31/497; A61K 31/501; A61K 31/5377; A61P 1/00; A61P 1/04; A61P 3/10; A61P 5/14; A61P 7/00; A61P 7/02; A61P 9/00; A61P 9/10;** (Cont.)

(86) International application number:
**PCT/CN2023/105745**

(87) International publication number:
**WO 2024/008088 (11.01.2024 Gazette 2024/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.07.2022 CN 202210777651**

(71) Applicant: **Medinno Pharmaceutical Technology (Zhuhai) Co., Ltd.**
**Zhuhai, Guangdong 519000 (CN)**

(72) Inventors:
- **LU, Liang**
  **Zhengzhou, Henan 450000 (CN)**
- **HUANG, Hai**
  **Zhengzhou, Henan 450000 (CN)**
- **ZHANG, Longzheng**
  **Zhengzhou, Henan 450000 (CN)**
- **ZHAO, Saisai**
  **Zhengzhou, Henan 450000 (CN)**
- **ZHANG, Jixuan**
  **Zhengzhou, Henan 450000 (CN)**

(74) Representative: **Kraus & Lederer PartGmbB**
**Thomas-Wimmer-Ring 15**
**80539 München (DE)**

# (54) COMPOUNDS AS TRK INHIBITORS AND/OR RET INHIBITORS AND USE THEREOF

(57) The present disclosure relates to a class of compound as a TRK-inhibiting medicine or RET-inhibiting medicine and uses thereof. Specifically, the present disclosure discloses use of a compound represented by formula (G), isotopically labeled compound thereof, or optical isomer thereof, geometric isomer thereof, a tautomer thereof or a mixture of various isomers, or a pharmaceutically acceptable salt thereof, or a prodrug thereof, or a metabolite thereof in the preparation of a TRK inhibiting medicine and/or a RET inhibiting medicine. The present disclosure also relates to the application of the compounds in medicine.

EP 4 553 076 A1

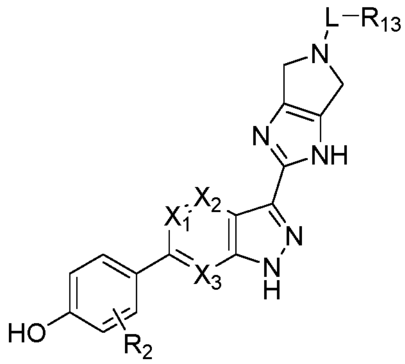

(G)

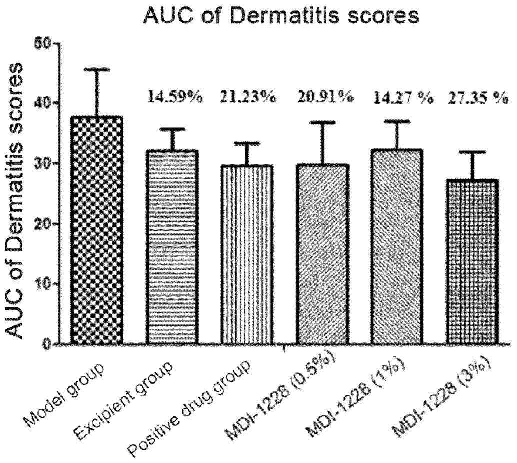


FIG. 23

(52) Cooperative Patent Classification (CPC): (Cont.)
**A61P 11/00; A61P 11/02; A61P 11/06; A61P 13/12; A61P 15/00; A61P 17/00; A61P 17/02; A61P 17/04; A61P 17/06; A61P 17/10; A61P 17/14; A61P 19/02; A61P 21/04; A61P 25/00; A61P 25/14; A61P 25/16; A61P 25/28; A61P 27/02; A61P 29/00; A61P 35/00; A61P 35/02; A61P 37/00; A61P 37/06; C07D 487/04; C07D 519/00**

## Description

### TECHNICAL FIELD

The present disclosure provides use of a class of compounds for TRK kinase and/or RET kinase. The present disclosure further relates to a composition comprising the compound, and use of the compound and the composition in the preparation of a medicament for the treatment and/or prevention of TRK and/or TRK-related diseases or disorders.

### BACKGROUND

Protein kinases are a family of enzymes that catalyze phosphorylation of specific residues in proteins, and are broadly classified into tyrosine and serine/threonine kinases. Inappropriate kinase activities caused by mutations, overexpression or inappropriate regulation, abnormal regulation or dysregulation, and excessive or insufficient production of growth factors or cytokines are involved in many diseases, including but not limited to cancers, cardiovascular diseases, allergies, asthma and other respiratory diseases, autoimmune diseases, inflammatory diseases, bone diseases, metabolic disorders and neurological and neurodegenerative disorders (such as Alzheimer's disease). Inappropriate kinase activity triggers a variety of biological cell responses associated with cell growth, cell differentiation, cell function, survival, apoptosis, and cell motility related to the aforementioned diseases and other related diseases. Therefore, protein kinases have become an important class of enzymes as targets for therapeutic intervention.

TRK kinase is a high-affinity receptor tyrosine kinase activated by a group of soluble growth factors known as neurotrophic factors (NT). The TRK family consists of three members: TRKA, TRKB, and TRKC in which TRKA is activated by nerve growth factor (NGF), TRKB is activated by brain-derived neurotrophic factor (BDNF) and NT-4/5, and TRKC is activated by NT3. TRK is widely expressed in neuronal tissues and is involved in the maintenance, signaling, and survival of neuronal cells.

It has been confirmed that inhibitors targeting the TRK/neurotrophic factor pathway are effective in numerous preclinical animal models of pain. It has also been shown that NGF secreted by tumor cells and tumor-infiltrating macrophages directly stimulates peripheral pain fibers, so inhibitors of TrkA and/or other Trk kinases may provide an effective treatment for chronic pain states and cancer-related pain. It has also been reported that the overexpression, activation, amplification, and/or mutation of Trk kinases are associated with many cancers, such as neuroblastoma, colorectal cancer, melanoma, gastric cancer, lung cancer, breast cancer, etc. In 2018, the FDA of USA officially approved the first oral TRK inhibitor, Vitrakvi (also known as larotrectinib, LOXO101) produced by Loxo Oncology, for the treatment of patients with solid tumors harboring neurotrophic tyrosine kinase (NTRK) gene fusions, which has been proven to have a high response rate in treating various cancers. It has also been found that TRK inhibitors can be used to treat inflammatory diseases and autoimmune diseases.

In addition, RET kinase is also a very important drug target. In May 2020, the FDA approved the world's first RET kinase inhibitor, Selpercatinib (Retevmo) developed by Loxo Oncology, for the treatment of non-small cell lung cancer, medullary thyroid cancer, and other types of thyroid cancer. Later, in September 2020, the FDA approved another RET kinase inhibitor, Pralsetinib developed by Blueprint Medicines, for the treatment of adult patients with RET fusion-positive metastatic non-small cell lung cancer.

Although some TRK or RET inhibitors have been approved for listing, and a large number of TRK or RET inhibitors are still in clinical research, these TRK/RET inhibitors are not satisfactory in terms of efficacy or safety. Therefore, there is always a need for TRK inhibitors or RET inhibitors with better efficacy and/or fewer side effects.

### SUMMARY

It is one object of the present disclosure to provide a novel TRK inhibitor alternative to existing TRK inhibitors, so as to provide more options for the treatment of JAK-related diseases.

A further object of the present disclosure is to provide a novel TRK inhibitor with better efficacy and/or better safety than existing TRK inhibitors.

It is one object of the present disclosure to provide a novel RET inhibitor alternative to existing TRK inhibitors, so as to provide more options for the treatment of RET-related diseases.

A further object of the present disclosure is to provide a novel RET inhibitor with better efficacy and/or better safety than existing RET inhibitors.

It is another object of the present invention is to provide an alternative or more effective therapy for diseases associated with TRK and/or RET, such as itching, psoriasis, atopic dermatitis, acne, vitiligo, alopecia areata, asthma, rhinitis, hemorrhoids, cervicitis, pneumonia, cancer (tumor), etc., thereby providing more options for the treatment of these diseases. The Chinese invention patent CN111606908B (equivalent to U.S. Patent Publication US2022/0073524A1) discloses a class of compounds as pan-JAK inhibitors. JAK is an abbreviation for Janus kinase, a cytoplasmic tyrosine

kinase that transduces cytokine signaling from membrane receptors to STAT transcription factors. JAK is a very important drug target, involving numerous significant biological processes such as cell proliferation, differentiation, apoptosis, and immune regulation. JAK inhibitors developed for this target are mainly used to treat blood system diseases, tumors, rheumatoid arthritis, and other disorders. The JAK protein family includes four members: JAK1, JAK2, JAK3, and TYK2. From the disclosure of CN111606908B, the disclosed compounds have high inhibitory activity against JAK1, JAK2, JAK3, TYK2, and are potent pan-JAK inhibitors.

Unexpectedly, it was found by the inventors that the compounds disclosed in CN111606908B also have very high inhibitory activity against TRK kinases, and have very good inhibitory activity against TRKA, TRKB, and TRKC, thus being a class of pan-TRK inhibitors.

Furthermore, it was also unexpectedly found by the inventors that the compounds disclosed in CN111606908B have very high inhibitory activity against RET kinases.

It has been reported that Pegcantratinib (SNA-125) developed by Sienna Pharm is capable of inhibiting both JAK3 and TRKA, but has poor inhibitory effects on other members of the JAK and TRK kinase families and ultimately failed in *clinical* trials for psoriasis. To the best of the inventors' knowledge, no compounds have been reported to date that have broad-spectrum and potent inhibitory activity against both JAK and TRK kinases, and the compounds according to the pesent application are the first reported potent pan-JAK/pan-TRK inhibitors.

Due to the dual or multiple broad-spectrum inhibitory activity against JAK, TRK, and RET, the compounds described in the prsent application can provide better efficacy than existing drugs for the treatment of numerous diseases and can treat acne, a side effect caused by other JAK inhibitors *(*Int. J. Dermatol. 2021 Aug 22. doi: 10.1111/ijd.15853. PMID: 34423443). It has been found that the compound according to the pesent application are particularly suitable for the treatment of autoimmune diseases, especially skin autoimmune diseases. In addition, through animal models, the therapeutic effects of the compounds involved in the present application on itching, psoriasis, atopic dermatitis, skin side effects caused by EGFR inhibitors (such as those diseases listed in CN112933095A), acne, vitiligo, alopecia areata, asthma, rhinitis, hemorrhoids, cervicitis, pneumonia, (diabetes-induced) slow wound healing, diabetes, and diabetes complications (such as diabetic foot) have been confirmed.

Surprisingly, it was further found by the inventors that the compounds disclosed in CN111606908B have a very good therapeutic effect on diabetic foot and chronic wound healing, including bedsores. As a common complication of diabetes, diabetic foot affects nearly 400 million people worldwide. Due to the difficulty of wound healing, about 30% of the patients will eventually undergo amputation. Currently, there is a lack of effective drugs for the treatment of diabetic foot. Recent studies have shown that wound healing and skin repair require adipocytes to undergo lipolysis and convert into myofibroblasts, and patients with diabetic foot lack this ability (Cell Stem Cell 26, 1-16, June 4, 2020). Surprisingly, the inventors found that the compounds disclosed in CN111606908B can effectively promote adipocyte lipolysis in vitro, while other JAK inhibitors such as Tofacitinib have no similar effect, and there have been no reports indicating that JAK inhibitors can promote adipocyte lipolysis. The inventors further found that the compounds disclosed in CN111606908B can significantly increase the number of myofibroblasts at the wound site in diabetic animals and promote wound healing in diabetic animals. Given that conventional JAK inhibitors do not have the effect of promoting lipolysis in adipocytes, the inventor believes that the efficacy of the compounds disclosed in CN111606908B in diabetic foot animals stems from their dual inhibitory activity against pan-JAK/pan-TRK, or multiple inhibitory activity against pan-JAK/pan-TRK/RET. Based on this, the inventor believes that: JAK/TRK dual inhibitors (preferably pan-JAK/pan-TRK dual inhibitors), or JAK/TRK/RET multiple inhibitors (preferably pan-JAK/pan-TRK/RET multiple inhibitors) would have a good effect on the healing of chronic wounds, including but not limited to diabetic foot and bedsores.

In a first aspect, the present disclosure provides a compound of Formula (G)

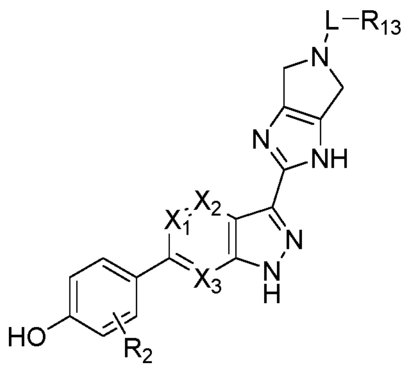

(G)

or an isotopically labeled compound thereof, or an optical isomer thereof, a geometric isomer thereof, a tautomer thereof or a mixture of various isomers, or a pharmaceutically acceptable salt thereof, or a prodrug thereof, or a

metabolite thereof, for use as a TRK inhibitor and/or RET inhibitor, or for use in the preparation of a TRK-inhibiting medicine and/or RET-inhibiting medicine, wherein

L is C=O, O=S=O, $CH_2$ or a bond; and

$X_1$ is N or $CR_{14}$; and

$X_2$ is N or $CR_{15}$; and

$X_3$ is N or $CR_{16}$; and

$R_{14}$, $R_{15}$, $R_{16}$ are each independently selected from H, -OH, -SH, -CN, halogen, $-NO_2$, $-SF_5$, -S-$C_{1-4}$ alkyl, $C_{1-6}$ alkyl, $C_{2-8}$ alkenyl, $C_{2-8}$ alkynyl, $C_{1-6}$ alkoxy, $C_{3-7}$ cycloalkyl, 3-7 membered heterocycloalkyl, $C_{5-7}$ aryl, 5-7 membered heteroaryl, -N($R_9$)($R_{10}$), -N($R_{11}$)(C(= O)$R_{12}$), -C(=O)-N($R_9$)($R_{10}$), -C(=O)-$R_{12}$, -C(=O)-O$R_{12}$, -OC(=O)$R_{12}$, -N($R_{11}$)(S(= O)$_2R_{12}$), -S(=O)$_2$-N($R_9$)($R_{10}$), -S$R_{12}$ and -O$R_{12}$, in which the -S-$C_{1-4}$ alkyl, $C_{1-6}$ alkyl, $C_{1-6}$ alkoxy, $C_{3-7}$ cycloalkyl, and 3-7 membered heterocycloalkyl are optionally substituted with 1, 2 or 3 substitutes selected from halogen, -OH, $-NH_2$, $-NH(CH_3)$, $-N(CH_3)_2$, -CN, $C_{1-4}$ alkyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, -C(=O)-$NH_2$, -C(=O)-N($C_{1-4}$ alkyl)$_2$, -N($C_{1-4}$ alkyl)(C(=O) $C_{1-4}$ alkyl), $C_{1-4}$ haloalkyl, $C_{1-4}$ alkoxy and $C_{1-4}$ haloalkoxy; and

$R_{13}$ is H, -N($R_{17}$)($R_{18}$), $C_{1-6}$ alkoxy, -S$R_{12}$, -O$R_{12}$, -CN, halogen, $-NO_2$, $-SF_5$, -S-$C_{1-4}$ alkyl, $C_{1-6}$ alkyl or $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicycloalkyl, or 5-11 membered bicyclic heteroalkyl, and $R_{13}$ is substituted with 0, 1, 2, 3 or 4 $R_1$(s), in which $R_{17}$ and $R_{18}$ are each independently selected from H, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, $C_{3-7}$ heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicycloalkyl, and 5-11 membered bicyclic heteroalkyl and are optionally substituted with one or more substitutes each independently selected from -OH, - CN, -SH, halogen, $-NO_2$, $-SF_5$, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, -N($R_9$)($R_{10}$), - N($R_{11}$)(C(=O)$R_{12}$), -C(=O)-N($R_9$)($R_{10}$), -C(=O)-$R_{12}$, -C(=O)-O$R_{12}$, -OC(=O)$R_{12}$, -N($R_{11}$)(S(=O)$_2R_{12}$), -S(=O)$_2$-N($R_9$)($R_{10}$), -S$R_{12}$ and -O$R_{12}$, in which the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2 or 3 substitutes each independently selected from halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), -C(=O)-O$R_{12}$, -C(=O)H, -C(=O)$R_{12}$, -C(= O)-N($R_9$)($R_{10}$), -N($R_{11}$)(S(=O)$_2R_{12}$), -S(=O)$_2$-N($R_9$)($R_{10}$), -S$R_{12}$ and -O$R_{12}$; or $R_{17}$, $R_{18}$ and the N atom connected thereto together form a 3-14 membered ring; and

0, 1, 2, 3 or 4 $R_2$(s) are present in formula (G), and $R_2$ is selected from H, halogen, - OH, $-NO_2$, -CN, $-SF_5$, -SH, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, -N($R_9$)($R_{10}$), -N($R_{11}$)(C(= O)$R_{12}$), -C(=O)-N($R_9$)($R_{10}$), -C(=O)-$R_{12}$, -C(=O)-O$R_{12}$, -OC(=O)$R_{12}$, -N($R_{11}$)(S(= O)$_2R_{12}$), -S(=O)$_2$-N($R_9$)($R_{10}$), -S$R_{12}$ and -O$R_{12}$, in which the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_0$)($R_{10}$), -N($R_{11}$)(C(=O)$R_{12}$), - C(=O)-O$R_{12}$, -C(=O)H, -C(=O)$R_{12}$, -C(=O)-N($R_9$)($R_{10}$), -N($R_{11}$)(S(=O)$_2R_{12}$), -S(=O)$_2$-N($R_9$)($R_{10}$), -S$R_{12}$ and -O$R_{12}$; and

$R_1$ is selected from H, halogen, -OH, $-NO_2$, -CN, $-SF_5$, -SH, -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$bicycloalkyl, 5-11 membered bicyclic heteroalkyl, -N($R_9$)($R_{10}$), -N($R_{11}$)(C(=O)$R_{12}$), -C(=O)-N($R_9$)($R_{10}$), -C(=O)-$R_{12}$, -C(=O)-O$R_{12}$, -OC(=O)$R_{12}$, - N($R_{11}$)(S(=O)$_2R_{12}$), -S(=O)$_2$-N($R_9$)($R_{10}$), -S$R_{12}$ and -O$R_{12}$, in which the -S-$C_{1-4}$ alkyl, $C_{1-8}$ alkyl, $C_{2-8}$ alkenyl, $C_{2-8}$ alkynyl, and $C_{1-8}$ alkoxy are optionally substituted with 1, 2, 3, or 4 $R_3$(s), and in which the $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2, 3, or 4 $R_4$(s); and

$R_3$ and $R_4$ are each independently selected from H, halogen, -OH, $-NO_2$, -CN, $-SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, $-N(R_5)(R_6)$, $-N(R_{11})(C(=O)R_{12})$, $-CON(R_7)(R_8)$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2, 3 or 4 substituent(s) each independently selected from the group consisting of halogen, -CN, -OH, $C_{1-4}$ alkyl, $C_{1-6}$ alkoxy, $C_{1-4}$ haloalkyl, $C_{1-4}$ haloalkoxy, $C_{3-6}$ cycloalkyl, $-N(R_0)(R_{10})$, $-N(R_{11})(C(=O)R_{12})$, $-C(=O)-OR_{12}$, -C(=O)H, $-C(=O)R_{12}$, - $C(=O)-N(R_9)(R_{10})$, $-N(R_{11})(S(=O)_2R_{12})$, $-S(=O)_2-N(R_9)(R_{10})$, $-SR_{12}$ and $-OR_{12}$; and $R_5$, $R_6$, $R_7$, $R_8$, $R_9$, $R_{10}$, $R_{11}$, and $R_{12}$ are each independently H or selected from the group consisting of $C_{1-6}$ alkyl, $C_{1-4}$ haloalkyl, $C_{3-7}$ cycloalkyl, 4-14 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein the options included in the above group are each optionally substituted with 1, 2, 3 or 4 substituent(s) each independently selected from the group consisting of halogen, $-CF_3$, -OH, $-NH_2$, $-NH(CH_3)$, $-N(CH_3)_2$, -CN, oxo, $C_{1-4}$ alkyl, $C_{2-6}$ alkenyl, $C_{2-6}$ alkynyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, $-C(= O)-NH_2$, -C(=O)-N($C_{1-4}$ alkyl)$_2$, $C_{1-4}$ haloalkyl, $C_{1-4}$ alkoxy and $C_{1-4}$ haloalkoxy.

In some preferred embodiments of the present disclosure, an isotopically labeled compound of the above-mentioned compound of formula (G) is used. In some more preferred embodiments of the present disclosure, an isotopically labeled compound of the compound of formula (G) is used, wherein all Hs are each independently and optionally substituted with D.

In some preferred embodiments of the present disclosure, in formula (G), $X_1$ is N. In some preferred embodiments of the present disclosure, in formula (G), $X_2$ is N. In some preferred embodiments of the present disclosure, in formula (G), $X_3$ is N. In some preferred embodiments of the present disclosure, in formula (G), $X_1$ is $CR_{14}$, $X_2$ is N or $CR_{15}$, and $X_3$ is $CR_{16}$. In some preferred embodiments of the present disclosure, in formula (G), $X_1$ is $CR_{14}$, $X_2$ is $CR_{15}$, and $X_3$ is $CR_{16}$. In some preferred embodiments of the present disclosure, in formula (G), $X_1$ is $CR_{14}$, $X_2$ is $CR_{15}$, $X_3$ is $CR_{16}$, and $R_{14}$, $R_{15}$, and $R_{16}$ are each independently selected from H, -OH, -CN, halogen, $C_{1-6}$ alkyl, $C_{1-6}$ alkoxy, $C_{3-7}$ cycloalkyl, and 3-7 membered heterocycloalkyl. In some preferred embodiments of the present disclosure, in formula (G), $X_1$ is $CR_{14}$, $X_2$ is N, $X_3$ is $CR_{16}$, and $R_{14}$ and $R_{16}$ are each independently selected from H, -OH, -CN, halogen, $C_{1-6}$ alkyl, $C_{1-6}$ alkoxy, $C_{3-7}$ cycloalkyl, and 3-7 membered heterocycloalkyl. In some preferred embodiments of the present disclosure, in formula (G), $X_1$, $X_2$, and $X_3$ are the same. In some preferred embodiments of the present disclosure, in formula (G), $X_1$, $X_2$ and $X_3$ are CH. In some preferred embodiments of the present disclosure, in formula (G), $X_1$, $X_2$ and $X_3$ are N. In some preferred embodiments of the present disclosure, in formula (G), $X_1$ is $C(CH_3)$, $X_2$ and $X_3$ are CH. In some preferred embodiments of the present disclosure, in formula (G), $X_2$ is $C(CH_3)$, $X_1$ and $X_3$ are CH. In some preferred embodiments of the present disclosure, in formula (G), $X_3$ is $C(CH_3)$, $X_1$ and $X_2$ are CH. In some preferred embodiments of the present disclosure, in formula (G), $X_1$ is N, $X_2$ and $X_3$ are CH. In some preferred embodiments of the present disclosure, in formula (G), $X_2$ is N, $X_1$ and $X_3$ are CH. In some preferred embodiments of the present disclosure, in formula (G), $X_3$ is N, $X_1$ and $X_2$ are CH.

In some more preferred embodiments of the present disclosure, an isotopically labeled compound of the compound of formula (G) is used, wherein all H are each independently and optionally substituted with D, and $X_1$, $X_2$ and $X_3$ are the same. In some more preferred embodiments of the present disclosure, an isotopically labeled compound of the compound of formula (G) is used, wherein all Hs are each independently and optionally substituted with D, and $X_1$, $X_2$ and $X_3$ are all CH. In some more preferred embodiments of the present disclosure, an isotopically labeled compound of the compound of formula (G) is used, wherein all Hs are each independently and optionally substituted with D, and $X_1$, $X_2$ and $X_3$ are N. In some more preferred embodiments of the present disclosure, an isotopically labeled compound of the compound of formula (G) is used, wherein all H are each independently and optionally substituted with D, and $X_1$ is $C(CH_3)$, $X_2$ and $X_3$ are both CH. In some more preferred embodiments of the present disclosure, an isotopically labeled compound of the compound of formula (G) is used, wherein all Hs are each independently and optionally substituted with D, and $X_2$ is $C(CH_3)$, $X_1$ and $X_3$ are both CH. In some more preferred embodiments of the present disclosure, an isotopically labeled compound of the compound of formula (G) is used, wherein all Hs are each independently and optionally substituted with D, and $X_3$ is $C(CH_3)$, and $X_1$ and $X_2$ are both CH. In some more preferred embodiments of the present disclosure, an isotopically labeled compound of the compound of formula (G) is used, wherein all Hs are each independently and optionally substituted with D, and $X_1$ is N, $X_2$ and $X_3$ are both CH. In some more preferred embodiments of the present disclosure, an isotopically labeled compound of the compound of formula (G) is used, wherein all Hs are each independently and optionally substituted with D, and $X_2$ is N, $X_1$ and $X_3$ are both CH. In some more preferred embodiments of the present disclosure, an isotopically labeled compound of the compound of formula (G) is used, wherein all Hs are each independently and optionally substituted with D, and $X_3$ is N, and $X_1$ and $X_2$ are both CH.

In some preferred embodiments of the present disclosure, in formula (G), L is C=O, O=S=O or $CH_2$. In some particularly preferred embodiments of the present disclosure, in formula (G), L is C=O. In some particularly preferred

embodiments of the present disclosure, in formula (G), L is O=S=O. In some particularly preferred embodiments of the present disclosure, in formula (G), L is $CH_2$. In other embodiments of the present disclosure, in formula (G), L is a bond.

In some particularly preferred embodiments of the present disclosure, in formula (G), $X_1$, $X_2$ and $X_3$ are all CH, and L is C=O.

In some particularly preferred embodiments of the present disclosure, in formula (G), $X_1$, $X_2$ and $X_3$ are all CH, and L is O=S=O.

In some particularly preferred embodiments of the present disclosure, in formula (G), $X_1$, $X_2$ and $X_3$ are all CH, and L is $CH_2$.

In some particularly preferred embodiments of the present disclosure, in formula (G), $X_1$, $X_2$ and $X_3$ are all CH, and L is a bond.

In some particularly preferred embodiments of the present disclosure, in formula (G), $X_1$, $X_2$ and $X_3$ are all N, and L is C=O.

In some particularly preferred embodiments of the present disclosure, in formula (G), $X_1$, $X_2$ and $X_3$ are all N, and L is O=S=O.

In some particularly preferred embodiments of the present disclosure, in formula (G), $X_1$, $X_2$ and $X_3$ are all N, and L is $CH_2$.

In some particularly preferred embodiments of the present disclosure, in formula (G), $X_1$, $X_2$ and $X_3$ are all N, and L is a bond.

In some particularly preferred embodiments of the present disclosure, in formula (G), $X_1$, $X_2$ and $X_3$ are all $CR_{14}$, wherein $R_{14}$ is selected from -OH, -CN, halogen, $C_{1-6}$ alkyl, $C_{1-6}$ alkoxy, $C_{3-7}$ cycloalkyl, and 3-7 membered heterocycloalkyl, and L is C=O.

In some particularly preferred embodiments of the present disclosure, in formula (G), $X_1$, $X_2$ and $X_3$ are all $CR_{14}$, wherein $R_{14}$ is selected from -OH, -CN, halogen, $C_{1-6}$ alkyl, $C_{1-6}$ alkoxy, $C_{3-7}$ cycloalkyl, and 3-7 membered heterocycloalkyl, and L is O=S=O.

In some particularly preferred embodiments of the present disclosure, in formula (G), $X_1$, $X_2$ and $X_3$ are all $CR_{14}$, wherein $R_{14}$ is selected from -OH, -CN, halogen, $C_{1-6}$ alkyl, $C_{1-6}$ alkoxy, $C_{3-7}$ cycloalkyl, and 3-7 membered heterocycloalkyl, and L is $CH_2$.

In some particularly preferred embodiments of the present disclosure, in formula (G), $X_1$, $X_2$ and $X_3$ are all $CR_{14}$, wherein $R_{14}$ is selected from -OH, -CN, halogen, $C_{1-6}$ alkyl, $C_{1-6}$ alkoxy, $C_{3-7}$ cycloalkyl, and 3-7 membered heterocycloalkyl, and L is a bond.

In some particularly preferred embodiments of the present disclosure, in formula (G), $X_1$ is $C(CH_3)$, $X_2$ and $X_3$ are both CH, and L is C=O.

In some particularly preferred embodiments of the present disclosure, in formula (G), $X_1$ is $C(CH_3)$, $X_2$ and $X_3$ are both CH, and L is O=S=O.

In some particularly preferred embodiments of the present disclosure, in formula (G), $X_1$ is $C(CH_3)$, $X_2$ and $X_3$ are both CH, and L is $CH_2$.

In some particularly preferred embodiments of the present disclosure, in formula (G), $X_1$ is $C(CH_3)$, $X_2$ and $X_3$ are both CH, and L is a bond.

In some particularly preferred embodiments of the present disclosure, in formula (G), $X_2$ is $C(CH_3)$, $X_1$ and $X_3$ are both CH, and L is C=O.

In some particularly preferred embodiments of the present disclosure, in formula (G), $X_2$ is $C(CH_3)$, $X_1$ and $X_3$ are both CH, and L is O=S=O.

In some particularly preferred embodiments of the present disclosure, in formula (G), $X_2$ is $C(CH_3)$, $X_1$ and $X_3$ are both CH, and L is $CH_2$.

In some particularly preferred embodiments of the present disclosure, in formula (G), $X_2$ is $C(CH_3)$, $X_1$ and $X_3$ are both CH, and L is a bond.

In some particularly preferred embodiments of the present disclosure, in formula (G), $X_3$is $C(CH_3)$, $X_1$ and $X_2$ are both CH, and L is C=O.

In some particularly preferred embodiments of the present disclosure, in formula (G), $X_3$ is $C(CH_3)$, $X_1$ and $X_2$ are both CH, and L is O=S=O.

In some particularly preferred embodiments of the present disclosure, in formula (G), $X_3$ is $C(CH_3)$, $X_1$ and $X_2$ are both CH, and L is $CH_2$.

In some particularly preferred embodiments of the present disclosure, in formula (G), $X_3$ is $C(CH_3)$, $X_1$ and $X_2$ are both CH, and L is a bond.

In some particularly preferred embodiments of the present disclosure, in formula (G), $X_1$ is N, $X_2$ and $X_3$ are both CH, and L is C=O.

In some particularly preferred embodiments of the present disclosure, in formula (G), $X_1$ is N, $X_2$ and $X_3$ are both CH, and L is O=S=O.

In some particularly preferred embodiments of the present disclosure, in formula (G), $X_1$ is N, $X_2$ and $X_3$ are both CH, and L is $CH_2$.

In some particularly preferred embodiments of the present disclosure, in formula (G), $X_1$ is N, $X_2$ and $X_3$ are both CH, and L is a bond.

In some particularly preferred embodiments of the present disclosure, in formula (G), $X_2$ is N, $X_1$ and $X_3$ are both CH, and L is C=O.

In some particularly preferred embodiments of the present disclosure, in formula (G), $X_2$ is N, $X_1$ and $X_3$ are both CH, and L is O=S=O.

In some particularly preferred embodiments of the present disclosure, in formula (G), $X_2$ is N, $X_1$ and $X_3$ are both CH, and L is $CH_2$.

In some particularly preferred embodiments of the present disclosure, in formula (G), $X_2$ is N, $X_1$ and $X_3$ are both CH, and L is a bond.

In some particularly preferred embodiments of the present disclosure, in formula (G), $X_3$ is N, $X_1$ and $X_2$ are both CH, and L is C=O.

In some particularly preferred embodiments of the present disclosure, in formula (G), $X_3$ is N, $X_1$ and $X_2$ are both CH, and L is O=S=O.

In some particularly preferred embodiments of the present disclosure, in formula (G), $X_3$ is N, $X_1$ and $X_2$ are both CH, and L is $CH_2$.

In some particularly preferred embodiments of the present disclosure, in formula (G), $X_3$ is N, $X_1$ and $X_2$ are both CH, and L is a bond.

In some preferred embodiments of the present disclosure, in formula (G), $R_{13}$ is H, - $N(R_{17})(R_{18})$, $C_{1-6}$ alkoxy, -OH, -SH, -CN, halogen, $-NO_2$, $-SF_5$, $-S-C_{1-4}$ alkyl, $C_{1-6}$ alkyl, or $C_{3-7}$ cycloalkyl, 3-7 membered heterocycloalkyl, $C_{5-7}$ aryl, 5-7 membered heteroaryl, $C_{7-11}$ bicyclic aryl, 7-11membered bicyclic heteroaryl, 11-15 membered tricyclyl, $C_{5-11}$bicycloalkyl, or 5-11 membered bicyclic heteroalkyl, in which $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of -OH, -CN, -SH, halogen, $-NO_2$,-and $SF_5$, wherein $R_{13}$ is optionally substituted with 1, 2, 3 or 4 $R_1$(s). In some preferred embodiments of the present disclosure, in formula (G), $R_{13}$ is H, $-N(R_{17})(R_{18})$, $C_{1-6}$ alkoxy, -OH, -SH, -CN, halogen, $-NO_2$, $-SF_5$, $-S-C_{1-4}$ alkyl, $C_{1-6}$ alkyl, or $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, or 11-15 membered tricyclyl and $R_{17}$ and $R_{18}$ are defined as above, wherein $R_{13}$ is optionally substituted with 1, 2, 3 or 4 $R_1$(s). In some preferred embodiments of the present disclosure, in formula (G), $R_{13}$ is H, $-N(R_{17})(R_{18})$, $C_{1-6}$ alkoxy, $C_{1-6}$ alkyl, or $C_{3-7}$ cycloalkyl, 3-7 membered heterocycloalkyl, $C_{5-7}$ aryl, or 5-7 membered heteroaryl, and $R_{17}$ and $R_{18}$ are defined as above, wherein $R_{13}$ is optionally substituted with 1, 2, 3 or 4 $R_1$(s). In some preferred embodiments of the present disclosure, in formula (G), $R_{13}$ is $-N(R_{17})(R_{18})$, $C_{1-6}$ alkoxy, $C_{1-6}$ alkyl, or $C_{3-7}$ cycloalkyl, 4-6 membered heterocycloalkyl, phenyl, or 5-6 membered heteroaryl, and $R_{17}$ and $R_{18}$ are defined as above, wherein $R_{13}$ is optionally substituted with 1, 2, or 3 $R_1$(s). In some preferred embodiments of the present disclosure, in formula (G), $R_{13}$ is $-N(R_{17})(R_{18})$, $C_{1-3}$ alkoxy, $C_{3-6}$ cycloalkyl, 4-6 membered heterocycloalkyl, phenyl, 5-6 membered heteroaryl or $C_{1-4}$ alkyl, and $R_{17}$ and $R_{18}$ are defined as above, wherein $R_{13}$ is optionally substituted with 1, 2, or 3 $R_1$(s). In some preferred embodiments of the present disclosure, in formula (G), $R_{13}$ is $-N(H)(C_{1-3}$ alkyl), -N(H)(3-6 membered cycloalkyl), -N(H) (4-6 membered heterocycloalkyl), -N ($C_{1-3}$ alkyl) ($C_{1-3}$ alkyl), $C_{1-3}$ alkoxy, C3-6 cycloalkyl, 4-6 membered azacycloalkyl or oxacycloalkyl, phenyl, 5-6 membered azaaryl or $C_{1-4}$ alkyl; or $R_{13}$ is $-N(R_{17})(R_{18})$, and $R_{17}$ and $R_{18}$ and the N atom connected thereto together form a 4-10 membered ring (where $R_{13}$ is optionally substituted with 1, 2, or 3 $R_1$(s)). In some particularly preferred embodiments of the present disclosure, in formula (G), $R_{13}$ is cyclopropyl, cyclobutyl, cyclopentyl, cyclohexyl, methyl, ethyl, propyl, butyl, methoxy, ethoxy, propoxy, $-N(H)(CH_3)$, $-N(H)(CH_2CH_3)$, $-N(H)(CH_2CH_2OH)$, - $N(H)(CH_2CH_2CN)$, $-N(CH_3)(CH_3)$, -N(H)(cyclopropyl), -N(H)(cyclobutyl), - N(H)(tetrahydrofuranyl), pyrazinyl, pyridazinyl, pyrrolidinyl, pyrazolyl, piperidinyl, phenyl, azetidinyl, morpholinyl, piperazinyl or tetrahydropyranyl; or $R_{13}$ is $-N(R_{17})(R_{18})$, and $R_{17}$ and $R_{18}$ and the N atom connected thereto together form a 7-membered ring (where $R_{13}$ is optionally substituted with 1, 2, or 3 $R_1$(s)). In some particularly preferred embodiments of the present disclosure, in formula (G), $R_{13}$ is cyclopropyl. In some particularly preferred embodiments of the present disclosure, in formula (G), $R_{13}$ is cyclobutyl. In some particularly preferred embodiments of the present disclosure, in formula (G), $R_{13}$ is cyclopentyl. In some particularly preferred embodiments of the present disclosure, in formula (G), $R_{13}$ is cyclohexyl. In some particularly preferred embodiments of the present disclosure, in formula (G), $R_{13}$ is methyl. In some particularly preferred embodiments of the present disclosure, in formula (G), $R_{13}$ is ethyl. In some particularly preferred embodiments of the present disclosure, in formula (G), $R_{13}$ is propyl. In some particularly preferred embodiments of the present disclosure, in formula (G), $R_{13}$ is butyl. In some particularly preferred embodiments of the present disclosure, in formula (G), $R_{13}$ is pyrazinyl. In some particularly preferred embodiments of the present disclosure, in formula (G), $R_{13}$ is pyridazinyl. In some particularly preferred embodiments of the present disclosure, in formula (G), $R_{13}$ is a pyrrolidinyl. In some particularly preferred embodiments of the present disclosure, in formula (G), $R_{13}$ is pyrazolyl. In some particularly preferred embodiments of the present disclosure, in formula (G), $R_{13}$ is piperidinyl. In some particularly preferred

embodiments of the present disclosure, in formula (G), $R_{13}$ is phenyl. In some particularly preferred embodiments of the present disclosure, in formula (G), $R_{13}$ is azetidinyl. In some particularly preferred embodiments of the present disclosure, in formula (G), $R_{13}$ is morpholinyl. In some particularly preferred embodiments of the present disclosure, in formula (G), $R_{13}$ is piperazinyl. In some particularly preferred embodiments of the present disclosure, in formula (G), $R_{13}$ is tetrahydropyranyl. In some particularly preferred embodiments of the present disclosure, in formula (G), $R_{13}$ is methoxy. In some particularly preferred embodiments of the present disclosure, in formula (G), $R_{13}$ is ethoxy. In some particularly preferred embodiments of the present disclosure, in formula (G), $R_{13}$ is $-N(H)(CH_3)$. In some particularly preferred embodiments of the present disclosure, in formula (G), $R_{13}$ is $-N(H)(CH_2CH_3)$. In some particularly preferred embodiments of the present disclosure, in formula (G), $R_{13}$ is $-N(H)(CH_2CH_2OH)$. In some particularly preferred embodiments of the present disclosure, in formula (G), $R_{13}$ is $-N(H)(CH_2CH_2CN)$. In some particularly preferred embodiments of the present disclosure, in formula (G), $R_{13}$ is - $N(CH_3)(CH_3)$. In some particularly preferred embodiments of the present disclosure, in formula (G), $R_{13}$ is -N(H) (cyclopropyl). In some particularly preferred embodiments of the present disclosure, in formula (G), $R_{13}$ is -N(H) (cyclobutyl). In some particularly preferred embodiments of the present disclosure, in formula (G), $R_{13}$ is -N(H) (tetrahydrofuranyl). In some particularly preferred embodiments of the present disclosure, in formula (G), $R_{13}$ is -$N(R_{17})(R_{18})$, and $R_{17}$ and $R_{18}$ and the N atom connected to them together form a 7-membered ring.

In some particularly preferred embodiments of the present disclosure, in formula (G), $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of -OH, -CN, -SH, halogen, $-NO_2$, and $SF_5$. In some particularly preferred embodiments of the present disclosure, in formula (G), $R_{17}$ and $R_{18}$ are each independently selected from H, $C_{1-6}$ alkyl, $C_{3-7}$ cycloalkyl, and $C_{3-7}$ heterocycloalkyl and are optionally substituted with one or more of -OH, -CN, -SH, halogen, $-NO_2$, and $SF_5$. In some particularly preferred embodiments of the present disclosure, in formula (G), $R_{17}$ and $R_{18}$ are each independently selected from H, $C_{1-6}$ alkyl, $C_{3-7}$ cycloalkyl, and $C_{3-7}$ heterocycloalkyl and are optionally substituted with one or more of -OH and -CN. In some preferred embodiments of the present disclosure, in formula (G), $R_{17}$ and $R_{18}$ are each independently selected from H, methyl, ethyl, propyl, 3-membered cycloalkyl, 4-membered cycloalkyl, 5-membered cycloalkyl, 5-membered heterocycloalkyl, and 6-membered heterocycloalkyl, and optionally substituted with one or more of -OH and -CN. In some preferred embodiments of the present disclosure, in formula (G), $R_{17}$, $R_{18}$ and the N atom connected thereto together form a 4-10 membered ring. In some preferred embodiments of the present disclosure, in formula (G), $R_{17}$, $R_{18}$ and the N atom connected thereto together form a 7-membered ring.

In some particularly preferred embodiments of the present disclosure, in formula (G), L is C=O, and $R_{13}$ is $-N(R_{17})(R_{18})$, $C_{1-6}$ alkoxy, -OH, -SH, -CN, halogen, $-NO_2$, $-SF_5$, or -S-$C_{1-4}$ alkyl, and $R_{13}$ is substituted with 0, 1, 2, 3 or 4 $R_1$(s) in which $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of - OH, -CN, -SH, halogen, $-NO_2$,-and $SF_5$, or $R_{17}$, $R_{18}$ and the N atom connected thereto together form a 3-14 membered ring. In some particularly preferred embodiments of the present disclosure, in formula (G), L is C=O, and $R_{13}$ is $-N(R_{17})(R_{13})$, or $C_{1-6}$ alkoxy, in which $R_{17}$ and $R_{18}$ are each independently selected from H, $C_{1-6}$ alkyl, $C_{3-7}$ cycloalkyl, and $C_{3-7}$ heterocycloalkyl and are optionally substituted with one or more of -OH, -CN, -SH, halogen, $-NO_2$,-and $SF_5$, or $R_{17}$, $R_{18}$ and the N atom connected thereto together form a 3-10 membered ring. In some particularly preferred embodiments of the present disclosure, in formula (G), L is C=O, and $R_{13}$ is methoxy, ethoxy, propoxy, $-N(H)(CH_3)$, - $N(H)(CH_2CH_3)$, - $N(H)(CH_2CH_2OH)$, $-N(H)(CH_2CH_2CN)$, $-N(CH_3)(CH_3)$, -N(H)(cyclopropyl), -N(H) (cyclobutyl), -N(H) (tetrahydrofuranyl); or $R_{13}$ is $-N(R_{17})(R_{18})$, and $R_{17}$, $R_{18}$ and the N atom connected thereto together form a 7-membered ring. In some particularly preferred embodiments of the present disclosure, in formula (G), L is C=O, and $R_{13}$ is methoxy. In some particularly preferred embodiments of the present disclosure, in formula (G), L is C=O, and $R_{13}$ is ethoxy. In some particularly preferred embodiments of the present disclosure, in formula (G), L is C=O, and $R_{13}$ is $-N(H)(CH_3)$. In some particularly preferred embodiments of the present disclosure, in formula (G), L is C=O, and $R_{13}$ is $-N(H)(CH_2CH_3)$. In some particularly preferred embodiments of the present disclosure, in formula (G), L is C=O, and $R_{13}$ is $-N(H)(CH_2CH_2OH)$. In some particularly preferred embodiments of the present disclosure, in formula (G), L is C=O, and $R_{13}$ is $-N(H)(CH_2CH_2CN)$. In some particularly preferred embodiments of the present disclosure, in formula (G), L is C=O, and $R_{13}$ is - $N(CH_3)(CH_3)$. In some particularly preferred embodiments of the present disclosure, in formula (G), L is C=O, and $R_{13}$ is -N(H)(cyclopropyl). In some particularly preferred embodiments of the present disclosure, in formula (G), L is C=O, and $R_{13}$ is -N(H)(cyclobutyl). In some particularly preferred embodiments of the present disclosure, in formula (G), L is C=O, and $R_{13}$ is -N(H)(tetrahydrofuranyl). In some particularly preferred embodiments of the present disclosure, in the formula (G), L is C=O, and $R_{13}$ is $-N(R_{17})(R_{18})$, and $R_{17}$, $R_{18}$ and the N atom connected thereto together form a 7-membered ring.

In some particularly preferred embodiments of the present disclosure, in formula (G), one, two or three $R_2$(s) are present and $R_2$ is selected from H, halogen, -OH, $-NO_2$, -CN, $-SF_5$, -SH, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, and 4-10 membered heterocycloalkyl, in which the -S-$C_{1-4}$ alkyl, $C_{1-6}$ alkyl, $C_{3-7}$ cycloalkyl, and 4-10 membered heterocycloalkyl are each optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in

formula (G), one, two or three $R_2$(s) are present and $R_2$ is selected from halogen, $C_{1-6}$ alkyl and $C_{3-6}$ cycloalkyl, in which the $C_{1-6}$ alkyl and $C_{3-6}$ cycloalkyl are each optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (G), one, two or three $R_2$(s) are present and $R_2$ is selected from halogen, and $C_{1-6}$ alkyl, in which the $C_{1-6}$ alkyl is optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (G), one, or two $R_2$(s) are present and $R_2$ is selected from halogen, and $C_{1-6}$ alkyl. In some particularly preferred embodiments of the present disclosure, in formula (G), one or two $R_2$(s) are present and $R_2$ is selected from fluorine, chlorine, bromine, methyl, ethyl, n-propyl, isopropyl, n-butyl, and isobutyl. In some preferred embodiments of the present disclosure, in formula (G), one or two $R_2$(s) are present, and $R_2$ is selected from fluorine, chlorine, methyl, ethyl, n-propyl, and isopropyl. In some preferred embodiments of the present disclosure, in formula (G), one or two $R_2$(s) are present, and $R_2$ is selected from fluorine, methyl, and ethyl. In some preferred embodiments of the present disclosure, in formula (G), one or two $R_2$ (s) are present, and $R_2$ is selected from fluorine and ethyl. In some preferred embodiments of the present disclosure, in formula (G), one $R_2$ is present, and $R_2$ is selected from fluorine and ethyl. In some preferred embodiments of the present disclosure, in formula (G), two $R_2$(s) are present, and $R_2$ is selected from fluorine and ethyl. In some particularly preferred embodiments of the present disclosure, in formula (G), two $R_2$(s) are present which are respectively fluorine and ethyl. In some particularly preferred embodiments of the present disclosure, in formula (G), one $R_2$ is present, and $R_2$ is an ethyl group.

In some preferred embodiments of the present disclosure, in formula (G), $R_{13}$ is substituted with 0, 1, 2, 3 or 4 $R_1$ (s), and each $R_1$ is independently selected from H, halogen, -OH, $-NO_2$,- CN, $-SF_5$, -SH, -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl, wherein the -S-$C_{1-4}$ alkyl, $C_{1-8}$ alkyl, $C_{2-8}$ alkenyl, $C_{2-8}$ alkynyl, and $C_{1-8}$ alkoxy are optionally substituted with 1, 2, 3, or 4 $R_3$(s), and wherein the $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2, 3 or 4 $R_4$(s). In some preferred embodiments of the present disclosure, in formula (G), $R_{13}$ is substituted with 0, 1, 2, 3 or 4 $R_1$(s), and each $R_1$ is independently selected from H, halogen, -OH, $-NO_2$,- CN, $-SF_5$, -SH, -S-$C_{1-4}$ alkyl, $C_{1-8}$ alkyl, $C_{3-7}$ cycloalkyl, 3-7 membered heterocycloalkyl, $C_{5-7}$ aryl, and 5-7 membered heteroaryl, wherein the -S-$C_{1-4}$ alkyl, and $C_{1-8}$ alkyl are optionally substituted with 1, 2, 3 or 4 $R_3$(s), and wherein the $C_{3-7}$ cycloalkyl, 3-7 membered heterocycloalkyl, $C_{5-7}$ aryl, and 5-7 membered heteroaryl are optionally substituted with 1, 2, 3, or 4 $R_4$(s). In some preferred embodiments of the present disclosure, in formula (G), $R_{13}$ is substituted with 0, 1, 2, 3 or 4 $R_1$(s), and each $R_1$ is independently selected from halogen, -OH, -CN, $C_{1-8}$ alkyl, $C_{3-7}$ cycloalkyl, 3-7 membered heterocycloalkyl, $C_{5-7}$ aryl, 5-7 membered heteroaryl, wherein the $C_{1-8}$ alkyl is optionally substituted with 1, 2 , 3 or 4 $R_3$(s), and wherein the $C_{3-7}$ cycloalkyl, 3-7 membered heterocycloalkyl, $C_{5-7}$ aryl, 5-7 membered heteroaryl are optionally substituted with 1, 2, 3 or 4 $R_4$(s). In some preferred embodiments of the present disclosure, in formula (G), $R_{13}$ is substituted with 0, 1, 2, 3 or 4 $R_1$(s), and each $R_1$ is independently selected from halogen, - OH, -CN, $C_{1-8}$ alkyl, $C_{3-7}$ cycloalkyl, and 3-7 membered heterocycloalkyl, wherein the $C_{1-8}$ alkyl is optionally substituted with 1, 2, or 3 $R_3$(s), and wherein the $C_{3-7}$ cycloalkyl, and 3-7 membered heterocycloalkyl are optionally substituted with 1, 2, or 3 $R_4$(s). In some preferred embodiments of the present disclosure, in formula (G), $R_{13}$ is substituted with 0 or 1 $R_1$, and each $R_1$ is independently selected from halogen, -OH, -CN, $C_{1-6}$ alkyl, $C_{3-7}$ cycloalkyl, 5-7 membered heterocycloalkyl, wherein the $C_{1-6}$ alkyl is optionally substituted with 1, 2, or 3 $R_3$(s), and wherein the $C_{3-7}$ cycloalkyl, and 5-7-membered heterocycloalkyl are optionally substituted with 1, 2, or 3 $R_4$(s). In some preferred embodiments of the present disclosure, in formula (G), $R_{13}$ is substituted with 0 or 1 $R_1$, and each $R_1$ is independently selected from halogen, -OH, -CN, $C_{1-4}$ alkyl, $C_{3-6}$ cycloalkyl, and 5-7 membered heterocycloalkyl, wherein the $C_{1-4}$ alkyl is optionally substituted with 1 or 2 $R_3$(s), and wherein the $C_{3-6}$ cycloalkyl and 5-7 membered heterocycloalkyl are optionally substituted with 1, 2, or 3 $R_4$(s). In some particularly preferred embodiments of the present disclosure, in formula (G), $R_{13}$ is substituted with 0 or 1 $R_1$, and each $R_1$ is independently selected from methyl, ethyl, hydroxyl, -CN, piperidinyl, morpholinyl, piperazinyl, and cyclopropyl, wherein the piperidinyl, morpholinyl, and piperazinyl are optionally substituted with 1, 2, 3 or 4 $C_{1-3}$ alkyl(s). In some particularly preferred embodiments of the present disclosure, in formula (G), $R_{13}$ is substituted with 0 or 1 $R_1$, and each $R_1$ is independently selected from methyl, ethyl, hydroxy, -CN, piperidinyl, morpholinyl, 1-methylpiperazinyl, and cyclopropyl. In some particularly preferred embodiments of the present disclosure, in formula (G), $R_1$ is absent. In some particularly preferred embodiments of the present disclosure, in formula (G), $R_1$ is 1-methylpiperazinyl. In some particularly preferred embodiments of the present disclosure, in formula (G), $R_1$ is methyl. In some particularly preferred embodiments of the present disclosure, in formula (G), $R_1$ is ethyl. In some particularly preferred embodiments of the present disclosure, in formula (G), $R_1$ is piperidinyl. In some particularly preferred embodiments of the present disclosure, in formula (G), $R_1$ is morpholinyl. In some particularly preferred embodiments of the present disclosure, in formula (G), $R_1$ is hydroxyl. In some particularly preferred embodiments of the present disclosure, in formula (G), $R_1$ is -CN. In some particularly preferred embodiments of the present disclosure, in formula (G), $R_1$ is cyclopropyl.

The preferred options of the respective substituents mentioned in the above various preferred embodiments can be combined with each other in any way, and various combinations thereof are within the scope of the present disclosure.

In the compound of formula (G), when $X_1$, $X_2$, and $X_3$ are the same, the compound of formula (G) can also be represented as a compound of formula (G'):

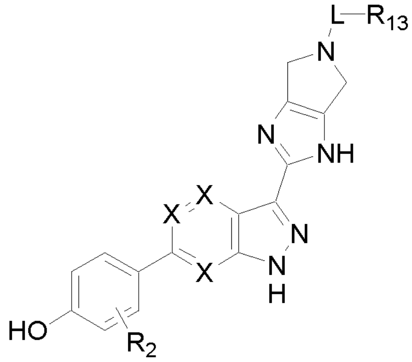

(G'),

wherein X is N or $CR_{14}$, and $R_{14}$, $R_{13}$, $R_1$, L, and $R_2$ are as defined in the compound of formula (G).

In a preferred embodiment, the present disclosure provides a compound of Formula (G)'

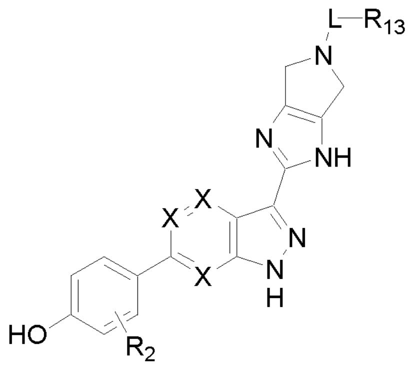

(G')

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, for use as a TRK inhibitor and/or RET inhibitor, or for use in the preparation of a TRK-inhibiting medicine and/or RET-inhibiting medicine, wherein

X is N or CH;

L is C=O, O=S=O, $CH_2$ or a bond; and

$R_{13}$ is H, $-N(R_{17})(R_{18})$, $C_{1-6}$ alkoxy, $-SR_{12}$, $-OR_{12}$, -CN, halogen, $-NO_2$, $-SF_5$, $-S-C_{1-4}$ alkyl, $C_{1-6}$ alkyl or $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicycloalkyl, or 5-11 membered bicyclic heteroalkyl, and $R_{13}$ is substituted with 0, 1, 2, 3 or 4 $R_1$(s), in which $R_{17}$ and $R_{18}$ are each independently selected from H, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, $C_{3-7}$ heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicycloalkyl, and 5-11 membered bicyclic heteroalkyl and are optionally substituted with one or more substitutes each independently selected from -OH, - CN, -SH, halogen, $-NO_2$, $-SF_5$, $-S-C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, $-N(R_9)(R_{10})$, $- N(R_{11})(C(=O)R_{12})$, $-C(=O)-N(R_9)(R_{10})$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $-S-C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2 or 3 substitutes each independently selected from halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, $-N(R_0)(R_{10})$, $-N(R_{11})(C(=O)R_{12})$, $-C(=O)-OR_{12}$, -C(=O)H, $-C(=O)R_{12}$, $-C(= O)-N(R_9)$

$(R_{10})$, $-N(R_{10})(S(=O)_2R_{12})$, $-S(=O)_2-N(R_9)(R_{10})$, $-SR_{12}$ and $-OR_{12}$; or $R_{17}$, $R_{18}$ and the N atom connected thereto together form a 3-14 membered ring; and

0, 1, 2, 3 or 4 $R_2$(s) are present in formula (G'), and $R_2$ is selected from H, halogen, - OH, $-NO_2$, -CN, $-SF_5$, -SH, $-S-C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, $-N(R_0)(R_{10})$, $-N(R_{11})(C(= O)R_{12})$, $-C(=O)-N(R_9)(R_{10})$, $-C(=O)-R_{12}$, $-C(=O)-OR_{12}$, $-OC(=O)R_{12}$, $-N(R_{11})(S(= O)_2R_{12})$, $-S(=O)_2-N(R_9)(R_{10})$, $-SR_{12}$ and $-OR_{12}$, in which the $-S-C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, $-N(R_0)(R_{10})$, $-N(R_{11})(C(=O)R_{12})$, $- C(=O)-OR_{12}$, -C(=O)H, $-C(=O)R_{12}$, $-C(=O)-N(R_9)(R_{10})$, $-N(R_{11})(S(=O)_2R_{12})$, $-S(=O)_2-N(R_9)(R_{10})$, $-SR_{12}$ and $-OR_{12}$; and

$R_1$ is selected from H, halogen, -OH, $-NO_2$, -CN, $-SF_5$, -SH, $-S-C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicycloalkyl, 5-11 membered bicyclic heteroalkyl, $-N(R_9)(R_{10})$, $-N(R_{11})(C(=O)R_{12})$, $-C(=O)-N(R_9)(R_{10})$, $-C(=O)-R_{12}$, $-C(=O)-OR_{12}$, $-OC(=O)R_{12}$, $- N(R_{11})(S(=O)_2R_{12})$, $-S(=O)_2-N(R_9)(R_{10})$, $-SR_{12}$ and $-OR_{12}$, in which the $-S-C_{1-4}$ alkyl, $C_{1-8}$ alkyl, $C_{2-8}$ alkenyl, $C_{2-8}$ alkynyl, and $C_{1-8}$ alkoxy are optionally substituted with 1, 2, 3, or 4 $R_3$(s), and in which the $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2, 3, or 4 $R_4$(s); and

$R_3$ and $R_4$ are each independently selected from H, halogen, -OH, $-NO_2$, -CN, $-SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, $-N(R_5)(R_6)$, $-N(R_{11})(C(=O)R_{12})$, $-CON(R_7)(R_8)$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2, 3 or 4 substituent(s) each independently selected from the group consisting of halogen, -CN, -OH, $C_{1-4}$ alkyl, $C_{1-6}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, $-C(=O)-OR_{12}$, -C(=O)H, $-C(=O)R_{12}$, $- C(=O)-N(R_9)(R_{10})$, $-N(R_{11})(S(=O)_2R_{12})$, $-S(=O)_2-N(R_9)(R_{10})$, $-SR_{12}$ and $-OR_{12}$; and

$R_5$, $R_6$, $R_7$, $R_8$, $R_9$, $R_{10}$, $R_{11}$, and $R_{12}$ are each independently H or selected from the group consisting of $C_{1-6}$ alkyl, $C_{1-4}$ haloalkyl, $C_{3-7}$ cycloalkyl, 4-14 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein the options included in the above group are each optionally substituted with 1, 2, 3 or 4 substituent(s) each independently selected from the group consisting of halogen, $-CF_3$, -OH, $-NH_2$, $-NH(CH_3)$, $-N(CH_3)_2$, -CN, oxo, $C_{1-4}$ alkyl, $C_{2-6}$ alkenyl, $C_{2-6}$ alkynyl, $C_{3-7}$ cycloalkyl, $C_{1-4}$ hydroxyalkyl, $-S-C_{1-4}$ alkyl, -C(=O)H, $-C(=O)-C_{1-4}$ alkyl, $-C(=O)-O-C_{1-4}$ alkyl, $-C(=O)-NH_2$, $-C(=O)-N(C_{1-4}\ alkyl)_2$, $C_{1-4}$ haloalkyl, $C_{1-4}$ alkoxy and $C_{1-4}$ haloalkoxy.

In some preferred embodiments of the present disclosure, an isotopically labeled compound of the above-mentioned compound of formula (G') is used. In some more preferred embodiments of the present disclosure, an isotopically labeled compound of the compound of formula (G') is used, wherein all Hs are each independently and optionally substituted with D.

In some preferred embodiments of the present disclosure, in formula (G), X is N. In some more preferred embodiments of the present disclosure, in formula (G), X is CH.

In some more preferred embodiments of the present disclosure, an isotopically labeled compound of the compound of formula (G') is used, wherein all H are each independently and optionally substituted with D, and X is N. In some more preferred embodiments of the present disclosure, an isotopically labeled compound of the compound of formula (G') is used, wherein all Hs are each independently and optionally substituted with D, and X is CH.

In some preferred embodiments of the present disclosure, in formula (G'), L is C=O, O=S=O or $CH_2$. In some particularly preferred embodiments of the present disclosure, in formula (G'), L is C=O. In some particularly preferred embodiments of the present disclosure, in formula (G'), L is O=S=O. In some particularly preferred embodiments of the present disclosure, in formula (G'), L is $CH_2$. In other embodiments of the present disclosure, in formula (G'), L is a bond.

In some particularly preferred embodiments of the present disclosure, in formula (G'), X is CH, and L is C=O.

In some particularly preferred embodiments of the present disclosure, in formula (G'), X is CH, and L is O=S=O.

In some particularly preferred embodiments of the present disclosure, in formula (G'), X is CH, and L is CH2.

In some particularly preferred embodiments of the present disclosure, in formula (G'), X is CH, and L is a bond.

In some particularly preferred embodiments of the present disclosure, in formula (G'), X is N, and L is C=O.

In some particularly preferred embodiments of the present disclosure, in formula (G'), X is N, and L is O=S=O.

In some particularly preferred embodiments of the present disclosure, in formula (G'), X is N, and L is $CH_2$.

In some particularly preferred embodiments of the present disclosure, in formula (G'), X is N, and L is a bond.

In some preferred embodiments of the present disclosure, in formula (G'), $R_{13}$ is H, - $N(R_{17})(R_{18})$, $C_{1-6}$ alkoxy, -OH, -SH, -CN, halogen, $-NO_2$, $-SF_5$, -S-$C_{1-4}$ alkyl, $C_{1-6}$ alkyl, or $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicycloalkyl, or 5-11 membered bicyclic heteroalkyl, in which $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of - OH, -CN, -SH, halogen, $-NO_2$, -and $SF_5$, wherein $R_{13}$ is optionally substituted with 1, 2, 3 or 4 $R_1$(s). In some preferred embodiments of the present disclosure, in formula (G'), $R_{13}$ is H, - $N(R_{17})(R_{18})$, $C_{1-6}$ alkoxy, -OH, -SH, -CN, halogen, $-NO_2$, $-SF_5$, -S-$C_{1-4}$ alkyl, $C_{1-6}$ alkyl, or $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, or 11-15 membered tricyclyl and $R_{17}$ and $R_{18}$ are defined as above, wherein $R_{13}$ is optionally substituted with 1, 2, 3 or 4 $R_1$(s). In some preferred embodiments of the present disclosure, in formula (G'), $R_{13}$ is H, $-N(R_{17})(R_{18})$, $C_{1-6}$ alkoxy, $C_{1-6}$ alkyl, or $C_{3-7}$ cycloalkyl, 3-7 membered heterocycloalkyl, $C_{5-7}$ aryl, or 5-7 membered heteroaryl, and $R_{17}$ and $R_{18}$ are defined as above, wherein $R_{13}$ is optionally substituted with 1, 2, 3 or 4 $R_1$(s). In some preferred embodiments of the present disclosure, in formula (G'), $R_{13}$ is $-N(R_{17})(R_{18})$, $C_{1-6}$ alkoxy, $C_{1-6}$ alkyl, or $C_{3-7}$ cycloalkyl, 4-6 membered heterocycloalkyl, phenyl, or 5-6 membered heteroaryl, and $R_{17}$ and $R_{18}$ are defined as above, wherein $R_{13}$ is optionally substituted with 1, 2, or 3 $R_1$(s). In some preferred embodiments of the present disclosure, in formula (G'), $R_{13}$ is $-N(R_{17})(R_{18})$, $C_{1-3}$ alkoxy, $C_{3-6}$ cycloalkyl, 4-6 membered heterocycloalkyl, phenyl, 5-6 membered heteroaryl or $C_{1-4}$ alkyl, and $R_{17}$ and $R_{18}$ are defined as above, wherein $R_{13}$ is optionally substituted with 1, 2, or 3 $R_1$(s). In some preferred embodiments of the present disclosure, in formula (G'), $R_{13}$ is -N(H)($C_{1-3}$ alkyl), - N(H)(3-6 membered cycloalkyl), -N(H) (4-6 membered heterocycloalkyl), -N ($C_{1-3}$ alkyl) ($C_{1-3}$ alkyl), $C_{1-3}$ alkoxy, $C_{3-6}$ cycloalkyl, 4-6 membered azacycloalkyl or oxacycloalkyl, phenyl, 5-6 membered azaaryl or $C_{1-4}$ alkyl; or $R_{13}$ is $-N(R_{17})(R_{18})$, and $R_{17}$ and $R_{18}$ and the N atom connected thereto together form a 4-10 membered ring, wherein $R_{13}$ is optionally substituted with 1, 2, or 3 $R_1$(s). In some particularly preferred embodiments of the present disclosure, in formula (G'), $R_{13}$ is cyclopropyl, cyclobutyl, cyclopentyl, cyclohexyl, methyl, ethyl, propyl, butyl, methoxy, ethoxy, propoxy, $-N(H)(CH_3)$, $-N(H)(CH_2CH_3)$, $-N(H)(CH_2CH_2OH)$, - $N(H)(CH_2CH_2CN)$, $-N(CH_3)(CH_3)$, -N(H)(cyclopropyl), -N(H)(cyclobutyl), - N(H)(tetrahydrofuranyl), pyrazinyl, pyridazinyl, pyrrolidinyl, pyrazolyl, piperidinyl, phenyl, azetidinyl, morpholinyl, piperazinyl or tetrahydropyranyl; or $R_{13}$ is $-N(R_{17})(R_{18})$, and $R_{17}$ and $R_{18}$ and the N atom connected thereto together form a 7-membered ring, wherein $R_{13}$ is optionally substituted with 1, 2, or 3 $R_1$(s). In some particularly preferred embodiments of the present disclosure, in formula (G'), $R_{13}$ is cyclopropyl. In some particularly preferred embodiments of the present disclosure, in formula (G'), $R_{13}$ is cyclobutyl. In some particularly preferred embodiments of the present disclosure, in formula (G'), $R_{13}$ is cyclopentyl. In some particularly preferred embodiments of the present disclosure, in formula (G'), $R_{13}$ is cyclohexyl. In some particularly preferred embodiments of the present disclosure, in formula (G'), $R_{13}$ is methyl. In some particularly preferred embodiments of the present disclosure, in formula (G'), $R_{13}$ is ethyl. In some particularly preferred embodiments of the present disclosure, in formula (G'), $R_{13}$ is propyl. In some particularly preferred embodiments of the present disclosure, in formula (G'), $R_{13}$ is butyl. In some particularly preferred embodiments of the present disclosure, in formula (G'), $R_{13}$ is pyrazinyl. In some particularly preferred embodiments of the present disclosure, in formula (G'), $R_{13}$ is a pyridazinyl. In some particularly preferred embodiments of the present disclosure, in formula (G'), $R_{13}$ is a pyrrolidinyl. In some particularly preferred embodiments of the present disclosure, in formula (G'), $R_{13}$ is pyrazolyl. In some particularly preferred embodiments of the present disclosure, in formula (G'), $R_{13}$ is piperidinyl. In some particularly preferred embodiments of the present disclosure, in formula (G'), $R_{13}$ is phenyl. In some particularly preferred embodiments of the present disclosure, in formula (G'), $R_{13}$ is azetidinyl. In some particularly preferred embodiments of the present disclosure, in formula (G'), $R_{13}$ is morpholinyl. In some particularly preferred embodiments of the present disclosure, in formula (G'), $R_{13}$ is piperazinyl. In some particularly preferred embodiments of the present disclosure, in formula (G'), $R_{13}$ is tetrahydropyranyl. In some particularly preferred embodiments of the present disclosure, in formula (G'), $R_{13}$ is methoxy. In some particularly preferred embodiments of the present disclosure, in formula (G'), $R_{13}$ is ethoxy. In some particularly preferred embodiments of the present disclosure, in formula (G'), $R_{13}$ is $-N(H)(CH_3)$. In some particularly preferred embodiments of the present disclosure, in formula (G'), $R_{13}$ is - $N(H)(CH_2CH_3)$. In some particularly preferred embodiments of the present disclosure, in formula (G'), $R_{13}$ is $-N(H)(CH_2CH_2OH)$. In some particularly preferred embodiments of the present disclosure, in formula (G'), $R_{13}$ is $-N(H)(CH_2CH_2CN)$. In some particularly preferred embodiments of the present disclosure, in formula (G'), $R_{13}$ is $-N(CH_3)(CH_3)$. In some particularly preferred embodiments of the present disclosure, in formula (G'), $R_{13}$ is -N(H) (cyclopropyl). In some particularly preferred embodiments of the present disclosure, in formula (G'), $R_{13}$ is -N(H) (cyclobutyl). In some particularly preferred embodiments of the present disclosure, in formula (G'), $R_{13}$ is -N(H) (tetrahydrofuranyl). In some particularly preferred embodiments of the present disclosure, in

formula (G'), $R_{13}$ is -N($R_{17}$)($R_{18}$), and $R_{17}$ and $R_{18}$ and the N atom connected to them together form a 7-membered ring.

In some particularly preferred embodiments of the present disclosure, in formula (G'), $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of -OH, -CN, -SH, halogen, $-NO_2$, and $SF_5$. In some particularly preferred embodiments of the present disclosure, in formula (G'), $R_{17}$ and $R_{18}$ are each independently selected from H, $C_{1-6}$ alkyl, $C_{3-7}$ cycloalkyl, and $C_{3-7}$ heterocycloalkyl and are optionally substituted with one or more of -OH, -CN, -SH, halogen, $-NO_2$, and $SF_5$. In some particularly preferred embodiments of the present disclosure, in formula (G'), $R_{17}$ and $R_{18}$ are each independently selected from H, $C_{1-6}$ alkyl, $C_{3-7}$ cycloalkyl, and $C_{3-7}$ heterocycloalkyl and are optionally substituted with one or more of -OH and -CN. In some preferred embodiments of the present disclosure, in formula (G'), $R_{17}$ and $R_{18}$ are each independently selected from H, methyl, ethyl, propyl, 3-membered cycloalkyl, 4-membered cycloalkyl, 5-membered cycloalkyl, 5-membered heterocycloalkyl, and 6-membered heterocycloalkyl, and optionally substituted with one or more of -OH and -CN. In some preferred embodiments of the present disclosure, in formula (G'), $R_{17}$, $R_{18}$ and the N atom connected thereto together form a 4-10 membered ring. In some preferred embodiments of the present disclosure, in formula (G'), $R_{17}$, $R_{18}$ and the N atom connected thereto together form a 7-membered ring.

In some particularly preferred embodiments of the present disclosure, in formula (G'), L is C=O, and $R_{13}$ is -N($R_{17}$)($R_{18}$), $C_{1-6}$ alkoxy, -OH, -SH, -CN, halogen, $-NO_2$, $-SF_5$, or -S-$C_{1-4}$ alkyl, and $R_{13}$ is substituted with 0, 1, 2, 3 or 4 $R_1$ (s) in which $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of -OH, -CN, -SH, halogen, $-NO_2$, -and $SF_5$, or $R_{17}$, $R_{18}$ and the N atom connected thereto together form a 3-14 membered ring. In some particularly preferred embodiments of the present disclosure, in formula (G'), L is C=O, and $R_{13}$ is -N($R_{17}$)($R_{18}$), or $C_{1-6}$ alkoxy, in which $R_{17}$ and $R_{18}$ are each independently selected from H, $C_{1-6}$ alkyl, $C_{3-7}$ cycloalkyl, and $C_{3-7}$ heterocycloalkyl and are optionally substituted with one or more of -OH, -CN, -SH, halogen, $-NO_2$, -and $SF_5$, or $R_{17}$, $R_{18}$ and the N atom connected thereto together form a 3-10 membered ring. In some particularly preferred embodiments of the present disclosure, in formula (G'), L is C=O, and $R_{13}$ is methoxy, ethoxy, propoxy, -N(H)($CH_3$), - N(H)($CH_2CH_3$), - N(H) ($CH_2CH_2OH$), -N(H)($CH_2CH_2CN$), -N($CH_3$)($CH_3$), -N(H)(cyclopropyl), -N(H) (cyclobutyl), -N(H) (tetrahydrofuranyl); or $R_{13}$ is -N($R_{17}$)($R_{18}$), and $R_{17}$, $R_{18}$ and the N atom connected thereto together form a 7-membered ring. In some particularly preferred embodiments of the present disclosure, in formula (G'), L is C=O, and $R_{13}$ is methoxy. In some particularly preferred embodiments of the present disclosure, in formula (G'), L is C=O, and $R_{13}$ is ethoxy. In some particularly preferred embodiments of the present disclosure, in formula (G'), L is C=O, and $R_{13}$ is -N(H)($CH_3$). In some particularly preferred embodiments of the present disclosure, in formula (G'), L is C=O, and $R_{13}$ is -N(H)($CH_2CH_3$). In some particularly preferred embodiments of the present disclosure, in formula (G'), L is C=O, and $R_{13}$ is -N(H)($CH_2CH_2OH$). In some particularly preferred embodiments of the present disclosure, in formula (G'), L is C=O, and $R_{13}$ is -N(H)($CH_2CH_2CN$). In some particularly preferred embodiments of the present disclosure, in formula (G'), L is C=O, and $R_{13}$ is - N($CH_3$)($CH_3$). In some particularly preferred embodiments of the present disclosure, in formula (G'), L is C=O, and $R_{13}$ is -N(H) (cyclopropyl). In some particularly preferred embodiments of the present disclosure, in formula (G'), L is C=O, and $R_{13}$ is - N(H)(cyclobutyl). In some particularly preferred embodiments of the present disclosure, in formula (G'), L is C=O, and $R_{13}$ is -N(H)(tetrahydrofuranyl). In some particularly preferred embodiments of the present disclosure, in the formula (G'), L is C=O, and $R_{13}$ is -N($R_{17}$)($R_{18}$), and $R_{17}$, $R_{18}$ and the N atom connected thereto together form a 7-membered ring.

In some particularly preferred embodiments of the present disclosure, in formula (G'), one, two or three $R_2$(s) are present and $R_2$ is selected from H, halogen, -OH, - $NO_2$, -CN, $-SF_5$, -SH, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, and 4-10 membered heterocycloalkyl, in which the -S-$C_{1-4}$ alkyl, $C_{1-6}$ alkyl, $C_{3-7}$ cycloalkyl, and 4-10 membered heterocycloalkyl are each optionally substituted with 1, 2 or 3 substituent(s) each independently selected from the group consisting of halogen, -OH, $-NH_2$, -NH($CH_3$), -N$(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (G'), one, two or three $R_2$(s) are present and $R_2$ is selected from halogen, $C_{1-6}$ alkyl and $C_{3-6}$ cycloalkyl, in which the $C_{1-6}$ alkyl and $C_{3-6}$ cycloalkyl are each optionally substituted with 1, 2 or 3 substituent(s) each independently selected from the group consisting of halogen, -OH, $-NH_2$, -NH($CH_3$), -N$(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (G'), one, two or three $R_2$(s) are present and $R_2$ is selected from halogen, and $C_{1-6}$ alkyl, in which the $C_{1-6}$ alkyl is optionally substituted with 1, 2 or 3 substituent(s) each independently selected from the group consisting of halogen, -OH, $-NH_2$, -NH($CH_3$), -N$(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (G'), one or two $R_2$(s) are present and $R_2$ is selected from halogen, and $C_{1-6}$ alkyl. In some particularly preferred embodiments of the present disclosure, in formula (G'), one or two $R_2$(s) are present and $R_2$ is selected from fluorine, chlorine, bromine, methyl, ethyl, n-propyl, isopropyl, n-butyl, and isobutyl. In some preferred embodiments of the present disclosure, in formula (G'), one or two $R_2$(s) are present, and $R_2$ is selected from fluorine, chlorine, methyl, ethyl, n-propyl, and isopropyl. In some preferred embodiments of the present disclosure, in formula (G'), one or two $R_2$ (s) are present, and $R_2$ is selected from fluorine, methyl, and ethyl. In some preferred embodiments of the present disclosure, in formula (G'), one or two $R_2$(s) are present, and $R_2$ is selected from fluorine and ethyl. In some

preferred embodiments of the present disclosure, in formula (G'), one $R_2$ is present, and $R_2$ is selected from fluorine and ethyl. In some preferred embodiments of the present disclosure, in formula (G'), two $R_2$s are present, and $R_2$ is selected from fluorine and ethyl. In some particularly preferred embodiments of the present disclosure, in formula (G'), two $R_2$s are present which are respectively fluorine and ethyl. In some particularly preferred embodiments of the present disclosure, in formula (G'), one $R_2$ is present, and $R_2$ is an ethyl group.

In some preferred embodiments of the present disclosure, in formula (G'), $R_{13}$ is substituted with 0, 1, 2, 3 or 4 $R_1$ (s), and each $R_1$ is independently selected from H, halogen, -OH, $-NO_2$, -CN, $-SF_5$, -SH, -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3 -10 membered heterocycloalkyl, $C_{5-7}$ aryl, 5-7 membered heteroaryl, $C_{7-11}$ bicyclic aryl, and 7-11 membered bicyclic heteroaryl, wherein the -S-$C_{1-4}$ alkyl, $C_{1-8}$ alkyl, $C_{2-8}$ alkenyl, $C_{2-8}$ alkynyl, and $C_{1-8}$ alkoxy are optionally substituted with 1, 2, 3, or 4 $R_3$(s), and wherein the $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2 , 3 or 4 $R_4$(s). In some preferred embodiments of the present disclosure, in formula (G'), $R_{13}$ is substituted with 0, 1, 2, 3 or 4 $R_1$(s), and each $R_1$ is independently selected from H, halogen, -OH, $-NO_2$,- CN, $-SF_5$, -SH, -S-$C_{1-4}$ alkyl, $C_{1-8}$ alkyl, $C_{3-7}$ cycloalkyl, 3-7 membered heterocycloalkyl, $C_{5-7}$ aryl, 5-7 membered heteroaryl, wherein the -S-$C_{1-4}$ alkyl, and $C_{1-8}$ alkyl are optionally substituted with 1, 2, 3 or 4 $R_3$(s), and wherein the $C_{3-7}$ cycloalkyl, 3-7 membered heterocycloalkyl, $C_{5-7}$ aryl, and 5-7 membered heteroaryl are optionally substituted with 1, 2, 3, or 4 $R_4$(s). In some preferred embodiments of the present disclosure, in formula (G'), $R_{13}$ is substituted with 0, 1, 2, 3 or 4 $R_1$(s), and each $R_1$ is independently selected from halogen, -OH, -CN, $C_{1-8}$ alkyl, $C_{3-7}$ cycloalkyl, 3-7 membered heterocycloalkyl, $C_{5-7}$ aryl, 5-7 membered heteroaryl, wherein the $C_{1-8}$ alkyl is optionally substituted with 1, 2 , 3 or 4 $R_3$(s), and wherein the $C_{3-7}$ cycloalkyl, 3-7 membered heterocycloalkyl, $C_{5-7}$ aryl, and 5-7 membered heteroaryl are optionally substituted with 1, 2, 3 or 4 $R_4$(s). In some preferred embodiments of the present disclosure, in formula (G'), $R_{13}$ is substituted with 0, 1, 2, 3 or 4 $R_1$ (s), and each $R_1$ is independently selected from halogen, -OH, -CN, $C_{1-8}$ alkyl, $C_{3-7}$ cycloalkyl, and 3-7 membered heterocycloalkyl, wherein the $C_{1-8}$ alkyl is optionally substituted with 1, 2, or 3 $R_3$(s), and wherein the $C_{3-7}$ cycloalkyl, and 3-7 membered heterocycloalkyl are optionally substituted with 1, 2, or 3 $R_4$. In some preferred embodiments of the present disclosure, in formula (G'), $R_{13}$ is substituted with 0 or 1 $R_1$, and each $R_1$ is independently selected from halogen, -OH, -CN, $C_{1-6}$ alkyl, $C_{3-7}$ cycloalkyl, and 5-7 membered heterocycloalkyl, wherein the $C_{1-6}$ alkyl is optionally substituted with 1, 2, or 3 $R_3$(s), and wherein the $C_{3-7}$ cycloalkyl, and 5-7-membered heterocycloalkyl are optionally substituted with 1, 2, or 3 $R_4$(s). In some preferred embodiments of the present disclosure, in formula (G'), $R_{13}$ is substituted with 0 or 1 $R_1$, and each $R_1$ is independently selected from halogen, -OH, -CN, $C_{1-4}$ alkyl, $C_{3-6}$ cycloalkyl, and 5-7 membered heterocycloalkyl, wherein the $C_{1-4}$ alkyl is optionally substituted with 1 or 2 $R_3$, and wherein the $C_{3-6}$ cycloalkyl and 5-7 membered heterocycloalkyl are optionally substituted with 1, 2, or 3 $R_4$(s). In some particularly preferred embodiments of the present disclosure, in formula (G'), $R_{13}$ is substituted with 0 or 1 $R_1$, and each $R_1$ is independently selected from methyl, ethyl, hydroxyl, -CN, piperidinyl, morpholinyl, piperazinyl, and cyclopropyl, wherein the piperidinyl, morpholinyl, and piperazinyl are optionally substituted with 1, 2, 3 or 4 $C_{1-3}$ alkyl(s). In some particularly preferred embodiments of the present disclosure, in formula (G'), $R_{13}$ is substituted with 0 or 1 $R_1$, and each $R_1$ is independently selected from methyl, ethyl, hydroxy, -CN, piperidinyl, morpholinyl, 1-methylpiperazinyl, and cyclopropyl. In some particularly preferred embodiments of the present disclosure, in formula (G'), $R_1$ is absent. In some particularly preferred embodiments of the present disclosure, in formula (G'), $R_1$ is 1-methylpiperazinyl. In some particularly preferred embodiments of the present disclosure, in formula (G'), $R_1$ is methyl. In some particularly preferred embodiments of the present disclosure, in formula (G'), $R_1$ is ethyl. In some particularly preferred embodiments of the present disclosure, in formula (G'), $R_1$ is piperidinyl. In some particularly preferred embodiments of the present disclosure, in formula (G'), $R_1$ is morpholinyl. In some particularly preferred embodiments of the present disclosure, in formula (G'), $R_1$ is hydroxyl. In some particularly preferred embodiments of the present disclosure, in formula (G'), $R_1$ is - CN. In some particularly preferred embodiments of the present disclosure, in formula (G'), $R_1$ is cyclopropyl.

The preferred options of the respective substituents mentioned in the above various preferred embodiments can be combined with each other in any way, and various combinations thereof are within the scope of the present disclosure.

In the compound of formula (G'), when $R_{13}$ is a ring, the compound of formula (G') can also be represented as a compound of the following formula (I):

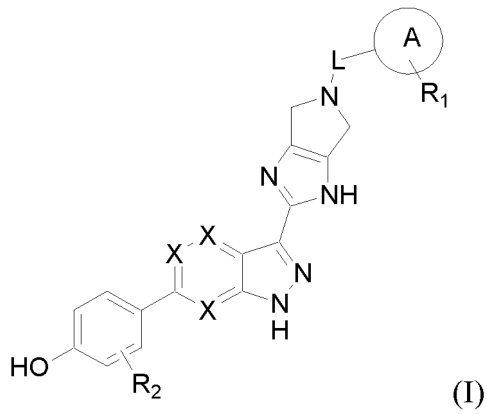

(I)

wherein the ring A is $C_{3-7}$ cycloalkyl, $_{3-7}$ membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicyclic alkyl group or 5-11 membered bicyclic heteroalkyl, which may be optionally substituted with $R_1$, and L, $R_1$, $R_2$, and X are defined as above in the compound of formula (G').

In particular, the present disclosure provides a compound of formula (I):

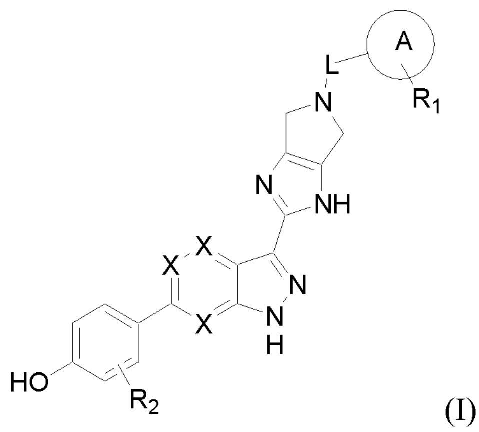

(I)

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, for use as a TRK inhibitor and/or RET inhibitor, or for use in the preparation of a TRK-inhibiting medicine and/or RET-inhibiting medicine, in which

L is C=O, O=S=O, $CH_2$ or a bond; and

X is CH or N;

the ring A is $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, or 11 -15 membered tricyclyl;

0, 1, 2, 3 or 4 $R_1$(s) are present in formula (I), and $R_1$ is selected from H, halogen, $C_{1-8}$ alkyl, $C_{2-8}$ alkenyl, $C_{2-8}$ alkynyl, $C_{1-8}$ alkoxy, $C_{3-7}$ cycloalkyl, 3-7 membered heterocycloalkyl, $C_{5-7}$ aryl, 5-7 membered heteroaryl, $C_{7-11}$ bicyclic aryl, and 7-11 membered bicyclic heteroaryl, in which the $C_{1-8}$ alkyl, $C_{2-8}$ alkenyl, $C_{2-8}$ alkynyl, and $C_{1-8}$ alkoxy are optionally substituted with 1, 2, 3 or 4 $R_3$(s), and in which the $C_{3-7}$ cycloalkyl, 3-7 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2, 3 or 4 $R_4$(s),

0, 1, 2, 3 or 4 $R_2$(s) are present in formula (I), and $R_2$ is selected from H, halogen, -OH, $-NO_2$, -CN, $-SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $-N(R_9)(R_{10})$, $-N(R_{11})(C(=O)R_{12})$, $-C(=O)-N(R_9)(R_{10})$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl and 4-10 membered heterocycloalkyl

are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), - C(=O)-O$R_{12}$, -C(=O)H, -C(=O)$R_{12}$, -C(=O)-N($R_9$)($R_{10}$), -N($R_{11}$)(S(=O)$_2R_{12}$), -S(=O)$_2$-N($R_9$)($R_{10}$), -S$R_{12}$ and -O$R_{12}$;

$R_3$ is selected from halogen, cyano, $C_{1-3}$ alkyl, hydroxy, $C_{1-6}$ alkoxy, - N($R_5$)($R_6$), -CON($R_7$)($R_8$) or 3-7 membered heterocycloalkyl, in which the 3-7 membered heterocycloalkyl is optionally substituted with 1, 2, 3 or 4 $R_4$(s);

$R_4$ is selected from halogen, $C_{1-3}$ alkyl, hydroxyl, $C_{1-6}$ alkoxy, -$NH_2$, -$NHCH_3$ or -N($CH_3$)$_2$;

$R_5$, $R_6$, $R_7$, $R_8$ are each independently hydrogen or $C_{1-4}$ alkyl;

$R_9$ is selected from H, $C_{1-4}$ alkyl, $C_{1-4}$ haloalkyl or $C_{3-7}$ cycloalkyl;

$R_{10}$ is H or selected from the group consisting of $C_{1-4}$ alkyl, $C_{1-4}$ haloalkyl, $C_{3-7}$ cycloalkyl, 4-10 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein the options included in the above group each are optionally substituted with 1, 2, 3 or 4 substituent(s) each independently selected from the group consisting of -OH, -$NH_2$, -NH($CH_3$), -N($CH_3$)$_2$, -CN, $C_{1-4}$ alkyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, -C(=O)-$NH_2$, -C( =O)-N($C_{1-4}$ alkyl)$_2$, $C_{1-4}$ haloalkyl, $C_{1-4}$ alkoxy and $C_{1-4}$ haloalkoxy;

$R_{11}$ is selected from H, $C_{1-4}$ alkyl and $C_{3-7}$ cycloalkyl; and

$R_{12}$ is selected from the group consisting of $C_{1-6}$ alkyl, $C_{3-7}$ cycloalkyl, 4- to 14-membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each option included in the above group is optionally substituted with 1, 2 or 3 substituent(s) each independently selected from the group consisting of halogen, -$CF_3$, -CN, -OH, -$NH_2$, -NH($CH_3$), -N($CH_3$)$_2$, oxo, -S-$C_{1-4}$ alkyl, $C_{1-4}$ alkyl, $C_{1-4}$ haloalkyl, $C_{2-6}$ alkenyl, $C_{2-6}$ alkynyl , $C_{3-7}$ cycloalkyl, $C_{1-4}$ alkoxy and $C_{1-4}$ haloalkoxy.

In some preferred embodiments of the present disclosure, in formula (I), L is C=O, O=S=O or $CH_2$. In some particularly preferred embodiments of the present disclosure, in formula (I), L is C=O. In some particularly preferred embodiments of the present disclosure, in formula (I), L is O=S=O. In some particularly preferred embodiments of the present disclosure, in formula (I), L is $CH_2$. In other embodiments of the present disclosure, in formula (I), L is a bond.

In some particularly preferred embodiments of the present disclosure, in formula (I), X is CH. In other some embodiments of the present disclosure, in formula (I), X is N.

In some particularly preferred embodiments of the present disclosure, in formula (I), X is CH, and L is C=O.

In some particularly preferred embodiments of the present disclosure, in formula (I), X is CH, and L is O=S=O.

In some particularly preferred embodiments of the present disclosure, in formula (I), X is CH, and L is $CH_2$.

In some particularly preferred embodiments of the present disclosure, in formula (I), X is CH, and L is a bond.

In some particularly preferred embodiments of the present disclosure, in formula (I), X is N, and L is C=O.

In some particularly preferred embodiments of the present disclosure, in formula (I), X is N, and L is O=S=O.

In some particularly preferred embodiments of the present disclosure, in formula (I), X is N, and L is $CH_2$.

In some particularly preferred embodiments of the present disclosure, in formula (I), X is N, and L is a bond.

In some preferred embodiments of the present disclosure, in formula (I), the ring A is $C_{3-7}$ cycloalkyl, 3-7 membered heterocycloalkyl, $C_{5-7}$ aryl, or 5-7 membered heteroaryl, where the ring A is optionally substituted with 1, 2, 3 or 4 $R_1$(s). In some preferred embodiments of the present disclosure, in formula (I), the ring A is $C_{5-6}$ cycloalkyl, 5-6 membered heterocycloalkyl, phenyl, or 5-6 membered heteroaryl wherein the ring A is optionally substituted with 1, 2, 3 or 4 $R_1$(s). In some preferred embodiments of the present disclosure, in formula (I), the ring A is 5-6 membered heterocycloalkyl, phenyl, or 5-6 membered heteroaryl wherein the ring A is optionally substituted with 1, 2, 3 or 4 $R_1$(s). In some preferred embodiments of the present disclosure, in formula (I), the ring A is 5-6 membered azacycloalkyl, phenyl, or 5-6 membered azaaryl, where the ring A is optionally substituted with 1, 2, 3 or 4 $R_1$(s). In some preferred embodiments of the present disclosure, in formula (I), the ring A is pyrazinyl, pyrazolyl, piperidinyl or phenyl, where the ring A is optionally substituted with 1, 2, 3 or 4 $R_1$(s). In some particularly preferred embodiments of the present disclosure, in formula (I), the ring A is pyrazinyl. In some particularly preferred embodiments of the present disclosure, in formula (I), the ring A is pyrazolyl. In some particularly preferred embodiments of the present disclosure, in formula (I), the ring A is piperidinyl. In some particularly preferred embodiments of the present disclosure, in formula (I), the ring A is phenyl.

In some preferred embodiments of the present disclosure, in formula (I), $R_1$ is absent or $R_1$ is selected from $C_{1-8}$ alkyl, $C_{2-8}$ alkenyl, $C_{2-8}$ alkynyl, $C_{1-8}$ alkoxy, $C_{3-7}$ cycloalkyl, 3-7 membered heterocycloalkyl, $C_{5-7}$ aryl, and 5-7 membered heteroaryl, wherein the $C_{1-8}$ alkyl, $C_{2-8}$ alkenyl, $C_{2-8}$ alkynyl, and $C_{1-8}$ alkoxy are optionally substituted with 1, 2, 3 or 4 $R_3$ (s), and wherein the $C_{3-7}$ cycloalkyl, 3-7 membered heterocycloalkyl, $C_{5-7}$ aryl, and 5-7 membered heteroaryl group are optionally substituted with 1, 2, 3, or 4 $R_4$(s). In some preferred embodiments of the present disclosure, in formula (I), $R_1$ is absent or $R_1$ is selected from $C_{1-8}$ alkyl, $C_{3-7}$ cycloalkyl, 3-7 membered heterocycloalkyl, $C_{5-7}$ aryl, 5-7 membered heteroaryl, wherein the $C_{1-8}$ alkyl group is optionally substituted with 1, 2, 3 or 4 $R_3$(s), and wherein the $C_{3-7}$ cycloalkyl group, 3-7 membered heterocycloalkyl, $C_{5-7}$ aryl, 5-7 membered heteroaryl are optionally substituted with 1, 2, 3, or 4 $R_4$ (s). In some preferred embodiments of the present disclosure, in formula (I), $R_1$ is absent or $R_1$ is selected from $C_{1-8}$ alkyl, and 3-7 membered heterocycloalkyl, wherein the $C_{1-8}$ alkyl is optionally substituted with 1, 2, 3, or 4 $R_3$(s), and wherein the 3-7 membered heterocycloalkyl is optionally substituted with 1, 2, 3, or 4 $R_4$(s). In some preferred embodiments of the present disclosure, in formula (I), $R_1$ is absent or $R_1$ is selected from $C_{1-6}$ alkyl, and 5-7 membered heterocycloalkyl, wherein the $C_{1-6}$ alkyl is optionally substituted with 1, 2, 3, or 4 $R_3$(s), and wherein the 5-7 membered heterocycloalkyl is optionally substituted with 1, 2, 3, or 4 $R_4$(s). In some preferred embodiments of the present disclosure, in formula (I), $R_1$ is absent or $R_1$ is selected from $C_{1-6}$ alkyl, and 5-7 membered heterocycloalkyl, wherein the $C_{1-6}$ alkyl is optionally substituted with 1 or 2 $R_3$(s), and wherein the 5-7 membered heterocycloalkyl is optionally substituted with 1, 2, 3 or 4 $C_{1-3}$ alkyl(s). In some preferred embodiments of the present disclosure, in formula (I), $R_1$ is absent or $R_1$ is selected from $C_{1-4}$ alkyl, and 5-7 membered heterocycloalkyl, wherein the $C_{1-4}$ alkyl is optionally substituted with 1 or 2 $R_3$(s), and wherein the 5-7 membered heterocycloalkyl is optionally substituted with 1, 2, 3 or 4 $C_{1-3}$ alkyl(s). In some preferred embodiments of the present disclosure, in formula (I), $R_1$ is absent or $R_1$ is selected from methyl, piperidinyl, morpholinyl, and piperazinyl, wherein the piperidinyl, morpholinyl, and piperazinyl are optionally substituted with 1, 2, 3 or 4 $C_{1-3}$ alkyl(s). In some preferred embodiments of the present disclosure, in formula (I), $R_1$ is absent or $R_1$ is selected from methyl, piperidinyl, morpholinyl, and 1-methylpiperazinyl. In some particularly preferred embodiments of the present disclosure, in formula (I), $R_1$ is absent. In some particularly preferred embodiments of the present disclosure, in formula (I), $R_1$ is 1-methylpiperazinyl. In some particularly preferred embodiments of the present disclosure, in formula (I), $R_1$ is methyl. In some particularly preferred embodiments of the present disclosure, in formula (I), $R_1$ is piperidinyl. In some particularly preferred embodiments of the present disclosure, in formula (I), $R_1$ is morpholinyl.

In some particularly preferred embodiments of the present disclosure, in formula (I), one, two or three $R_2$(s) are present and $R_2$ is selected from halogen, $C_{1-6}$ alkyl, and $C_{3-6}$ cycloalkyl, in which the $C_{1-6}$ alkyl, and $C_{3-6}$ cycloalkyl are each optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (I), one, two or three $R_2$(s) are present and $R_2$ is selected from halogen, and $C_{1-6}$ alkyl, in which the $C_{1-6}$ alkyl is optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (I), one or two $R_2$(s) are present and $R_2$ is selected from fluorine, chlorine, bromine, methyl, ethyl, n-propyl, isopropyl, n-butyl, and isobutyl. In some preferred embodiments of the present disclosure, in formula (I), one or two $R_2$(s) are present, and $R_2$ is selected from fluorine, chlorine, methyl, ethyl, n-propyl, and isopropyl. In some preferred embodiments of the present disclosure, in formula (I), one or two $R_2$ (s) are present, and $R_2$ is selected from fluorine, methyl, and ethyl. In some preferred embodiments of the present disclosure, in formula (I), one or two $R_2$ (s) are present, and $R_2$ is selected from fluorine and ethyl. In some preferred embodiments of the present disclosure, in formula (I), one $R_2$ is present, and $R_2$ is selected from fluorine and ethyl. In some preferred embodiments of the present disclosure, in formula (I), two $R_2$(s) are present, and $R_2$ is selected from fluorine and ethyl. In some particularly preferred embodiments of the present disclosure, in formula (I), two $R_2$(s) are present which are respectively fluorine and ethyl. In some particularly preferred embodiments of the present disclosure, in formula (I), one $R_2$ is present, and $R_2$ is an ethyl group.

The preferred options of the respective substituents mentioned in the above various preferred embodiments can be combined with each other in any way, and various combinations thereof are within the scope of the present disclosure.

In particular, the present disclosure provides a compound of formula (I):

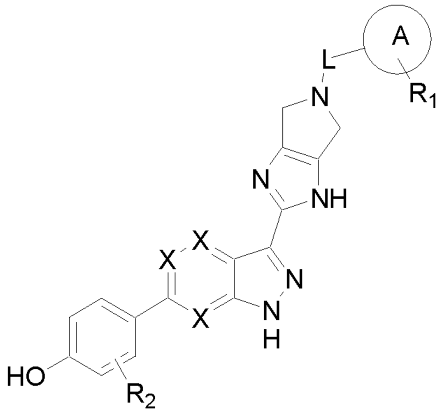

(I)

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, for use as a TRK inhibitor and/or RET inhibitor, or for use in the preparation of a TRK-inhibiting medicine and/or RET-inhibiting medicine, in which

L is C=O, and O=S=O;

X is CH;

the ring A is 5-7 membered heteroaryl or $C_{5-7}$ aryl;

0, 1, 2, 3 or 4 $R_1$(s) are present in formula (I), and $R_1$ is selected from $C_{1-8}$ alkyl, and 3-7 membered heterocycloalkyl, in which the $C_{1-8}$ alkyl is optionally substituted with 1, 2, 3 or 4 $R_3$(s), and in which the 3-7 membered heterocycloalkyl is optionally substituted with 1, 2, 3 or 4 $R_4$(s),

1, 2, or 3 $R_2$(s) are present in formula (I), and $R_2$ is selected from H, halogen, -OH, -$NO_2$, -CN, -$SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, -N($R_9$)($R_{10}$), -N($R_{11}$)(C(=O)$R_{12}$), -C(=O)-N($R_9$)($R_{10}$), -C(=O)-$R_{12}$, -C(=O)-O$R_{12}$, -OC(=O)$R_{12}$, -N($R_{11}$)(S(=O)$_2R_{12}$), -S(=O)$_2$-N($R_9$)($R_{10}$), -S$R_{12}$ and -O$R_{12}$, in which the $C_{1-6}$ alkyl, $C_{3-7}$ cycloalkyl and 4-10 membered heterocycloalkyl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), - C(=O)-O$R_{12}$, -C(=O)H, -C(=O)$R_{12}$, -C(=O)-N($R_9$)($R_{10}$), -N($R_{11}$)(S(=O)$_2R_{12}$), -S(=O)$_2$-N($R_9$)($R_{10}$), -S$R_{12}$ and -O$R_{12}$;

$R_3$ is selected from halogen, cyano, $C_{1-3}$ alkyl, hydroxy, $C_{1-6}$ alkoxy, - N($R_5$)($R_6$), -CON($R_7$)($R_8$) or 3-7 membered heterocycloalkyl, in which the 3-7 membered heterocycloalkyl is optionally substituted with 1, 2, 3 or 4 $R_4$(s);

$R_4$ is selected from halogen, $C_{1-3}$ alkyl, hydroxyl, $C_{1-6}$ alkoxy, -$NH_2$, -$NHCH_3$ or -N($CH_3$)$_2$;

$R_5$, $R_6$, $R_7$, $R_8$ are each independently hydrogen or $C_{1-4}$ alkyl;

$R_9$ is selected from H, $C_{1-4}$ alkyl, $C_{1-4}$ haloalkyl or $C_{3-7}$ cycloalkyl;

$R_{10}$ is H or selected from the group consisting of $C_{1-4}$ alkyl, $C_{1-4}$ haloalkyl, $C_{3-7}$ cycloalkyl, 4-10 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each option included in the above group is optionally substituted with 1, 2, 3 or 4 substituent(s) each independently selected from the group consisting of -OH, -$NH_2$, -NH($CH_3$), -N($CH_3$)$_2$, -CN, $C_{1-4}$ alkyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, -C(=O)-$NH_2$, -C( =O)-N($C_{1-4}$ alkyl)$_2$, $C_{1-4}$ haloalkyl, $C_{1-4}$ alkoxy and $C_{1-4}$ haloalkoxy;

$R_{11}$ is selected from H, $C_{1-4}$ alkyl and $C_{3-7}$ cycloalkyl; and

$R_{12}$ is selected from the group consisting of $C_{1-6}$ alkyl, $C_{3-7}$ cycloalkyl, 4- to 14-membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each option included in the above group is optionally substituted with 1, 2 or 3 substituent(s) each independently selected from the group consisting of halogen, $-CF_3$, -CN, -OH, $-NH_2$, $-NH(CH_3)$, $-N(CH_3)_2$, oxo, -S-$C_{1-4}$ alkyl, $C_{1-4}$ alkyl, $C_{1-4}$ haloalkyl, $C_{2-6}$ alkenyl, $C_{2-6}$ alkynyl , $C_{3-7}$ cycloalkyl, $C_{1-4}$ alkoxy and $C_{1-4}$ haloalkoxy.

In some preferred embodiments of the present disclosure, in formula (I), L is O=S=O. In some preferred embodiments of the present disclosure, in formula (I), L is C=O.

In some preferred embodiments of the present disclosure, in formula (I), the ring A is 5-6 membered heteroaryl or phenyl wherein the ring A is optionally substituted with 1, 2, 3 or 4 $R_1$(s). In some preferred embodiments of the present disclosure, in formula (I), the ring A is pyrazinyl, pyrazolyl, or phenyl, where the ring A is optionally substituted with 1, 2, 3 or 4 $R_1$(s). In some particularly preferred embodiments of the present disclosure, in formula (I), the ring A is pyrazinyl. In some particularly preferred embodiments of the present disclosure, in formula (I), the ring A is pyrazolyl. In some particularly preferred embodiments of the present disclosure, in formula (I), the ring A is phenyl.

In some preferred embodiments of the present disclosure, in formula (I), $R_1$ is absent or $R_1$ is selected from $C_{1-8}$ alkyl, and 3-7 membered heterocycloalkyl, wherein the $C_{1-8}$ alkyl is optionally substituted with 1, 2, 3, or 4 $R_3$(s), and wherein the 3-7 membered heterocycloalkyl is optionally substituted with 1, 2, 3, or 4 $R_4$(s). In some preferred embodiments of the present disclosure, in formula (I), $R_1$ is absent or $R_1$ is selected from $C_{1-6}$ alkyl, and 5-7 membered heterocycloalkyl, wherein the $C_{1-6}$ alkyl is optionally substituted with 1, 2, 3, or 4 $R_3$(s), and wherein the 5-7 membered heterocycloalkyl is optionally substituted with 1, 2, 3, or 4 $R_4$(s). In some preferred embodiments of the present disclosure, in formula (I), $R_1$ is absent or $R_1$ is selected from $C_{1-6}$ alkyl, and 5-7 membered heterocycloalkyl, wherein the $C_{1-6}$ alkyl is optionally substituted with 1 or 2 $R_3$(s), and wherein the 5-7 membered heterocycloalkyl is optionally substituted with 1, 2, 3 or 4 $C_{1-3}$ alkyl(s). In some preferred embodiments of the present disclosure, in formula (I), $R_1$ is absent or $R_1$ is selected from $C_{1-4}$ alkyl, and 5-7 membered heterocycloalkyl, wherein the $C_{1-4}$ alkyl is optionally substituted with 1 or 2 $R_3$, and wherein the 5-7 membered heterocycloalkyl is optionally substituted with 1, 2, 3 or 4 $C_{1-3}$ alkyl(s). In some preferred embodiments of the present disclosure, in formula (I), $R_1$ is absent or $R_1$ is selected from methyl, piperidinyl, and morpholinyl, wherein the piperidinyl, and morpholinyl are optionally substituted with 1, 2, 3 or 4 $C_{1-3}$ alkyl(s). In some preferred embodiments of the present disclosure, in formula (I), $R_1$ is absent or $R_1$ is selected from methyl, piperidinyl, and morpholinyl. In some particularly preferred embodiments of the present disclosure, in formula (I), $R_1$ is absent. In some particularly preferred embodiments of the present disclosure, in formula (I), $R_1$ is methyl. In some particularly preferred embodiments of the present disclosure, in formula (I), $R_1$ is piperidinyl. In some particularly preferred embodiments of the present disclosure, in formula (I), $R_1$ is morpholinyl.

In some particularly preferred embodiments of the present disclosure, in formula (I), one or two $R_2$(s) are present and $R_2$ is selected from halogen, $C_{1-6}$ alkyl, and $C_{3-6}$ cycloalkyl, in which the $C_{1-6}$ alkyl, and $C_{3-6}$ cycloalkyl are each optionally substituted with 1, 2 or 3 substituent(s) each independently selected from the group consisting of halogen, - OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (I), one, or two $R_2$(s) are present and $R_2$ is selected from halogen, and $C_{1-6}$ alkyl, in which the $C_{1-6}$ alkyl is optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (I), two $R_2$s are present and $R_2$ is selected from fluorine, chlorine, bromine, methyl, ethyl, n-propyl, isopropyl, n-butyl, and isobutyl. In some preferred embodiments of the present disclosure, in formula (I), two $R_2$s are present, and $R_2$ is selected from fluorine, chlorine, methyl, ethyl, n-propyl, and isopropyl. In some preferred embodiments of the present disclosure, in formula (I), two $R_2$s are present, and $R_2$ is selected from fluorine, methyl, and ethyl. In some preferred embodiments of the present disclosure, in formula (I), two $R_2$s are present, and $R_2$ is selected from fluorine and ethyl. In some preferred embodiments of the present disclosure, in formula (I), two $R_2$(s) are present, and $R_2$ is selected from fluorine and ethyl. In some particularly preferred embodiments of the present disclosure, in formula (I), two $R_2$(s) are present which are respectively fluorine and ethyl.

The preferred options of the respective substituents mentioned in the above various preferred embodiments can be combined with each other in any way, and various combinations thereof are within the scope of the present disclosure.

In a preferred embodiment of the present disclosure, the compound of formula (I) for use as a TRK-inhibiting medicine and/or RET-inhibiting medicine is selected from

(2-(6-(2-ethyl-5-fluoro-4-hydroxyphenyl)-1H-indazol-3-yl)-4,6-dihydropyrrolo[3,4-d]imidazol-5(1H)-yl)(5-(piperidin-1-yl)pyrazin-2-yl)ketone (MDI-2);

(2-(6-(2-ethyl-5-fluoro-4-hydroxyphenyl)-1H-indazol-3-yl)-4,6-dihydropyrrolo[3,4-d]imidazol-5(1H)-yl)(5-morpholinylpyrazin-2-yl)ketone (MDI-201);

(2-(6-(2-ethyl-5-fluoro-4-hydroxyphenyl)-1H-indazol-3-yl)-4,6-dihydropyrrolo[3,4-d]imidazol-5(1H)-yl)(1-methyl-1H-pyrazol-4-yl)ketone (MDI-202);

(2-(6-(2-ethyl-5-fluoro-4-hydroxyphenyl)-1H-indazol-3-yl)pyrrolo[3,4-d]imidazol-5(1H, 4H,6H)-yl)(1-methylpiperidin-4-yl)ketone (MDI-203);

(2-(6-(2-ethyl-5-fluoro-4-hydroxyphenyl)-1H-indazol-3-yl)pyrrolo[3,4-d]imidazol-5(1H,4H,6H)-yl)(5-(4-methylpiperzin-1-yl)pyrazin-2-yl)ketone (MDI-204);

(2-(6-(2-ethyl-4-hydroxyphenyl)-1H-indazol-3-yl)pyrrolo[3,4-d]imidazol-5(1H,4H,6H)-yl)(5-(4-methylpiperzin-1-yl) pyrazin-2-yl)ketone (MDI-205);

5-ethyl-2-fluoro-4-(3-(5-(benzenesulfonyl)-1,4,5,6-tetrahydropyrrolo[3,4-d]imidazol-2-yl)-1H-indazol-6-yl)phenol (MDI-206);

5-ethyl-2-fluoro-4-(3-(5-(pyrazin-2ylmethyl)-1,4,5,6-tetrahydropyrrolo[3,4-d]imidazol-2-yl)-1H-indazol-6-yl)phenol (MDI-207);

4-(3-(5-(cyclopropylmethyl)-1,4,5,6-tetrahydropyrrolo[3,4-d]imidazol-2-yl)-1H-indazol-6-yl)-5-ethyl-2-fluorophenol (MDI-208);

Cyclopropyl (2-(6-(2-ethyl-5-fluoro-4-hydroxyphenyl)-1H-indazol-3-yl)pyrrolo[3,4-d]imidazol-5(1H,4H,6H)-yl)ketone (MDI-1233);

4-(3-(5-(cyclobutylmethyl)-1,4,5,6-tetrahydropyrrolo[3,4-d]imidazol-2-yl)-1H-indazol-6-yl)-5-ethyl-2-fluorophenol (MDI-210);

Cyclobutyl (2-(6-(2-ethyl-5-fluoro-4-hydroxyphenyl)-1H-indazol-3-yl)pyrrolo[3,4-d]imidazol-5(1H,4H,6H)-yl)ketone (MDI-211);

(2-(6-(2-ethyl-5-fluoro-4-hydroxyphenyl)-1H-indazol-3-yl)-pyrrolo[3,4-d]imidazol-5-(1H,4H,6H)-yl)(3-hydroxycyclobutyl)ketone (MDI-213);

(2-(6-(2-ethyl-5-fluoro-4-hydroxyphenyl)-1H-indazol-3-yl)-pyrrolo[3,4-d]imidazol-5-(1H,4H,6H)-yl)(pyridazin-4-yl) ketone (MDI-214);

(2-(6-(2-ethyl-5-fluoro-4-hydroxyphenyl)-1H-indazol-3-yl)-pyrrolo[3,4-d]imidazol-5-(1H,4H,6H)-yl)(pyridazin-3-yl) ketone (MDI-215);

(S)-(2-(6-(2-ethyl-5-fluoro-4-hydroxyphenyl)-1H-indazol-3-yl)-4,6-dihydropyrrolo[3,4-d]imidazol-5(1H)-yl)(3-hydroxypyrrolidin-1-yl)keone (MDI-1228);

5-ethyl-2-fluoro-4-(3-(5-(4-hydroxycyclohexyl)-1,4,5,6-tetrahydropyrrolo[3,4-d]imidazol-2-yl)-1H-indazol-6-yl)phenol (MDI-217);

4-(3-(5-(cyclopropanesulfonyl)-1,4,5,6-tetrahydropyrrolo[3,4-d]imidazol-2-yl)-1H-indazol-6-yl)-5-ethyl-2-fluorophenol (MDI-218);

4-(3-(5-(cyclobutylsulfonyl)-1,4,5,6-tetrahydropyrrolo[3,4-d]imidazol-2-yl)-1H-indazol-6-yl)-5-ethyl-2-fluorophenol (MDI-219);

4-(3-(5-(cyclopentylsulfonyl)-1,4,5,6-tetrahydropyrrolo[3,4-d]imidazol-2-yl)-1H-indazol-6-yl)-5-ethyl-2-fluorophenol (MDI-220);

5-ethyl-2-fluoro-4-(3-(5-((1-methyl-1H-pyrazol-4-yl)methyl)-1,4,5,6-tetrahydropyrrolo[3,4-d]imidazol-2-yl)-1H-indazol-6-yl)phenol (MDI-221);

4-(3-(5-(cyclopentyl-1,4,5,6-tetrahydropyrrolo[3,4-d]imidazol-2-yl)-1H-indazol-6-yl)-5-ethyl-2-fluorophenol

(MDI-224);

5-ethyl-2-fluoro-4-(3-(5-(tetrahydro-2H-pyran-4-yl)-1,4,5,6-tetrahydropyrrolo[3,4-d]imidazol-2-yl)-1H-indazol-6-yl) phenol (MDI-225);

1-(2-(6-(2-ethyl-5-fluoro-4-hydroxyphenyl)-1H-indazol-3-yl)-4,6-dihydropyrrolo[3,4-d]imidazol-5(1H)-yl)ethan-1-one (MDI-226);

1-(2-(6-(2-ethyl-5-fluoro-4-hydroxyphenyl)-1H-indazol-3-yl)-4,6-dihydropyrrolo[3,4-d]imidazol-5(1H)-yl)propan-1-one (MDI-227);

(1-(2-(6-(2-ethyl-5-fluoro-4-hydroxyphenyl)-1H-indazol-3-yl)-4,6-dihydropyrrolo[3,4-d]imidazol-5(1H)-yl)-2-methylpropan-1-one) (MDI-228);

2-cyclopropyl-1-(2-(6-(2-ethyl-5-fluoro-4-hydroxyphenyl)-1H-indazol-3 -yl)-4,6-dihydropyrrolo[3,4-d]imidazol -5(1H)-yl)ethan-1-one (MDI-229);

1-(2-(6-(2-ethyl-5-fluoro-4-hydroxyphenyl)-1H-indazol-3-yl)-4,6-dihydropyrrolo[3,4-d]imidazol -5(1H)-yl)-3-methylbutan-1-one (MDI-230);

(2-(6-(2-ethyl-5-fluoro-4-hydroxyphenyl)-1H-indazol-3-yl)-4,6-dihydropyrrolo[3,4-d]imidazol-5-(1H)-yl)(pyrrolidin-1-yl)ketone (MDI-231);

N-(3-Chloro-2-hydroxypropyl)-2-(6-(2-ethyl-5-fluoro-4-hydroxyphenyl)-1H-indazol-3-yl)-4,6-dihydropyrrolo[3,4-d] pyrimidin-5(1H)-carboxamide (MDI-1288);

(2-(6-(2-ethyl-5-fluoro-4-hydroxyphenyl)-1H-indazol-3-yl)-4,6-dihydropyrrolo[3,4-d]imidazol-5-(1H)-yl)(piperidin-1-yl)ketone (MDI-233);

(2-(6-(2-ethyl-5-fluoro-4-hydroxyphenyl)-1H-indazol-3-yl)-4,6-dihydropyrrolo[3,4-d]imidazol-5-(1H)-yl)(morpholino) ketone (MDI-234);

(2-(6-(2-ethyl-5-fluoro-4-hydroxyphenyl)-1H-indazol-3-yl)-4,6-dihydropyrrolo[3,4-d]imidazol-5-(1H)-yl)(4-methylpiperzin-1-yl)ketone (MDI-235);

(2-(6-(2-ethyl-5-fluoro-4-hydroxyphenyl)-1H-indazol-3-yl)-4,6-dihydropyrrolo[3,4-d]imidazol-5-(1H)-yl)(4-ethylpiperzin-1-yl)ketone (MDI-236);

Cyclopropyl(2-(6-(2-ethyl-5-fluoro-4-hydroxyphenyl)-1H-pyrazolo [4,3-b]pyridin-3-yl)-4,6-dihydropyrrolo[3,4-d]imidazol-5(1H)-yl)ketone (MDI-237);

Cyclopropyl(2-(6-(2-ethyl-5-fluoro-4-hydroxyphenyl)-4-methyl-1H-indazol-3-yl)-4,6-dihydropyrrolo[3,4-d]imidazol-5(1H)-yl)ketone (MDI-239);

(S)-(2-(6-(2-ethyl-5-fluoro-4-hydroxyphenyl)-4-methyl-1H-indazol-3-yl)-4,6-dihydropyrrolo[3,4-d]imidazol-5(1H)-yl) (3-hydroxylpyrrolidin-1-yl)ketone (MDI-240);

Cyclopropyl(2-(6-(2-ethyl-5-fluoro-4-hydroxyphenyl)-1H-pyrazolo[4,3-c]pyridin-3-yl)-4,6-dihydropyrrolo[3,4-d]imidazol-5(1H)-yl)ketone (MDI-242);

(R)-(2-(6-(2-ethyl-5-fluoro-4-hydroxyphenyl)-1H-indazol-3-yl)-4,6-dihydropyrrolo[3,4-d]imidazol-5(1H )-yl)(3-hydroxylpyrrolidin-1-yl)ketone (MDI-243);

(2-(6-(2-ethyl-5-fluoro-4-hydroxyphenyl)-1H-indazol-3-yl)-4,6-dihydropyrrolo[3,4-d]imidazol-5(1H)-yl)(4-hydroxylpiperidin-1-yl)ketone (MDI-245);

2-(6-(2-ethyl-5-fluoro-4-hydroxyphenyl)-1H-indazol-3-yl)-N-methyl-4, 6-dihydropyrrolo[3,4-d]imidazol-5-(1H)- carboxamide (MDI-246);

2-(6-(2-ethyl-5-fluoro-4-hydroxyphenyl)-1H-indazol-3-yl)-N-ethyl-4, 6-dihydropyrrolo[3,4-d]imidazol-5-(1H)- carboxamide (MDI-247);

2-(6-(2-ethyl-5-fluoro-4-hydroxyphenyl)-1H-indazol-3-yl)-N-(2-hydroxyethyl)-4, 6-dihydropyrrolo[3,4-d]imidazol-5-(1H)- carboxamide (MDI-248);

1-(2-(6-(2-ethyl-5-fluoro-4-hydroxyphenyl)-1H-indazol-3-yl)-1,4,5,6-tetrahydropyrrolo[3,4-d]imidazol-5-carbonyl) pyrrolidin-3-nitrile (MDI-250);

2-(6-(2-ethyl-5-fluoro-4-hydroxyphenyl)-1H-indazol-3-yl)-N-(tetrahydrofuran-3-yl)-4,6-dihydropyrrolo[3,4-d]imidazol-5(1H)-carboxamide (MDI-251);

Methyl 2-(6-(2-ethyl-5-fluoro-4-hydroxyphenyl)-1H-indazol-3-yl)-4,6-dihydropyrrolo[3,4-d]imidazol-5(1H)-carboxylate (MDI-252);

Ethyl 2-(6-(2-ethyl-5-fluoro-4-hydroxyphenyl)-1H-indazol-3-yl)-4,6-dihydropyrrolo[3,4-d]imidazol-5(1H)-carboxylate (MDI-253);

(S)-(2-(6-(2-ethyl-5-fluoro-4-hydroxyphenyl)-1H-pyrazolo[3,4-b]pyridin-3-yl)-4,6-dihydropyrrolo[3,4-d]imidazol-5(1H)-yl)(3-hydroxylpyrrolidin-1-yl)ketone (MDI-255);

3-(2-(6-(2-ethyl-5-fluoro-4-hydroxyphenyl)-1H-indazol-3-yl)-4,6-dihydropyrrolo[3,4-d]imidazol-5(1H)-yl)-3-oxopropanenitrile (MDI-256);

2-(6-(2-ethyl-5-fluoro-4-hydroxyphenyl)-1H-indazol-3-yl)-N,N-dimethyl-4,6-dihydropyrrolo[3,4-d]imidazol-5(1H)-carboxamide (MDI-257);

N-(2-cyanoethyl)-2-(6-(2-ethyl-5-fluoro-4-hydroxyphenyl)-1H-indazol-3-yl)-4,6-dihydropyrrolo[3,4-d]imidazol-5(1H)-carboxamide (MDI-258);

N-cyclopropyl-2-(6-(2-ethyl-5-fluoro-4-hydroxyphenyl)-1H-indazol-3-yl)-4,6-dihydropyrrolo[3,4-d]imidazol-5(1H)-carboxamide (MDI-259);

N-cyclobutyl-2-(6-(2-ethyl-5-fluoro-4-hydroxyphenyl)-1H-indazol-3-yl)-4,6-dihydropyrrolo[3,4-d]imidazol-5(1H)-carboxamide (MDI-260);

(S)-6-(2-ethyl-5-fluoro-4-hydroxyphenyl)-3-(5-prolyl-1,4,5,6-tetrahydropyrrolo[3,4-d]imidazol-2-yl)- 1H-indazol (MDI-262); and

(R)-6-(2-ethyl-5-fluoro-4-hydroxyphenyl)-3-(5-prolyl-1,4,5,6-tetrahydropyrrolo[3,4-d]imidazol-2-yl)- 1H-indazol (MDI-263).

In the most preferred embodiments of the present disclosure, the compound as used is selected from

(2-(6-(2-ethyl-5-fluoro-4-hydroxyphenyl)-1H-indazol-3-yl)-4,6-dihydropyrrolo[3,4-d]imidazol-5(1H)-yl)(5-morpholinylpyrazin-2-yl)ketone (MDI-201)

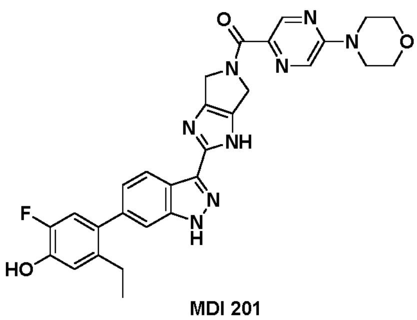

MDI 201 ;

Cyclopropyl (2-(6-(2-ethyl-5-fluoro-4-hydroxyphenyl)-1H-indazol-3-yl)pyrrolo[3,4-d]imidazol-5(1H,4H,6H)-yl)ketone (MDI-1233)

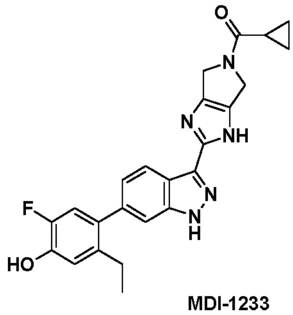

MDI-1233 ;

(S)-(2-(6-(2-ethyl-5-fluoro-4-hydroxyphenyl)-1H-indazol-3-yl)-4,6-dihydropyrrolo[3,4-d]imidazol-5(1H)-yl)(3-hydroxypyrrolidin-1-yl)keone (MDI-1228)

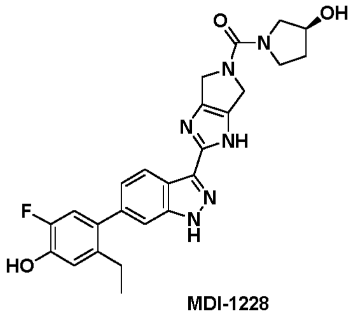

MDI-1228 ;

4-(3-(5-(cyclopropanesulfonyl)-1,4,5,6-tetrahydropyrrolo[3,4-d]imidazol-2-yl)-1H-indazol-6-yl)-5-ethyl-2-fluorophenol (MDI-218)

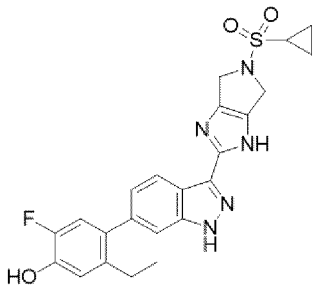

MDI-218 ;

N-(3-Chloro-2-hydroxypropyl)-2-(6-(2-ethyl-5-fluoro-4-hydroxyphenyl)-1H-indazol-3-yl)-4,6-dihydropyrrolo[3,4-d]pyrimidin-5(1H)-carboxamide (MDI-1288)

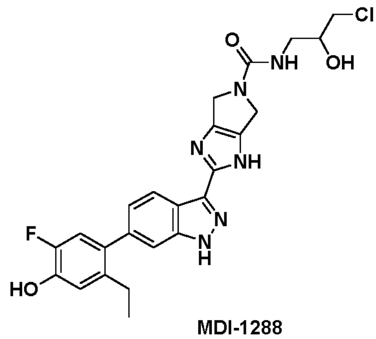

MDI-1288 ;

(2-(6-(2-ethyl-5-fluoro-4-hydroxyphenyl)-1H-indazol-3-yl)-4,6-dihydropyrrolo[3,4-d]imidazol-5-(1H)-yl)(pyrrolidin-1-yl)ketone (MDI-231)

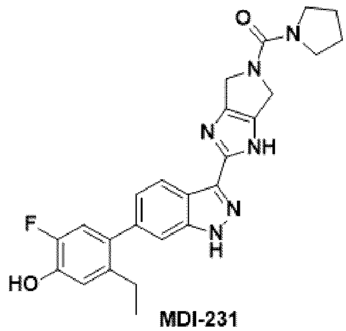

MDI-231 ;

(2-(6-(2-ethyl-5-fluoro-4-hydroxyphenyl)-1H-indazol-3-yl)-4,6-dihydropyrrolo[3,4-d]imidazol-5-(1H)-yl)(piperidin-1-yl)ketone (MDI-233)

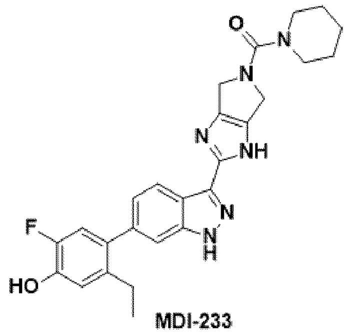

MDI-233 ;

(2-(6-(2-ethyl-5-fluoro-4-hydroxyphenyl)-1H-indazol-3-yl)-4,6-dihydropyrrolo[3,4-d]imidazol-5-(1H)-yl)(morpholino) ketone (MDI-234)

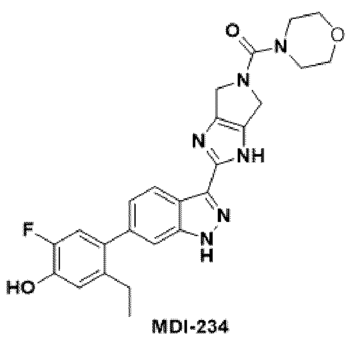

MDI-234 ;

(2-(6-(2-ethyl-5-fluoro-4-hydroxyphenyl)-1H-indazol-3-yl)-4,6-dihydropyrrolo[3,4-d]imidazol-5-(1H)-yl)(4-methylpiperzin-1-yl)ketone (MDI-235)

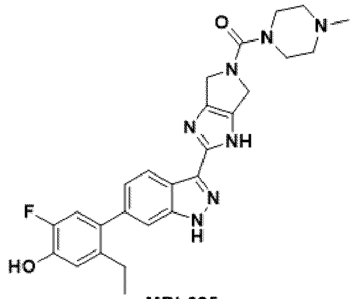

MDI-235

;

(2-(6-(2-ethyl-5-fluoro-4-hydroxyphenyl)-1H-indazol-3-yl)-4,6-dihydropyrrolo[3,4-d]imidazol-5-(1H)-yl)(4-ethylpiperzin-1-yl)ketone (MDI-236)

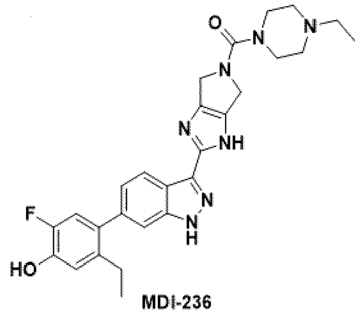

MDI-236

;

(2-(6-(2-ethyl-5-fluoro-4-hydroxyphenyl)-1H-indazol-3-yl)-4,6-dihydropyrrolo[3,4-d]imidazol-5(1H)-yl)(4-hydroxylpiperidin-1-yl)ketone (MDI-245)

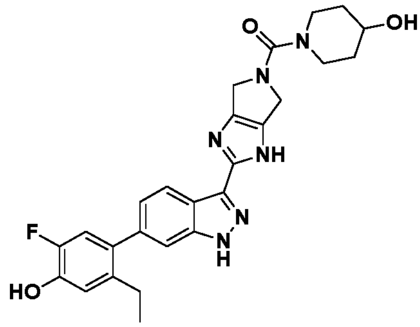

MDI-245

;

2-(6-(2-ethyl-5-fluoro-4-hydroxyphenyl)-1H-indazol-3-yl)-N-ethyl-4, 6-dihydropyrrolo[3,4-d]imidazol-5-(1H)- carboxamide (MDI-247)

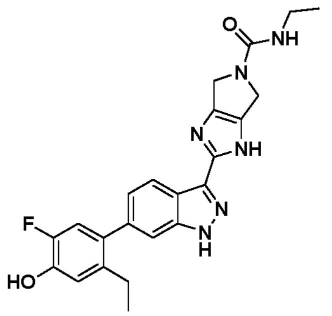

MDI-247 ;

2-(6-(2-ethyl-5-fluoro-4-hydroxyphenyl)-1H-indazol-3-yl)-N-(2-hydroxyethyl)-4, 6-dihydropyrrolo[3,4-d]imidazol-5-(1H)- carboxamide (MDI-248)

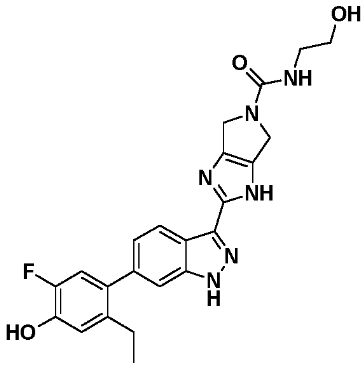

MDI-248 ;

Ethyl 2-(6-(2-ethyl-5-fluoro-4-hydroxyphenyl)-1H-indazol-3-yl)-4,6-dihydropyrrolo[3,4-d]imidazol-5(1H)-carboxylate (MDI-253)

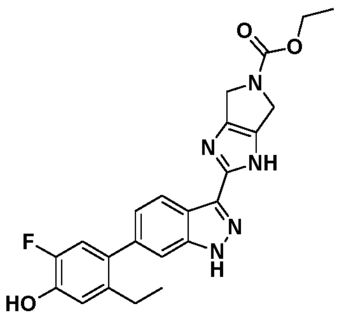

MDI-253 ;

2-(6-(2-ethyl-5-fluoro-4-hydroxyphenyl)-1H-indazol-3-yl)-N,N-dimethyl-4,6-dihydropyrrolo[3,4-d]imidazol-5(1H)-carboxamide (MDI-257)

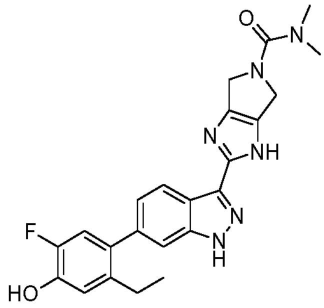

**MDI-257**

For simplicity, hereinafter, the term "a compound as shown by Formula (G)" or "a compound of Formula (G)" or "a compound of the invention" or "a compound according to the invention" also encompasses any optical isomer, geometric isomer, tautomer or a mixture of various isomers of the compound of Formula (G); the term "a compound as shown by Formula (G')" or "a compound of Formula (G')" or "a compound of the invention" or "a compound according to the invention" also encompasses any optical isomer, geometric isomer, tautomer or a mixture of various isomers of the compound of Formula (G'); and the term "a compound as shown by Formula (I)" or "a compound of Formula (I)" or "a compound of the invention" or "a compound according to the invention" also encompasses any optical isomer, geometric isomer, tautomer or a mixture of various isomers of the compound of Formula (I).

The term "optical isomer" refers that when a compound has one or more chiral centers, each chiral center may have an R configuration or an S configuration, and the various isomers thus constituted are known as an optical isomer. Optical isomers comprise all diastereomers, enantiomers, meso forms, racemates or mixtures thereof. For example, optical isomers can be separated by a chiral chromatography or by chiral synthesis.

The term "geometric isomer" refers that when a double bond is present in a compound, the compound may exist as a cis isomer, a trans isomer, an E isomer, or a Z isomer. A geometric isomer comprises a cis isomer, trans isomer, E isomer, Z isomer, or a mixture thereof.

The term "tautomer" refers to an isomer that is formed by rapid movement of an atom at two positions in a single molecule. It will be understood by those skilled in the art that tautomers can be mutually transformed, and in a certain state, may coexist by reaching an equilibrium state. As used herein, the term "a compound as shown by Formula (G)" also encompasses any tautomer of the compound of Formula (G); "a compound as shown by Formula (G')" also encompasses any tautomer of the compound of Formula (G'); and "a compound as shown by Formula (I)" also encompasses any tautomer of the compound of Formula (I).

Unless otherwise indicated, reference to "a compound as shown by Formula (G)" or "a compound of Formula (G)" or "a compound of the invention" or "a compound according to the invention" herein also encompasses isotopically-labeled compounds obtained by replacing any atom of the compound with its isotopic atom; reference to "a compound as shown by Formula (G')" or "a compound of Formula (G')" or "a compound of the invention" or "a compound according to the invention" herein also encompasses isotopically-labeled compounds obtained by replacing any atom of the compound with its isotopic atom; and reference to "a compound as shown by Formula (I)" or "a compound of Formula (I)" or "a compound of the invention" or "a compound according to the invention" herein also encompasses isotopically-labeled compounds obtained by replacing any atom of the compound with its isotopic atom.

The invention comprises all pharmaceutically acceptable isotopically-labeled compounds of Formula (G) wherein one or more atoms are replaced by atoms having the same atomic number but different atomic mass or mass number than those normally found in nature. The invention comprises all pharmaceutically acceptable isotopically-labeled compounds of Formula (G') wherein one or more atoms are replaced by atoms having the same atomic number but different atomic mass or mass number than those normally found in nature. The invention comprises all pharmaceutically acceptable isotopically-labeled compounds of Formula (I) wherein one or more atoms are replaced by atoms having the same atomic number but different atomic mass or mass number than those normally found in nature.

Examples of isotopes suitable for inclusion in the compounds of the invention include isotopes of hydrogen, such as $^{2}H$ (D) and $^{3}H$ (T), of carbon, such as $^{11}C$, $^{13}C$ and $^{14}C$, of chlorine, such as $^{36}Cl$, of fluorine, such as $^{18}F$, of iodine, such as $^{123}I$ and $^{125}I$, of nitrogen, such as $^{13}N$ and $^{15}N$, of oxygen, such as $^{15}O$, $^{17}O$ and $^{18}O$, and of sulphur, such as $^{35}S$.

Certain isotopically-labelled compounds of formula (G), for example, those incorporating a radioactive isotope, are useful in drug and/or substrate tissue distribution studies. Certain isotopically-labelled compounds of formula (G'), for example, those incorporating a radioactive isotope, are useful in drug and/or substrate tissue distribution studies. Certain isotopically-labelled compounds of formula (I), for example, those incorporating a radioactive isotope, are useful in drug and/or substrate tissue distribution studies. The radioactive isotopes deuterium, i.e. $^{2}H$, and carbon-14, i.e. $^{14}C$, are particularly useful for this purpose in view of their ease of incorporation and ready means of detection.

Substitution with heavier isotopes such as deuterium, i.e. $^{2}H$, may afford certain therapeutic advantages resulting from greater metabolic stability, for example, increased in vivo half-life or reduced dosage requirements, and hence may be preferred in some circumstances.

Substitution with positron emitting isotopes, such as $^{11}C$, $^{18}F$, $^{15}O$ and $^{13}N$, can be useful in Positron Emission Topography (PET) studies for examining substrate receptor occupancy.

Isotopically-labeled compounds of formula (G) can generally be prepared by conventional techniques known to those skilled in the art or by processes analogous to those described in the accompanying Examples and Preparations using an appropriate isotopically-labeled reagents in place of the non-labeled reagent previously employed. Isotopically-labeled compounds of formula (G') can generally be prepared by conventional techniques known to those skilled in the art or by processes analogous to those described in the accompanying Examples and Preparations using an appropriate isotopically-labeled reagents in place of the non-labeled reagent previously employed. Isotopically-labeled compounds of formula (I) can generally be prepared by conventional techniques known to those skilled in the art or by processes analogous to those described in the accompanying Examples and Preparations using an appropriate isotopically-labeled reagents in place of the non-labeled reagent previously employed.

The compound of formula (G) may exist in the form of a pharmaceutically acceptable salt, for example, an acid addition salt and/or a base addition salt of the compound of formula (G). Unless otherwise indicated, "a pharmaceutically acceptable salt" as used herein includes acid addition salts or base addition salts that may appear in the compound of formula (G). The compound of formula (G') may exist in the form of a pharmaceutically acceptable salt, for example, an acid addition salt and/or a base addition salt of the compound of formula (G'). Unless otherwise indicated, "a pharmaceutically acceptable salt" as used herein includes acid addition salts or base addition salts that may appear in the compound of formula (G'). The compound of formula (I) may exist in the form of a pharmaceutically acceptable salt, for example, an acid addition salt and/or a base addition salt of the compound of formula (I). Unless otherwise indicated, "a pharmaceutically acceptable salt" as used herein includes acid addition salts or base addition salts that may appear in the compound of formula (I).

The pharmaceutically acceptable salt of the compound of formula (G), the compound of formula (G') and the compound of formula (I) include acid addition salts and base addition salts thereof. Suitable acid addition salts are formed from acids that form non-toxic salts. Examples include but are not limited to: acetate, adipate, aspartate, benzoate, benzenesulfonate, bicarbonate/carbonate, bisulfate/sulfate, borate, camphor sulfonate, citrate, cyclohexamine sulfonate, ethanedisulfonate, formate, fumarate, glucoheptonate, gluconate, glucuronate, hexafluorophosphate, 2-(4-hydroxybenzyl) benzoate, hydrochloride/chloride, hydrobromide/bromide, hydroiodide/iodide, 2-isethionate, lactate, malate, maleate, malonate, methanesulfonate, methyl sulfate, naphthalate, 2-naphthalenesulfonate, nicotinate, nitrate, orotate, oxalate, palmitate, phosphate/hydrogen phosphate/dihydrogen phosphate, pyroglutamate, glucarate, stearate, salicylate, tannate, tartrate, tosylate and trifluoroacetate. Suitable base addition salts are formed from bases that form non-toxic salts. Examples thereof include, but are not limited to: aluminum, arginine, calcium, choline, diethylamine, diethanolamine, glycine, lysine, magnesium, meglumine, ethanolamine, potassium, sodium, tromethamine, and zinc salts. It is also possible to form half salts of acids and bases, such as hemisulfate and hemicalcium salts. For a review of suitable salts, please refer to Handbook of Pharmaceutical Salts: Properties, Selection and Use by Stahl and Wermuth (Wiley-VCH, 2002). Methods for preparing pharmaceutically acceptable salts of the compounds described herein are known to those skilled in the art.

Certain compounds of the invention may exist in unsolvated form as well as solvated forms, including hydrated forms. In general, the compounds of formula (G), the compounds of formula (G') and the compounds of formula (I), whether present in solvated form or in unsolvated form, are included within the scope of the invention.

Certain compounds of the invention may exist in different crystalline or amorphous forms, and the compounds of formula (G), the compounds of formula (G') and the compounds of Formula (I) present in any forms, are included within the scope of the invention.

To avoid ambiguity, the definitions of some terms used herein are given below. Unless otherwise stated, the meanings of the terms used herein are as follows.

The term "pharmaceutically acceptable" means that the corresponding compound, carrier or molecule is suitable for administration to humans. Preferably, the term refers to it is approved by regulatory agencies such as CFDA (China), EMEA (Europe), FDA (United States), and other national regulatory agencies to be suitable for mammals, preferably humans.

The "prodrug" refers to a derivative that is converted into a compound of the present disclosure by a reaction with enzymes, gastric acid, and the like in the living body under physiological conditions, for example, through oxidation, reduction, hydrolysis, and the like catalyzed by enzymes.

The "metabolite" refers to all molecules derived from any compound of the present disclosure in a cell or organism, preferably a human.

The term "hydroxy" refers to -OH.

The term "halogen" or "halo" refers to -F, -Cl, -Br, or -I.

The term "cyano" refers to -CN.

In the present disclosure, when there are multiple substituents of a certain type, each substituent is independently selected from each other, and these substituents may be the same or different. For example, when there are 2, 3, or 4 $R_1$s, these $R_1$s may be the same or different. For example, when there are 2, 3, or 4 $R_2$s, these $R_2$s may be the same or different. For example, when $R_1$ and $R_2$ are both -N($R_9$)($R_{10}$), $R_9$ and $R_{10}$ contained in $R_1$ and $R_2$ can be independently selected, that is, $R_9$ in $R_1$ and $R_9$ in $R_2$ can be the same or different, and $R_{10}$ in $R_1$ and $R_{10}$ in $R_2$ may be the same or different. For example, when there are two $R_1$s, and the two $R_1$s are both -N($R_9$)($R_{10}$), $R_9$ and $R_{10}$ in the two $R_1$s can be selected independently, that is, $R_9$ in the first $R_1$ and $R_9$ in the second $R_1$ may be the same or different, and $R_{10}$ in the first $R_1$ and $R_{10}$ in the second $R_1$ may be the same or different. The above statement applies to $R_1$, $R_2$ , $R_3$, $R_4$ , $R_5$, $R_6$, $R_7$, $R_8$ , $R_9$, $R_{10}$, $R_{11}$, $R_{12}$, $R_{13}$, $R_{14}$, $R_{15}$, $R_{16}$, $R_{17}$, $R_{18}$.

As used herein, the term "substituted" means that one or more (preferably 1 to 5, more preferably 1 to 3) hydrogen atoms in a group are independently replaced by a corresponding number of substituents.

As used herein, the term "independently" means that when the number of substituents is more than one, these substituents may be the same or different.

As used herein, the term "optional" or "optionally" means that the event described therein may or may not occur. For example, an "optionally substituted" group means that the group may be unsubstituted or substituted.

As used herein, the term "heteroatom" as used herein refers to oxygen (O), nitrogen (N), or $S(O)_m$in which m may be 0, 1 or 2, i.e. a sulfur atom S, or a sulfoxide group SO, or a sulfonyl group $S(O)_2$).

As used herein, the term "alkyl" refers to saturated aliphatic hydrocarbons, including straight and branched chains. In some embodiments, the alkyl group has 1-8, or 1-6, or 1-3 carbon atoms. For example, the term "$C_{1-8}$ alkyl" refers to a straight or branched chain group of atoms having 1-8 carbon atoms. The term "$C_{1-8}$ alkyl" includes the terms "$C_{1-6}$ alkyl", "$C_1$-$C_3$ alkyl" and "$C_1$-$C_4$ alkyl" in its definition. Examples of alkyl groups include, but are not limited to, methyl, ethyl, propyl, isopropyl, butyl, isobutyl, sec-butyl, tert-butyl, pentyl, 2-pentyl, 3-pentyl, isopentyl, neopentyl, (R)-2-methylbutyl, (S)-2-methylbutyl, 3-methylbutyl, 2,3-dimethylpropyl, 2,3-dimethylbutyl, hexyl, and the like. The alkyl group may be optionally substituted with one or more (for example, 1 to 5) suitable substituent(s).

As used herein, the term "alkenyl" refers to an aliphatic hydrocarbon having at least one carbon-carbon double bond, including straight and branched chains having at least one carbon-carbon double bond. In some embodiments, alkenyl groups have 2-8 carbon atoms, 2-6 carbon atoms, 3-6 carbon atoms, or 2-4 carbon atoms. For example, the term "$C_{2-8}$ alkenyl" refers to a linear or branched unsaturated atomic group (having at least one carbon-carbon double bond) having 2-8 carbon atoms. The double bond may or may not be the point of attachment of another group. Alkenyl groups include, but are not limited to, vinyl, 1-propenyl, 2-propenyl, 2-methyl-2-propenyl, butenyl, pentenyl, 3-hexenyl, and the like. Alkenyl groups may be optionally substituted with one or more (for example, 1 to 5) suitable substituent(s). When the compound of formula (I) contains an alkenyl group, the alkenyl group may be present in the pure E form, the pure Z form, or any mixture thereof.

As used herein, the term "alkynyl" refers to an aliphatic hydrocarbon having at least one carbon-carbon triple bond, including straight and branched chains having at least one carbon-carbon triple bond. In some embodiments, an alkynyl group has 2-8 carbon atoms, 2-6 carbon atoms, 3-6 carbon atoms, or 2-4 carbon atoms. For example, the term "$C_{2-8}$ alkynyl" refers to a linear or branched unsaturated atomic group (having at least one carbon-carbon triple bond) having 2-8 carbon atoms. The triple bond may or may not be the point of attachment of another group. Alkynyl groups include, but are not limited to, ethynyl, 1-propynyl, 2-propynyl, 2-methyl-2-propynyl, butynyl, pentynyl, 3-hexynyl, and the like. The alkynyl group may be optionally substituted with one or more (for example, 1 to 5) suitable substituent(s).

As used herein, the term "$C_{3-7}$ cycloalkyl" refers to a cycloalkyl group having 3-7 carbon atoms forming a ring, such as cyclopropyl, cyclobutyl, cyclopentyl, cyclohexyl, cyclohexenyl, cycloheptyl. The cycloalkyl may be optionally substituted with one or more suitable substituent(s).

As used herein, the term "n-membered heterocycloalkyl" refers to a cycloalkyl group having m ring-forming carbon atoms and (n-m) ring-forming heteroatoms, the heteroatoms being selected from O, S and N. For example, 3-7 membered heterocycloalkyl includes, but not limited to, oxetane, thietane, azetidine, tetrahydrofuran, tetrahydrothiophene, pyrrolidine, tetrahydropyran, tetrahydrothiopyran, piperidine, morpholine, piperazine, oxepane, thiepane, and azepine. The heterocycloalkyl may be optionally substituted with one or more suitable substituent(s).

As used herein, the term "$C_{5-7}$ aryl" refers to an aryl group having an aromatic ring containing 5-7 carbon atoms, preferably phenyl.

As used herein, the term "n-membered heteroaryl" refers to a heteroaryl group having m carbon atoms forming an aromatic ring and (n-m) heteroatoms forming an aromatic ring, the heteroatoms being selected from O, S and N. For example, 5-7 membered heteroaryl includes but not limited to pyrazine, pyrazole, pyrrole, furan, thiophene, thiazole, and pyridine. The heteroaryl may be optionally substituted with one or more suitable substituent(s).

As used herein, the term "$C_{7-11}$ bicyclic aryl" refers to a bicyclic aryl group having 7-11 carbon atoms, such as naphthalene, indene and the like. The bicyclic aryl may be optionally substituted with one or more suitable substituent(s).

As used herein, the term "n-membered bicyclic heteroaryl" refers to a bicyclic heteroaryl group having m carbon

atoms forming an aromatic bicyclic ring and (n-m) heteroatoms forming an aromatic bicyclic ring, and the heteroatoms are selected from O, S and N. For example, 7-11 membered bicyclic heteroaryl includes, but not limited to, quinoline, isoquinoline, benzothiazole, and the like. The bicyclic heteroaryl may be optionally substituted with one or more suitable substituent(s).

As used herein, the term "11-15 membered tricyclyl" includes but not limited to acridine and the like. The 11-15 membered tricyclyl may be optionally substituted with one or more suitable substituent(s).

As used herein, the term "haloalkyl" refers to an alkyl group having one or more halogen substituent(s) (up to perhaloalkyl, that is, each hydrogen atom of the alkyl is replaced by a halogen atom). For example, the term "$C_{1-6}$ haloalkyl" refers to a $C_{1-6}$ alkyl group with one or more halogen substituent(s) (up to perhaloalkyl, that is, each hydrogen atom of the alkyl group is replaced by a halogen atom). As another example, the term "$C_{1-4}$ haloalkyl" refers to a $C_{1-4}$ alkyl group with one or more halogen substituent(s) (up to perhaloalkyl, that is, each hydrogen atom of the alkyl group is replaced by a halogen atom); the term "$C_{1-3}$ haloalkyl" refers to a $C_{1-3}$ alkyl group with one or more halogen substituent(s) (up to perhaloalkyl, that is, each hydrogen atom of the alkyl group is replaced by a halogen atom); and the term "$C_{1-2}$ haloalkyl" refers to a $C_{1-2}$ alkyl group (i.e. methyl or ethyl) with one or more halogen substituent(s) (up to perhaloalkyl, that is, each hydrogen atom of the alkyl group is replaced by a halogen atom). As another example, the term "$C_1$ haloalkyl" refers to a methyl group with 1, 2, or 3 halogen substituent(s). Examples of haloalkyl groups include: $CF_3$, $C_2F_5$, $CHF_2$, $CH_2F$, $CH_2CF_3$, $CH_2Cl$, and the like.

As used herein, the term "alkoxy" refers to alkyl with a single bond attached to an oxygen atom. The point of attachment of the alkoxy group to a molecule is through the oxygen atom. Alkoxy can be described as alkyl-O-. The term "$C_{1-6}$ alkoxy" refers to a linear or branched alkoxy group containing 1 to 6 carbon atoms. The term "$C_{1-6}$ alkoxy" includes the term "$C_{1-3}$ alkoxy" in its definition. Alkoxy includes, but not limited to, methoxy, ethoxy, propoxy, isopropoxy, butoxy, hexoxy, and the like. The alkoxy group may be optionally substituted with one or more suitable substituent(s).

Herein, a numerical range relating to the number of substituents, the number of carbon atoms, or the number of ring members represents an enumeration of all integers in the range, and the range is only a simplified representation thereof. For example:

"1-4 substituent(s)" means 1, 2, 3 or 4 substituent(s);

"1-3 substituent(s)" means a 1, 2 or 3 substituent(s);

"3 to 12-membered ring" means a 3, 4, 5, 6, 7, 8, 9, 10, 11 or 12-membered ring;

"3 to 14-membered ring" means a 3, 4, 5, 6, 7, 8, 9, 10, 11, 12, 13 or 14-membered ring;

"3 to 8 membered ring" means a 3, 4, 5, 6, 7, or 8 membered ring;

"1-12 carbon atoms" or "$C_{1-12}$" means 1 ($C_1$), 2 ($C_2$), 3 ($C_3$), 4 ($C_4$), 5 ($C_5$), 6 ($C_6$), 7 ($C_7$), 8 ($C_8$), 9 ($C_9$), 10 ($C_{10}$), 11 ($C_{11}$) or 12 ($C_{12}$) carbon atoms;

"1-6 carbon atoms" or "$C_{1-6}$" means 1 ($C_1$), 2 ($C_2$), 3 ($C_3$), 4 ($C_4$), 5 ($C_5$) or 6 ($C_6$) carbon atoms;

"1-4 carbon atoms" or "$C_{1-4}$" means 1 ($C_1$), 2 ($C_2$), 3 ($C_3$), 4 ($C_4$) carbon atoms;

"2-6 carbon atoms" or "$C_{2-6}$" means 2 ($C_2$), 3 ($C_3$), 4 ($C_4$), 5 ($C_5$) or 6 ($C_6$) carbon atoms;

"$C_{3-8}$" means 3 ($C_3$), 4 ($C_4$), 5 ($C_5$), 6 ($C_6$), 7 ($C_7$), 8 ($C_8$) carbon atoms; and

"3 to 8 ring members" means 3, 4, 5, 6, 7, or 8 ring members.

Thus, a numerical range associated with the number of substituents, the number of carbon atoms, or the number of ring members also encompasses any one of its subranges, and each subrange is also considered to be disclosed herein.

The compounds of formula (G), formula (G') or formula (I) used in the present disclosure are known compounds and can be synthesized through various methods familiar to those skilled in the art of organic synthesis, such as the methods disclosed in Chinese Invention Patent CN111606908B.

Those skilled in the art can understand that the compounds of formula (G), formula (G') or formula (I) used in the present disclosure can be expressed as "a TRK inhibitor and/or RET inhibitor", indicating that they can be a TRK inhibitor, a RET inhibitor, or even a TRK/RET dual inhibitor. Moreover, according to the content disclosed in Chinese Invention Patent

CN111606908B, these compounds can also act as a JAK inhibitor. Therefore, the compounds of formula (G), formula (G') or formula (I) used in the present disclosure can function as a JAK/TRK/RET multiple inhibitor, which means they can be a JAK/TRK dual inhibitor, a JAK/RET dual inhibitor, a TRK/RET dual inhibitor, or a JAK/TRK/RET triple inhibitor. Since the multiple inhibitor simultaneously inhibits multiple disease targets, the compounds used in the present disclosure are more effective in treating certain diseases than traditional single-target inhibitors (as shown in the examples below). Therefore, the compounds of formula (G), formula (G') or formula (I) used in the present disclosure are preferably used as a JAK/TRK/RET multiple inhibitors. In a particularly preferred embodiment, the compounds of formula (G), formula (G') or formula (I) used in the present disclosure function as a JAK/TRK dual inhibitor; and some particularly preferred compounds can act as a Pan-JAK/Pan-TRK dual inhibitor (i.e., inhibiting all members of the JAK kinase family and the TRK kinase family simultaneously), thereby exhibiting exceptional performance in the treatment of autoimmune diseases, pruritus and other skin diseases, as well as diabetic foot and other diseases.

In a second aspect, the present disclosure provides a pharmaceutical composition for use as TRK inhibitor comprising the compounds of formula (G), formula (G') or formula (I), or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, and one or more pharmaceutically acceptable carriers, adjuvants or excipients.

In a third aspect, the present disclosure provides a pharmaceutical composition for use as a RET inhibitor comprising the compounds of formula (G), formula (G') or formula (I), or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, and one or more pharmaceutically acceptable carriers, adjuvants or excipients.

The pharmaceutical compositions of the invention may be formulated as suitable dosage forms for oral, external (including not limited to external application, spraying, and the like), parenteral (including subcutaneous, intramuscular, intradermal and intravenous), bronchial or nasal administration as desire. Preferably, the pharmaceutical compositions of the invention may be formulated as suitable dosage forms for oral or external administration. More preferably, the pharmaceutical compositions of the invention may be formulated as suitable dosage forms for oral administration.

If a solid carrier is used, the preparation may be tableted, placed in a hard gelatin capsule in powder or pellet form, or in the form of a troche or lozenge. The solid carrier may contain conventional excipients such as binding agents, fillers, tableting lubricants, disintegrants, wetting agents and the like. The tablet may, if desired, be film coated by conventional techniques. If a liquid carrier is employed, the preparation may be in the form of a syrup, emulsion, paste, soft gelatin capsule, sterile vehicle for injection, an aqueous or non-aqueous liquid suspension, or may be a dry product for reconstitution with water or other suitable vehicle before use. Liquid preparations may contain conventional additives such as suspending agents, emulsifying agents, wetting agents, non-aqueous vehicle (including edible oils), preservatives, as well as flavoring and/or coloring agents. For parenteral administration, a vehicle normally will comprise sterile water, at least in large part, although saline solutions, glucose solutions and like may be utilized. Injectable suspensions also may be used, in which case conventional suspending agents may be employed. Conventional preservatives, buffering agents and the like also may be added to the parenteral dosage forms. The pharmaceutical compositions are prepared by conventional techniques appropriate to the desired preparation containing appropriate amounts of the active ingredient, that is, the compound of Formula (G), the compound of Formula (G') or the compound of Formula (I) according to the invention.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil), and injectable organic esters such as ethyl oleate.

These compositions may also contain excipients such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

Solid dosage forms for oral administration include capsules, tablets, pills, powders, and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid; (b) binders, as for example, carboxymethylcellulose, alignates, gelatin, polyvinylpyrrolidone, sucrose, and acacia; (c) humectants, as for example, glycerol; (d) disintegrating agents, as for example, agar-agar, calcium carbonate, potato or tapioca starch, alginic acid, certain complex silicates, and sodium carbonate; (e) solution retarders, as for example paraffin; (f) absorption accelerators, as for example, quaternary ammonium compounds; (g) wetting agents, as for example, cetyl alcohol and glycerol monostearate; (h) adsorbents, as for example, kaolin and bentonite; and (i)

lubricants, as for example, talc, calcium stearate, magnesium stearate, solid polyethylene glycols, sodium lauryl sulfate, or mixtures thereof.

Solid compositions of a similar type may also be employed as fillers in soft and hard-filled gelatin capsules using such excipients as lactose or milk sugar as well as high molecular weight polyethyleneglycols, and the like.

Solid dosage forms such as tablets, dragees, capsules, pills, and granules can be prepared with coatings and shells, such as enteric coatings and others well-known in the art. They may contain opacifying agents, and can also be of such composition that they release the active compound or compounds in a certain part of the intestinal tract in a delayed manner. Examples of embedding compositions which can be used are polymeric substances and waxes. The active compounds can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to the active compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan or mixtures of these substances, and the like.

Besides such inert diluents, the composition can also include adjuvants, such as wetting agents, emulsifying and suspending agents, sweetening, flavoring, and perfuming agents.

Suspensions, in addition to the active compounds, may contain suspending agents, as for example, ethoxylatedisostearyl alcohols, polyoxyethylene sorbitol and sorbitan esters, microcrystalline cellulose, aluminum metahydroxide, bentonite, agar-agar and tragacanth, or mixtures of these substances, and the like.

Dosage forms for topical administration of a compound of the invention include paste, powders, sprays, and inhalants. The active component is admixed under sterile conditions with a physiologically acceptable carrier and any preservatives, buffers, or propellants as may be required. Ophthalmic formulations, eye ointments, powders, and solutions are also contemplated as being within the scope of this invention.

The external dosage form of the compound of the present disclosure may be in the form of a water-in-oil (W/O) or oil-in-water (O/W) emulsion, a multi-emulsion form, such as a water-in-oil-in-water (W/O/W) form or an oil-in-water-oil (O/W/O) emulsion, or in the form of water dispersion or lipid dispersion, gel or aerosol.

The external dosage form of the compound of the present disclosure may contain additives and aids, such as emulsifiers, thickeners, gelling agents, water fixatives, spreading agents, stabilizers, dyes, fragrances, and preservatives. Suitable emulsifiers include stearic acid, triethanolamine and PEG-40-stearate. Suitable thickeners include glyceryl monostearate and PEG600. Suitable preservatives include propyl paraben and chlorocresol. Suitable spreading agents include dimethicone and polydimethylcyclosiloxane. Suitable water fixatives include polyethylene glycol, preferably polyethylene glycol 600.

The external dosage form of the compound of the present disclosure may include pastes, lotions, gels, emulsions, microemulsions, sprays, skin patches, and the like, which can be applied topically to treat atopic dermatitis, EGFR Inhibitor-induced skin side effects, acne, eczema, psoriasis, and scleroderma, itching, vitiligo, hair loss and other skin diseases. In particular, the external dosage form of the compound of the present disclosure is pastes, which can be applied topically to treat skin diseases such as atopic dermatitis, EGFR Inhibitor-induced skin side effects, acne, eczema, psoriasis, scleroderma, itching, vitiligo, and hair loss and other skin diseases.

The amount of the compound of formula (G), the compound of formula (G') or the compound of formula (I) in the pharmaceutical composition and dosage form can be appropriately determined by those skilled in the art as needed. For example, the compound of formula (G), the compound of formula (G') or the compound of formula (I) can be present in the pharmaceutical composition or dosage form in a therapeutically effective amount.

In a fourth aspect, the present disclosure provides a method for the treatment of TRK or RET-related diseases or disorders, the method comprising administrating a therapeutically effective amount of the compounds of formula (G), formula (G') or formula (I), or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, or the composition as described above to patients in need. The patients are preferably a mammal, and more preferably a human patient. The route of administration can be oral, topical (including but not limited to external application, spraying, and the like), parenteral (including subcutaneous, intramuscular, cortical, and intravenous) administration, bronchial administration, or nasal administration. It is preferred that it is preferably administered orally or topically. It is more preferably administered orally.

As used herein, "diseases or disorders related to TRK or RET" include but not limited to:

Arthritis, including rheumatoid arthritis, juvenile arthritis and psoriatic arthritis;

Autoimmune diseases or disorders, including single organ or single cell type autoimmune disorders, such as

Hashimoto's thyroiditis, autoimmune hemolytic anemia, pernicious anemia of autoimmune atrophic gastritis, autoimmune encephalomyelitis, autoimmune orchitis, Goodpasture's disease, autoimmune thrombocytopenia, sympathetic ophthalmia, myasthenia gravis, Graves' disease, primary biliary cirrhosis, chronic aggressive hepatitis, ulcerative colitis and membranous glomerulopathy, those involving systemic autoimmune disorders (e.g. systemic lupus erythematosus, rheumatoid arthritis, Sjogren's syndrome, Reiter's syndrome, polymyositis-dermatomyositis, systemic sclerosis, polyarteritis nodosa, multiple sclerosis and bullous pemphigoid) and other O-cell (humoral) or T-cell autoimmune diseases (including Kogan syndrome), ankylosing spondylitis, Wegener's Granuloma, autoimmune alopecia (alopecia areata), type I diabetes or juvenile-onset diabetes or thyroiditis;

Cancer or tumor, including digestive/gastrointestinal cancer, colorectal cancer, liver cancer, skin cancer (including mast cell tumor and squamous cell carcinoma), breast cancer, ovarian cancer, prostate cancer, lymphoma, leukemia (including acute myeloid leukemia and chronic myeloid leukemia), kidney cancer, lung cancer, muscle cancer, bone cancer, bladder cancer, brain cancer, melanoma (including oral and metastatic melanoma), Kaposi's sarcoma, myeloma (including multiple myeloma), myeloproliferative disorders, proliferative diabetic retinopathy or disorders related to angiogenesis (including solid tumors);

Diabetes, including type I diabetes or diabetic complications;

Eye diseases, disorders or conditions, including autoimmune diseases of eyes, keratoconjunctivitis, vernal conjunctivitis, uveitis (including uveitis and lens uveitis related to Behcet's disease), keratitis, herpetic keratitis, keratitis conus, corneal epithelial dystrophy, leukoplakia, ocular pemphigus, Moran ulcer, scleritis, Grave's eye disease, Vogt-Koyanagi-Harada syndrome, keratoconjunctivitis sicca (dry eye) , blisters, iridocyclitis, sarcoidosis, endocrine ophthalmopathy, sympathetic ophthalmia, allergic conjunctivitis, or ocular neovascularization;

Intestinal inflammation, allergies or conditions, including Crohn's disease and/or ulcerative colitis, inflammatory bowel disease, celiac disease, proctitis, eosinophilic gastroenteritis or mastocytosis;

Neurodegenerative diseases, including motor neuron disease, Alzheimer's disease, Parkinson's disease, amyotrophic lateral sclerosis, Huntington's disease, neurodegenerative disease caused by cerebral ischemia or traumatic injury, stroke, glutamate neurotoxicity or hypoxia; stroke ischemia/reperfusion injury, myocardial ischemia, renal ischemia, heart attack, cardiac hypertrophy, atherosclerosis and arteriosclerosis, organ hypoxia or platelet aggregation;

Skin diseases, conditions or disorders, including atopic dermatitis, EGFR Inhibitor-induced skin side effects, acne, eczema, psoriasis, scleroderma, itching or other pruritic conditions, vitiligo, alopecia (alopecia areata);

Allergies, including mammalian allergic dermatitis (including equine allergic diseases, such as bite allergies), summer eczema, Culex mosquito itch syndrome (sweet itch), emphysema, inflammatory airway disease, recurrent airway obstruction, airway overreaction, or chronic obstructive pulmonary disease;

Asthma and other obstructive airway diseases, including chronic or refractory asthma, advanced asthma, bronchitis, bronchial asthma, allergic asthma, endogenous asthma, exogenous asthma or dusty asthma; and

Transplant rejection, including islet transplant rejection, bone marrow transplant rejection, graft versus host disease, organ and cell transplant rejection (for example bone marrow, cartilage, cornea, heart, intervertebral disc, pancreatic islets, kidney, limbs, liver, lung, muscle, myoblasts , nerve, pancreas, skin, small intestine or trachea) or xenotransplantation.

It has been found that the compounds of formula (G), formula (G') or formula (I) as described in the present disclosure are particularly suitable for the treatment of diseases or conditions selected from arthritis, autoimmune diseases or conditions, cancer or tumors, diabetes, delayed wound healing caused by diabetes, eye diseases, conditions or disorders, inflammatory bowel disease, allergic reactions or conditions, neurodegenerative diseases, skin diseases, conditions or disorders, allergies, asthma and other obstructive airway diseases, and transplant rejection.

Animal model experiments have revealed that the compounds of formula (G), formula (G') or formula (I) as mentioned above are optimally used in the treatment of itching, psoriasis, atopic dermatitis, acne, vitiligo, alopecia areata, asthma, rhinitis, hemorrhoids, cervicitis, pneumonia, and other diseases. In addition, these compounds can also be used to treat allergic conjunctivitis, cancer (tumor), delayed wound healing caused by diabetes, diabetic foot, and other diseases.

In some particularly preferred embodiments, the compounds of formula (G), formula (G') or formula (I) as described above can be used as a JAK/panTRK inhibitor, particularly a panJAK/panTRK inhibitor or a panJAK/panTRK/-RET inhibitors.

In some particularly preferred embodiments, the compounds of formula (G), formula (G') or formula (I) as described above can be used to treat autoimmune diseases, chronic wound healing, and diabetic complications, such as selected from: arthritis, inflammatory bowel disease, allergic reactions or conditions, neurodegenerative diseases, skin diseases, conditions or disorders, allergies, asthma and other obstructive airway diseases, transplant rejection, bedsores, delayed wound healing caused by diabetes, eye diseases, conditions or disorders, and the like. In some most preferred embodiments, the compounds of formula (G), formula (G') or formula (I) as described above can be used to treat diseases or conditions selected from itching, psoriasis, atopic dermatitis, skin side effects caused by EGFR inhibitors, acne, vitiligo, alopecia areata, asthma, rhinitis, hemorrhoids, cervicitis, pneumonia, bedsores, delayed wound healing caused by diabetes, diabetic foot, diabetic retinopathy, and the like.

In a fifth aspect, the present invention provides a compound of formula (G), formula (G') or formula (I) as described above, or an isotopically labeled compound thereof, or an optical isomer, geometric isomer, tautomer or isomer mixture thereof, or a pharmaceutically acceptable salt thereof, or a prodrug thereof, or a metabolite thereof, which is used as a TRK inhibitor and/or RET inhibitor, or which is used as a medicine for treating TRK or RET related diseases or conditions. The substituents and preference of the compounds of formula (G), formula (G') or formula (I) are as described previously, and the TRK or RET related diseases or conditions are as described previously.

Preferably, the present disclosure provides the following embodiments:

1. Use of a compound of Formula (G),

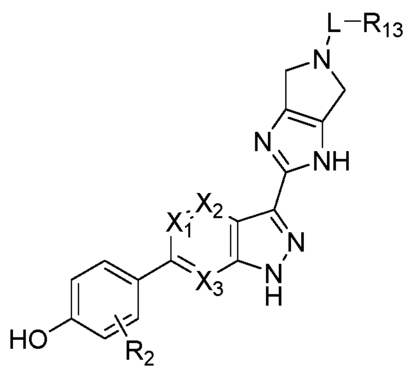

(G)

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, in the preparation of a TRK-inhibiting medicine and/or a RET-inhibiting medicine, in which

L is C=O, O=S=O, $CH_2$ or a bond; and

$X_1$ is N or $CR_{14}$; and

$X_2$ is N or $CR_{15}$; and

$X_3$ is N or $CR_{16}$; and

$R_{14}$, $R_{15}$, $R_{16}$ are each independently selected from H, -OH, -SH, -CN, halogen, $-NO_2$, $-SF_5$, -S-$C_{1-4}$ alkyl, $C_{1-6}$ alkyl, $C_{2-8}$ alkenyl, $C_{2-8}$ alkynyl, $C_{1-6}$ alkoxy, $C_{3-7}$ cycloalkyl, 3-7 membered heterocycloalkyl, $C_{5-7}$ aryl, 5-7 membered heteroaryl, $-N(R_9)(R_{10})$, - $N(R_{11})(C(=O)R_{12})$, $-C(=O)-N(R_9)(R_{10})$, $-C(=O)-R_{12}$, $-C(=O)-OR_{12}$, $-OC(=O)R_{12}$, - $N(R_{11})(S(=O)_2R_{12})$, $-S(=O)_2-N(R_9)(R_{10})$, $-SR_{12}$ and $-OR_{12}$, in which the -S-$C_{1-4}$ alkyl, $C_{1-6}$ alkyl, $C_{1-6}$ alkoxy, $C_{3-7}$ cycloalkyl, and 3-7 membered heterocycloalkyl are optionally substituted with 1, 2 or 3 substitutes selected from halogen, -OH, $-NH_2$, $-NH(CH_3)$, $-N(CH_3)_2$, -CN, $C_{1-4}$ alkyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, $-C(=O)-NH_2$, -C(=O)-N($C_{1-4}$ alkyl$)_2$, -N($C_{1-4}$ alkyl)(C(=O) $C_{1-4}$ alkyl), $C_{1-4}$ haloalkyl, $C_{1-4}$ alkoxy and $C_{1-4}$ haloalkoxy; and

$R_{13}$ is H, $-N(R_{17})(R_{18})$, $C_{1-6}$ alkoxy, $-SR_{12}$, $-OR_{12}$, -CN, halogen, $-NO_2$, $-SF_5$, - S-$C_{1-4}$ alkyl, $C_{1-6}$ alkyl or $C_{3-7}$ cycloalkyl, 3-7 membered heterocycloalkyl, $C_{5-7}$ aryl, 5-7 membered heteroaryl, $C_{7-11}$ bicyclic aryl, 7-11

membered bicyclic heteroaryl, 11-15 membered tricyclyl, $C_{5-11}$bicycloalkyl, or 5-11 membered bicyclic heteroalkyl, and $R_{13}$ is substituted with 0, 1, 2, 3 or 4 $R_1$(s), in which $R_{17}$ and $R_{18}$ are each independently selected from H, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, $C_{3-7}$ heterocycloalkyl, $C_{5-7}$ aryl, 5-7 membered heteroaryl, $C_{7-11}$ bicyclic aryl, 7-11 membered bicyclic heteroaryl, 11-15membered tricyclyl, $C_{5-11}$ bicycloalkyl, and 5-11 membered bicyclic heteroalkyl and are optionally substituted with one or more substitutes each independently selected from -OH, -CN, -SH, halogen, $-NO_2$, $-SF_5$, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, - $N(R_9)(R_{10})$, $-N(R_{11})(C(=O)R_{12})$, $-C(=O)-N(R_9)(R_{10})$, $-C(=O)-R_{12}$, $-C(=O)-OR_{12}$, $-OC(= O)R_{12}$, $-N(R_{11})(S(=O)_2R_{12})$, $-S(=O)_2-N(R_9)(R_{10})$, $-SR_{12}$ and $-OR_{12}$, wherein the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2 or 3 substitutes each independently selected from halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, $-C(=O)-OR_{12}$, -C(=O)H, - $C(=O)R_{12}$, $-C(=O)-N(R_9)(R_{10})$, $-N(R_{11})(S(=O)_2R_{12})$, $-S(=O)_2-N(R_9)(R_{10})$, $-SR_{12}$ and - $OR_{12}$; or $R_{17}$, $R_{18}$ and the N atom connected thereto together form a 3-14 membered ring; and

0, 1, 2, 3 or 4 $R_2$(s) are present in formula (G), and $R_2$ is selected from H, halogen, -OH, $-NO_2$, -CN, $-SF_5$, -SH, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, $-N(R_9)(R_{10})$, - $N(R_{11})(C(=O)R_{12})$, $-C(=O)-N(R_9)(R_{10})$, $-C(=O)-R_{12}$, $-C(=O)-OR_{12}$, $-OC(=O)R_{12}$, - $N(R_{11})(S(=O)_2R_{12})$, $-S(=O)_2-N(R_9)(R_{10})$, $-SR_{12}$ and $-OR_{12}$, in which the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, - $C(=O)-OR_{12}$, -C(=O)H, $-C(=O)R_{12}$, $-C(=O)-N(R_9)(R_{10})$, $-N(R_{11})(S(=O)_2R_{12})$, $-S(=O)_2-N(R_9)(R_{10})$, $-SR_{12}$ and $-OR_{12}$; and

$R_1$ is selected from H, halogen, -OH, $-NO_2$, -CN, $-SF_5$, -SH, -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$bicycloalkyl, 5-11 membered bicyclic heteroalkyl, $-N(R_9)(R_{10})$, $-N(R_{11})(C(=O)R_{12})$, $-C(=O)-N(R_9)(R_{10})$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the -S-$C_{1-4}$ alkyl, $C_{1-8}$ alkyl, $C_{2-8}$ alkenyl, $C_{2-8}$ alkynyl, and $C_{1-8}$ alkoxy are optionally substituted with 1, 2, 3, or 4 $R_3$(s), and in which the $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2, 3, or 4 $R_4$(s); and

$R_3$ and $R_4$ are each independently selected from H, halogen, -OH, $-NO_2$, -CN, $-SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, $-N(R_5)(R_6)$, $-N(R_{11})(C(=O)R_{12})$, $-CON(R_7)(R_8)$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2, 3 or 4 substituent(s) each independently selected from the group consisting of halogen, -CN, -OH, $C_{1-4}$ alkyl, $C_{1-6}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, $-C(=O)-OR_{12}$, -C(=O)H, $-C(=O)R_{12}$, - $C(=O)-N(R_9)(R_{10})$, $-N(R_{11})(S(=O)_2R_{12})$, $-S(=O)_2-N(R_9)(R_{10})$, $-SR_{12}$ and $-OR_{12}$; and $R_5$, $R_6$, $R_7$, $R_8$, $R_9$, $R_{10}$, $R_{11}$, and $R_{12}$ are each independently H or selected from the group consisting of $C_{1-6}$ alkyl, $C_{1-4}$ haloalkyl, $C_{3-7}$ cycloalkyl, 4-14 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein the options included in the above group are each optionally substituted with 1, 2, 3 or 4 substituent(s) each independently selected from the group consisting of halogen, $-CF_3$, -OH, $-NH_2$, $-NH(CH_3)$, $-N(CH_3)_2$, -CN, oxo, $C_{1-4}$ alkyl, $C_{2-6}$ alkenyl, $C_{2-6}$ alkynyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, $-C(= O)-NH_2$, -C(=O)-N($C_{1-4}$ alkyl$)_2$, $C_{1-4}$ haloalkyl, $C_{1-4}$ alkoxy and $C_{1-4}$ haloalkoxy.

2. The use according to embodiment 1, wherein all Hs are each independently optionally substituted with D.

3. The use according to embodiment 1 or 2, wherein only one of $X_1$, $X_2$, and $X_3$ is N.

4. The use according to embodiment 1 or 2, wherein only two of $X_1$, $X_2$, and $X_3$ are N.

5. The use according to embodiment 1 or 2, wherein $X_1$, $X_2$ and $X_3$ are the same.

6. The use according to embodiment 5, wherein $X_1$ is $CR_{14}$, $X_2$ is $CR_{15}$, $X_3$ is $CR_{16}$ and $R_{14}$, $R_{15}$ and $R_{16}$ are the same.

7. The use according to embodiment 6, wherein $R_{14}$, $R_{15}$, and $R_{16}$ are selected from H, -OH, -SH, -CN, halogen, $-NO_2$, and $C_{1-6}$ alkyl.

8. The use according to embodiment 7, wherein $R_{14}$, $R_{15}$, and $R_{16}$ are selected from H, -OH, and $C_{1-6}$ alkyl.

9. The use according to embodiment 7, wherein $X_1$, $X_2$ and $X_3$ are CH.

10. The use according to embodiment 5, wherein $X_1$, $X_2$ and $X_3$ are N.

11. The use according to any one of embodiments 1 to 10, wherein L is C=O, O=S=O or $CH_2$.

12. The use according to any one of embodiments 1 to 10, wherein $R_{13}$ is H, - $N(R_{17})(R_{18})$, $C_{1-6}$ alkoxy, -OH, -SH, -CN, halogen, $-NO_2$, $-SF_5$, $-S-C_{1-4}$ alkyl, $C_{1-6}$ alkyl, or $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$bicycloalkyl, or 5-11 membered bicyclic heteroalkyl, and $R_{13}$ is substituted with 0, 1, 2, 3 or 4 $R_1$(s), in which $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of -OH, -CN, -SH, halogen, $-NO_2$,-and $SF_5$.

13. The use according to any one of embodiments 1 to 10, wherein $R_{13}$ is H, - $N(R_{17})(R_{18})$, $C_{1-6}$ alkoxy, -OH, -SH, -CN, halogen, $-NO_2$, $-SF_5$, $-S-C_{1-4}$ alkyl, $C_{1-6}$ alkyl, $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, or 11-15 membered tricyclyl, and $R_{13}$ is substituted with 0, 1, 2, 3 or 4 $R_1$(s).

14. The use according to any one of embodiments 1 to 10, wherein $R_{13}$ is H, - $N(R_{17})(R_{18})$, $C_{1-6}$ alkoxy, $C_{1-6}$ alkyl, $C_{3-7}$ cycloalkyl, 3-7 membered heterocycloalkyl, $C_{5-7}$ aryl, or 5-7 membered heteroaryl, and $R_{13}$ is substituted with 0, 1, 2, 3, or 4 $R_1$(s).

15. The use according to any one of embodiments 1 to 10, wherein $R_{13}$ is - $N(R_{17})(R_{18})$, $C_{1-6}$ alkoxy, $C_{1-6}$ alkyl, $C_{3-7}$ cycloalkyl, 4-6 membered heterocycloalkyl, phenyl, or 5-6 membered heteroaryl, and $R_{13}$ is substituted with 0, 1, 2, or 3 $R_1$(s).

16. The use according to any one of embodiments 1 to 10, wherein $R_{17}$ and $R_{18}$ are each independently selected from H, $C_{1-6}$ alkyl, $C_{3-7}$ cycloalkyl, and $C_{3-7}$ heterocycloalkyl, and are optionally substituted with one or more of -OH, -CN, -SH, halogen, $-NO_2$,-and $-SF_5$.

17. The use according to any one of embodiments 1 to 10, wherein $R_{17}$, $R_{18}$ and the N atom connected thereto together form a 4-10 membered ring.

18. The use according to any one of embodiments 1 to 10, wherein L is C=O, and $R_{13}$ is $-N(R_{17})(R_{18})$, $C_{1-6}$ alkoxy, -OH, -SH, -CN, halogen, $-NO_2$, $-SF_5$, or $-S-C_{1-4}$ alkyl, and $R_{13}$ is substituted with 0, 1, 2, 3 or 4 $R_1$(s) in which $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of -OH, -CN, -SH, halogen, $-NO_2$,-and $-SF_5$, or $R_{17}$, $R_{18}$ and the N atom connected thereto together form a 3-14 membered ring.

19. The use according to any one of embodiments 1 to 10, wherein 1, 2 or 3 $R_2$(s) are present and $R_2$ is selected from H, halogen, -OH, $-NO_2$, -CN, $-SF_5$, -SH, $-S-C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, and 4-10 membered heterocycloalkyl, in which the $-S-C_{1-4}$ alkyl, $C_{1-6}$ alkyl, $C_{3-7}$ cycloalkyl, and 4-10 membered heterocycloalkyl are each optionally substituted with 1, 2 or 3 substituent(s) each independently selected from the group consisting of halogen, -OH, $-NH_2$, $-NH(CH_3)$, - $N(CH_3)_2$, -CN, $C_{1-4}$ alkyl, $C_{1-4}$ haloalkyl, $C_{1-4}$ alkoxy, and $C_{1-4}$ haloalkoxy.

20. The use according to any one of embodiments 1 to 10, wherein 1, 2 or 3 $R_2$(s) are present, and $R_2$ is selected from

halogen, $C_{1-6}$ alkyl, and $C_{3-6}$ cycloalkyl in which the $C_{1-6}$ alkyl and $C_{3-6}$ cycloalkyl are each optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy.

21. The use according to embodiment 15, wherein 1 or 2 $R_2$(s) are present, and $R_2$ is selected from halogen, and $C_{1-6}$ alkyl.

22. The use according to any one of embodiments 1 to 10, wherein $R_{13}$ is substituted with 0 or 1 $R_1$, and $R_1$ is selected from halogen, -OH, CN, $C_{1-6}$ alkyl, 5-7 membered heterocycloalkyl, and $C_{3-7}$ cycloalkyl, in which the $C_{1-6}$ alkyl is optionally substituted with 1, 2, or 3 $R_3$(s) and in which the 5-7 membered heterocycloalkyl, and $C_{3-7}$ cycloalkyl is optionally substituted with 1, 2, 3 or 4 $C_{1-3}$ alkyl(s).

23. The use according to embodiment 1, wherein the compound is a compound of Formula (I),

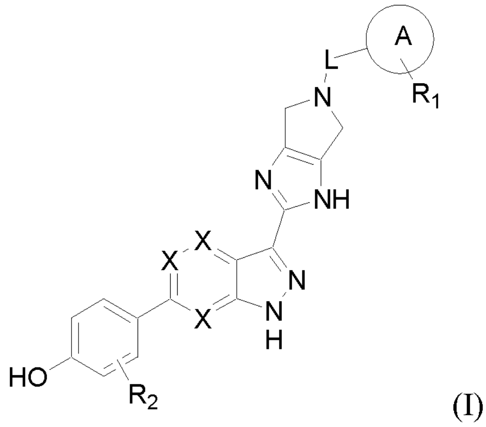

(I)

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, in which

L is C=O, O=S=O, $CH_2$ or a bond; and

X is CH or N;

the ring A is $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, or 11 -15 membered tricyclyl;

0, 1, 2, 3 or 4 $R_1$(s) are present in formula (I), and $R_1$ is selected from H, halogen, $C_{1-8}$ alkyl, $C_{2-8}$ alkenyl, $C_{2-8}$ alkynyl, $C_{1-8}$ alkoxy, $C_{3-7}$ cycloalkyl, 3-7 membered heterocycloalkyl, $C_{5-7}$ aryl, 5-7 membered heteroaryl, $C_{7-11}$ bicyclic aryl, and 7-11 membered bicyclic heteroaryl, in which the $C_{1-8}$ alkyl, $C_{2-8}$ alkenyl, $C_{2-8}$ alkynyl, and $C_{1-8}$ alkoxy are optionally substituted with 1, 2, 3 or 4 $R_3$(s), and in which the $C_{3-7}$ cycloalkyl, 3-7 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2, 3 or 4 $R_4$(s),

0, 1, 2, 3 or 4 $R_2$(s) are present in formula (I), and $R_2$ is selected from H, halogen, -OH, $-NO_2$, -CN, $-SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $-N(R_9)(R_{10})$, $-N(R_{11})(C(=O)R_{12})$, $-C(=O)-N(R_9)(R_{10})$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl and 4-10 membered heterocycloalkyl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, $- C(=O)-OR_{12}$, -C(=O)H, $-C(=O)R_{12}$, $-C(=O)-N(R_9)(R_{10})$, $-N(R_{11})(S(=O)_2R_{12})$, $-S(=O)_2-N(R_9)(R_{10})$, $-SR_{12}$ and $-OR_{12}$;

$R_3$ is selected from halogen, cyano, $C_{1-3}$ alkyl, hydroxy, $C_{1-6}$ alkoxy, $-N(R_5)(R_6)$, $-CON(R_7)(R_8)$ or 3-7 membered heterocycloalkyl, in which the 3-7 membered heterocycloalkyl is optionally substituted with 1, 2, 3 or 4 $R_4$(s);

$R_4$ is selected from halogen, $C_{1-3}$ alkyl, hydroxyl, $C_{1-6}$ alkoxy, $-NH_2$, $-NHCH_3$ or $-N(CH_3)_2$;

$R_5$, $R_6$, $R_7$, $R_8$ are each independently hydrogen or $C_{1-4}$ alkyl;

$R_9$ is selected from H, $C_{1-4}$ alkyl, $C_{1-4}$ haloalkyl or $C_{3-7}$ cycloalkyl;

$R_{10}$ is H or selected from the group consisting of $C_{1-4}$ alkyl, $C_{1-4}$ haloalkyl, $C_{3-7}$ cycloalkyl, 4-10 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each option included in the above group is optionally substituted with 1, 2, 3 or 4 substituent(s) each independently selected from the group consisting of -OH, $-NH_2$, $-NH(CH_3)$, $-N(CH_3)_2$, -CN, $C_{1-4}$ alkyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, $-C(=O)-NH_2$, -C( =O)-N($C_{1-4}$ alkyl)$_2$, $C_{1-4}$ haloalkyl, $C_{1-4}$ alkoxy and $C_{1-4}$ haloalkoxy;

$R_{11}$ is selected from H, $C_{1-4}$ alkyl and $C_{3-7}$ cycloalkyl; and

$R_{12}$ is selected from the group consisting of $C_{1-6}$ alkyl, $C_{3-7}$ cycloalkyl, 4- to 14-membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each option included in the above group is optionally substituted with 1, 2 or 3 substituent(s) each independently selected from the group consisting of halogen, $-CF_3$, -CN, -OH, $-NH_2$, $-NH(CH_3)$, $-N(CH_3)_2$, oxo, -S-$C_{1-4}$ alkyl, $C_{1-4}$ alkyl, $C_{1-4}$ haloalkyl, $C_{2-6}$ alkenyl, $C_{2-6}$ alkynyl, $C_{3-7}$ cycloalkyl, $C_{1-4}$ alkoxy and $C_{1-4}$ haloalkoxy.

24. The use according to embodiment 23, wherein L is C=O, O=S=O or $CH_2$.

25. The use according to embodiment 23, wherein X is CH.

26. The use according to any one of embodiments 23 to 25, wherein the ring A is $C_{3-7}$ cycloalkyl, 3-7 membered heterocycloalkyl, $C_{5-7}$ aryl, or 5-7 membered heteroaryl.

27. The use according to any one of embodiments 23 to 25, wherein the ring A is 5-6 membered heteroaryl, or phenyl.

28. The use according to any one of embodiments 23 to 25, wherein 0, or 1 $R_1$ is present, and $R_1$ is selected from $C_{1-6}$ alkyl, and 5-7 membered heterocycloalkyl in which the $C_{1-6}$ alkyl is optionally substituted with 1 or 2 $R_3$(s), and in which the 5-7 membered heterocycloalkyl is optionally substituted with 1, 2, 3 or 4 $C_{1-3}$ alkyl(s).

29. The use according to any one of embodiments 23 to 25, wherein 1 or 2 $R_2$(s) are present, and $R_2$ is selected from halogen, $C_{1-6}$ alkyl and $C_{3-6}$ cycloalkyl, in which the $C_{1-6}$ alkyl and $C_{3-6}$ cycloalkyl are each optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy.

30. The use according to any one of embodiments 23 to 25, wherein

L is C=O, and O=S=O;

X is CH;

the ring A is 5-7 membered heteroaryl or $C_{5-7}$ aryl;

0, 1, 2, 3 or 4 $R_1$(s) are present in formula (I), and $R_1$ is selected from $C_{1-8}$ alkyl, and 3-7 membered heterocycloalkyl, in which the $C_{1-8}$ alkyl is optionally substituted with 1, 2, 3 or 4 $R_3$(s), and in which the 3-7 membered heterocycloalkyl is optionally substituted with 1, 2, 3 or 4 $R_4$(s),

1, 2, or 3 $R_2$(s) are present in formula (I), and $R_2$ is selected from H, halogen, -OH, $-NO_2$, -CN, $-SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $-N(R_9)(R_{10})$, $-N(R_{11})(C(=O)R_{12})$, $-C(=O)-N(R_9)(R_{10})$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl and 4-10 membered heterocycloalkyl are each optionally substituted with 1, 2 or 3 substituent(s) each independently selected from the group

consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), - C(=O)-O$R_{12}$, -C(=O)H, -C(=O)$R_{12}$, -C(=O)-N($R_9$)($R_{10}$), -N($R_{11}$)(S(=O)$_2R_{12}$), -S(=O)$_2$-N($R_9$)($R_{10}$), -S$R_{12}$ and -O$R_{12}$;

$R_3$ is selected from halogen, cyano, $C_{1-3}$ alkyl, hydroxy, $C_{1-6}$ alkoxy, - N($R_5$)($R_6$), -CON($R_7$)($R_8$) or 3-7 membered heterocycloalkyl, in which the 3-7 membered heterocycloalkyl is optionally substituted with 1, 2, 3 or 4 $R_4$(s);

$R_4$ is selected from halogen, $C_{1-3}$ alkyl, hydroxyl, $C_{1-6}$ alkoxy, -$NH_2$, -$NHCH_3$ or -N$(CH_3)_2$;

$R_5$, $R_6$, $R_7$, $R_8$ are each independently hydrogen or $C_{1-4}$ alkyl;

$R_9$ is selected from H, $C_{1-4}$ alkyl, $C_{1-4}$ haloalkyl or $C_{3-7}$ cycloalkyl;

$R_{10}$ is H or selected from the group consisting of $C_{1-4}$ alkyl, $C_{1-4}$ haloalkyl, $C_{3-7}$ cycloalkyl, 4-10 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each option included in the above group is optionally substituted with 1, 2, 3 or 4 substituent(s) each independently selected from the group consisting of -OH, -$NH_2$, -NH($CH_3$), -N$(CH_3)_2$, -CN, $C_{1-4}$ alkyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, -C(=O)-$NH_2$, -C( =O)-N($C_{1-4}$ alkyl)$_2$, $C_{1-4}$ haloalkyl, $C_{1-4}$ alkoxy and $C_{1-4}$ haloalkoxy;

$R_{11}$ is selected from H, $C_{1-4}$ alkyl and $C_{3-7}$ cycloalkyl; and

$R_{12}$ is selected from the group consisting of $C_{1-6}$ alkyl, $C_{3-7}$ cycloalkyl, 4- to 14-membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each option included in the above group is optionally substituted with 1, 2 or 3 substituent(s) each independently selected from the group consisting of halogen, -$CF_3$, -CN, -OH, -$NH_2$, -NH($CH_3$), -N$(CH_3)_2$, oxo, -S-$C_{1-4}$ alkyl, $C_{1-4}$ alkyl, $C_{1-4}$ haloalkyl, $C_{2-6}$ alkenyl, $C_{2-6}$ alkynyl, $C_{3-7}$ cycloalkyl, $C_{1-4}$ alkoxy and $C_{1-4}$ haloalkoxy.

31. The use according to embodiment 30, wherein the ring A is 5-6 membered heteroaryl, or phenyl.

32. The use according to embodiment 30, wherein 0 or 1 $R_1$ is present, and $R_1$ is selected from $C_{1-6}$ alkyl, and 5-7 membered heterocycloalkyl, wherein the $C_{1-6}$ alkyl is optionally substituted by 1 or 2 $R_3$(s), and wherein the 5-7 membered heterocycloalkyl group is optionally substituted with 1, 2, 3, or 4 $C_{1-3}$ alkyl(s).

33. The use according to embodiment 30, wherein 1 or 2 $R_2$(s) are present, and $R_2$ is selected from halogen, $C_{1-6}$ alkyl and $C_{3-6}$ cycloalkyl, wherein the $C_{1-6}$ alkyl and $C_{3-6}$ cycloalkyl are each optionally substituted with 1, 2 or 3 substituent(s) each independently selected from the group consisting of halogen, -OH, -$NH_2$, -NH($CH_3$), -N$(CH_3)_2$, -CN, $C_{1-4}$ alkyl, $C_{1-4}$ haloalkyl, $C_{1-4}$ alkoxy and $C_{1-4}$ haloalkoxy.

34. The use according to embodiment 1, wherein the compound is selected from a group consisting of:

(2-(6-(2-ethyl-5-fluoro-4-hydroxyphenyl)-1H-indazol-3-yl)-4,6-dihydropyrrolo[3,4-d]imidazol-5(1H)-yl)(5-(piperidin-1-yl)pyrazin-2-yl)ketone (MDI-2);

(2-(6-(2-ethyl-5-fluoro-4-hydroxyphenyl)-1H-indazol-3-yl)-4,6-dihydropyrrolo[3,4-d]imidazol-5(1H)-yl)(5-morpholinylpyrazin-2-yl)ketone (MDI-201);

(2-(6-(2-ethyl-5-fluoro-4-hydroxyphenyl)-1H-indazol-3-yl)-4,6-dihydropyrrolo[3,4-d]imidazol-5(1H)-yl)(1-methyl-1H-pyrazol-4-yl)ketone (MDI-202);

(2-(6-(2-ethyl-5-fluoro-4-hydroxyphenyl)-1H-indazol-3-yl)pyrrolo[3,4-d]imidazol-5(1H, 4H,6H)-yl)(1-methylpiperidin-4-yl)ketone (MDI-203);

(2-(6-(2-ethyl-5-fluoro-4-hydroxyphenyl)-1H-indazol-3-yl)pyrrolo[3,4-d]imidazol-5(1H,4H,6H)-yl)(5-(4-methylpiperzin-1-yl)pyrazin-2-yl)ketone (MDI-204);

(2-(6-(2-ethyl-4-hydroxyphenyl)-1H-indazol-3-yl)pyrrolo[3,4-d]imidazol-5(1H,4H,6H)-yl)(5-(4-methylpiperzin-1-yl)pyrazin-2-yl)ketone (MDI-205);

5-ethyl-2-fluoro-4-(3-(5-(benzenesulfonyl)-1,4,5,6-tetrahydropyrrolo[3,4-d]imidazol-2-yl)-1H-indazol-6-yl)phenol (MDI-206);

5-ethyl-2-fluoro-4-(3-(5-(pyrazin-2ylmethyl)-1,4,5,6-tetrahydropyrrolo[3,4-d]imidazol-2-yl)-1H-indazol-6-yl) phenol (MDI-207);

4-(3-(5-(cyclopropylmethyl)-1,4,5,6-tetrahydropyrrolo[3,4-d]imidazol-2-yl)-1H-indazol-6-yl)-5-ethyl-2-fluorophenol (MDI-208);

Cyclopropyl (2-(6-(2-ethyl-5-fluoro-4-hydroxyphenyl)-1H-indazol-3-yl)pyrrolo[3,4-d]imidazol-5(1H,4H,6H)-yl) ketone (MDI-1233);

4-(3-(5-(cyclobutylmethyl)-1,4,5,6-tetrahydropyrrolo[3,4-d]imidazol-2-yl)-1H-indazol-6-yl)-5-ethyl-2-fluorophenol (MDI-210);

Cyclobutyl (2-(6-(2-ethyl-5-fluoro-4-hydroxyphenyl)-1H-indazol-3-yl)pyrrolo[3,4-d]imidazol-5(1H,4H,6H)-yl)ketone (MDI-211);

(2-(6-(2-ethyl-5-fluoro-4-hydroxyphenyl)-1H-indazol-3-yl)-pyrrolo[3,4-d]imidazol-5-(1H,4H,6H)-yl)(3-hydroxycyclobutyl)ketone (MDI-213);

(2-(6-(2-ethyl-5-fluoro-4-hydroxyphenyl)-1H-indazol-3-yl)-pyrrolo[3,4-d]imidazol-5-(1H,4H,6H)-yl)(pyridazin-4-yl)ketone (MDI-214);

(2-(6-(2-ethyl-5-fluoro-4-hydroxyphenyl)-1H-indazol-3-yl)-pyrrolo[3,4-d]imidazol-5-(1H,4H,6H)-yl)(pyridazin-3-yl)ketone (MDI-215);

(S)-(2-(6-(2-ethyl-5-fluoro-4-hydroxyphenyl)-1H-indazol-3-yl)-4,6-dihydropyrrolo[3,4-d]imidazol-5(1H)-yl)(3-hydroxypyrrolidin-1-yl)keone (MDI-1228);

5-ethyl-2-fluoro-4-(3-(5-(4-hydroxycyclohexyl)-1,4,5,6-tetrahydropyrrolo[3,4-d]imidazol-2-yl)-1H-indazol-6-yl) phenol (MDI-217);

4-(3-(5-(cyclopropanesulfonyl)-1,4,5,6-tetrahydropyrrolo[3,4-d]imidazol-2-yl)-1H-indazol-6-yl)-5-ethyl-2-fluorophenol (MDI-218);

4-(3-(5-(cyclobutylsulfonyl)-1,4,5,6-tetrahydropyrrolo[3,4-d]imidazol-2-yl)-1H-indazol-6-yl)-5-ethyl-2-fluorophenol (MDI-219);

4-(3-(5-(cyclopentylsulfonyl)-1,4,5,6-tetrahydropyrrolo[3,4-d]imidazol-2-yl)-1H-indazol-6-yl)-5-ethyl-2-fluorophenol (MDI-220);

5-ethyl-2-fluoro-4-(3-(5-((1-methyl-1H-pyrazol-4-yl)methyl)-1,4,5,6-tetrahydropyrrolo[3,4-d]imidazol-2-yl)-1H-indazol-6-yl)phenol (MDI-221);

4-(3-(5-(cyclopentyl-1,4,5,6-tetrahydropyrrolo[3,4-d]imidazol-2-yl)-1H-indazol-6-yl)-5-ethyl-2-fluorophenol (MDI-224);

5-ethyl-2-fluoro-4-(3-(5-(tetrahydro-2H-pyran-4-yl)-1,4,5,6-tetrahydropyrrolo[3,4-d]imidazol-2-yl)-1H-indazol-6-yl)phenol (MDI-225);

1-(2-(6-(2-ethyl-5-fluoro-4-hydroxyphenyl)-1H-indazol-3-yl)-4,6-dihydropyrrolo[3,4-d]imidazol-5(1H)-yl) ethan-1-one (MDI-226);

1-(2-(6-(2-ethyl-5-fluoro-4-hydroxyphenyl)-1H-indazol-3-yl)-4,6-dihydropyrrolo[3,4-d]imidazol-5(1H)-yl)propan-1-one (MDI-227);

(1-(2-(6-(2-ethyl-5-fluoro-4-hydroxyphenyl)-1H-indazol-3-yl)-4,6-dihydropyrrolo[3,4-d]imidazol-5(1H)-yl)-2-methylpropan-1-one) (MDI-228);

2-cyclopropyl-1-(2-(6-(2-ethyl-5-fluoro-4-hydroxyphenyl)-1H-indazol-3 -yl)-4,6-dihydropyrrolo[3,4-d]imidazol -5(1H)-yl)ethan-1-one (MDI-229);

1-(2-(6-(2-ethyl-5-fluoro-4-hydroxyphenyl)-1H-indazol-3-yl)-4,6-dihydropyrrolo[3,4-d]imidazol -5(1H)-yl)-3-methylbutan-1-one (MDI-230);

(2-(6-(2-ethyl-5-fluoro-4-hydroxyphenyl)-1H-indazol-3-yl)-4,6-dihydropyrrolo[3,4-d]imidazol-5-(1H)-yl)(pyrrolidin-1-yl)ketone (MDI-231);

N-(3-Chloro-2-hydroxypropyl)-2-(6-(2-ethyl-5-fluoro-4-hydroxyphenyl)-1H-indazol-3-yl)-4,6-dihydropyrrolo [3,4-d]pyrimidin-5(1H)-carboxamide (MDI-1288);

(2-(6-(2-ethyl-5-fluoro-4-hydroxyphenyl)-1H-indazol-3-yl)-4,6-dihydropyrrolo[3,4-d]imidazol-5-(1H)-yl)(piperidin-1-yl)ketone (MDI-233);

(2-(6-(2-ethyl-5-fluoro-4-hydroxyphenyl)-1H-indazol-3-yl)-4,6-dihydropyrrolo[3,4-d]imidazol-5-(1H)-yl)(morpholino)ketone (MDI-234);

(2-(6-(2-ethyl-5-fluoro-4-hydroxyphenyl)-1H-indazol-3-yl)-4,6-dihydropyrrolo[3,4-d]imidazol-5-(1H)-yl)(4-methylpiperzin-1-yl)ketone (MDI-235);

(2-(6-(2-ethyl-5-fluoro-4-hydroxyphenyl)-1H-indazol-3-yl)-4,6-dihydropyrrolo[3,4-d]imidazol-5-(1H)-yl)(4-ethylpiperzin-1-yl)ketone (MDI-236);

Cyclopropyl(2-(6-(2-ethyl-5-fluoro-4-hydroxyphenyl)-1H-pyrazolo [4,3-b]pyridin-3-yl)-4,6-dihydropyrrolo[3,4-d] imidazol-5(1H)-yl)ketone (MDI-237);

Cyclopropyl(2-(6-(2-ethyl-5-fluoro-4-hydroxyphenyl)-4-methyl-1H-indazol-3-yl)-4,6-dihydropyrrolo[3,4-d]imidazol-5(1H)-yl)ketone (MDI-239);

(S)-(2-(6-(2-ethyl-5-fluoro-4-hydroxyphenyl)-4-methyl-1H-indazol-3-yl)-4,6-dihydropyrrolo[3,4-d]imidazol-5(1H)-yl)(3-hydroxylpyrrolidin-1-yl)ketone (MDI-240);

Cyclopropyl(2-(6-(2-ethyl-5-fluoro-4-hydroxyphenyl)-1H-pyrazolo[4,3-c]pyridin-3-yl)-4,6-dihydropyrrolo[3,4-d] imidazol-5(1H)-yl)ketone (MDI-242);

(R)-(2-(6-(2-ethyl-5-fluoro-4-hydroxyphenyl)-1H-indazol-3-yl)-4,6-dihydropyrrolo[3,4-d]imidazol-5(1H )-yl)(3-hydroxylpyrrolidin-1-yl)ketone (MDI-243);

(2-(6-(2-ethyl-5-fluoro-4-hydroxyphenyl)-1H-indazol-3-yl)-4,6-dihydropyrrolo[3,4-d]imidazol-5(1H)-yl)(4-hydroxylpiperidin-1-yl)ketone (MDI-245);

2-(6-(2-ethyl-5-fluoro-4-hydroxyphenyl)-1H-indazol-3-yl)-N-methyl-4, 6-dihydropyrrolo[3,4-d]imidazol-5-(1H)-carboxamide (MDI-246);

2-(6-(2-ethyl-5-fluoro-4-hydroxyphenyl)-1H-indazol-3-yl)-N-ethyl-4, 6-dihydropyrrolo[3,4-d]imidazol-5-(1H)-carboxamide (MDI-247);

2-(6-(2-ethyl-5-fluoro-4-hydroxyphenyl)-1H-indazol-3-yl)-N-(2-hydroxyethyl)-4, 6-dihydropyrrolo[3,4-d]imidazol-5-(1H)- carboxamide (MDI-248);

1-(2-(6-(2-ethyl-5-fluoro-4-hydroxyphenyl)-1H-indazol-3-yl)-1,4,5,6-tetrahydropyrrolo[3,4-d]imidazol-5-carbonyl)pyrrolidin-3-nitrile (MDI-250);

2-(6-(2-ethyl-5-fluoro-4-hydroxyphenyl)-1H-indazol-3-yl)-N-(tetrahydrofuran-3-yl)-4,6-dihydropyrrolo[3,4-d]imidazol-5(1H)-carboxamide (MDI-251);

Methyl 2-(6-(2-ethyl-5-fluoro-4-hydroxyphenyl)-1H-indazol-3-yl)-4,6-dihydropyrrolo[3,4-d]imidazol-5(1H)-carboxylate (MDI-252);

Ethyl 2-(6-(2-ethyl-5-fluoro-4-hydroxyphenyl)-1H-indazol-3-yl)-4,6-dihydropyrrolo[3,4-d]imidazol-5(1H)-carboxylate (MDI-253);

(S)-(2-(6-(2-ethyl-5-fluoro-4-hydroxyphenyl)-1H-pyrazolo[3,4-b]pyridin-3-yl)-4,6-dihydropyrrolo[3,4-d]imidazol-5(1H)-yl)(3-hydroxylpyrrolidin-1-yl)ketone (MDI-255);

3-(2-(6-(2-ethyl-5-fluoro-4-hydroxyphenyl)-1H-indazol-3-yl)-4,6-dihydropyrrolo[3,4-d]imidazol-5(1H)-yl)-3-oxopropanenitrile (MDI-256);

2-(6-(2-ethyl-5-fluoro-4-hydroxyphenyl)-1H-indazol-3-yl)-N,N-dimethyl-4,6-dihydropyrrolo[3,4-d]imidazol-5(1H)-carboxamide (MDI-257);

N-(2-cyanoethyl)-2-(6-(2-ethyl-5-fluoro-4-hydroxyphenyl)-1H-indazol-3-yl)-4,6-dihydropyrrolo[3,4-d]imidazol-5(1H)-carboxamide (MDI-258);

N-cyclopropyl-2-(6-(2-ethyl-5-fluoro-4-hydroxyphenyl)-1H-indazol-3-yl)-4,6-dihydropyrrolo[3,4-d]imidazol-5(1H)-carboxamide (MDI-259);

N-cyclobutyl-2-(6-(2-ethyl-5-fluoro-4-hydroxyphenyl)-1H-indazol-3-yl)-4,6-dihydropyrrolo[3,4-d]imidazol-5(1H)-carboxamide (MDI-260);

(S)-6-(2-ethyl-5-fluoro-4-hydroxyphenyl)-3-(5-prolyl-1,4,5,6-tetrahydropyrrolo[3,4-d]imidazol-2-yl)- 1H-indazol (MDI-262); and

(R)-6-(2-ethyl-5-fluoro-4-hydroxyphenyl)-3-(5-prolyl-1,4,5,6-tetrahydropyrrolo[3,4-d]imidazol-2-yl)- 1H-indazol (MDI-263),

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof.

35. The use according to embodiment 1, wherien the compound is selected from

(2-(6-(2-ethyl-5-fluoro-4-hydroxyphenyl)-1H-indazol-3-yl)-4,6-dihydropyrrolo[3,4-d]imidazol-5(1H)-yl)(5-morpholinylpyrazin-2-yl)ketone (MDI-201)

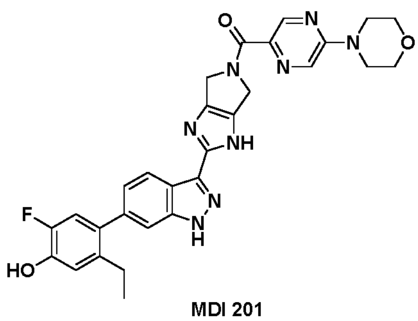

MDI 201 ;

Cyclopropyl (2-(6-(2-ethyl-5-fluoro-4-hydroxyphenyl)-1H-indazol-3-yl)pyrrolo[3,4-d]imidazol-5(1H,4H,6H)-yl) ketone (MDI-1233)

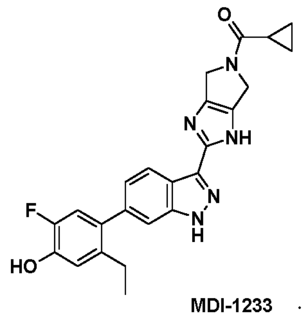

MDI-1233 ;

(S)-(2-(6-(2-ethyl-5-fluoro-4-hydroxyphenyl)-1H-indazol-3-yl)-4,6-dihydropyrrolo[3,4-d]imidazol-5(1H)-yl)(3-hydroxypyrrolidin-1-yl)keone (MDI-1228)

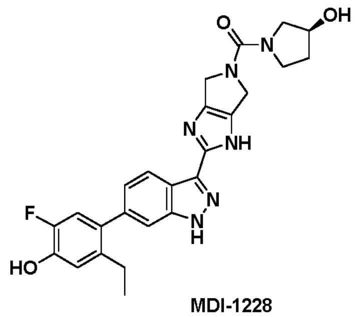

MDI-1228 ;

4-(3-(5-(cyclopropanesulfonyl)-1,4,5,6-tetrahydropyrrolo[3,4-d]imidazol-2-yl)-1H-indazol-6-yl)-5-ethyl-2-fluorophenol (MDI-218)

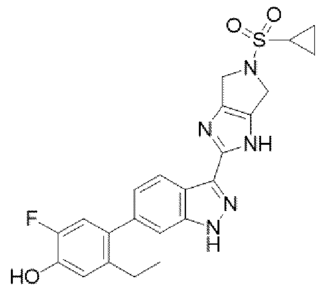

MDI-218 ;

N-(3-Chloro-2-hydroxypropyl)-2-(6-(2-ethyl-5-fluoro-4-hydroxyphenyl)-1H-indazol-3-yl)-4,6-dihydropyrrolo [3,4-d]pyrimidin-5(1H)-carboxamide (MDI-1288)

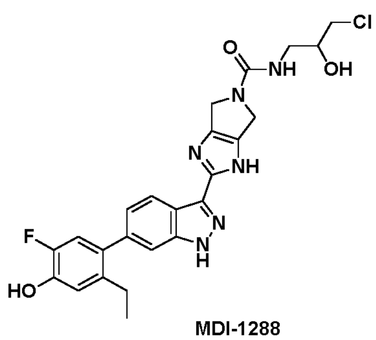

MDI-1288 ;

(2-(6-(2-ethyl-5-fluoro-4-hydroxyphenyl)-1H-indazol-3-yl)-4,6-dihydropyrrolo[3,4-d]imidazol-5-(1H)-yl)(pyrrolidin-1-yl)ketone (MDI-231)

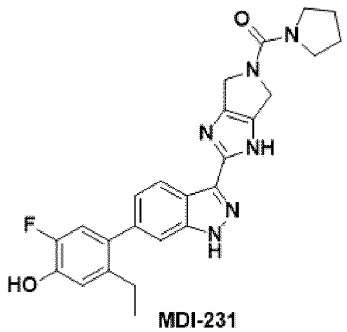

MDI-231

;

(2-(6-(2-ethyl-5-fluoro-4-hydroxyphenyl)-1H-indazol-3-yl)-4,6-dihydropyrrolo[3,4-d]imidazol-5-(1H)-yl)(piperidin-1-yl)ketone (MDI-233)

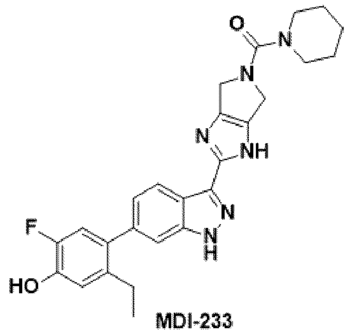

MDI-233

;

(2-(6-(2-ethyl-5-fluoro-4-hydroxyphenyl)-1H-indazol-3-yl)-4,6-dihydropyrrolo[3,4-d]imidazol-5-(1H)-yl)(morpholino)ketone (MDI-234)

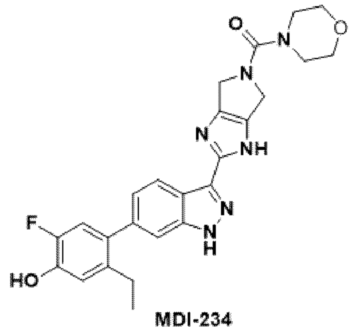

MDI-234

;

(2-(6-(2-ethyl-5-fluoro-4-hydroxyphenyl)-1H-indazol-3-yl)-4,6-dihydropyrrolo[3,4-d]imidazol-5-(1H)-yl)(4-methylpiperzin-1-yl)ketone (MDI-235)

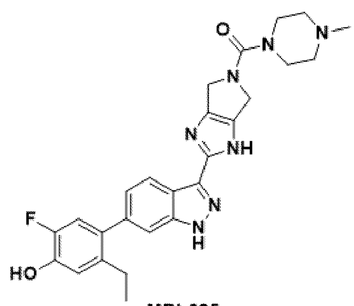

MDI-235

;

(2-(6-(2-ethyl-5-fluoro-4-hydroxyphenyl)-1H-indazol-3-yl)-4,6-dihydropyrrolo[3,4-d]imidazol-5-(1H)-yl)(4-ethylpiperzin-1-yl)ketone (MDI-236)

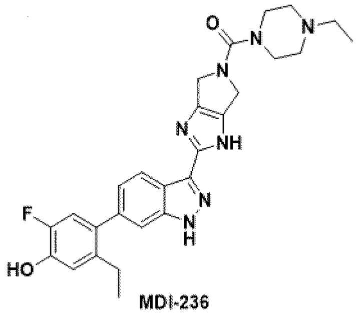

MDI-236 ;

(2-(6-(2-ethyl-5-fluoro-4-hydroxyphenyl)-1H-indazol-3-yl)-4,6-dihydropyrrolo[3,4-d]imidazol-5(1H)-yl)(4-hydroxylpiperidin-1-yl)ketone (MDI-245)

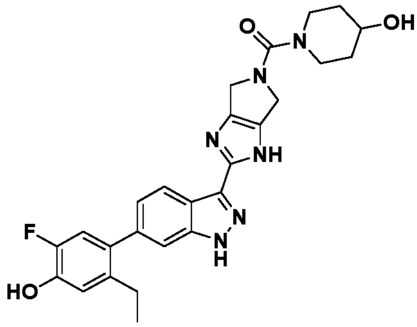

MDI-245 ;

2-(6-(2-ethyl-5-fluoro-4-hydroxyphenyl)-1H-indazol-3-yl)-N-ethyl-4, 6-dihydropyrrolo[3,4-d]imidazol-5-(1H)-carboxamide (MDI-247)

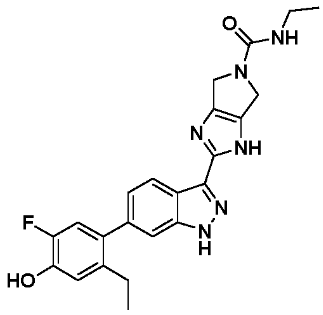

MDI-247 ;

2-(6-(2-ethyl-5-fluoro-4-hydroxyphenyl)-1H-indazol-3-yl)-N-(2-hydroxyethyl)-4, 6-dihydropyrrolo[3,4-d]imidazol-5-(1H)- carboxamide (MDI-248)

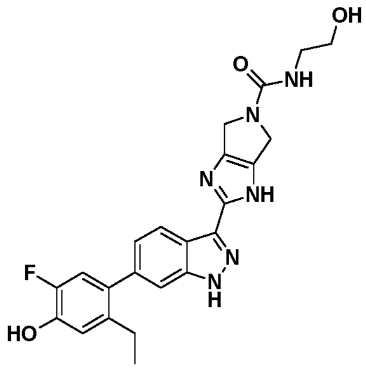

MDI-248 ;

Ethyl 2-(6-(2-ethyl-5-fluoro-4-hydroxyphenyl)-1H-indazol-3-yl)-4,6-dihydropyrrolo[3,4-d]imidazol-5(1H)-carboxylate (MDI-253)

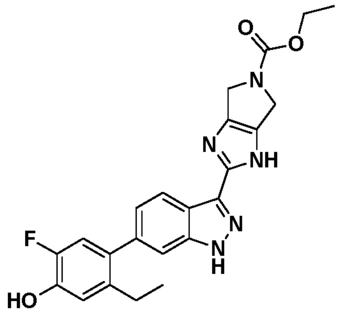

MDI-253 ;

2-(6-(2-ethyl-5-fluoro-4-hydroxyphenyl)-1H-indazol-3-yl)-N,N-dimethyl-4,6-dihydropyrrolo[3,4-d]imidazol-5(1H)-carboxamide (MDI-257)

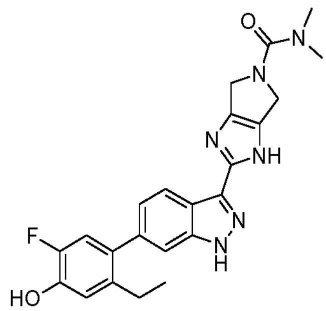

MDI-257 ,

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof.

36. The use according to any one of embodiments 1 to 35, wherein the compound is used as a JAK/TRK dual inhibitor (preferably used as a pan-JAK/pan-TRK dual inhibitor) or as a JAK/TRK/RET multiple inhibitor (preferably used as a pan-JAK/pan-TRK/RET multiple inhibitor).

37. A pharmaceutical composition for use as a TRK inhibitor and/or RET inhibitor, comprising the compound, or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof as defined in any one of embodiments 1-35, and one or more pharmaceutically acceptable carriers, adjuvants

or excipients.

38. The pharmaceutical composition according to embodiment 37, which is formulated as an oral dosage form or as a topical dosage form suitable for external application.

39. The pharmaceutical composition according to embodiment 37, which is formulated as a JAK/TRK dual inhibiting medicine (preferably used as a pan-JAK/pan-TRK dual inhibiting medicine) or as a JAK/TRK/RET multiple inhibiting medicine (preferably formulated as a pan-JAK/pan-TRK/RET multiple inhibiting medicine).

40. Use of the compound, or isotopically labeled compound thereof, or optical isomer thereof, geometric isomer thereof, a tautomer thereof or a mixture of various isomers, or a pharmaceutically acceptable salt thereof, or a prodrug thereof, or a metabolite thereof as defined in any one of embodiments 1-35 or the pharmaceutical composition of embodiment 37, 38 or 39 in the manufacture of a medicament for the treatment and/or prevention of a TRK- and/or RET-related disease or disorder.

41. The use according to embodiment 40, wherein the TRK- and/or RET-related disease or disorder is selected from the group consisting of arthritis, autoimmune diseases or disorders, cancer or tumor, diabetes and its complications, (diabetes-induced) delayed wound healing, eye diseases, disorders or conditions, intestinal inflammation, allergies or conditions, neurodegenerative diseases, skin diseases, conditions or disorders, allergies, asthma and other obstructive airway diseases, and transplant rejection.

42. The use according to embodiment 40, wherein the TRK- and/or RET-related disease or disorder is selected from the group consisting of itching, psoriasis, atopic dermatitis, skin side effects caused by EGFR inhibitors, acne, vitiligo, alopecia areata, asthma, rhinitis, hemorrhoids, cervicitis, pneumonia, delayed wound healing caused by diabetes, diabetic foot, diabetic retinopathy, cancer (tumor), and bedsores.

The present invention will be further illustrated and described below in conjunction with the drawings and specific examples.

## DESCRIPTION OF THE DRAWINGS

FIG. 1 shows the results of IL-5 Elisa in the lung lavage fluid of an ovalbumin-induced asthma model in mice in which the data are expressed as mean ± standard error (Mean± SEM) (*p<0.05; **p<0.01; ***p<0.001 vs. vehicle control group, One-way ANOVA, Bonferroni's Multiple Comparison Test).

FIG. 2 shows the results of inflammatory cell count in the lung lavage fluid of an ovalbumin-induced asthma model in mice in which the data are expressed as mean ± standard error (Mean± SEM) (*p<0.05; **p<0.01; ***p<0.001 vs. vehicle control group, One-way ANOVA, Bonferroni's Multiple Comparison Test).

FIG. 3 shows the results of IL-5 Elisa in the serum of an ovalbumin-induced asthma model in mice in which the data are expressed as mean ± standard error (Mean± SEM) (*p<0.05; **p<0.01; ***p<0.001 vs. vehicle control group, One-way ANOVA, Bonferroni's Multiple Comparison Test).

FIG. 4 shows representative images of lung tissue histopathology staining in an ovalbumin-induced asthma model in mice.

FIG. 5 shows the results of lung tissue histopathology scoring in an ovalbumin-induced asthma model in mice in which the data are expressed as mean ± standard error (Mean± SEM) (*p<0.05; **p<0.01; ***p<0.001 vs. vehicle control group, One-way ANOVA, Bonferroni's Multiple Comparison Test).

FIG. 6 shows the scoring results of drug intervention in male mice of an allergic rhinitis model in mice (*, p<0.05).

FIG. 7 shows the scoring results of drug intervention in female mice of an allergic rhinitis model in mice (*, p<0.05).

FIG. 8 shows the HE staining results of each sections of different gender mice in an allergic rhinitis model in mice.

FIG. 9 shows the detection results of IL-4 level in the serum of male mice in an allergic rhinitis model in mice (*, $p<0.05$).

FIG. 10 shows the detection results of IL-4 level in the serum of female mice in an allergic rhinitis model in mice (*, $p<0.05$).

FIG. 11 shows the HE staining results of lung tissue in each group in a chronic obstructive pulmonary disease model in rats.

FIG. 12 shows the changes in IL-$\beta$ expression of serum and lavage fluid in a chronic obstructive pulmonary disease model in rats.

FIG. 13 shows the modeling effect of external hemorrhoids in rats and the effect of positive control drugs.

FIG. 14 shows the HE staining scoring of pathological sections in each group in an external hemorrhoid model in rats.

FIG. 15 shows the detection of TNF-$\alpha$ expression levels in each group in an external hemorrhoid model in rats.

FIG. 16 shows the effect of the drugs on the inflammation of external genitalia in phenol-induced cervicitis rats.

FIG. 17 shows the effect of the drugs on the erythema symptoms in phenol-induced cervicitis rats.

FIG. 18 shows the effect of the drugs on the edema symptoms in phenol-induced cervicitis rats.

FIG. 19 shows the effect of the drugs on the secretion symptoms in phenol-induced cervicitis rats.

FIG. 20 shows the effect of the drugs on the cervical index in phenol-induced cervicitis rats.

FIG. 21 shows the HE staining results of vagina in phenol-induced cervicitis rats.

FIG. 22 shows the scoring results of skin redness, bleeding, eruption, and desquamation in an atopic dermatitis/itchy skin disease model in mice.

FIG. 23 shows the area under curve (AUC) of the scoring for skin redness, bleeding, eruption, and desquamation in an atopic dermatitis/itchy skin disease model in mice.

FIG. 24 shows the HE staining experimental results ($\times 400$) in an atopic dermatitis/itchy skin disease model in mice.

FIG. 25 shows the toluidine blue staining experimental results ($\times 400$) in an atopic dermatitis/itchy skin disease model in mice.

FIG. 26 shows the effect of the drugs on IL-4 in the serum of atopic dermatitis/itchy skin disease mice (* compared to the normal group, $p<0.05$).

FIG. 27 shows the effect of the drugs on IL-13 in the serum of atopic dermatitis/itchy skin disease mice (* compared to the normal group, $p<0.05$).

FIG. 28 shows the effect of the drugs on IFN-$\gamma$ in the serum of atopic dermatitis/itchy skin disease mice (* compared to the normal group, $p<0.05$).

FIG. 29 shows the effect of the drugs on TNF-$\alpha$ in the serum of atopic dermatitis/itchy skin disease mice (* indicates $p<0.05$ compared to the normal group).

FIG. 30 shows the effect of the drugs on the skin&hair depigmentation area score in vitiligo mice at the treated site i.e. skin&hair depigmentation area).

FIG. 31 shows the effect of the drugs on the skin&hair depigmentation area scoreof vitiligo mice at non-treated sites including ears, trunk without administration, and tail.

FIG. 32 shows the HE staining results of vitiligo mice.

FIG. 33 shows the effect of the drugs on TNF-α in the serum of vitiligo mice (* indicates p<0.01 compared to the blank control group, # indicates p<0.01 compared to the model group).

FIG. 34 shows the effect of the drugs on IL-6 in the serum of vitiligo mice (* indicates p<0.01 compared to the blank control group, # indicates p<0.01 compared to the model group).

FIG. 35 shows the comparison of the area under curve (AUC) of skin&hair growth scores of alopecia areata mice.

FIG. 36 shows the skin&hair growth condition of alopecia areata mice on day 21.

FIG. 37 shows the skin&hair growth condition of female alopecia areata mice.

FIG. 38 shows the skin&hair growth condition of male alopecia areata mice.

FIG. 39 shows the skin thickness score of Imiquimod-induced psoriasis mice.

FIG. 40 shows the clinical score of Imiquimod-induced psoriasis mice.

FIG. 41 shows the area under curve (AUC) of skin thickness scores of Imiquimod-induced psoriasis mice (* indicates p<0.05, ** indicates p<0.01, **** indicates p<0.0001 vs. the vehicle control group, One-way ANOVA).

FIG. 42 shows the area under curve (AUC) of clinical scores of Imiquimod-induced psoriasis mice (*** indicates p<0.001, **** indicates p<0.0001 vs. the vehicle control group, One-way ANOVA).

FIG. 43 shows the spleen weight of Imiquimod-induced psoriasis mice at the end of the experiment (*** indicates p<0.001, **** indicates p<0.0001 vs. the vehicle control group, One-way ANOVA).

FIG. 44 shows the pathological scoring results of psoriasis mice (**** indicates p<0.0001 vs. the vehicle control group, One-way ANOVA).

FIG. 45 shows the detection results of the cytokine TNF-α of psoriasis mice (* indicates p<0.05 vs. the vehicle control group, One-way ANOVA).

FIG. 46 shows the effect of the drugs on the ear slices weight of acne rabbits (## indicates p<0.01 compared to the blank control, * indicates p<0.05 compared to the model (vehicle) group).

FIG. 47 shows the histopathology of an ear tissue of acne rabbits.

FIG. 48 shows the effect of the drugs on the diameter of sebaceous glands in ear tissue (## indicates p<0.01 compared to the blank control, * indicates p<0.05 compared to the model (vehicle) group).

FIG. 49 shows the effect of the drugs on the hair follicle area in an ear tissue of acne rabbits (## indicates p<0.01 compared to the blank control, # indicates p<0.05 compared to the blank control, ** indicates p<0.01 compared to the model (vehicle) group, * indicates p<0.05 compared to the model (vehicle) group).

FIG. 50 shows the effect of the drugs on serum IL-1α of acne rabbits (## indicates p<0.01 compared to the blank control, ** indicates p<0.01 compared to the model (vehicle) group).

FIG. 51 shows the effect of the drugs on serum IL-6 of acne rabbits (## indicates p<0.01 compared to the blank control, ** indicates p<0.01 compared to the model (vehicle) group).

FIG. 52 shows the effect of the drugs on serum DHT of acne rabbits.

FIG. 53 shows the experimental process and results determing the effect of the compound on lipolysis in vitro fat cells.

FIG. 54 shows the HE staining results of non-wounded skin and wound tissue in mice.

FIG. 55 shows the experimental results of the compound promoting epithelial healing in wounds.

## EXAMPLES

In order to make the inventive objects, technical solutions, and beneficial technical effects of the present invention clearer, the following describes the embodiments of the present invention in further detail with reference to examples. However, it should be understood that the examples of the present invention are provided merely for the purpose of explaining the invention and are not intended to limit the invention. Moreover, the embodiments of the present invention are not limited to those given in the specification. Reagents or instruments without a specified source in the examples are conventional reagents or instruments used in chemical or biological laboratories. Operations without specified experimental conditions or operating conditions in the examples are carried out under conventional conditions known to those skilled in the art, or according to the conditions recommended by material suppliers or instrument manufacturers.

### Example 1:(2-(6-(2-ethyl-5-fluoro-4-hydroxyphenyl)-1H-indazol-3-yl)-4,6-dihydropyrrolo[3,4-d]imidazol-5(1H)-yl)(5-(piperidin-1-yl)pyrazin-2-yl)ketone (MDI-2)

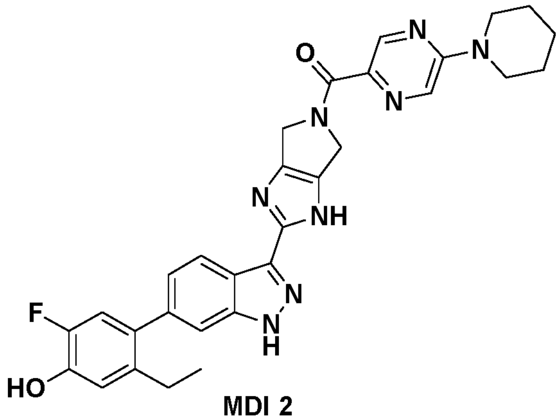

MDI 2

It was synthesised according to the method shown in Example 1 of Chinese invention patent CN111606908B, and the NMR hydrogen spectral data of the resulting product were as follows:

$^1$H NMR (400 MHz, MeOD-d4) δ 8.67 (s, 1H), 8.28 (dd, J = 8.0 Hz, J = 4.0 Hz, 1H), 8.21 (s, 1H), 7.40 (s, 1H), 7.18 (dd, J = 8.0 Hz, J = 4.0 Hz, 1H), 6.96-6.89 (m, 2H), 5.14 (s, 2H), 4.82 (s, 2H), 3.76-3.73 (m, 4H), 2.58 ( dd, J = 12.0 Hz, J = 8.0 Hz, 2H), 1.76-1.66 (m, 6H), 1.10 (t, J = 8.0 Hz, 3H).

### Example 1: (2-(6-(2-ethyl-5-fluoro-4-hydroxyphenyl)-1H-indazol-3-yl)-4,6-dihydropyrrolo[3,4-d]imidazol-5(1H)-yl)(5-morpholinepyrazin-2-yl)ketone (MDI-201)

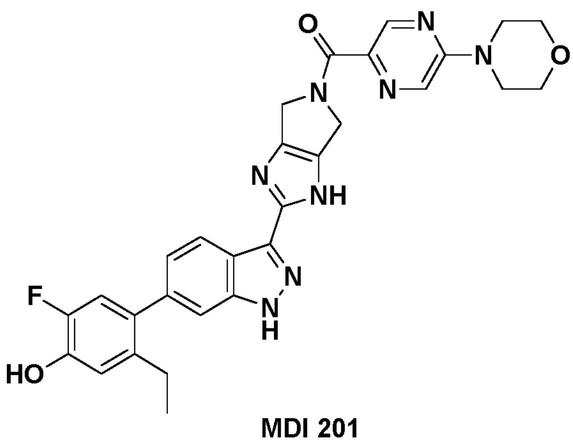

MDI 201

It was synthesised according to the method shown in Example 2 of Chinese invention patent CN111606908B as follows.

**Synthetic route of MDI-201:**

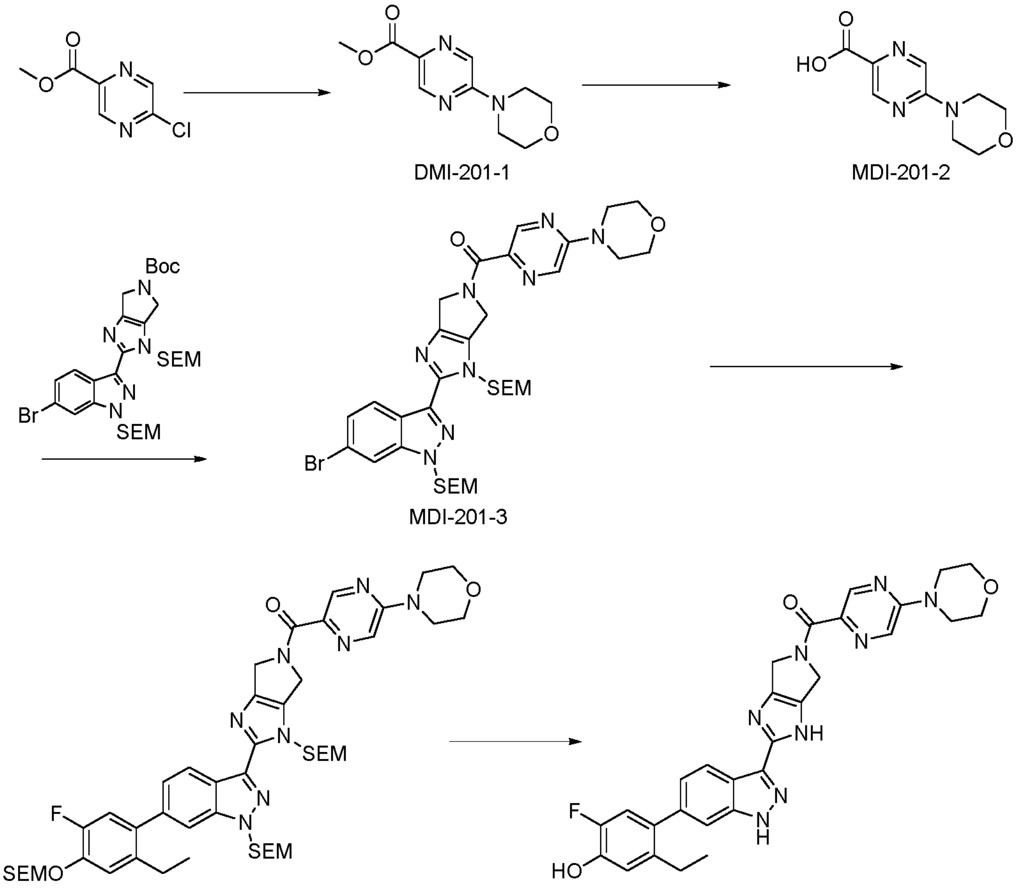

DMI-201-1

MDI-201-2

MDI-201-3

MDI-201-4

MDI-201

**Synthesis method:**

**Synthesis of intermediate MDI-201-1: Methyl 5-morpholine pyrazin-2-carboxylate**

Methyl 5-chloro-pyrazine-2-carboxylate (1.5 g, 8.7 mmol) was dissolved in 10ml DMF, and N,N-diisopropylethylamine (3.0 ml, 17.4 mmol) and morpholine (0.91 g, 10.4 mmol) were added. The mixture was stirred overnight at room temperature. Under vigorous stirring, water was added and a solid precipitated out, and filtered. The resulting filter cake was washed with water, and dried to afford the intermediate MDI-201-1 with a yield of 72.2%.

**Synthesis of intermediate MDI-201-2: 5-morpholinepyrazin-2-carboxylic acid**

The intermediate MDI-201-1 (1.4 g, 6.27 mmol) was dissolved in 20 ml of tetrahydrofuran and 20 ml of water, lithium hydroxide (0.32 g, 7.53 mmol) was added, and the reaction was carried out at room temperature for 4 hours. The reaction mixture was concentrated by distilling off tetrahydrofuran under reduced pressure and adjusted with 1N HCl to pH=4. A solid precipitated out, and filtered. The resulting filter cake was washed with water, and dried to afford the intermediate MDI-201-2 with a yield of 99.1%.

$^{1}H$ NMR (400 MHz, CDCl3) $\delta$ 8.92 (s, 1H), 8.04 (s, 1H), 3.88-3.86 (m, 4H), 3.80-3.77 (m, 4H).

**Synthesis of intermediate MDI-201-3: (2-(6-bromo1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-yl)(5-morpholinepyrazin-2-yl)ketone**

The intermediate MDI-201-2 (27.4 mg, 0.13 mmol) and N,N-diisopropylethylamine (46.0 mg, 0.36 mmol) was

dissolved in DMF, to which HATU (67.8 mg, 0.18 mmol) was added. It was allowed to react at room temperature for 10 minutes. Intermediate tert-butyl 2-(6-bromol-((2-(trimethylsilyl)ethoxy)methyl)-1H-indazol-3-yl)-1-((2-(trimethylsilyl) ethoxy)methyl)-4,6-dihydropyrrolo[3,4-d]imidazole-5(1H)-carboxylate (80 mg, 0.12 mmol) was dissolved in 5ml dichloromethane, to which 1ml of trifluoroacetic acid was added. The mixture was stirred at room temperature for 30 minutes, and concentrated to give a residue. The residue was dissolved in dichloromethane and concentrated to dryness, which was repeated 3 times. The resulting residue was dissolved in DMF, and then slowly added to the previous reaction solution. It was allowed to react at room temperature overnight, and water was added to quench the reaction. The mixture was extracted twice with ethyl acetate and the organic phases were combined, washed with water and saturated brine, dried over anhydrous sodium sulfate, concentrated, and purified on a silica gel column to afford intermediate MDI-201-3 with a yield of 47.8%.

$^1H$ NMR (400 MHz, $CDCl_3$) δ 8.91 (d, J = 8.0 Hz, 1H), 8.44-8.36 (m, 1H), 8.10 (d, J = 8.0 Hz, 1H), 7.80 (s, 1H), 7.46 -7.41 (m, 1H), 5.96 (s, 2H), 5.74 (d, J = 4.0 Hz, 2H), 5.27 (s, 1H), 5.19 (s, 1H), 5.00 (s, 1H), 4.92 ( s, 1H), 3.90-3.88 (m, 4H), 3.75-3.72 (m, 4H), 3.64-3.58 (m, 4H), 0.96-0.89 (m, 4H), 0.03 (s, 9H), 0.02 ( s, 9H).

**Synthesis of intermediate MDI-201-4: (2-(6-(2-ethyl-5-fluoro-4-((2-(trimethylsilyl)ethoxy)methyl)hydroxyphenyl) 1-((2-(trimethylsilyl)ethoxy)methyl)-1H-indazol-3-yl)-1-((2-(trimethylsilyl)ethoxy)methyl)-4,6-dihydropyrrolo [3,4-d]imidazol-5(1H)-yl)(5-morpholinepyrazin-2-yl)ketone**

The intermediate MDI-201-3 (43.0 mg, 0.06 mmol), (2-((5-ethyl-2-fluoro-4-(4,4,5,5-tetramethyl-1,3,2-dioxaborolan-2-yl)phenoxy)methoxy)ethyl) trimethylsilane (27.1 mg, 0.07 mmol), Pd(dppf)Cl2 (4.2 mg, 0.006 mmol) and potassium phosphate ( 36.2 mg, 0.17 mmol) were dissolved in 1,4-dioxane (10ml) and water (2ml). The atmosphere was replaced with nitrogen, which was repeated 3 times. The mixture was heated to 100°C, reacted overnight, cooled to room temperature. Water was added and the resulting mixture was extracted with ethyl acetate twice. The organic phases were combined, washed with water and saturated brine, dried over anhydrous sodium sulfate, concentrated, and purified by silica gel column to afford intermediate MDI-201-4 with a yield of 40.9%.

$^1H$ NMR (400 MHz, $CDCl_3$) δ 8.91 (dd, J = 4.0 Hz, J = 4.0 Hz, 1H), 8.52 (dd, J = 8.0 Hz, J = 16.0 Hz, 1H), 8.10 (dd, J = 8.0 Hz, J = 4.0 Hz, 1H), 7.49 (s, 1H), 7.27 (s, 1H), 7.20 (d, J = 8.0 Hz, 1H), 7.06 (d, J = 12.0 Hz, 1H), 6.00 ( s, 2H), 5.79 (d, J = 4.0 Hz, 2H), 5.35 (s, 2H), 5.29 (s, 1H), 5.20 (s, 1H), 5.02(s, 1H), 4.94 (s, 1H) ), 3.91-3.86 (m, 6H), 3.76-3.72 (m, 4H), 3.65-3.61 (m, 4H), 2.58 (t, J = 8.0 Hz, 2H), 1.10-1.03 (m, 3H), 0.95-0.91 (m, 6H), 0.06 (s, 9H), 0.04 (s, 9H), 0.03 (s, 9H).

**Synthesis of MDI-201:(2-(6-(2-ethyl-5-fluoro-4-hydroxyphenyl)-1H-indazol-3-yl)--4,6-dihydropyrrolo[3,4-d]imidazol-5(1H)-yl)(5-morpholinepyrazin-2-yl)ketone**

The intermediate MDI-201-4 (22.0 mg, 0.02 mmol) was dissolved in methanol (4ml), to which concentrated hydrochloric acid (2ml) was added. The mixture was heated to 50°C, reacted for 6 hours, and concentrated. The resulting solid was dissolved with 1ml methanol, to which 2ml concentrated aqueous ammonia was added. The resulting mixture was concentrated to a residue. The residue was dissolved in methanol and concentrated to dryness, which was repeated 3 times. The resulting residue was and purified by a preparation plate to afford 8 mg of the final product, with a yield of 61.9%.

$^1H$ NMR (400 MHz, DMSO-d6) δ 13.35 (s, 1H), 9.89 (s, 1H), 8.66 (d, J = 4.0 Hz, 1H), 8.38-8.33 (m, 2H), 7.42 (s, 1H), 7.15 (d, J = 8.0 Hz, 1H), 7.06 (d, J = 12.0 Hz, 1H), 6.95 (d, J = 8.0 Hz, 1H), 5.05 (s, 2H), 4.72 (s, 2H), 3.76-3.74 (m, 4H), 3.71-3.68 (m, 4H), 2.52 (dd, J = 12.0 Hz, J = 4.0 Hz, 2H), 1.05 (t, J = 8.0 Hz, 3H).

**Example 3: (2-(6-(2-ethyl-5-fluoro-4-hydroxyohenyl)-1H-indazol-3-yl)-4,6-dihydropyrrolo[3,4-d]imidazol-5(1H)-yl)(1-methyl-1H-pyrazol-4-yl)ketone (MDI-202)**

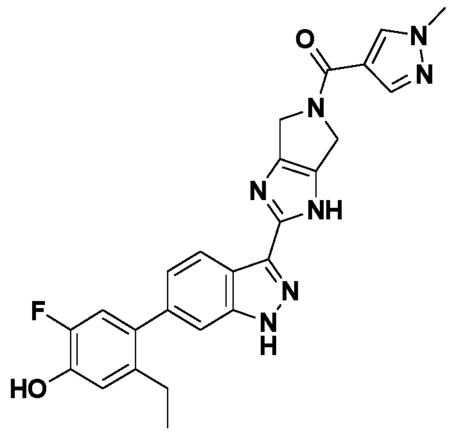

**MDI 202**

It was synthesised according to the method shown in Example 3 of Chinese invention patent CN111606908B, and the NMR hydrogen spectral data of the resulting product were as follows:

$^1$H NMR (400 MHz, DMSO-d6) δ 13.33 (s, 1H), 12.87 (s, 1H), 9.89 (s, 1H), 8.35 (d, J = 8.0 Hz, 2H), 7.94 (s, 1H) , 7.42 (s, 1H), 7.15 (d, J = 8.0 Hz, 1H), 7.06 (d, J = 12.0 Hz, 1H), 6.95 (d, J = 12.0 Hz, 1H), 4.89(s, 2H) , 4.67 (s, 2H), 3.92 (s, 3H), 2.51-2.48 (m, 2H), 1.05 (t, J = 8.0 Hz, 3H).

**<u>Example 4: (2-(6-(2-ethyl-5-fluoro-4-hydroxyphenyl)-1H-indazol-3-yl)pyrrolo[3,4-d]imidazol-5(1H, 4H,6H)-yl)(1-methylpiperidin-4-yl)ketone (MDI-203)</u>**

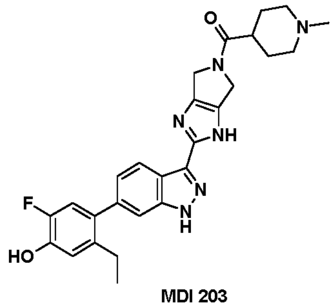

**MDI 203**

MDI-203 may also be named as 5-ethyl-2-fluoro-4-{3-[5-(1-methylpiperidin-4-carbonyl)-1H,4H,5H,6H-pyrrolo [3,4-d]imidazol-2-yl]-1H-indazol-6-yl}phenol. It was synthesised according to the method shown in Example 4 of Chinese invention patent CN111606908B, and the NMR hydrogen spectral data of the resulting product were as follows:

$^1$H NMR (400 MHz, DMSO-d6) δ 13.35 (s, 1H), 9.87 (s, 1H), 9.24 (s, 1H), 8.32 (d, J = 8.0 Hz, 1H), 7.42 (s, 1H) , 7.22 (d, J = 8.0 Hz, 1H), 7.03 (d, J = 12.0 Hz, 1H), 6.96 (d, J = 12.0 Hz, 1H), 4.80 (s, 2H), 4.48 (s, 2H) , 3.04-3.01 (m, 2H), 2.79 (s, 3H), 2.55-2.51 (m, 2H), 2.05-1.99 (m, 3H), 1.85-1.78 (m, 2H), 1.01-0.98 (m, 3H). The signals of the two H were masked by the water peak (δ=3.37).

**<u>Example 5: (2-(6-(2-ethyl-5-fluoro-4-hydroxyphenyl)-1H-indazol-3-yl)pyrrolo[3,4-dlimidazol-5(1H,4H,6H)-yl)(5-(4-methylpiperazin-1-yl)pyrazin-2-yl)ketone (MDI-204)</u>**

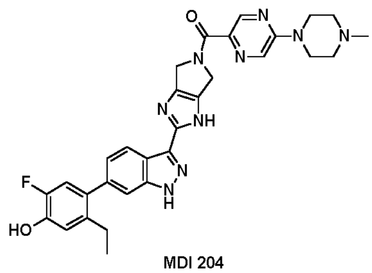

**MDI 204**

MDI-204 may also be named as 5-ethyl-2-fluoro-4-{3-[5-(4-methylpiperazine-1-carbonyl)-1H,4H,5H,6H-pyrrolo[3,4-d]imidazol-2-yl]-1H-indazol-6-yl}phenol. It was synthesised according to the method shown in Example 5 of Chinese invention patent CN111606908B, and the NMR hydrogen spectral data of the resulting product were as follows:

$^1$H NMR (400 MHz, DMSO-d6) δ 13.29 (s, 1H), 12.79 (d, J =16.0 Hz, 1H), 9.85 (s, 1H), 8.62 (s, 1H), 8.36 (s, 1H) ,8.34-8.30 (m, 1H),7.40 (s, 1H), 7.14-7.10 (m, 1H), 7.03 (d, J = 12.0 Hz, 1H), 6.92 (d, J = 12.0 Hz, 1H), 5.08-4.65 (m, 4H), 2.55-2.49 (m, 6H), 2.24 (s, 3H), 2.03-1.97 (m, 4H), 1.04-1.02 (m, 3H).

**Example 6: (2-(6-(2-ethyl-4-hydroxyphenyl)-1H-indazol-3-yl)pyrrolo[3,4-d]imidazol-5(1H,4H,6H)-yl)(5-(4-methylpiperazin-1-yl)pyrazin-2-yl)ketone (MDI-205)**

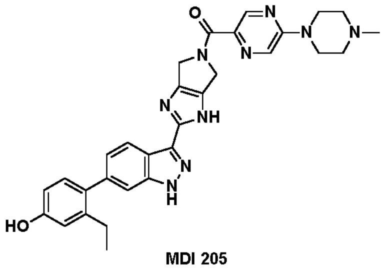

MDI 205

MDI-205 may also be named as 3-ethyl-4-{3-[5-(4-methylpiperazine-1-carbonyl)-1H,4H,5H,6H-pyrrolo[3,4-d]imidazol-2-yl]-1H-indazol-6-yl}phenol. It was synthesised according to the method shown in Example 6 of Chinese invention patent CN111606908B, and the NMR hydrogen spectral data of the resulting product were as follows:

$^1$H NMR (400 MHz, MeOD-d4) δ 8.72 (d, J = 4.0 Hz, 1H), 8.28 (dd, J = 4.0 Hz, J = 8.0 Hz,2H), 7.40 (s, 1H), 7.18 (dd , J = 4.0 Hz, J = 8.0 Hz, 1H), 7.09 (d, J = 8.0 Hz, 1H), 6.80 (d, J = 4.0 Hz, 1H), 6.72-6.69 (m, 1H), 5.17 (s , 2H), 4.85 (s, 2H), 3.83-3.81 (m, 4H), 2.67-2.64 (m, 4H), 2.60 (dd, J = 4.0 Hz, J = 8.0 Hz, 2H), 2.43 (s, 3H), 1.10 (t, J = 8.0 Hz, 3H).

**Example 7: 5-ethyl-2-fluoro-4-(3-(5-(benzenesulfonyl)-1,4,5,6-tetrahydro pyrrolo[3,4-d]imidazol-2-yl)-1H-indazol-6-yl)phenol (MDI-206)**

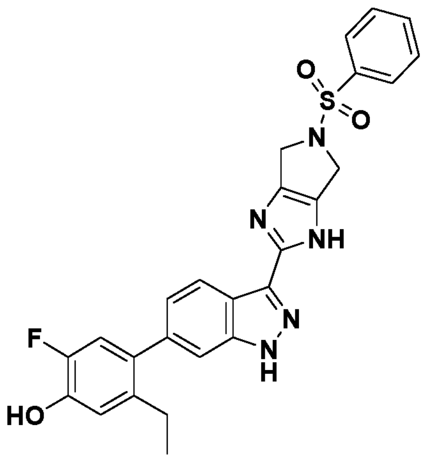

MDI 206

It was synthesised according to the method shown in Example 7 of Chinese invention patent CN111606908B, and the NMR hydrogen spectral data of the resulting product were as follows:

$^1$H NMR (400 MHz, MeOD-d4) δ 8.22 (d, J = 8.0 Hz, 1H), 7.98-7.96 (m, 2H), 7.69-7.65 (m, 3H), 7.41(s, 1H), 7.16 ( d, J = 8.0 Hz, 1H), 6.96-6.89 (m, 2H), 4.61-4.52 (m, 4H), 2.57 (dd, J = 16.0 Hz, J = 8.0 Hz, 2H), 1.08 (t, J = 8.0 Hz, 3H).

**Example 8: 5-ethyl-2-fluoro-4-(3-(5-(pyrazin-2ylmethyl)-1,4,5,6-tetrahydropyrrolo[3,4-dlimidazol-2-yl)-1H-indazol-6-yl)phenol (MDI-207)**

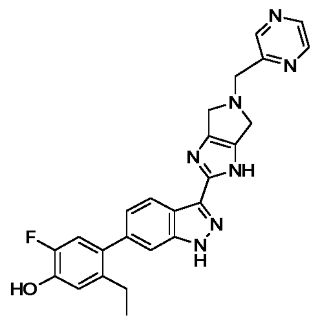

MDI 207

It was synthesised according to the method shown in Example 8 of Chinese invention patent CN111606908B, and the NMR hydrogen spectral data of the resulting product were as follows:

$^1$H NMR (400 MHz, MeOD-d4) δ 8.80 (d, J = 4.0 Hz, 1H), 8.65 (dd, J = 4.0 Hz, J = 4.0 Hz, 1H), 8.56 (d, J = 4.0 Hz, 1H ), 8.26 (dd, J = 4.0 Hz, J = 4.0 Hz, 1H), 7.41 (d, J = 4.0 Hz, 1H), 7.17(dd, J = 12.0 Hz, J = 4.0 Hz, 1H), 6.91- 6.89(m, 2H), 4.30 (s, 2H), 4.07 (s, 4H), 2.56 (dd, J = 8.0 Hz, J = 16.0 Hz, 2H), 1.07 (t, J = 8.0 Hz, 3H).

**Example 9: 4-(3-(5-(cyclopropylmethyl)-1,4,5,6-tetrahydropyrrolo[3,4-d]imidazol-2-yl)-1H-indazol-6-yl)-5-ethyl-2-fluorophenol (MDI-208)**

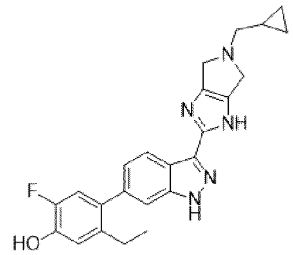

MDI-208

It was synthesised according to the method shown in Example 9 of Chinese invention patent CN111606908B, and the NMR hydrogen spectral data of the resulting product were as follows:

$^1$H NMR (400 MHz, MeOD-d4) δ 8.27 (dd, J = 4.0 Hz, J = 8.0 Hz,1H), 7.42 (s, 1H), 7.17 (dd, J = 4.0 Hz, J = 8.0 Hz, 1H ), 6.97-6.89 (m, 2H), 4.02 (s, 4H), 2.80(d, J = 8.0 Hz, 2H), 2.59-2.53 (m, 2H), 1.10( m, 4H), 0.66-0.61( m, 2H), 0.30-0.27(m, 2H).

**Example 10: cyclopropyl (2-(6-(2-ethyl-5-fluoro-4-hydroxyphenyl)-1H-indazol-3-yl)pyrrolo[3,4-d]imidazol-5(1H, 4H,6H)-yl)ketone (MDI-1233)**

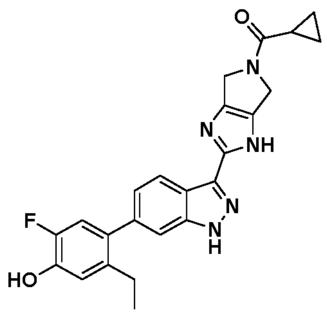

MDI-1233

It was synthesised according to the method shown in Example 10 of Chinese invention patent CN111606908B. In

CN111606908B, the compound was marked as MDI-209 while it was marked as MDI-1233 in the present application. The synthesis was as follows.

**Synthetic route of MDI-1233:**

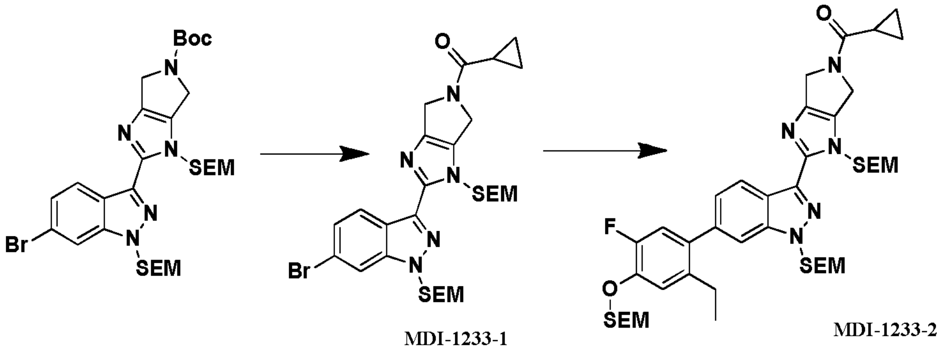

MDI-1233-1 MDI-1233-2

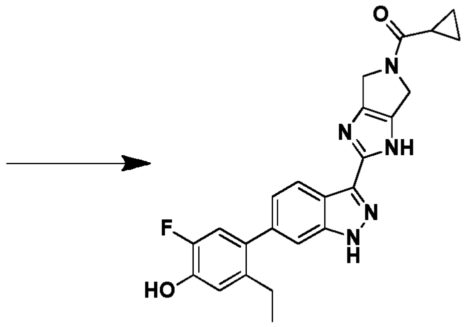

MDI-1233

**Synthesis method:**

**Synthesis of intermediate MDI-1233-1: (2-(6-bromo-1-((2-(trimethylsilyl)ethoxy)methyl)-1H-indazol-3-yl)-1-((2-(trimethylsilyl)ethoxy)methyl) pyrrolo[3,4-d]imidazol-5(1H,4H,6H)-yl)(cyclopropyl)ketone**

The intermediate tert-butyl 2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-carboxylate (80 mg, 0.12 mmol) was dissolved in 5ml of dichloromethane, to which 1ml of trifluoroacetic acid was added. The mixture was stirred at room temperature for 30 minutes, and concentrated to give a residue. The residue was dissolved in dichloromethane and was concentrated to dryness, which was repeated 3 times. The resulting residue was dissolved in 5ml of DCM, to which triethylamine (24.3 mg, 0.24mmol) was added. The temperature was lowered to 0°C, and cyclopropylformyl chloride (18.8 mg, 0.18mmol) was slowly added dropwise. After the dropwise addition was completed, the reaction was warmed up to room temperature and was allowed to react for 1-2h. Water was added to quench the reaction and liquids were separated. The organic phase was dried over sodium sulfate and concentrated by column chromatography to afford compound MDI-1233-1 with a yield of 45%.

$^{1}$H NMR (400 MHz, CDCl3) δ 8.36 (dd, J = 17.8Hz, J =8.6 Hz, 1H), 7.80-7.79 (m, 1H), 7.41 (d, J = 8.6Hz, 1H), 5.97-5.92 (m, 2H), 5.71 (d, J = 2.4 Hz, 2H), 4.96-4.66 (m, 4H), 3.62-3.54 (m, 4H), 1.78-1.67(m, 1H), 1.10 - 1.07 (m , 2H), 0.94 - 0.84 (m, 6H), -0.05 (s, 9H), -0.08 (s, 9H).

**Synthesis of intermediate MDI-1233-2: cyclopropyl(2-(6-(2-ethyl-5-fluoro-4-((2-(trimethylsilyl)ethoxy)methoxy) phenyl)1-((2-(trimethylsilyl)ethoxy) methyl)-1H-indazol-3-yl)-1-((2-(trimethylsilyl)ethoxy)methyl)pyrrolo[3,4-d] imidazol-5(1H,4H,6H)-yl)ketone**

The intermediate MDI-1233-1 (50.5 mg, 0.08mmol), (2-((5-ethyl-2-fluoro-4-(4, 4,5,5-tetramethyl-1,3,2-dioxaboran-2-yl)phenoxy)methoxy)ethyl) trimethylsilane (34.8 mg, 0.1mmol), Pd(dppf)$Cl_2$ (5.9 mg, 0.008mmol) and potassium phosphate (50.9 mg, 0.24mmol) were dissolved in 1,4-dioxane (10ml) and water (2ml). The atmosphere was replaced with

nitrogen, which was repeated 3 times. The mixture was heated to 100°C, reacted overnight, and cooled to room temperature. Water was added and the resulting mixture was extracted twice with ethyl acetate and the organic phases were combined, washed with water and saturated brine, dried over anhydrous sodium sulfate, concentrated, and purify by silica gel column to afford intermediate MDI-1233- 2 with a yield of 76.1%.

$^{1}$H NMR (400 MHz, CDCl3) δ 8.50-8.43 (m,1H), 7.46-7.45 (m,1H), 7.25-7.22 (m,1H), 7.16 (d, J = 8.0Hz, 1H), 7.02 ( d, J = 12.0Hz, 1H), 5.99-5.94 (m, 2H), 5.76(s, 2H), 5.32 (s, 2H), 4.98-4.67(m, 4H), 3.88-3.84 (m, 2H) , 3.64-3.55 (m, 4H), 2.57-2.51 (m, 2H), 1.79-1.68 (m, 1H), 1.07-1.02 (m, 6H), 0.95-0.87 (m, 5H), 0.03 (s, 9H),-0.06--0.08(m, 18H).

**Synthesis of compound MDI-1233: cyclopropyl (2-(6-(2-ethyl-5-fluoro-4-hydroxyphenyl)-1H-indazol-3-yl)pyrrolo[3,4-d]imidazol-5(1H, 4H,6H)-yl)ketone**

The intermediate MDI-1233-2 (50 mg, 0.06mmol) was dissolved in methanol (4ml), to which concentrated hydrochloric acid (2ml) was added. The mixture was heated to 50°C, reacted for 6 hours, and concentrated. The solid was dissolved in 1ml methanol, to which 2ml concentrated aqueous ammonia was added. The mixture was concentrated to give a residue. The residue was dissolved in methanol and was concentrated to dryness, which was repeated 3 times. The resulting residue was purified by a preparation plate to afford 10.0 mg of the final product with a yield of 38.1%.

$^{1}$H NMR (400 MHz, MeOD -d4) δ 8.28(d, J = 8.0 Hz,1H), 7.43(s, 1H), 7.18 (dd, J = 8.4Hz, J = 1.4 Hz, 1H), 6.98 (d, J =12.0 Hz, 1H), 6.92 (d, J =12.0 Hz, 1H), 4.95 (s, 2H), 4.65 (s, 2H), 2.59-2.53 (m, 2H), 1.98-1.89 (m , 1H), 1.08 (t, J = 8.0 Hz, 3H), 1.02-1.00 (m, 2H), 0.98-0.92 (m, 2H).

**Example 11: 4-(3-(5-(cyclobutylmethyl)-1,4,5,6-tetrahydropyrrolo[3,4-d]imidazol-2-yl)-1H-indazol-6-yl)-5-ethyl-2-fluorophenol (MDI-210)**

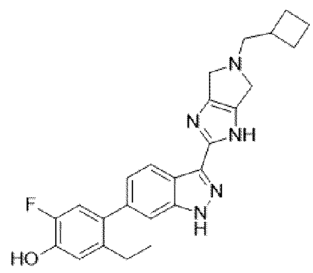

MDI-210

It was synthesised according to the method shown in Example 11 of Chinese invention patent CN111606908B, and the NMR hydrogen spectral data of the resulting product were as follows:

$^{1}$H NMR (400 MHz, MeOD-d4) δ 8.27 (dd, J = 4.0 Hz, J = 8.0 Hz,1H), 7.42 (s, 1H), 7.17 (dd, J = 4.0 Hz, J = 8.0 Hz, 1H ), 6.97-6.89 (m, 2H), 3.98 (s, 4H), 3.00(d, J = 8.0 Hz, 2H), 2.72-2.68 (m, 1H), 2.59-2.53 (m, 2H), 2.21- 2.18 (m, 2H), 1.89-1.85 (m, 4H), 1.07 (t, J = 8.0 Hz, 3H).

**Example 12: cyclobutyl (2-(6-(2-ethyl-5-fluoro-4-hydroxyphenyl)-1H-indazol-3-yl)pyrrolo[3,4-d]imidazol-5(1H, 4H,6H)-yl)ketone (MDI-211)**

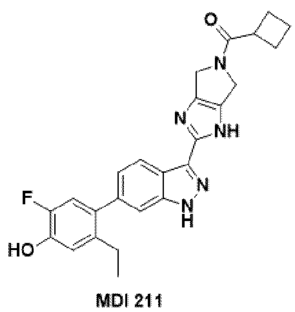

MDI 211

It was synthesised according to the method shown in Example 12 of Chinese invention patent CN111606908B, and the NMR hydrogen spectral data of the resulting product were as follows:

$^1H$ NMR (400 MHz, MeOD-d4) δ 8.27(d, J = 8.0 Hz, 1H), 7.43 (s, 1H),, 7.17 (dd, J = 8.4, 1.4 Hz, 1H), 6.93 (dd, J = 20.0Hz, J =12.0 Hz, 2H), 4.68-4.63 (m, 4H), 3.54-3.46 (m, 1H), 2.57-2.53 (m, 2H), 2.43-2.26 (m, 4H), 2.16- 2.04 (m, 1H), 1.98-1.89 (m, 1H), 1.08 (t, J = 8.0 Hz, 3H).

**Example 13: (2-(6-(2-ethyl-5-fluoro-4-hydroxyphenyl)-1H-indazol-3-yl)pyrrolo[3,4-dlimidazol-5(1H,4H,6H)-yl)(3-hydroxylcyclobutyl)ketone (MDI-213)**

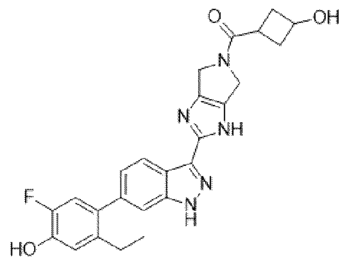

MDI-213

It was synthesised according to the method shown in Example 13 of Chinese invention patent CN111606908B, and the NMR hydrogen spectral data of the resulting product were as follows:

$^1H$ NMR (400 MHz, MeOD-d4) δ 8.28 (dd, J = 4.0 Hz, J = 8.0 Hz, 1H), 7.43-7.42 (m, 1H), 7.19 (dd, J = 4.0 Hz, J = 8.0 Hz , 1H), 6.97-6.89 (m, 2H), 4.72-4.62 (m, 4H), 4.23-4.20 (m, 1H), 2.95-2.91 (m, 1H), 2.63-2.53 (m, 4H), 2.26 -2.18 (m, 2H), 1.10(t, J = 8.0 Hz, 3H).

**Example 14: (2-(6-(2-ethyl-5-fluoro-4-hydroxyphenyl)-1H-indazol-3-yl)-pyrrolo[3,4-dlimidazol-5-(1H,4H,6H)-yl)(pyridazin-4-yl)ketone (MDI-214)**

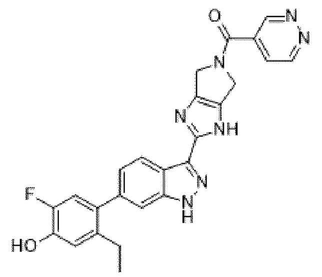

MDI-214

It was synthesised according to the method shown in Example 14 of Chinese invention patent CN111606908B, and the NMR hydrogen spectral data of the resulting product were as follows:

$^1H$ NMR (400 MHz, MeOD-d4) δ 9.48 (dd, J = 2.3, J = 1.3 Hz, 1H), 9.42 (dd, J = 5.2, J = 1.3 Hz, 1H), 8.26 (s, 1H), 8.02 (dd, J = 5.3, J = 2.2 Hz, 1H), 7.43 (d, J = 1.1 Hz, 1H), 7.17 (d, J = 8.2 Hz, 1H), 6.93 (dd, J = 19.7, J = 10.4 Hz, 2H), 4.90 (s, 2H), 4.73 (s, 2H), 2.55 (q, J = 7.5 Hz, 2H), 1.08 (t, J = 7.5 Hz, 3H).

**Example 15: (2-(6-(2-ethyl-5-fluoro-4-hydroxyphenyl)-1H-indazol-3-yl)-pyrrolo[3,4-d]imidazol-5-(1H,4H,6H)-yl)(pyridazin-3-yl)ketone (MDI-215)**

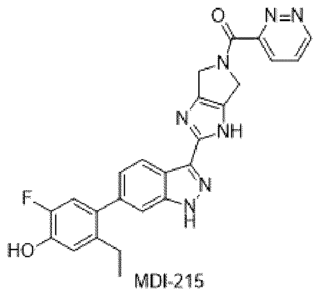

MDI-215

It was synthesised according to the method shown in Example 15 of Chinese invention patent CN111606908B, and the NMR hydrogen spectral data of the resulting product were as follows:

$^1$H NMR (400 MHz,DMSO-d6) δ 13.31 (s, 1H), 12.83 (d, J = 33.0 Hz, 1H), 9.85 (s, 1H), 9.39 (dd, J = 5.0Hz, J =1.7 Hz, 1H), 8.37 - 8.31 (m, 1H), 8.07 (s, 1H), 7.92 (dd, J = 8.5Hz, J = 5.0 Hz, 1H), 7.40 (s, 1H), 7.13 (d, J = 8.1 Hz, 1H), 7.03 (d, J = 11.9 Hz, 1H), 6.92 (d, J = 9.1 Hz, 1H), 4.84 - 4.45 (m, 4H), 2.49 (q, J = 7.5 Hz, 2H) , 1.02 (t, J = 7.5 Hz, 3H).

**Example 16: (S)-(2-(6-(2-ethyl-5-fluoro-4-hydroxyphenyl)-1H-indazol-3-yl)-4,6-dihydropyrrolo[3,4-d]imidazol-5-(1H)-yl)(3-hydroxylpyrrolidin-1-yl)ketone (MDI-1228)**

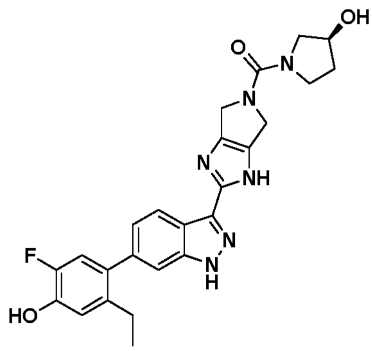

**MDI-1228**

It was synthesised according to the method shown in Example 16 of Chinese invention patent CN111606908B. In CN111606908B, the compound was marked as MDI-216 while it was marked as MDI-1228 in the present application. The synthesis was as follows.

**Synthetic route of MDI-1228:**

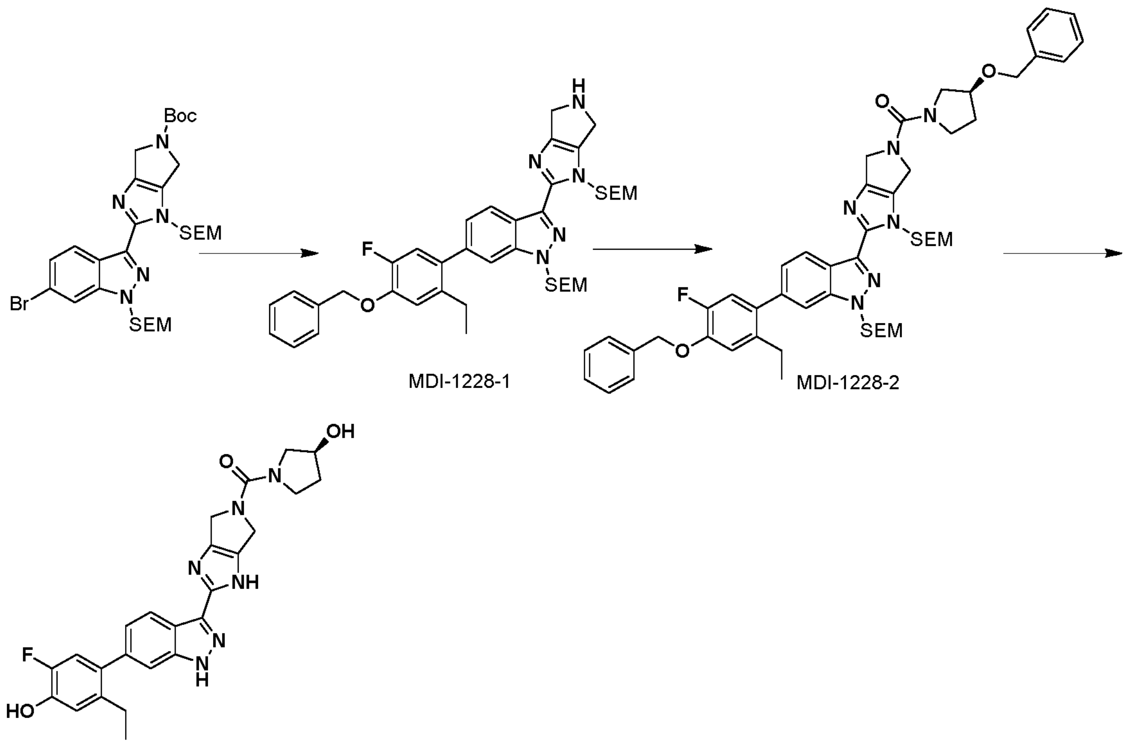

MDI-1228-1

MDI-1228-2

MDI-1228

**Synthesis method:**

**Synthesis of intermediate MDI-1228-1: 6-(4-(benzyloxy)-2-ethyl-5-fluorophenyl)-1-((2-(trimethylsilyl)ethoxy) methyl)-3-(1-((2-(trimethylsilyl)ethoxy) methyl)- 1,4,5,6-tetrahydropyrrolo[3,4-d]imidazol-2-yl)-1H-indazole**

Tert-butyl 2-(6-bromo-1-((2-(trimethylsilyl)ethoxy)methyl)-1H-indol-3-yl)-1-((2-(trimethylsilyl)ethoxy) methyl)-4,6-dihydropyrrolo[3,4-d]imidazol-5(1H)-carboxylate (500 mg, 0.75 mmol), 2-(4-(benzyloxy)-2-ethyl-5-fluorophenyl)-4,4,5,5-tetramethyl-1,3,2-dioxaborane (401 mg, 1.13 mmol), Pd(dppf)Cl2 (75 mg, 0.075 mmol) and potassium phosphate (495 mg, 2.25 mmol) were dissolved in 1,4-dioxane (30 ml) and water (6 ml). The atmosphere was replaced with nitrogen, which was repeated 3 times. The mixture was heated to 100°C, reacted for 16 hours and cooled to room temperature. Water was added and the resulting mixture was extracted twice with ethyl acetate, and the organic phases were combined, washed with water and saturated brine, dried over anhydrous sodium sulfate, concentrated, and purified by silica gel column. The purified product was dissolved in 25 ml of dichloromethane, and 5 ml of trifluoroacetic acid was added dropwise. The mixture was stirred at room temperature for 30 minutes, and concentrated to give a residue. The residue was dissolved in dichloromethane and was concentrated to dryness, which was repeated 3 times. The resulting residue was purified with silica gel column to afford 210 mg of intermediate MDI-1228-1 with a yield of 39.2%.

$^1$H NMR (400 MHz, CDCl3) δ 8.48 (d, J = 8.3 Hz, 1H), 7.52 (d, J = 7.4 Hz, 1H), 7.49 - 7.37 (m, 5H), 7.25 (d, J = 8.4 Hz , 1H), 7.23-6.96(m, 2H), 5.93 (s, 2H), 5.77 (s, 2H), 5.23 (s, 2H), 4.21 (d, J = 35.1 Hz, 4H), 3.66 - 3.52 ( m, 4H), 2.54 (q, J = 7.6 Hz, 2H), 1.05 (t, J = 7.5 Hz, 3H), 0.95 - 0.89 (m, 4H), 0.02 (s, 9H), -0.05 (d, J = 3.4 Hz, 9H).

**Synthesis of intermediate MDI-1228-2: (S)-(2-(6-(4-(benzyloxy)-2-ethyl-5-fluorophenyl)-1-((2-(trimethylsilyl) ethoxy)methyl)-1H-indazol-3-yl)-1-((2-(trimethylsilyl)ethoxy)methyl)-4,6-dihydropyrrolo[3,4-d]imidazol-5-(1H)-yl)(3-(benzyloxy)pyrrolidin-1-yl)ketone**

Triphosgene (25.8 mg, 0.09 mmol) was dissolved in 5 ml of tetrahydrofuran, and intermediate MDI-1228-1 (80 mg, 0.09 mmol) in tetrahydrofuran (5 ml) was added dropwise at 0°C. The mixture was stirred at room temperature for 10 minutes and (S)-3-(benzyloxy) pyrrolidine (31.9 mg, 0.18 mmol) in tetrahydrofuran was added. The mixture was stirred at room temperature for 5 minutes, and water was added. The resulting mixture was extracted twice with ethyl acetate, and the organic phases were combined, washed with water and saturated brine, dried over aqueous sodium sulfate, concentrated, and purified by silica gel column to afford 71 mg of intermediate MDI-1228-2 with a yield of 86.1%.

$^1$H NMR (400 MHz, CDCl3) δ 8.46 (d, J = 8.3 Hz, 1H), 7.53 - 7.51 (m, 2H), 7.47 - 7.42 (m, 3H), 7.39 - 7.29 (m, 6H), 7.24 (dd, J = 8.4 Hz, J = 4.0 Hz,1H), 7.07 - 6.97 (m, 2H), 5.95 (s, 2H), 5.77 (s, 2H), 5.23 (s, 2H), 4.92 - 4.88 (m, 2H), 4.76 -

4.69 (m, 2H), 4.60 (s, 2H), 4.23 (s, 1H), 3.76 - 3.70 (m, 2H), 3.66 - 3.58 (m, 6H), 2.54 (q, J = 7.5 Hz, 2H), 2.15 - 2.13 (m, 1H), 2.06 - 2.02 (m, 1H), 1.05 (t, J = 7.5 Hz, 3H), 0.95 - 0.91 (m, 4H) ,-0.01 --- 0.11(m, 18H).

**Synthesis of compound MDI-1228: (S)-(2-(6-(2-ethyl-5-fluoro-4-hydroxyphenyl)-1H-indazol-3-yl)-4,6-dihydropyrrolo[3,4-d]imidazol-5-(1H)-yl)(3-hydroxylpyrrolidin-1-yl)ketone**

Intermediate MDI-1228-2 (83 mg, 0.11 mmol) was dissolved in methanol (10ml), to which 10 mg Pd/C and concentrated hydrochloric acid (5ml) were added. The mixture was heated to 50 °C, reacted for 6 hours, filtered and concentrated to give a residue. The residue was dissolved in methanol and was concentrated to dryness, which was repeated 3 times. The resulting residue was dissolve in methanol, 1ml of aqueous ammonia was added, and then the mixture was concentrated, and purified by a preparation plate to afford 8 mg of the final product with a yield of 15.2%.

$^1$H NMR (400 MHz, MeOD-d4) δ 8.27 (d, *J* = 8.4 Hz, 1H), 7.43 (d, *J* = 1.0 Hz, 1H), 7.17 (d, *J* = 8.4 Hz, 1H), 6.97 - 6.90 (m, 2H), 4.81 - 4.61 (m, 4H), 4.46 - 4.44 (m, 1H), 3.79 - 3.69 (m, 2H), 3.50 - 3.43 (m, 2H), 2.56 (q, *J* = 7.5 Hz, 2H), 2.09 - 1.99 (m, 2H), 1.08 (t, *J* = 7.5 Hz, 3H).

**Example 17: 5-ethyl-2-fluoro-4-(3-(5-(4-hydroxylcyclohexyl)-1,4,5,6-tetrahydropyrrolo[3,4-d]imidazol-2-yl)-1H-indazol-6-yl)phenol (MDI-217)**

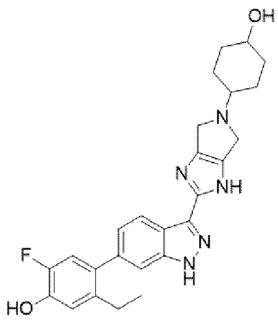

MDI-217

It was synthesised according to the method shown in Example 17 of Chinese invention patent CN111606908B, and the NMR hydrogen spectral data of the resulting product were as follows:

$^1$H NMR (400 MHz, MeOD-d4) δ 8.27 (dd, J = 4.0 Hz, J = 8.0 Hz,1H), 7.43-7.42 (m, 1H), 7.19 (dd, J = 4.0 Hz, J = 8.0 Hz, 1H), 6.96-6.88 (m, 2H), 3.98 (s, 4H), 3.93 (m, 1H), 2.74-2.72 (m, 1H), 2.58(q, J = 8.0 Hz, 2H), 2.04- 2.05 (m, 1H), 1.90-1.80 (m, 5H), 1.64-1.62 (m, 2H), 1.10 (t, J = 8.0 Hz, J = 16.0 Hz, 3H).

**Example 18: 4-(3-(5-(cyclopropanesulfonyl)-1,4,5,6-tetrahydropyrrolo [3,4-dlimidazol-2-yl)-1H-indazol-6-yl)-5-ethyl-2-fluorophenol (MDI-218)**

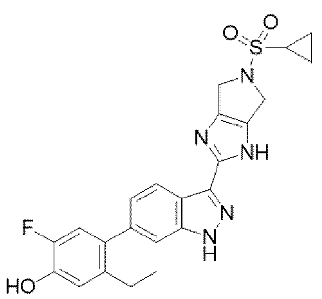

MDI-218

It was synthesised according to the method shown in Example 18 of Chinese invention patent CN111606908B. The synthesis was as follows.

**Synthetic route of MDI-218:**

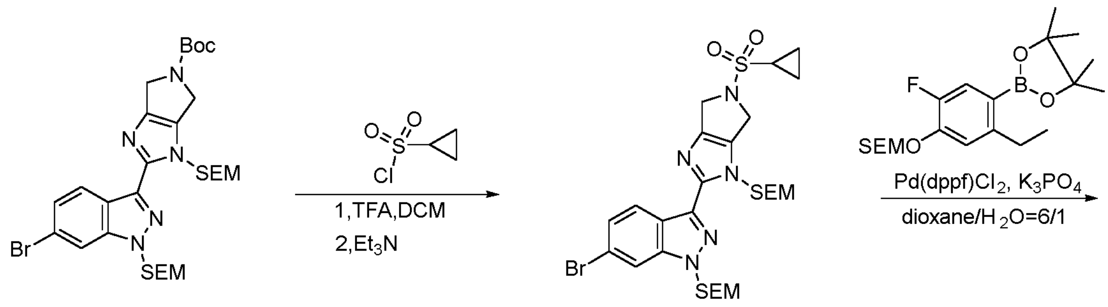


MDI-218-1

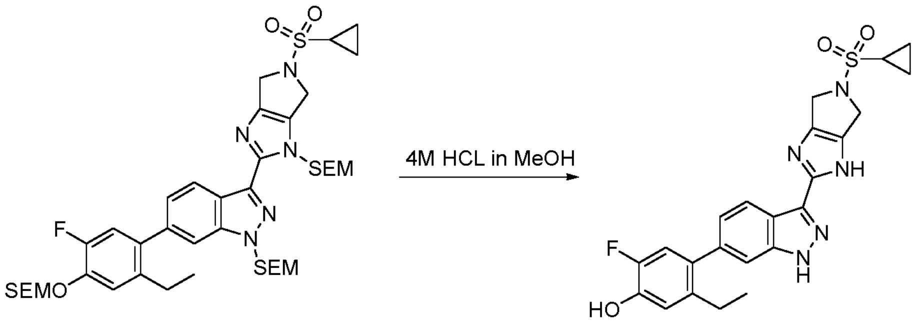


MDI-218-2

MDI-218

**Synthesis method:**

**Synthesis of intermediate MDI-218-1: 6-bromo-3-(5-(cyclopropanesulfonyl)-1-((2-(trimethylsilyl)ethoxy) methyl)-1,4,5,6-tetrahydropyrrolo[3,4-d]imidazol-2-yl)-1-((2-(trimethylsilyl)ethoxy)methyl)-1H-indazol**

Tert-butyl 2-(6-bromol-((2-(trimethylsilyl)ethoxy)methyl)-1H-indazol-3-yl)-1-((2-(trimethylsilyl)ethoxy) methyl)-4,6-dihydropyrrolo[3,4-d]imidazol-5(1H)-carboxylate (100 mg, 0.15 mmol) was dissolved in 5ml dichloromethane, and 1ml trifluoroacetic acid was added. The mixture was stirred at room temperature for 30 minutes, concentrated, quenched with sodium bicarbonate, and extracted twice with dichloromethane. The organic phases were combined, washed with water and saturated brine, dried over anhydrous sodium sulfate and concentrated. The obtained compound was dissolved in 5ml DCM and Et3N (0.08ml, 0.59mmol), and cooled to 0°C. Cyclopropylsulfonyl chloride (22.4 mg, 0.16mmol) was slowly added. It was allowed to react at room temperature for 2 hours, and water was added to quench the reaction. The resulting mixture was extracted with DCM twice, and the organic phases were combined, washed with water and saturated brine, dried over anhydrous sodium sulfate, concentrated, and purified by silica gel column to afford intermediate MDI-218-1 with a yield of 36.0%.

$^1$H NMR (400 MHz, CDCl3) δ 8.37 (d, J = 8.0 Hz, 1H), 7.80 (d, J = 4.0 Hz, 1H), 7.43 (d, J = 8.0 Hz, 1H), 5.91 (s, 2H ), 5.73 (s, 2H), 4.75-4.74 (m, 2H), 4.66-4.65 (m, 2H), 3.63-3.58 (m, 4H), 2.50-2.44 (m, 1H), 1.33-1.31 (m, 2H), 1.06-1.02 (m, 2H), 0.96-0.91 (m, 4H), 0.00--0.05 (m, 18H).

**Synthesis of intermediate MDI-218-2: 3-(5-cyclopropanesulfonyl)-1-((2-(trimethylsilyl)ethoxy)methyl)-1,4,5,6-tetrahydropyrrolo[3,4-d]imidazol-2-yl)-6-(2-ethyl-5-fluoro-4-((2-(trimethylsilyl)ethoxy)methoxy)phenyl)-1-((2-(trimethylsilyl) ethoxy)methyl)-1H-indazole**

The intermediate MDI-218-1 (36.0 mg, 0.05 mmol), (2-((5-ethyl-2-fluoro-4-(4, 4,5,5-tetramethyl-1,3,2-dioxaboran-2-yl)phenoxy)methoxy)ethyl)trimethylsilane (25.5 mg, 0.06 mmol), Pd(dppf)Cl2 (3.9 mg, 0.005 mmol) and potassium phosphate (34.2 mg, 0.16 mmol) were dissolved in 1,4-dioxane (6 ml) and water (1 ml). The atmosphere was replaced with nitrogen, which was repeated 3 times. The mixture was heat to 100°C, reacted overnight, and cooled to room temperature. Water was added, and the resulting mixture was extracted twice with ethyl acetate, and the organic phases were combined, washed with water and saturated brine, dried over anhydrous sodium sulfate, concentrated, and purified by

silica gel column to afford the intermediate MDI- 218-2, the yield was 70.0%.

$^1$H NMR (400 MHz, CDCl3) δ 8.47 (d, J = 8.0 Hz, 1H), 7.48 (s, 1H), 7.26 (d, J = 7.9 Hz, 1H), 7.18 (d, J = 8.0 Hz, 1H ), 7.04 (d, J = 12.0 Hz, 1H), 5.95 (s, 2H), 5.78 (s, 2H), 5.34 (s, 2H), 4.76 (s, 2H), 4.68 (s, 2H), 3.88 (t, J = 8.0 Hz, 2H), 3.68- 3.57 (m, 4H), 2.56 (q, J = 7.6 Hz, 2H), 2.24 (t, J = 7.7 Hz, 1H), 1.12- 0.86 (m, 13H),-0.01--0.06 (m, 27H).

**Synthesis of compound MDI-218: 4-(3-(5-(cyclopropanesulfonyl)-1,4,5,6-tetrahydropyrrolo[3,4-d]imidazol-2-yl)-1H-indazol-6-yl)-5-ethyl-2-fluorophenol**

Intermediate MDI-218-2 (36.0 mg, 0.04 mmol) was dissolved in methanol (4ml), to which concentrated hydrochloric acid (2ml) was added. The mixture was heated to 50°C, reacted for 6 hours, and concentrated. The solid was dissolved in 1ml methanol, and pH was adjusted with sodium bicarbonate to 8-9, and the resulting mixture was extracted 4 times with dichloromethane. The organic phases were combined, dried over anhydrous sodium sulfate, and purified by a preparation plate to afford 16 mg of the final product with a yield of 81.4%.

$^1$H NMR (400 MHz, MeOD-d4) δ 8.27 (d, J = 8.0 Hz, 1H), 7.43 (s, 1H), 7.18 (dd, J = 8.0Hz, J = 4.0 Hz, 1H), 6.93 (dd , J = 20.0Hz, J = 12.0 Hz, 2H), 4.65 (s, 4H), 2.76-2.69(m, 1H), 2.60- 2.51 (m, 2H), 1.20- 1.18 (m, 2H), 1.10- 1.06 (m, 5H).

**Example 19: 4-(3-(5-(cyclobutylsulfonyl)-1,4,5,6-tetrahydropyrrolo[3,4-dlimidazol-2-yl)-1H-indazol-6-yl)-5-ethyl-2-fluorophenol (MDI-219)**

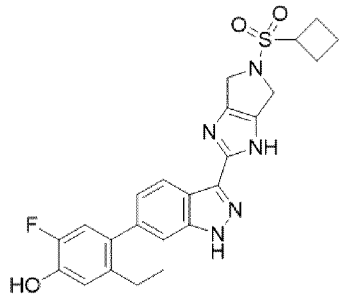

MDI-219

It was synthesised according to the method shown in Example 19 of Chinese invention patent CN111606908B, and the NMR hydrogen spectral data of the resulting product were as follows:

$^1$H NMR (400 MHz, MeOD-d4) δ 8.26 (d, J = 8.0 Hz, 1H), 7.43 (s, 1H), 7.17 (dd, J = 8.4Hz, J =1.4 Hz, 1H), 6.93 (dd, J = 20.0Hz, J =12.0 Hz, 2H), 4.60 (s, 4H), 4.26-4.18 (m, 1H), 2.68- 2.52 (m, 4H), 2.40- 2.31 (m, 2H), 2.13- 2.02 (m, 2H), 1.08 (t, J = 7.5 Hz, 3H).

**Example 20: 4-(3-(5-(cyclopentylsulfonyl)-1,4,5,6-tetrahydropyrrolo[3,4-d]imidazol-2-yl)-1H-indazol-6-yl)-5-ethyl-2-fluorophenol (MDI-220)**

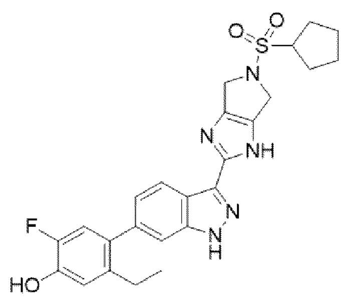

MDI-220

It was synthesised according to the method shown in Example 20 of Chinese invention patent CN111606908B, and the NMR hydrogen spectral data of the resulting product were as follows:

$^1$H NMR (400 MHz, MeOD-d4) δ 8.27 (dd, J = 8.0 Hz, J = 4.0 Hz,1H), 7.43 (s, 1H), 7.17 (dd, J = 8.0Hz, J = 1.4 Hz, 1H ), 6.93 (dd, J = 20.0Hz, J =12.0 Hz, 2H), 4.65 (s, 4H), 3.91-3.83 (m, 1H), 2.58- 2.52 (m, 2H), 2.13 - 2.03 (m, 4H), 1.89-1.78 (m, 2H), 1.75-1.64 (m, 2H), 1.08 (t, J = 8.0 Hz, 3H).

**Example 21: 5-ethyl-2-fluoro-4-(3-(5-((1-methyl-1H-pyrazol-4-yl)methyl) -1,4,5,6-tetrahydropyrrolo[3,4-d]imidazol-2-yl)-1H-indazol-6-yl)phenol (MDI-221)**

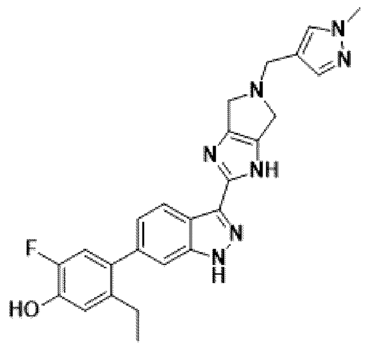

MDI-221

It was synthesised according to the method shown in Example 21 of Chinese invention patent CN111606908B, and the NMR hydrogen spectral data of the resulting product were as follows:

$^1$H NMR (400 MHz, MeOD-d4) δ 8.25 (d, J = 8.0 Hz, 1H), 7.68 (s, 1H), 7.56 (s, 1H), 7.42 (s, 1H), 7.16 (dd, J= 8.4Hz, J=1.4 Hz, 1H), 6.93 (dd, J = 20.0Hz, J=12.0 Hz, 2H), 4.03-3.96 (m, 6H), 3.92 (s, 3H), 2.58-2.53 (m, 2H), 1.08 (t, J = 8.0 Hz, 3H).

**Example 22: 4-(3-(5-cyclopentyl-1,4,5,6-tetrahydropyrrolo[3,4-dlimidazol-2-yl)-1H-indazol-6-yl)-5-ethyl-2-fluorophenol (MDI-224)**

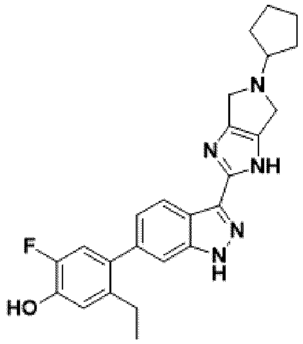

MDI-224

It was synthesised according to the method shown in Example 22 of Chinese invention patent CN111606908B, and the NMR hydrogen spectral data of the resulting product were as follows:

$^1$H NMR (400 MHz, MeOD-d4) δ 8.26(d, *J* = 8.0 Hz, 1H), 7.42(s, 1H),7.17 (d, *J* = 8.0 Hz, 1H),6.93 (dd, *J* = 20.0Hz, *J* =12.0 Hz, 2H),4.05-3.94 (m, 4H), 3.27-3.25 (m, 1H), 2.59-2.54 (m, 2H), 2.08-2.01(m, 2H), 1.87-1.79(m, 2H), 1.73-1.56(m,4H), 1.08 (t, *J* = 8.0 Hz, 3H).

**Example 23: 5-ethyl-2-fluoro-4-(3-(5-(tetrahydro-2H-pyran-4-yl)-1,4,5,6-tetrahydropyrrolo[3,4-d]imidazol-2-yl)-1H-indazol-6-yl)phenol (MDI-225)**

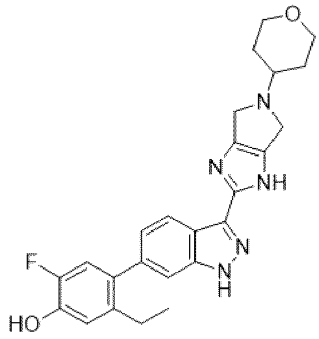

MDI-225

It was synthesised according to the method shown in Example 23 of Chinese invention patent CN111606908B, and the NMR hydrogen spectral data of the resulting product were as follows:

$^1$H NMR (400 MHz, MeOD-d4) δ 8.26 (dd, J = 12.0 Hz, J = 4.0 Hz,1H), 7.42 (s, 1H), 7.16 (dd, J = 8.0Hz, J = 4.0 Hz, 1H ), 6.93 (dd, J = 20.0Hz, J =12.0 Hz, 2H), 4.07-3.99 (m, 6H), 3.52- 3.49 (m, 2H), 2.95- 2.90 (m, 1H), 2.58- 2.53 (m, 2H), 2.00- 1.97 (m, 2H), 1.69- 1.59 (m, 2H), 1.08 (t, J = 8.0 Hz, 3H).

**Example 24: 1-(2-(6-(2-ethyl-5-fluoro-4-hydroxyphenyl)-1H-indazol-3-yl)-4,6-dihydropyrrolo[3,4-d]imidazol-5-(1H) - yl)ethan-1-one (MDI-226)**

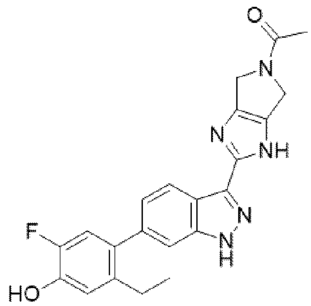

MDI-226

It was synthesised according to the method shown in Example 24 of Chinese invention patent CN111606908B, and the NMR hydrogen spectral data of the resulting product were as follows:

$^1$H NMR (400 MHz, methanol-d4) δ 8.28 (d, J = 8 Hz, 1H), 7.43 (s, 1H), 7.18 (d, J = 8 Hz, 1H), 6.94 (dd, J = 22, 10 Hz, 2H), 4.79 (s, 2H), 4.65 (s, 2H), 2.59-2.53 (m, 2H), 2.23 (s, 3H), 1.08 (t, J = 7.5 Hz, 3H).

**Example 25: 1-(2-(6-(2-ethyl-5-fluoro-4-hydroxyphenyl)-1H-indazol-3-yl)-4,6-dihydropyrrolo[3,4-d]imidazol-5-(1H)-yl)propan-1-one (MDI-227)**

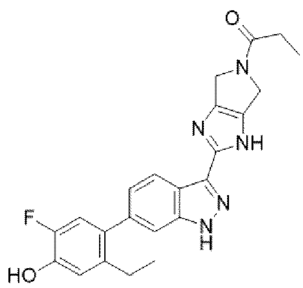

MDI-227

It was synthesised according to the method shown in Example 25 of Chinese invention patent CN111606908B, and the NMR hydrogen spectral data of the resulting product were as follows:

$^1$H NMR (400 MHz, DMSO-d6) δ 13.29 (s, 1H), 12.80 (s, 1H), 9.85 (s, 1H), 8.33 (d, J = 8 Hz, 1H), 7.40 (s, 1H) , 7.12 (d, J = 8 Hz, 1H), 7.03 (d, J = 12 Hz, 1H), 6.92 (d, J = 12 Hz, 1H), 4.73-4.58 (m, 2H), 4.50-4.42 (m, 2H), 2.50-2.47 (m, 2H), 2.43-2.37 (m, 2H), 1.08-1.01 (m, 6H).

**Example 26: 1-(2-(6-(2-ethyl-5-fluoro-4-hydroxyphenyl)-1H-indazol-3-yl)-4,6-dihydropyrrolo[3,4-d]imidazol-5-(1H)-yl)-2-methylpropan-1-one (MDI-228)**

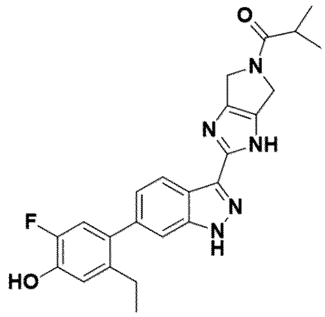

MDI 228

It was synthesised according to the method shown in Example 26 of Chinese invention patent CN111606908B, and the NMR hydrogen spectral data of the resulting product were as follows:

$^1$H NMR (400 MHz, MeOD-d4) δ 8.26 (d, J = 8.0 Hz, 1H), 7.43 (s, 1H), 7.17 (d, J = 8Hz, 1H), 6.93 (dd, J = 20, 12 Hz, 2H), 4.83-4.59 (m, 4H), 2.94-2.90 (m, 1H), 2.58-2.52 (m, 2H), 1.22 (d, J = 8.0 Hz, 6H ), 1.08 (t, J = 8.0 Hz, 3H ).

**Example 27: 2-cyclopropyl-1-(2-(6-(2-ethyl-5-fluoro-4-hydroxyphenyl)-1H-indazol-3-yl)-4,6-dihydropyrrolo[3,4-d]imidazol-5-(1H)-yl)ethan-1-one** (MDI-229)

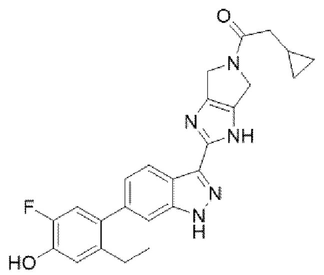

MDI-229

It was synthesised according to the method shown in Example 27 of Chinese invention patent CN111606908B, and the NMR hydrogen spectral data of the resulting product were as follows:

$^1$H NMR (400 MHz, MeOD-d4) δ 8.29 (d, *J* = 8.0 Hz, 1H), 7.43 (s, 1H), 7.19-7.17 (m, 1H), 6.98-6.90 (m, 2H), 4.73-4.61 (m, 4H), 2.59-2.53 (m, 2H), 2.46 (d, *J* = 8.0 Hz, 2H), 1.17 (m, 1H), 1.08 (t, *J* = 8.0 Hz, 3H), 0.64-0.59 (m, 2H), 0.30-0.26(m, 2H).

**Example 28: 1-(2-(6-(2-ethyl-5-fluoro-4-hydroxyphenyl)-1H-indazol-3-yl)-4,6-dihydropyrrolo[3,4-d]imidazol-5-(1H)-yl)-3-methylbutan-1-one (MDI-230)**

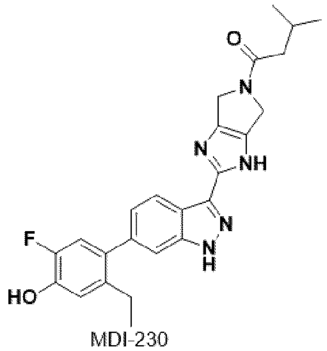

MDI-230

It was synthesised according to the method shown in Example 14 of Chinese invention patent CN111606908B, and the NMR hydrogen spectral data of the resulting product were as follows:

$^{1}$H NMR (400 MHz, MeOD-d4) δ 8.29 - 8.26 (m, 1H), 7.43 (s, 1H), 7.19 - 7.16 (m, 1H), 6.97 - 6.89 (m,2H), 4.75 - 4.70 (m , 4H), 2.56 (q, J = 7.5 Hz, 2H), 2.36 (m, 2H), 2.29 - 2.20 (m, 1H), 1.10 - 1.05 (m, 9H).

**Example 29: (2-(6-(2-ethyl-5-fluoro-4-hydroxyphenyl)-1H-indazol-3-yl)-4,6-dihydropyrrolo[3,4-d]imidazol-5-(1H) - yl)(pyrrolidin-1-yl)ketone (MDI-231)**

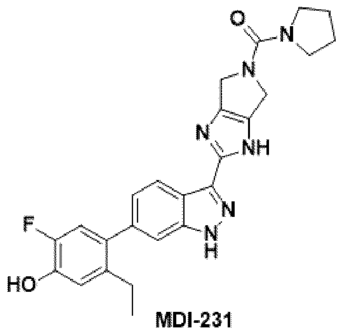

**MDI-231**

It was synthesised according to the method shown in Example 29 of Chinese invention patent CN111606908B. The synthesis was as follows.

**Synthetic route of MDI-231:**

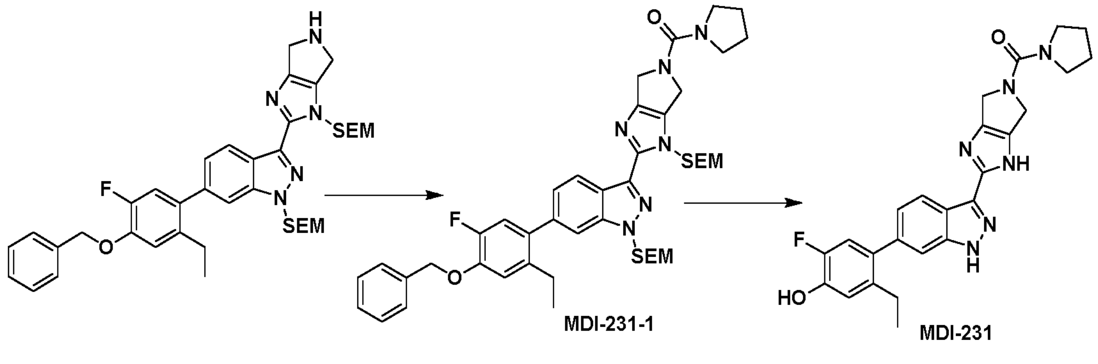

**Synthesis method:**

**Synthesis of intermediateMDI-231-1: (2-(6-(4-(benzyloxy)-2-ethyl-5-fluorophenyl)-1-((2-(trimethylsilyl)ethoxy) methyl)-1H-indazol-3-yl)-1-((2-(trimethylsilyl)ethoxy)methyl)-4,6-dihydropyrrolo[3,4-d]imidazol-5-(1H)-y1) (pyrrolidin-1-yl)ketone**

Triphosgene (64.4 mg, 0.21 mmol) was dissolved in 15 ml of dichloromethane, to which the intermediate 6-(4-(benzyloxy)-2-ethyl-5-fluorophenyl)-1- ((2-(trimethylsilyl) ethoxy)methyl)-3-(1-((2-(Trimethylsilyl)ethoxy) methyl)-1-1,4,5,6-tetrahydropyrrolo[3,4-d]imidazol-2-yl)-1H-indazole (150 mg, 0.21 mmol) in dichloromethane (5 ml) was added dropwise at 0°C, followed by addition of triethylamine (63.6 mg, 0.63 mmol). The mixture was stirred at room temperature for 5 minutes and pyrrolidine (29.8 mg, 0.42 mmol) in dichloromethane was added. The resulting mixture was stirred at room temperature for 10 minutes, and water was added. The resulting mixture was extracted twice with ethyl acetate, and the organic phases were combined, washed with water and saturated brine, dried over anhydrous sodium sulfate, concentrated, and purified by silica gel column to afford 140 mg of intermediate MDI-231-1 with a yield of 82.4%.

$^1$H NMR (400 MHz, CDCl3) δ 8.48 (d, J = 8 Hz, 1H), 7.53-7.38 (m, 6H), 7.22 (d, J = 8 Hz, 1H), 7.03 (d, J = 12 Hz, 1H), 6.95 (d, J = 8 Hz, 1H), 5.96 (s, 2H), 5.77 (s, 2H), 5.23 (s, 2H), 4.81 (s, 2H), 4.67 (s, 2H), 3.66-3.59 (m, 4H), 3.53-3.51 (m, 4H), 2.56-2.52 (m, 2H), 1.93-1.88 (m, 4H), 1.03 (t, J = 8 Hz, 3H), 0.93 -0.87 (m, 4H), -0.05--0.09 (m, 18H).

**Synthesis of compound MDI-231: (2-(6-(2-ethyl-5-fluoro-4-hydroxyphenyl)-1H-indazol-3-yl)-4,6-dihydropyrrolo [3,4-d]imidazol-5-(1H) - yl)(pyrrolidin-1-yl)ketone**

Intermediate MDI-231-1 (140 mg, 0.173 mmol) was dissolved in methanol (6 ml), to which 15 mg Pd/C was added and concentrated hydrochloric acid (3 ml) was added dropwise. The mixture was heated to 50 °C, reacted for 6 hours, filtered, and concentrated to give a residue. The residue was dissolved in methanol and was concentrated to dryness, which was repeated 3 times. The resulting residue was dissolved in methanol, and 1 ml of ammonia was added. The resulting mixture was concentrated, and purified by a preparation plate to afford 21 mg of the final product with a yield of 26.3%.

$^1$H NMR (400 MHz, DMSO-d6) δ 13.25 (s, 1H), 12.69 (s, 1H), 9.83 (s, 1H), 8.31 (d, J = 8 Hz, 1H), 7.39 (s, 1H) , 7.11 (d, J = 8 Hz, 1H), 7.02 (d, J = 12 Hz, 1H), 6.91 (d, J = 12 Hz, 1H), 4.57-4.56 (m, 2H), 4.49-4.48 (m, 2H), 3.32-3.31 (m, 4H), 2.48-2.44 (m, 2H), 1.85-1.79 (m, 4H), 1.02 (t, J = 7 Hz, 3H).

**Example 30: N-(3-chloro-2-hydroxypropyl)-(2-(6-(2-ethyl-5-fluoro-4-hydroxyphenyl)-1H-indazol-3-yl)-4,6-dihidropyrrolo[3,4-d]imidazole-5-(1H)-carboxamide (MDI-1288)**

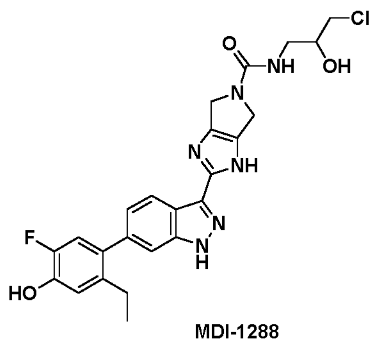

MDI-1288

**Synthetic route of MDI-1288:**

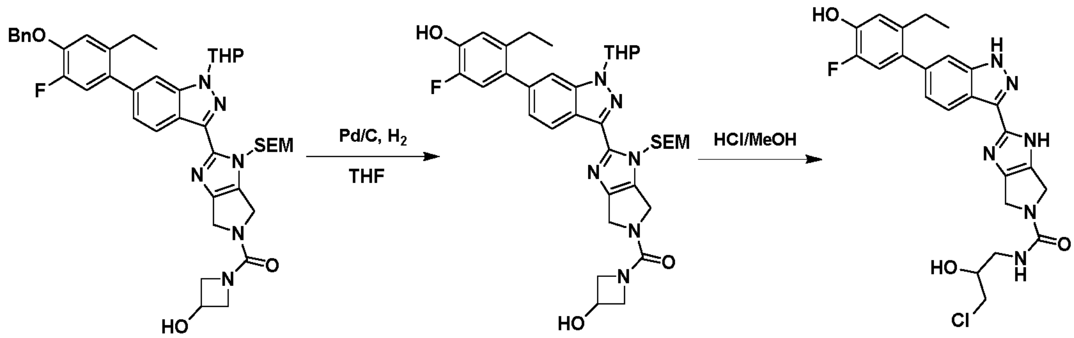


MDI-1288-1 MDI-1288-2 MDI-1288

**Synthesis method:**

**Synthesis of compound MDI-1288-2: (2-(6-(2-ethyl-5-fluoro-4-hydroxyphenyl)-1-(tetrahydro-2H-pyran-2-yl)-1H-indazol-3-yl)-1-((2-trimethylsilyl)ethoxy)methyl)-4,6-dihydropyrrolo[3,4-d]imidazol-5(1H)-yl)(3-hydroxyazetidin-1-yl)ketone**

MDI-1288-1 (51.00 g, 66.49 mmol), which was prepared according to the method for synthesising MDI-231-1 using suitable starting materials, was dissolved in 500 ml of tetrahydrofuran, to which 7 g of palladium carbon was added. The atmosphere was replaced with hydrogen. It was allowed to react at 40°C for 16 hours. After the reaction was completed, the reaction mixture was filtrated and the filtrate was concentrated to give a total of 44.10 g of MDI-1288-I07 in a yield of 97.8%.

**Synthesis of compound MDI-1288: (2-(6-(2-ethyl-5-fluoro-4-hydroxyphenyl)-1H-indazol-3-yl)-4,6-dihydropyrrolo[3,4-d]imidazol-5(1H)-yl) (3-hydroxyazetidin-1-yl)ketone**

MDI-1288-I07 (44.10 g, 65.01 mmol) was dissolved in 240 ml methanol, to which 120 ml concentrated hydrochloric acid was added. It was allowed to react at 50 degrees Celsius overnight. After the reaction was completed, the reaction solution was filtered, and the filter cake was dried in a drying oven at 50° C, to afford 29.8 g. 27.2 g of crude hydrochloride salt was obtained by pulping it with 150 ml of methanol for 0.5 h, and then purified by recrystallisation.

$^1$H NMR (400 MHz, DMSO-$d_6$) δ 14.25 (s, 1H), 9.90 (s, 1H), 8.43 (d, J = 8.4 Hz, 1H), 7.56 (s, 1H), 7.27 (dd, J = 8.5, 1.4 Hz, 1H), 7.04 (d, J = 11.8 Hz, 1H), 6.97 (d, J = 9.1 Hz, 1H), 6.71 (s, 1H), 4.59 (s, 4H), 3.83 - 3.78 (m, 1H), 3.68 (dd, J = 11.2, 4.2 Hz, 1H), 3.54 (dd, J = 11.2, 6.0 Hz, 1H), 3.28 - 3.15 (m, 2H), 2.48 (q, J = 7.5 Hz, 2H), 1.02 (t, J = 7.5 Hz, 3H).

**Example 31: (2-(6-(2-ethyl-5-fluoro-4-hydroxyphenyl)-1H-indazol-3-yl)-4,6-dihydropyrrolo[3,4-d]imidazol-5-(1H)-yl)(piperidin-1-yl)ketone (MDI-233)**

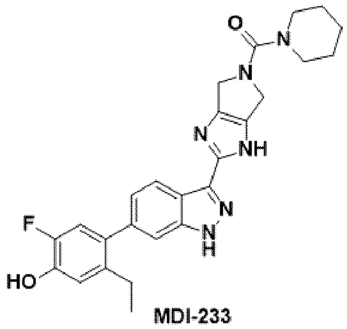

MDI-233

It was synthesised according to the method shown in Example 31 of Chinese invention patent CN111606908B. The synthesis was as follows.

**Synthetic route of MDI-233:**

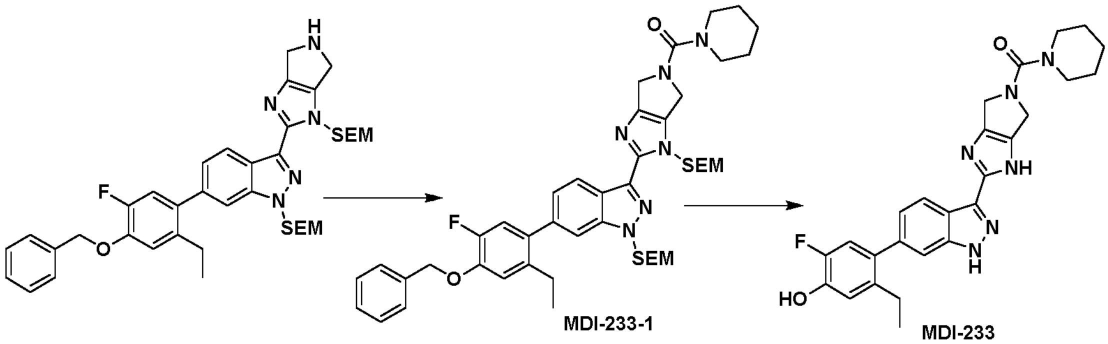

MDI-233-1

MDI-233

**Synthesis method:**

**Synthesis of intermediate MDI-233-1: (2-(6-(4-(benzyloxy)-2-ethyl-5-fluorophenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)(piperidin-1-yl)ketone**

Triphosgene (54.1 mg, 0.182 mmol) was dissolved in 5 ml of tetrahydrofuran, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (130 mg, 0.182 mmol) in tetrahydrofuran (5 ml) was added dropwise at 0°C, followed by addition of triethylamine (55.2 mg, 0.550 mmol). The mixture was stirred at room temperature for 5 minutes, and piperidine hydrochloride (44.4 mg, 0.364 mmol) in tetrahydrofuran was added. The resulting mixture was stirred at room temperature for 10 minutes. Water was added and the resulting mixture was extracted twice with ethyl acetate, and the organic phases were combined, washed with water and saturated brine, dried over anhydrous sodium sulfate, concentrated, and purified by silica gel column to afford 105 mg of intermediate MDI-233-1, with a yield of 66.6%.

$^1$H NMR (400 MHz, CDCl3) δ 8.44 (d, *J* = 8.3 Hz, 1H), 7.50 - 7.48 (m, 2H), 7.44 - 7.35 (m, 4H), 7.23 - 7.20 (m, 1H), 7.04 - 6.94 (m, 2H), 5.93 (s, 2H), 5.74 (s, 2H), 5.20 (s, 2H), 4.69 (d, *J* = 54.8 Hz, 4H), 3.64 - 3.56 (m, 4H), 3.31 (s, 4H) ,2.54 (q, *J* = 7.5 Hz, 2H), 1.64 (s, 6H), 1.03 (t, *J* = 7.5 Hz, 3H), 0.93 - 0.86 (m, 4H) , -0.07(d, *J* = 2.7 Hz, 18H).

**Synthesis of compound MDI-233: (2-(6-(2-ethyl-5-fluoro-4-hydroxyphenyl)-1H-indazol-3-yl)-4,6-dihydropyrrolo[3,4-d]imidazol-5-(1H)-yl)(piperidin-1-yl)ketone**

Intermediate MDI-233-1 (100 mg, 0.121 mmol) was dissolved in methanol (6 ml), to which 10 mg Pd/C was added and concentrated hydrochloric acid (3 ml) was added dropwise. The mixture was heated to 50°C, reacted for 6 hours, filtered, and concentrated to give a residue. The residue was dissolved in methanol and was concentrated to dryness, which was repeated 3 times. The resulting residue was dissolved in methanol, and 1 ml of ammonia was added. The resulting mixture was concentrated, and purified by a preparation plate to afford 33 mg of the final product with a yield of 57.3%.

$^1$H NMR (400 MHz, MeOD-d4) δ 8.25 (d, *J* = 8.4 Hz, 1H), 7.40 (s, 1H), 7.16 - 7.14 (m, 1H), 6.95 - 6.87 (m, 2H), 4.83 - 4.65 (m, 4H), 3.35 - 3.33 (m, 4H), 2.54 (q, *J*= 7.5 Hz, 2H), 1.67 - 1.65 (m, 6H), 1.06 (t, *J* = 7.5 Hz, 3H).

**Example 32: (2-(6-(2-ethyl-5-fluoro-4-hydroxyphenyl)-1H-indazol-3-yl)-4,6-dihydropyrrolo[3,4-d]imidazol-5-(1H)-yl)(morpholino)ketone (MDI-234)**

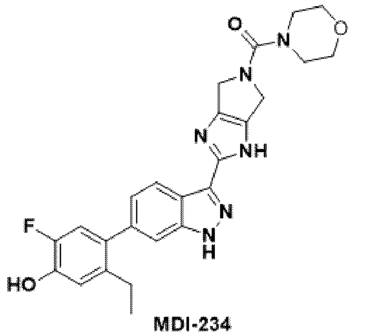

MDI-234

It was synthesised according to the method shown in Example 32 of Chinese invention patent CN111606908B. The synthesis was as follows.

**Synthetic route of MDI-234:**

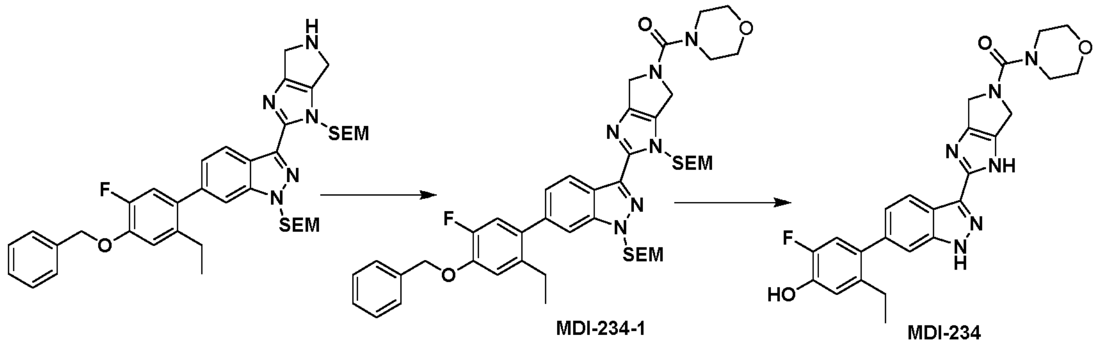

MDI-234-1 MDI-234

**Synthesis method:**

**Synthesis of intermediate MDI-234-1: (2-(6-(4-(benzyloxy)-2-ethyl-5-fluorophenyl)-1-((2-(trimethylsilyl)ethoxy) methyl)-1H-indazol-3-yl)-1-((2-(trimethylsilyl)ethoxy)methyl)-4,6-dihydropyrrolo[3,4-d]imidazol-5-(1H)-yl)(morpholino)ketone**

Triphosgene (54.1 mg, 0.182 mmol) was dissolved in 5 ml of tetrahydrofuran, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy) methyl)-1-1,4,5,6-tetrahydropyrrolo[3,4-d]imidazol-2-yl)-1H-indazole (130 mg, 0.182 mmol) in tetrahydrofuran (5 ml) was added dropwise at 0°C, followed by addition of triethylamine (55.1 mg, 0.546 mmol). The mixture was stirred at room temperature for 5 minutes, and morpholine (31.7 mg, 0.364 mmol) in tetrahydrofuran was added. The resulting mixture was stirred at room temperature for 10 minutes. Water was added and the resulting mixture was extracted twice with ethyl acetate, and the organic phases were combined, washed with water and saturated brine, dried over anhydrous sodium sulfate, concentrated, and purified by silica gel column to afford 120 mg of intermediate MDI-234-1, with a yield of 79.7%.

$^1$H NMR (400 MHz, CDCl3) δ 8.45 (d, *J* = 8.3 Hz, 1H), 7.53 - 7.51 (m, 2H), 7.47 - 7.35 (m, 4H), 7.26 - 7.23 (m, 1H), 7.06 - 6.97 (m, 2H), 5.96 (s, 2H), 5.77 (s, 2H), 5.23 (s, 2H), 4.68 (d, *J* = 54.8 Hz, 4H), 3.80 - 3.78 (m, 3H),3.67 - 3.59 (m, 4H), 3.43 - 3.40 (m, 3H), 3.27 - 3.21 (m, 6H), 2.54 (q, *J* = 7.5 Hz, 2H), 1.05 (t, *J* = 7.5 Hz, 3H), 0.96 - 0.89 (m, 4H) , -0.04(d, *J* = 2.7 Hz, 18H).

**Synthesis of compound MDI-234: (2-(6-(2-ethyl-5-fluoro-4-hydroxyphenyl)-1H-indazol-3-yl)-4,6-dihydropyrrolo [3,4-d]imidazol-5-(1H)-yl)(morpholino)ketone**

**Intermediate MDI-234-1 (120 mg, 0.145 mmol) was dissolved in methanol (6** ml), to which 12 mg Pd/C was added and concentrated hydrochloric acid (3 ml) was added dropwise. The mixture was heated to 50°C, reacted for 6 hours, filtered, and concentrated to give a residue. The residue was dissolved in methanol and was concentrated to dryness, which was repeated 3 times. The resulting residue was dissolved in methanol, and 1 ml of ammonia was added. The resulting mixture was concentrated, and purified by a preparation plate to afford 42 mg of the final product with a yield

of 60.9%.

$^1$H NMR (400 MHz, MeOD-d4) δ 8.28 (d, *J* = 8.4 Hz, 1H), 7.43 (s, 1H), 7.19 - 7.16 (m, 1H), 6.97 - 6.90 (m, 2H), 4.71 - 4.66 (m, 4H), 3.78 - 3.75 (m, 4H), 3.41-3.39 (m, 4H), 2.54 (q, *J* = 7.5 Hz, 2H), 1.06 (t, *J* = 7.5 Hz, 3H).

**Example 33: (2-(6-(2-ethyl-5-fluoro-4-hydroxyphenyl)-1H-indazol-3-yl)-4,6-dihydropyrrolo[3,4-d]imidazol-5-(1H)-yl)(4-methylpiperazin-1-yl)ketone ( MDI-235)**

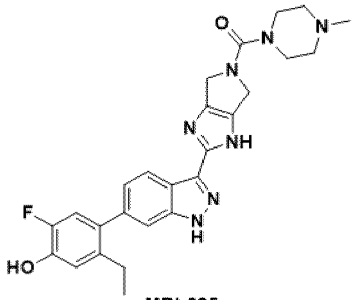

MDI-235

It was synthesised according to the method shown in Example 33 of Chinese invention patent CN111606908B. The synthesis was as follows.

**Synthetic route of MDI-235:**

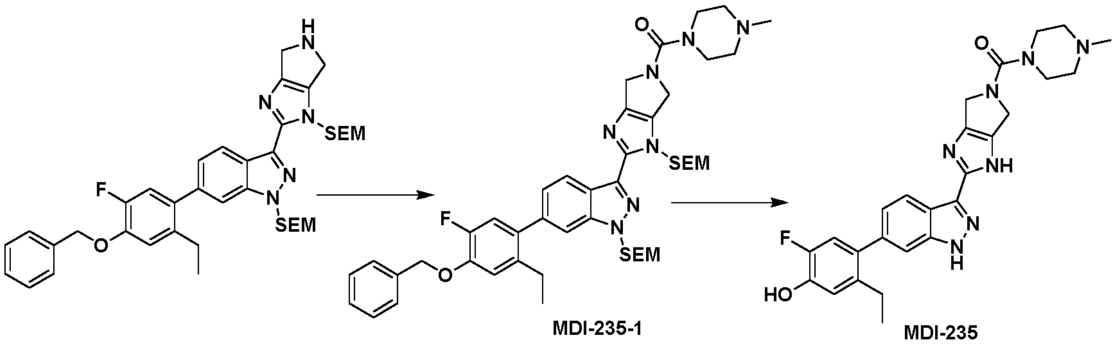

MDI-235-1

MDI-235

**Synthesis method:**

**Synthesis of intermediate MDI-235-1: (2-(6-(4-(benzyloxy)-2-ethyl-5-fluorophenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)(4-methylpiperazin-1-yl)ketone**

Triphosgene (8.3 mg, 0.028 mmol) was dissolved in 5 ml of tetrahydrofuran, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (20 mg, 0.028 mmol) in tetrahydrofuran (5 ml) was added dropwise at 0°C, followed by addition of triethylamine (8.5 mg, 0.084 mmol). The mixture was stirred at room temperature for 5 minutes, and 1-methylpiperazine (5.60 mg, 0.056 mmol) in tetrahydrofuran was added. The resulting mixture was stirred at room temperature for 10 minutes. Water was added and the resulting mixture was extracted twice with ethyl acetate, and the organic phases were combined, washed with water and saturated brine, dried over anhydrous sodium sulfate, concentrated, and purified by silica gel column to afford 20 mg of intermediate MDI-235-1, with a yield of 85.1%.

$^1$H NMR (400 MHz, CDCl3) δ 8.44 (d, *J* = 8.3 Hz, 1H), 7.50 - 7.48 (m, 2H), 7.44 - 7.33 (m, 4H), 7.23 - 7.21 (m, 1H), 7.04 - 6.94 (m, 2H), 5.93 (s, 2H), 5.75 (s, 2H), 5.20 (s, 2H), 4.70 (d, *J* = 54.8 Hz, 4H), 3.64 - 3.56 (m, 4H), 3.43 - 3.41 (m, 4H), 2.56 - 2.49 (m, 6H) , 2.34 (s, 3H), 1.03 (t, *J* = 7.5 Hz, 3H), 0.93 - 0.86 (m, 4H) , -0.07(d, *J* = 2.7 Hz, 18H).

**Synthesis of compound MDI-235: (2-(6-(2-ethyl-5-fluoro-4-hydroxyphenyl)-1H-indazol-3-yl)-4,6-dihydropyrrolo[3,4-d]imidazol-5-(1H)-yl)(4-methylpiperazin-1-yl)ketone**

Intermediate MDI-235-1 (20 mg, 0.024 mmol) was dissolved in methanol (6 ml), to which 5 mg Pd/C was added and concentrated hydrochloric acid (3 ml) was added dropwise. The mixture was heated to 50°C, reacted for 6 hours, filtered, and concentrated to give a residue. The residue was dissolved in methanol and was concentrated to dryness, which was repeated 3 times. The resulting residue was dissolved in methanol, and 1 ml of ammonia was added. The resulting mixture was concentrated, and purified by a preparation plate to afford 3 mg of the final product with a yield of 14.8%.

$^1$H NMR (400 MHz, MeOD-d4) δ 8.25 (d, *J* = 8.4 Hz, 1H), 7.40 (s, 1H), 7.16 - 7.14 (m, 1H), 6.95 - 6.87 (m, 2H), 4.83 - 4.66 (m, 4H), 3.44 - 3.41 (m, 4H), 2.56 - 2.51 (m, 6H), 2.35 (s, 3H), 1.06 (t, *J* = 7.5 Hz, 3H).

**Example 34: (2-(6-(2-ethyl-5-fluoro-4-hydroxyphenyl)-1H-indazol-3-yl)-4,6-dihydropyrrolo[3,4-dlimidazol-5-(1H) - yl)(4-ethylpiperazin-1-yl)ketone (MDI-236)**

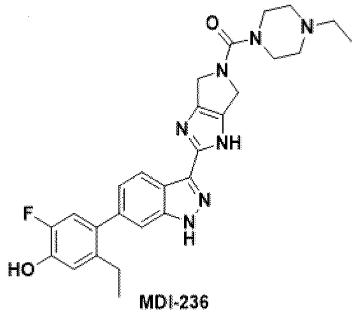

MDI-236

It was synthesised according to the method shown in Example 29 of Chinese invention patent CN111606908B. The synthesis was as follows.

**Synthetic route of MDI-236:**

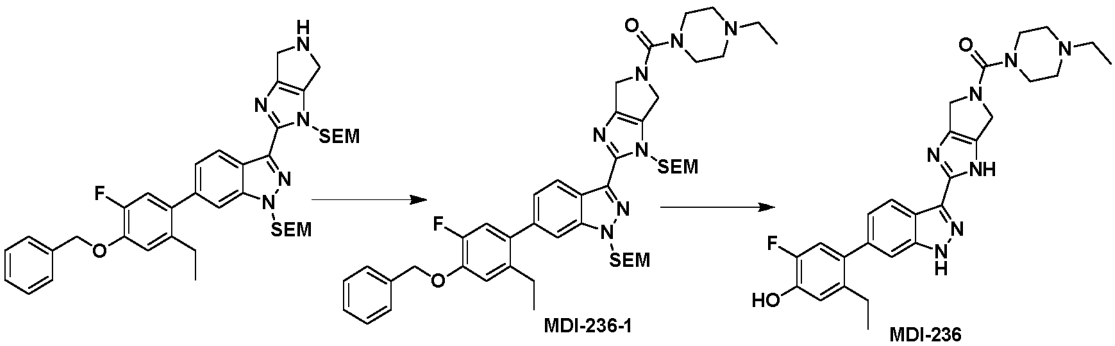

MDI-236-1

MDI-236

**Synthesis method:**

**Synthesis of intermediate MDI-236-1: (2-(6-(4-(benzyloxy)-2-ethyl-5-fluorophenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)(4-ethyl piperazin-1-yl)ketone**

Triphosgene (54.07 mg, 0.182 mmol) was dissolved in 15 ml of dichloromethane, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (20 mg, 0.028 mmol) in dichloromethane (5 ml) was added dropwise at 0°C, followed by addition of triethylamine (55.2 mg, 0.55 mmol). The mixture was stirred at room

temperature for 5 minutes, and 1-ethylpiperazine (41.5 mg, 0.364 mmol) in dichloromethane was added. The resulting mixture was stirred at room temperature for 10 minutes. Water was added and the resulting mixture was extracted twice with ethyl acetate, and the organic phases were combined, washed with water and saturated brine, dried over anhydrous sodium sulfate, concentrated, and purified by silica gel column to afford 100 mg of intermediate MDI-236-1, with a yield of 64.3%.

$^1$H NMR (400 MHz, CDCl3) δ 8.44 (d, *J* = 8 Hz, 1H), 8.28 (s, 1H), 7.50 - 7.33 (m, 5H), 7.22 (d, *J* = 8 Hz, 1H), 7.03 (d, *J* = 12 Hz, 1H), 6.95 (d, *J* = 8 Hz, 1H), 5.93 (s, 2H), 5.74 (s, 2H), 5.20 (s, 2H), 4.77 (s, 2H), 4.63 (s, 2H), 3.63-3.61 (m, 4H), 3.43 - 3.42 (m, 4H), 2.53 - 2.46 (m, 8H), 1.03 (t, *J* = 6 Hz, 3H), 0.93 - 0.86 (m, 7H), -0.06 - -0.08 (m, 18H).

**Synthesis of compound MDI-236: (2-(6-(2-ethyl-5-fluoro-4-hydroxyphenyl)-1H-indazol-3-yl)-4,6-dihydropyrrolo [3,4-d]imidazol-5-(1H)-yl)(4-ethylpiperazin-1-yl)ketone**

Intermediate MDI-236-1 (100 mg, 0.117 mmol) was dissolved in methanol (10 ml), to which 10 mg Pd/C was added and concentrated hydrochloric acid (5 ml) was added dropwise. The mixture was heated to 50°C, reacted for 6 hours, filtered, and concentrated to give a residue. The residue was dissolved in methanol and was concentrated to dryness, which was repeated 3 times. The resulting residue was dissolved in methanol, and 1 ml of ammonia was added. The resulting mixture was concentrated, and purified by a preparation plate to afford 21 mg of the final product with a yield of 35.6%.

$^1$H NMR (400 MHz, MeOD-d4) δ 8.27 (d, *J* = 8 Hz, 1H), 7.43 (s, 1H), 7.17 (d, *J* = 8 Hz, 1H), 6.96 (d, *J* = 12 Hz, 1H), 6.91 (d, *J* = 8 Hz, 1H), 4.75 - 4.60 (m, 4H), 3.48 - 3.44 (m, 4H), 2.61 - 2.48 (m, 8H), 1.17 (t, *J* = 8 Hz, 3H), 1.08 (t, *J* = 8 Hz, 3H).

**Example 35: cyclopropyl (2-(6-(2-ethyl-5-fluoro-4-hydroxyphenyl)-1H-pyrazolo[4,3-b]pyridine-3-yl)-4,6-dihydropyrrolo[3,4-dlimidazol-5(1H)-yl)ketone (MDI-237)**

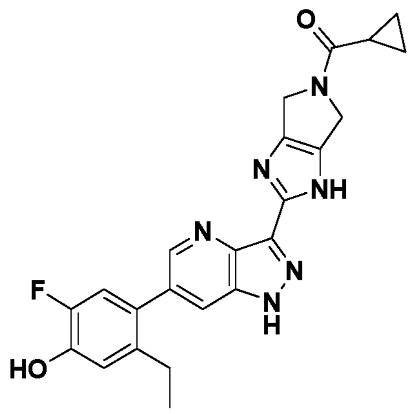

**MDI-237**

It was synthesised according to the method shown in Example 35 of Chinese invention patent CN111606908B, and the NMR hydrogen spectral data of the resulting product were as follows:

$^1$H NMR (400 MHz, DMSO) δ 13.60 (s, 1H), 12.60-12.48 (m, 1H), 10.02 (s, 1H), 8.53 (d, J = 1.6 Hz, 1H), 7.95 (s, 1H), 7.16 (d, J = 11.8 Hz, 1H), 6.98 (d, J = 9.1 Hz, 1H), 4.91-4.41 (m, 4H), 2.51-2.47 (m, 2H), 1.96-1.84 (m, 1H) ), 1.03 (t, J = 8.0 Hz, 3H), 0.87-0.80 (m, 4H). LC-MS m/z (ESI) [M+H]+ calculated value for $C_{23}H_{22}FN_6O_2$: 433.2; measured value: 433.2.

**Example 36: cyclopropyl (2-(6-(2-ethyl-5-fluoro-4-hydroxyphenyl)-4-methyl-1H-indazol-3-yl)-4,6-dihydropyrrolo[3,4-d]imidazol-5(1H)-yl)ketone(MDI-239)**

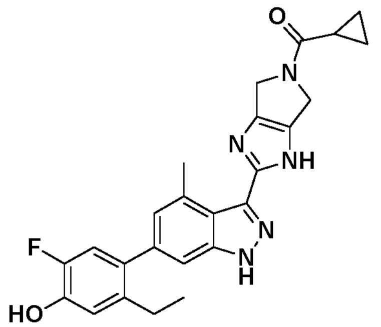

MDI-239

It was synthesised according to the method shown in Example 36 of Chinese invention patent CN111606908B, and the NMR hydrogen spectral data of the resulting product were as follows:

$^1$H NMR (400 MHz, MeOD) δ 7.27 (s, 1H), 6.95-6.88 (m, 3H), 4.96 (s, 2H), 4.66 (s, 2H), 2.63 (s, 3H), 2.55 (q, J = 7.5 Hz, 2H), 1.98-1.92 (m, 1H), 1.07 (t, J = 7.5 Hz, 3H), 1.04-0.92 (m, 4H).

**Example 37: (S)-(2-(6-(2-ethyl-5-fluoro-4-hydroxyphenyl)-4-methyl-1H-indazol-3-yl)-4,6-dihydropyrrolo[3,4-d]imidazol-5(1H)-yl)(3-hydroxylpyrrolidin-1-yl)ketone(MDI-240)**

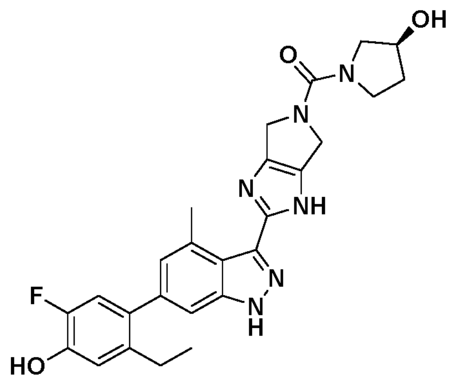

MDI-240

It was synthesised according to the method shown in Example 37 of Chinese invention patent CN111606908B, and the NMR hydrogen spectral data of the resulting product were as follows:

$^1$H NMR (400 MHz, MeOD) δ 7.27 (s, 1H), 6.95-6.88 (m, 3H), 4.85 -4.82 (m, 2H), 4.62-4.59 (m, 2H), 4.46-4.45 (m, 1H), 3.79-3.69 (m, 2H), 3.64-3.57 (m, 1H), 3.46-3.42 (m, 1H), 2.61 (s, 3H), 2.56 (q, J = 7.5 Hz, 2H), 2.09- 1.98 (m, 2H), 1.07 (t, J = 7.5 Hz, 3H).

**Example 38: cyclopropyl(2-(6-(2-ethyl-5-fluoro-4-hydroxyphenyl)-1H-pyrazolo[4,3-c]pyridine-3-yl)-4,6-dihydropyrrolo[3,4-d]imidazol-5(1H)-yl)ketone (MDI-242)**

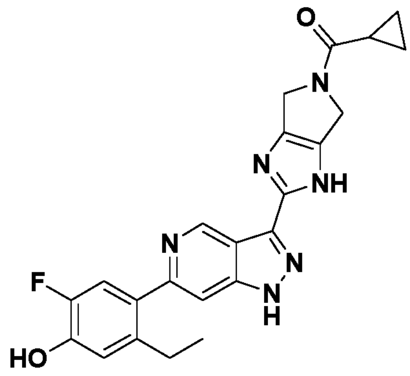

**MDI-242**

It was synthesised according to the method shown in Example 38 of Chinese invention patent CN111606908B, and the NMR hydrogen spectral data of the resulting product were as follows:

$^1$H NMR (400 MHz, MeOD) δ 9.61 (d, J = 1.0 Hz, 1H), 7.77 (d, J = 1.1 Hz, 1H), 7.16 (d, J = 11.6 Hz, 1H), 6.93 (d, J = 8.8 Hz, 1H), 5.07-4.88 (m, 2H), 4.68-4.62 (m, 2H), 2.69-2.64 (m, 2H), 1.98-1.89 (m, 1H), 1.09-1.05 (m, 3H) ), 1.01-0.98 (m, 2H), 0.96-0.94 (m, 2H).

**Example 39: (R)-(2-(6-(2-ethyl-5-fluoro-4-hydroxyphenyl)-1H-indazol-3-yl)-4,6-dihydropyrrolo[3,4-d]imidazol-5(1H)-yl)(3-hydroxylpyrrolidin-1-yl)ketone (MDI-243)**

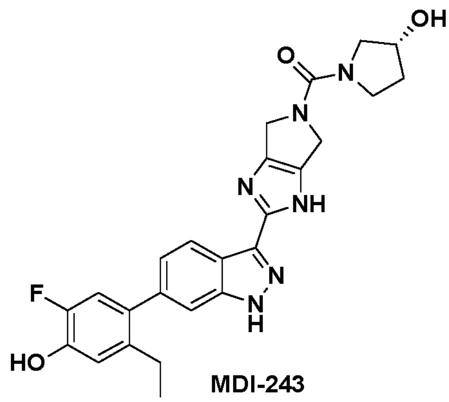

**MDI-243**

It was synthesised according to the method shown in Example 39 of Chinese invention patent CN111606908B, and the NMR hydrogen spectral data of the resulting product were as follows:

$^1$H NMR (400 MHz, MeOD) δ 8.27 (d, *J* = 8.4 Hz, 1H), 7.43 (d, *J* = 1.0 Hz, 1H), 7.17 (d, *J* = 8.4 Hz, 1H), 6.98 - 6.90 (m, 2H), 4.82 - 4.60 (m, 4H), 4.47 - 4.45 (m, 1H), 3.79 - 3.70 (m, 2H), 3.60 - 3.57 (m, 1H), 3.46 - 3.43 (m, 1H), 2.56 (q, *J* = 7.5 Hz, 2H), 2.09 - 1.98 (m, 2H), 1.08 (t, *J* = 7.5 Hz, 3H).

**Example 41: (2-(6-(2-ethyl-5-fluoro-4-hydroxyphenyl)-1H-indazol-3-yl)-4,6-dihydropyrrolo[3,4-d]imidazol-5(1H)-yl)(4-hydroxylpiperidin-1-yl)ketone(MDI-245)**

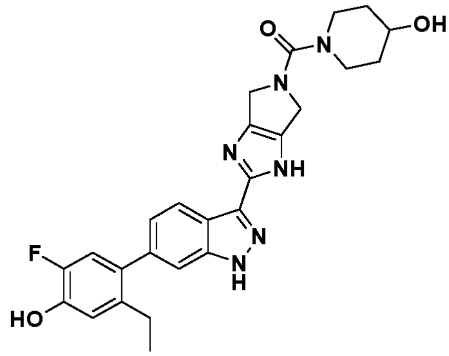

MDI-245

It was synthesised according to the method shown in Example 41 of Chinese invention patent CN111606908B. The synthesis was as follows.

**Synthetic route of MDI-245:**

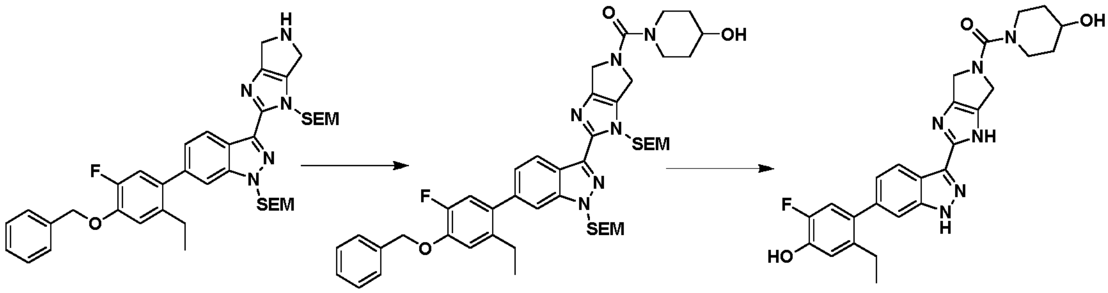

MDI-245-1

MDI-245

**Synthesis method:**

**Synthesis of intermediate MDI-245-1: (2-(6-(4-(benzyloxy)-2-ethyl-5-fluorophenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)(4-hydroxylpiperidin-1-yl)ketone**

Triphosgene (54.1 mg, 0.18 mmol) was dissolved in 10 ml of dichloromethane, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (130 mg, 0.18 mmol) in dichloromethane (5 ml) was added dropwise at 0°C, followed by addition of anhydrous triethylamine (185 mg, 1.8 mmol). The mixture was stirred at room temperature for 10 minutes. TLC monitored that the raw materials disappeared. Piperidin-4-ol (36.9 mg, 0.36 mmol) in dichloromethane (5ml) was added. The resulting mixture was stirred at room temperature for 20 minutes. Water was added to quench the reaction and the resulting mixture was extracted twice with ethyl acetate, and the organic phases were combined, washed with saturated brine, dried over anhydrous sodium sulfate, concentrated, and purified by silica gel column to afford 116 mg of intermediate MDI-245-1, with a yield of 75.7%.

$^1$H NMR (400 MHz, CDCl3) $\delta$ 8.46 (d, *J* = 8.3 Hz, 1H), 7.53 - 7.51 (m, 2H), 7.47 - 7.35(m, 4H), 7.25 (d, *J* = 8.4 Hz, 1H), 7.06-6.97(m, 2H), 5.96 (s, 2H), 5.75 (s, 2H), 5.37 (s, 2H), 4.79 - 4.66 (m, 4H), 3.95 - 3.92 (m, 1H), 3.75 - 3.72 (m, 2H), 3.66-3.52 (m, 4H), 3.12 - 3.07 (m, 2H), 2.54 (q, *J* = 7.6 Hz, 2H), 2.02 - 1.91 (m, 2H), 1.68 - 1.63 (m, 2H), 1.06 (t, *J* = 7.5 Hz, 3H), 0.99 - 0.89 (m, 4H), 0.02 (s, 9H),-0.05 (d, *J* = 3.4 Hz, 9H).

**Synthesis of compound MDI-245: (2-(6-(2-ethyl-5-fluoro-4-hydroxyphenyl)-1H-indazol-3-yl)-4,6-dihydropyrrolo[3,4-d]imidazol-5-(1H)-yl)(4-hydroxylpiperidin-1-yl)ketone**

MDI-243-1 (116 mg, 0.14 mmol) was dissolved in 20 ml methanol, and 20 mg palladium on carbon was added. The atmosphere was replaced hydrogen. It was allowed to react at 40 °C for 1 hour. After the reaction was completed, the mixture was filtered, and the filtrate was concentrated. The concentrate was dissolved in 12 ml methanol and 6ml

concentrated hydrochloric acid was added. It was allowed to react at 50°C for 7 hours and the mixture was concentrated to give a residue. The residue was dissolved in methanol and was concentrated to dryness, which was repeated 3 times. The resulting residue was dissolved in 8ml methanol, and 0.8ml aqueous ammonia was added. The resulting mixture was concentrated, and purified to afford 30 mg of the final product with a yield of 44.4%.

$^1H$ NMR (400 MHz, MeOD) δ 8.27 (d, *J* = 8.4 Hz, 1H), 7.43 (s, 1H), 7.18 (d, *J*= 8.4 Hz, 1H), 6.97 - 6.90 (m, 2H), 4.72 - 4.65 (m, 4H), 3.88 - 3.82 (m, 1H), 3.76 - 3.73 (m, 2H), 3.13 - 3.06 (m, 2H), 2.56 (q, *J* = 7.5 Hz, 2H), 1.97 - 1.95 (m, 2H), 1.63 - 1.55 (m, 2H), 1.08 (t, *J* = 7.5 Hz, 3H).

**Example 42: 2-(6-(2-ethyl-5-fluoro-4-hydroxyphenyl)-1H-indazol-3-yl)-N-methyl-4,6-dihydropyrrolo[3,4-d]imidazol-5-(1H)-carboxamide(MDI-246)**

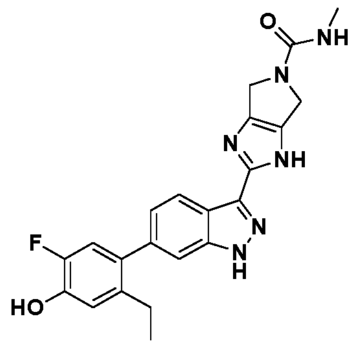

**MDI-246**

It was synthesised according to the method shown in Example 42 of Chinese invention patent CN111606908B, and the NMR hydrogen spectral data of the resulting product were as follows:

$^1H$ NMR (400 MHz, MeOD) δ 8.27 (d, *J* = 8.4 Hz, 1H), 7.43 (s, 1H), 7.18 (d, *J*= 8.4 Hz, 1H), 6.97 - 6.90 (m, 2H), 4.56 (s, 4H), 2.84 (s, 3H), 2.56 (q, *J* = 7.5 Hz, 2H), 1.08 (t, *J* = 7.5 Hz, 3H).

**Example 43: 2-(6-(2-ethyl-5-fluoro-4-hydroxyphenyl)-1H-indazol-3-yl)-N-ethyl-4,6-dihydropyrrolo[3,4-d]imidazol-5-(1H)-carboxamide(MDI-247)**

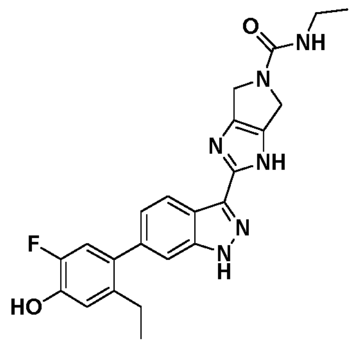

**MDI-247**

It was synthesised according to the method shown in Example 43 of Chinese invention patent CN111606908B. The synthesis was as follows.

**Synthetic route of MDI-247:**

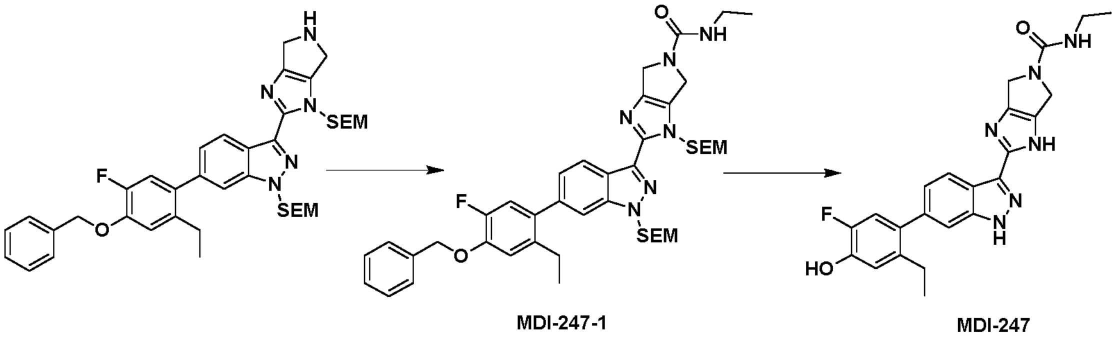


**Synthesis method:**

**Synthesis of intermediate MDI-247-1: 2-(6-(4-(benzyloxy)-2-ethyl-5-fluorophenyl)-1- ((2-(trimethylsilyl)ethoxy) methyl)-1H-indazol-3-yl)-N-ethyl-1-((2-(trimethylsilyl)ethoxy)methyl)-4,6-dihydropyrrolo[3,4-d]imidazol-5-(1H)-carboxamide**

Triphosgene (22.9 mg, 0.08 mmol) was dissolved in 6 ml of dry dichloromethane, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy) methyl)-1-1,4,5,6-tetrahydropyrrolo[3,4-d]imidazol-2-yl)-1H-indazole (55 mg, 0.08 mmol) in dichloromethane (5 ml) was added dropwise at 0°C, then anhydrous triethylamine (78.0 mg, 0.8 mmol) was added slowly. The mixture was stirred at room temperature for 10 minutes. TLC monitored that the raw materials disappeared. Ethylamine hydrochloride (12.6 mg, 0.16 mmol) was added. The resulting mixture was stirred at room temperature for 20 hours. Water was added to quench the reaction and the resulting mixture was extracted twice with ethyl acetate, and the organic phases were combined, washed with saturated brine, dried over anhydrous sodium sulfate, concentrated, and purified by silica gel column to afford 47 mg of intermediate MDI-247-1, with a yield of 77.7%.

$^1$H NMR (400 MHz, CDCl3) δ 8.46 (d, *J* = 8.3 Hz, 1H), 7.53 - 7.51 (m, 2H), 7.47 - 7.35(m, 4H), 7.25 (d, *J* = 8.4 Hz, 1H), 7.07 - 6.97(m, 2H), 5.96 (s, 2H), 5.78 (s, 2H), 5.23 (s, 2H), 4.72 - 4.54 (m, 4H), 3.65 - 3.58 (m, 4H), 3.45 - 3.38 (m, 2H), 2.54 (q, *J* = 7.6 Hz, 2H), 1.18 - 1.14 (m, 3H), 1.05 (t, *J* = 7.5 Hz, 3H), 0.95 - 0.89 (m, 4H), 0.02 (s, 9H),-0.05 (d, *J* = 3.4 Hz, 9H).

**Synthesis of compound MDI-247: 2-(6-(2-ethyl-5-fluoro-4-hydroxyphenyl)-1H-indazol-3-yl)-N-ethyl-4,6-dihydropyrrolo[3,4-d]imidazol-5-(1H)-carboxamide**

MDI-247-1 (47 mg, 0.06 mmol) was dissolved in 10 ml methanol, and 8 mg palladium on carbon was added. The atmosphere was replaced hydrogen. It was allowed to react at 40 °C for 1 hour. After the reaction was completed, the mixture was filtered, and the filtrate was concentrated. The concentrate was dissolved in 6 ml methanol and 3ml concentrated hydrochloric acid was added. It was allowed to react at 50°C for 7 hours and the mixture was concentrated to give a residue. The residue was dissolved in methanol and was concentrated to dryness, which was repeated 3 times. The resulting residue was dissolved in 5ml methanol, and 0.5ml aqueous ammonia was added. The resulting mixture was concentrated, and purified to afford 11 mg of the final product with a yield of 42.4%.

$^1$H NMR (400 MHz, MeOD) δ 8.27 (d, *J* = 8.4 Hz, 1H), 7.43 (s, 1H), 7.18 (d, *J* = 8.4 Hz, 1H), 6.98 - 6.90 (m, 2H), 4.57 (s, 4H), 3.38 - 3.28 (m, 2H), 2.56 (q, *J* = 7.5 Hz, 2H), 1.21 (t, *J* = 7.2 Hz, 3H), 1.08 (t, *J* = 7.5 Hz, 3H).

**Example 44: 2-(6-(2-ethyl-5-fluoro-4-hydroxyphenyl)-1H-indazol-3-yl)-N-(2-hydroxylethyl)-4,6-dihydropyrrolo [3,4-d]imidazol-5(1H)-carboxamide(MDI-248)**

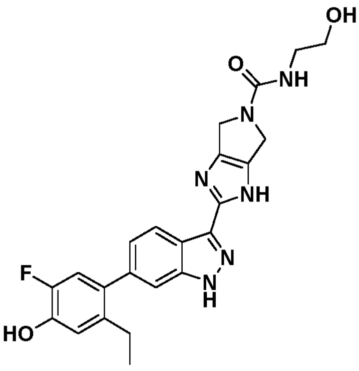

MDI-248

It was synthesised according to the method shown in Example 44 of Chinese invention patent CN111606908B. The synthesis was as follows.

**Synthetic route of MDI-248:**

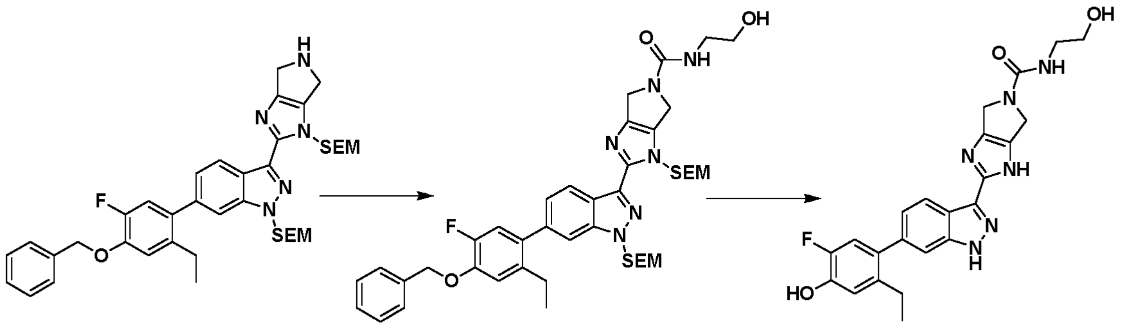

MDI-248-1 MDI-248

**Synthesis method:**

**Synthesis of intermediate MDI-248-1: 2-(6-(4-(benzyloxy)-2-ethyl-5-fluorophenyl)-1-((2-(trimethylsilyl)ethoxy) methyl)-1H-indazol-3-yl)-N-(2-hydroxylethyl)-1-((2-(trimethylsilyl)ethoxy)methyl)-4,6-dihydropyrrolo[3,4-d]imidazol-5-(1H)-carboxamide**

Triphosgene (22.9 mg, 0.08 mmol) was dissolved in 6 ml of dichloromethane, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy) methyl)-1-1,4,5,6-tetrahydropyrrolo[3,4-d]imidazol-2-yl)-1H-indazole (55 mg, 0.08 mmol) in dichloromethane (5 ml) was added dropwise at 0°C, then anhydrous triethylamine (78 mg, 0.8 mmol) was added slowly. The mixture was stirred at room temperature for 10 minutes. TLC monitored that the raw materials disappeared. Ethanolamine (9.4 mg, 0.16 mmol) in dichloromethane (5ml) was added. The resulting mixture was stirred at room temperature for 1 hour. Water was added to quench the reaction and the resulting mixture was extracted twice with dichloromethane, and the organic phases were combined, washed with saturated brine, dried over anhydrous sodium sulfate, concentrated, and purified by silica gel column to afford 44 mg of intermediate MDI-248-1, with a yield of 71.3%.

$^1$H NMR (400 MHz, CDCl3) δ 8.46 (d, *J* = 8.3 Hz, 1H), 7.73 - 7.51 (m, 2H), 7.48 - 7.35(m, 4H), 7.27 - 7.24 (m, 1H), 7.07 - 6.97(m, 2H), 5.96 (s, 2H), 5.78 (s, 2H), 5.23 (s, 2H), 4.73 - 4.57 (m, 4H), 3.65 - 3.53 (m, 6H), 3.33 - 3.29 (m, 2H), 2.54 (q, *J* = 7.6 Hz, 2H), 1.05 (t, *J* = 7.5 Hz, 3H), 0.95 - 0.89 (m, 4H), 0.02 (s, 9H),-0.05 (s, 9H).

**Synthesis of compound MDI-248: 2-(6-(2-ethyl-5-fluoro-4-hydroxyphenyl)-1H-indazol-3-yl)-N-(2-hydroxylethyl)-4,6-dihydropyrrolo[3,4-d]imidazol-5(1H)-carboxamide**

MDI-248-1 (44 mg, 0.06 mmol) was dissolved in 10 ml methanol, and 8 mg palladium on carbon was added. The atmosphere was replaced hydrogen. It was allowed to react at 40 °C for 1 hour. After the reaction was completed, the

mixture was filtered, and the filtrate was concentrated. The concentrate was dissolved in 6 ml methanol and 3ml concentrated hydrochloric acid was added. It was allowed to react at 50°C for 7 hours and the mixture was concentrated to give a residue. The residue was dissolved in methanol and was concentrated to dryness, which was repeated 3 times. The resulting residue was dissolved in 5ml methanol, and 0.5ml aqueous ammonia was added. The resulting mixture was concentrated, and purified to afford 14 mg of the final product with a yield of 56.6%.

$^1$H NMR (400 MHz, MeOD) δ 8.28 (d, *J* = 8.4 Hz, 1H), 7.43 (s, 1H), 7.18 (d, *J* = 8.4 Hz, 1H), 6.97 - 6.90 (m, 2H), 4.59 (s, 4H), 3.68 (t, *J* = 5.8 Hz, 2H), 3.40 (t, *J* = 5.8 Hz, 2H), 2.56 (q, *J* = 7.5 Hz, 2H), 1.08 (t, *J*= 7.5 Hz, 3H)

**Example 46: 1-(2-(6-(2-ethyl-5-fluoro-4-hydroxyphenyl)-1H-indazol-3-yl)-1,4,5,6-tetrahydropyrrolo[3,4-d]imidazol-5 -carbonyl)pyrrolidin-3-nitrile(MDI-250)**

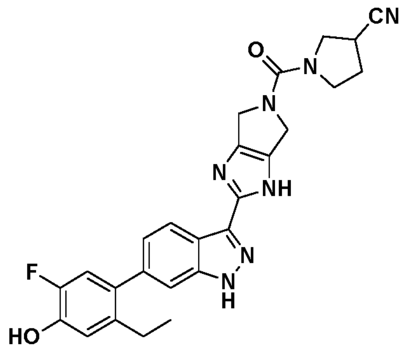

MDI-250

It was synthesised according to the method shown in Example 46 of Chinese invention patent CN111606908B, and the NMR hydrogen spectral data of the resulting product were as follows:

$^1$H NMR (400 MHz, MeOD) δ 8.27 (d, *J* = 8.4 Hz, 1H), 7.43 (s, 1H), 7.18 (d, *J* = 8.4 Hz, 1H), 6.98 - 6.90 (m, 2H), 4.70 (s, 4H), 3.89 - 3.85 (m, 1H), 3.78 - 3.76 (m, 1H), 3.70 - 3.59 (m, 2H), 3.23 - 3.18 (m, 1H), 2.56 (q, *J* = 7.5 Hz, 2H), 2.42 - 2.19 (m, 2H), 1.08 (t, *J* = 7.5 Hz, 3H).

**Example 47: 2-(6-(2-ethyl-5-fluoro-4-hydroxyphenyl)-1H-indazol-3-yl)-N-(tetrahydrofuran-3-yl)-4,6-dihydropyrrolo[3,4-d]imidazol-5(1H)-carboxamide(MDI-251)**

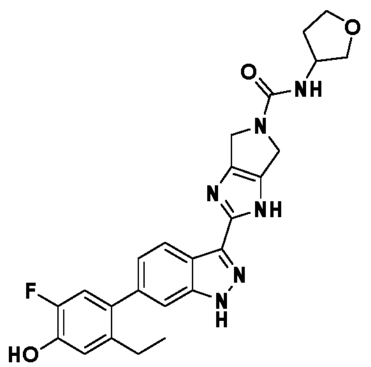

MDI-251

It was synthesised according to the method shown in Example 47 of Chinese invention patent CN111606908B, and the NMR hydrogen spectral data of the resulting product were as follows:

$^1$H NMR (400 MHz, MeOD) δ 8.28 (d, *J* = 8.4 Hz, 1H), 7.43 (s, 1H), 7.18 (d, *J*= *8.4* Hz, 1H), 6.97 - 6.90 (m, 2H), 4.63 (s, 4H), 4.45 - 4.40 (m, 1H), 4.03 - 3.94 (m, 2H), 3.87 - 3.81 (m, 1H), 3.71 - 3.68 (m, 1H), 2.56 (q, *J* = 7.5 Hz, 2H), 2.32 - 1.87 (m, 2H), 1.08 (t, *J* = 7.5 Hz, 3H).

**Example 48: Methyl 2-(6-(2-ethyl-5-fluoro-4-hydroxyphenyl)-1H-indazol-3-yl)-4,6-dihydropyrrolo[3,4-d]imidazol-5(1H)-carboxylate (MDI-252)**

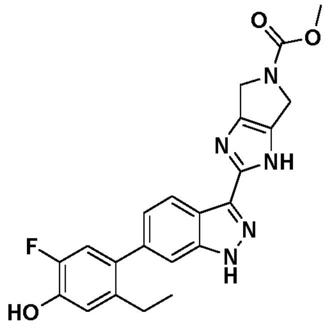

**MDI-252**

It was synthesised according to the method shown in Example 48 of Chinese invention patent CN111606908B, and the NMR hydrogen spectral data of the resulting product were as follows:

$^1$H NMR (400 MHz, MeOD) δ 8.28 (d, *J* = 8.4 Hz, 1H), 7.43 (s, 1H), 7.18 (d, *J* = 8.4 Hz, 1H), 6.97 - 6.90 (m, 2H), 4.61 (s, 4H), 3.83 (s, 3H), 2.56 (q, *J* = 7.5 Hz, 2H), 1.08 (t, *J* = 7.5 Hz, 3H).

**Example 49: Ethyl 2-(6-(2-ethyl-5-fluoro-4-hydroxyphenyl)-1H-indazol-3-yl)-4,6-dihydropyrrolo[3,4-d]imidazol-5(1H)-carboxylate (MDI-253)**

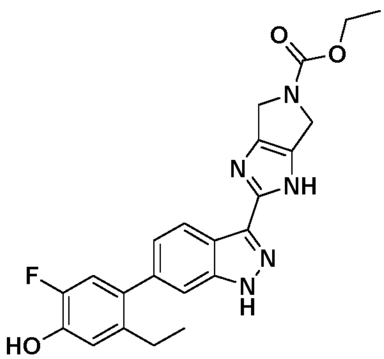

**MDI-253**

It was synthesised according to the method shown in Example 49 of Chinese invention patent CN111606908B. The synthesis was as follows.

**Synthetic route of MDI-253:**

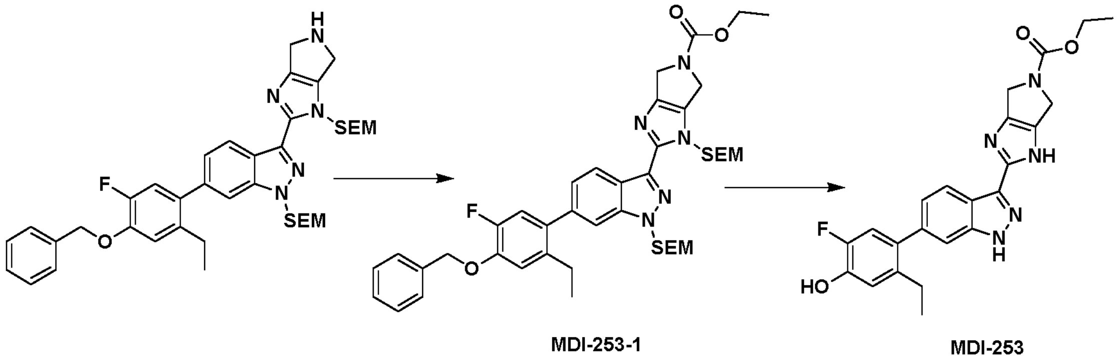


**Synthesis method:**

**Synthesis of intermediate MDI-253-1: Ethyl 2-(6-(4-(benzyloxy)-2-ethyl-5-fluorophenyl)-1-((2-(trimethylsilyl) ethoxy)methyl)-1H-indazol-3-yl)-1-((2 - (trimethylsilyl)ethoxy)methyl)-4,6-dihydropyrrolo[3,4-d]imidazole-5(1H)-carboxylate**

Triphosgene (20.1 mg, 0.07 mmol) was dissolved in 5 ml of dry dichloromethane, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy) methyl)-1-1,4,5,6-tetrahydropyrrolo[3,4-d]imidazol-2-yl)-1H-indazole (48 mg, 0.07 mmol) in dichloromethane (5 ml) was added dropwise at 0°C, then anhydrous triethylamine (68.1 mg, 0.67 mmol) was added slowly. The mixture was stirred at room temperature for 10 minutes. TLC monitored that the raw materials disappeared. The reaction mixture was concentrated and was dissolved in 10 ml ethanol. DMAP (8.2 mg, 0.07 mmol) was added. It was allowed to react at 80 °C for 4 hours. The reaction mixture was concentrated to which water was added. The resulting mixture was extracted twice with dichloromethane, and the organic phases were combined, washed with saturated brine, dried over anhydrous sodium sulfate, concentrated, and purified by silica gel column to afford 33 mg of intermediate MDI-253-1, with a yield of 62.5%.

$^1$H NMR (400 MHz, CDCl3) δ 8.50 - 8.45 (m, 1H), 7.53 - 7.51 (m, 2H), 7.47 - 7.37(m, 4H), 7.25 (d, *J* = 8.4 Hz, 1H), 7.07 - 6.96(m, 2H), 5.96 (s, 2H), 5.77 (s, 2H), 5.23 (s, 2H), 4.72 - 4.59 (m, 4H), 4.29 - 4.25 (m, 2H), 3.65 - 3.58 (m, 4H), 2.54 (q, *J* = 7.6 Hz, 2H), 1.38 - 1.34 (m, 3H), 1.05 (t, *J* = 7.5 Hz, 3H), 0.95 - 0.89 (m, 4H), 0.02 (s, 9H),-0.05 (s, 9H).

**Synthesis of compound MDI-253: Ethyl 2-(6-(2-ethyl-5-fluoro-4-hydroxyphenyl)-1H-indazol-3-yl)-4,6-dihydropyrrolo[3,4-d]imidazole-5(1H)-carboxylate**

MDI-253-1 (33 mg, 0.04 mmol) was dissolved in 10 ml ethanol, and 6 mg palladium on carbon was added. The atmosphere was replaced hydrogen. It was allowed to react at 40 °C for 1 hour. After the reaction was completed, the mixture was filtered, and the filtrate was concentrated. The concentrate was dissolved in 6 ml ethanol and 3ml concentrated hydrochloric acid was added. It was allowed to react at 50°C for 7 hours and the mixture was concentrated to give a residue. The residue was dissolved in ethanol and was concentrated to dryness, which was repeated 3 times. The resulting residue was dissolved in 5ml ethanol, and 0.5ml aqueous ammonia was added. The resulting mixture was concentrated, and purified to afford 10 mg of the final product with a yield of 54.8%.

$^1$H NMR (400 MHz, MeOD) δ 8.27 (d, *J* = 8.4 Hz, 1H), 7.43 (s, 1H), 7.18 (d, *J* = 8.4 Hz, 1H), 6.97 - 6.90 (m, 2H), 4.60 (s, 4H), 4.26 (q, *J* = 7.1 Hz, 2H), 2.57 (q, *J* = 7.5 Hz, 2H), 1.36 (t, *J* = 7.1 Hz, 3H), 1.08 (t, *J* = 7.5 Hz, 3H).

**Example 50: (S)-(2-(6-(2-ethyl-5-fluoro-4-hydroxyphenyl)-1H-pyrazolo [3,4-b]pyridine-3-yl)-4,6-dihydropyrrolo [3,4-d]imidazol-5(1H)-yl)(3-hydroxylpyrrolidin-1-yl)ketone (MDI-255)**

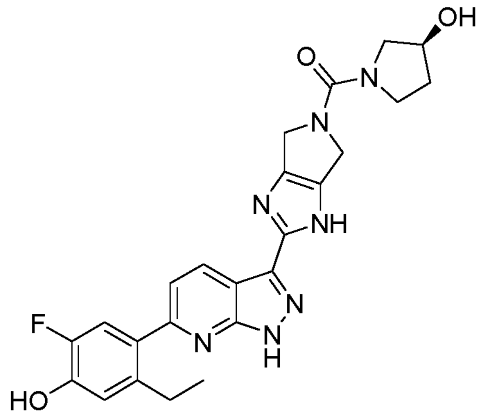

**MDI-255**

It was synthesised according to the method shown in Example 50 of Chinese invention patent CN111606908B, and the NMR hydrogen spectral data of the resulting product were as follows:

$^1$H NMR (400 MHz, DMSO) δ 13.61 (s, 1H), 10.22 (s, 1H), 8.78 (d, J = 8.0 Hz, 1H), 7.71 (d, J = 8.0 Hz, 1H), 7.34 (d, J = 12.0 Hz, 1H), 6.97 (d, J = 8.0 Hz, 1H), 4.93 (d, J = 4.0 Hz, 1H), 4.75-4.42 (m, 4H), 4.30-4.27 (m, 1H) , 3.58-3.53 (m, 2H), 3.41-3.40 (m, 1H), 3.26-3.23 (m, 1H), 2.73-2.71 (m, 2H), 2.01-1.79 (m, 2H), 1.09 (t, J = 8.0 Hz, 3H).

**Example 51: 3-(2-(6-(2-ethyl-5-fluoro-4-hydroxyphenyl)-1H-indazol-3-yl) -4,6-dihydropyrrolo[3,4-d]imidazol-5(1H)-yl)-3-oxypropionitrile (MDI-256)**

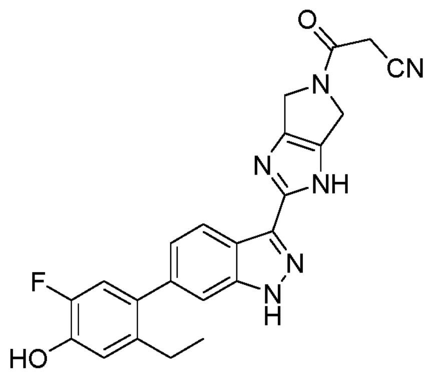

**MDI-256**

It was synthesised according to the method shown in Example 51 of Chinese invention patent CN111606908B, and the NMR hydrogen spectral data of the resulting product were as follows:

$^1$H NMR (400 MHz, MeOD) δ 8.27 (d, J = 8.0 Hz, 1H), 7.43 (s, 1H), 7.18 (d, J = 8.0 Hz, 1H), 6.97 (dd, J = 8.0 Hz, J = 20.0 Hz, 2H), 4.77-4.70 (m, 4H), 3.62 (s, 2H), 2.59-2.53 (m, 2H), 1.09 (t, J = 8.0 Hz, 3H).

**Example 52: 2-(6-(2-ethyl-5-fluoro-4-hydroxyphenyl)-1H-indazol-3-yl)-N,N-dimethyl-4,6-dihydropyrrolo[3,4-d] imidazol-5(1H)-carboxamide(MDI-257)**

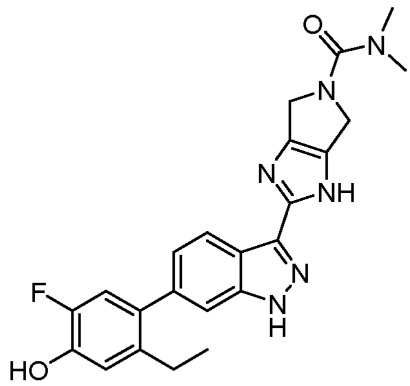

**MDI-257**

It was synthesised according to the method shown in Example 39 of Chinese invention patent CN111606908B. The synthesis was as follows.

**Synthetic route of MDI-257:**

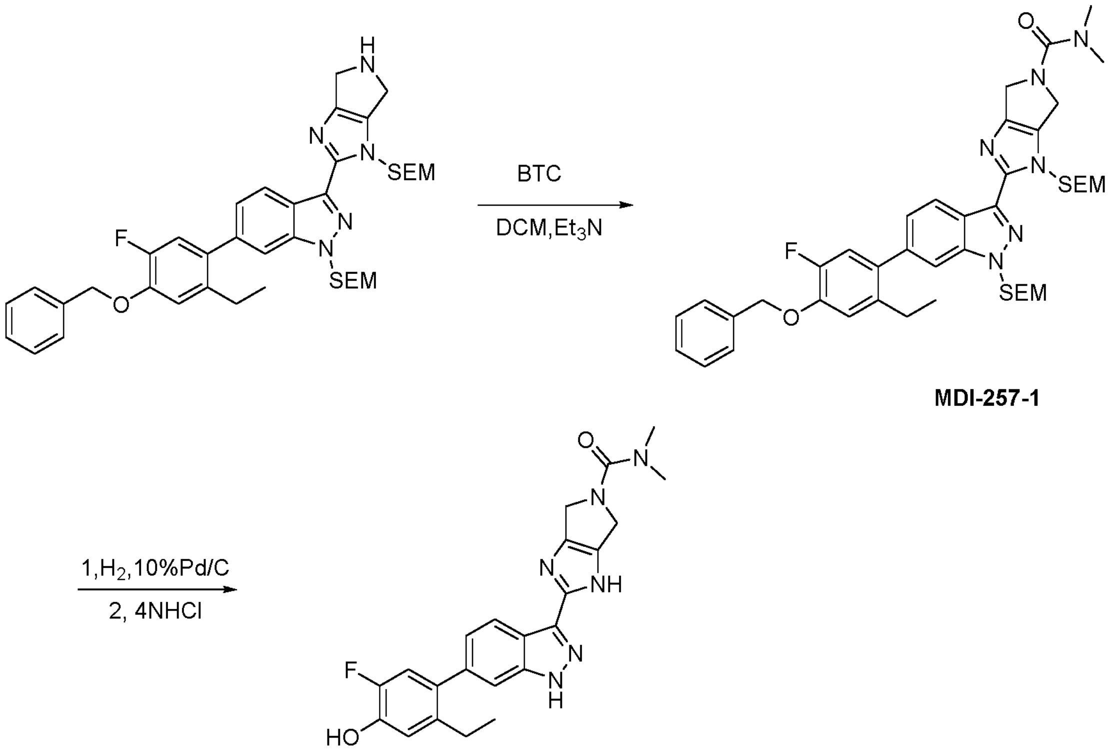


MDI-257-1

**MDI-257**

**Synthesis method:**

**Synthesis of intermediate MDI-257-1: 2-(6-(4-(benzyloxy)-2-ethyl-5-fluorophenyl)-1-((2-(trimethylsilyl)ethoxy) methyl)-1H-indazol-3-yl)-N,N-dimethyl-1-((2-(trimethylsilyl)ethoxy)methyl)-4,6-dihydropyrrolo[3,4-d]imidazol-5(1H)-carboxamide**

The synthesis process was similar to that of the intermediate MDI-246-1 of CN111606908B with the exception that dimethylamine hydrochloride was used instead of methylamine hydrochloride.

**Synthesis of compound MDI-257: 2-(6-(2-ethyl-5-fluoro-4-hydroxyphenyl)-1H-indazol-3-yl)-N,N-dimethyl-4,6-dihydropyrrolo[3,4-d]imidazol-5(1H)-carboxamide**

Intermediate MDI-257-1 (41 mg, 0.05 mmol) was dissolved in methanol (6 ml), and 8 mg 10% Pd/C was added. The atmosphere was replaced with hydrogen 3 times. The mixture was heated to 40 °C, reacted for 1 hour, filtered, and concentrated, to which 4 ml of methanol and 1 ml of concentrated hydrochloric acid were added. The mixture was heated to 50 °C, reacted for 6 hours, and concentrated to give a residue. The residue was dissolved in methanol, and was concentrated to dryness, which was repeated 3 times. The resulting residue was dissolved in methanol, to which 1 ml of ammonia was added to neutralize. The resulting mixture was concentrated and purified by a preparation plate to afford 8 mg of the final product with a yield of 35.2%.

$^1$H NMR (400 MHz, DMSO) δ 13.28 (s, 1H), 9.85 (s, 1H), 8.32 (d, J = 8.0 Hz, 1H), 7.40 (s, 1H), 7.13 (d, J = 8.0 Hz , 1H), 7.03 (d, J = 12.0 Hz, 1H), 6.93 (d, J = 12.0 Hz, 1H), 4.54-4.53 (m, 4H), 2.85 (s, 6H), 2.50-2.46 (m, 2H), 1.04 (t, J = 8.0 Hz, 3H).

**Example 53: N-(2-cyanoethyl)-2-(6-(2-ethyl-5-fluoro-4-hydroxyphenyl)-1H-indazol-3-yl)-4,6-dihydropyrrolo[3,4-d]imidazole-5(1H)-carboxamide(MDI-258)**

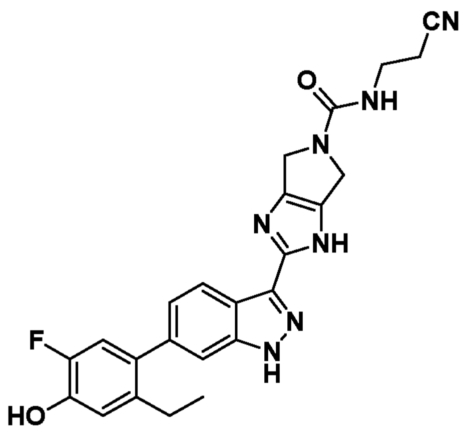

**MDI-258**

It was synthesised according to the method shown in Example 53 of Chinese invention patent CN111606908B, and the NMR hydrogen spectral data of the resulting product were as follows:

$^1$H NMR (400 MHz, MeOD) δ 8.25 (d, *J* = 8.4 Hz, 1H), 7.41 (s, 1H), 7.16 (d, *J* = 8.4 Hz, 1H), 6.91 (dd, *J* = 20.8, 10.3 Hz, 2H), 4.61 - 4.54 (m, 4H), 3.55 - 3.50 (m, 2H), 2.66 - 2.51 (m, 4H), 1.06 (t, *J* = 7.5 Hz, 3H).

**Example 54: N-cyclopropyl-2-(6-(2-ethyl-5-fluoro-4-hydroxyphenyl)-1H-indazol-3-yl)-4,6-dihydropyrrolo[3,4-d] imidazol-5(1H) -carboxamide(MDI-259)**

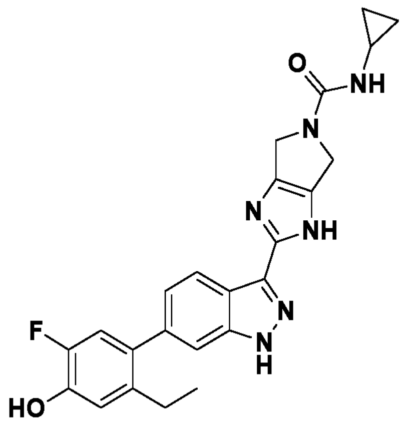

**MDI-259**

It was synthesised according to the method shown in Example 54 of Chinese invention patent CN111606908B, and the NMR hydrogen spectral data of the resulting product were as follows:

$^1H$ NMR (400 MHz, MeOD) δ 8.25 (d, *J* = 8.4 Hz, 1H), 7.43 (s, 1H), 7.16 (dd, *J* = 8.4, 1.4 Hz, 1H), 6.91 (dd, *J* = 20.6, 10.4 Hz, 2H), 4.66 - 4.48 (m, 4H), 2.68 - 2.62 (m, 1H), 2.59 - 2.53 (m, 2H), 1.08 (t, *J* = 7.5 Hz, 3H), 0.76 - 0.71 (m, 2H), 0.60 - 0.56 (m, 2H).

**Example 55: N-cyclobutyl-2-(6-(2-ethyl-5-fluoro-4-hydroxyphenyl)-1H-indazol-3-yl)-4,6-dihydropyrrolo[3,4-d]imidazol-5(1H)-carboxamide (MDI-260)**

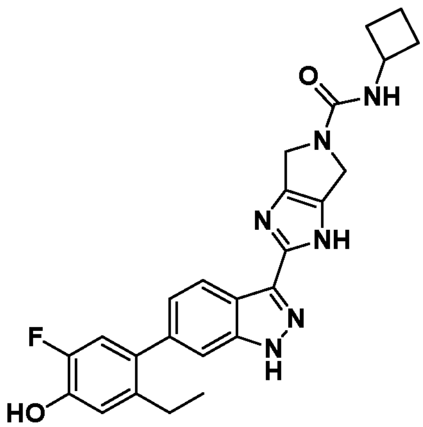

**MDI-260**

It was synthesised according to the method shown in Example 55 of Chinese invention patent CN111606908B, and the NMR hydrogen spectral data of the resulting product were as follows:

$^1H$ NMR (400 MHz, MeOD) δ 8.27 (d, J = 8.4 Hz, 1H), 7.43 (s, 1H), 7.18 (d, J = 8.4 Hz, 1H), 7.01-6.85 (m, 2H), 4.57 (s, 4H), 4.35-4.31 (m, 1H), 2.59-2.53 (m, 2H), 2.36-2.30 (m, 2H), 2.11-2.04 (m, 2H), 1.76-1.69 (m, 2H), 1.08 (t, J = 7.5 Hz, 3H).

**Example 57: (S)-6-(2-ethyl-5-fluoro-4-hydroxyphenyl)-3-(5-prolyl-1,4,5,6-tetrahydropyrrolo[3,4-d]imidazol-2-yl)-1H-indazol (MDI-262)**

It was synthesised according to the method shown in Example 57 of Chinese invention patent CN111606908B, and the NMR hydrogen spectral data of the resulting product were as follows:

$^1H$ NMR (400 MHz, MeOD) δ 8.28 (d, J = 8.0 Hz, 1H), 7.44 (s, 1H), 7.18 (d, J = 8.5 Hz, 1H), 6.96 (d, J = 11.7 Hz, 1H ), 6.91 (d, J = 8.9 Hz, 1H), 4.80-4.64 (m, 4H), 4.09-4.05 (m, 1H), 3.26-3.22 (m, 2H), 2.59-2.53 (m, 2H), 2.06-1.86 (m, 4H), 1.08 (t, J = 8.0 Hz, 3H). LC-MS m/z (ESI) $[M+H]^+$ calculated value for $C_{25}H_{26}FN_6O_2$: 461.2; measured value: 461.2.

**Example 58: (R)-6-(2-ethyl-5-fluoro-4-hydroxyphenyl)-3-(5-prolyl-1,4,5,6-tetrahydropyrrolo[3,4-d]imidazol-2-yl)-1H-indazol(MDI-263)**

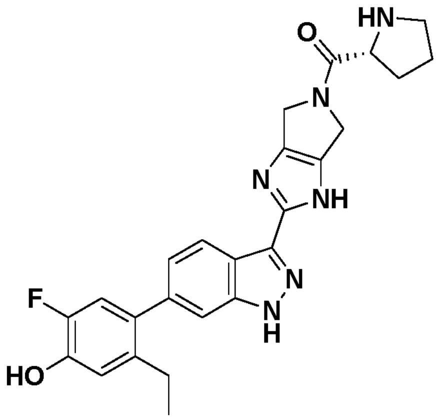

**MDI-263**

It was synthesised according to the method shown in Example 58 of Chinese invention patent CN111606908B, and the NMR hydrogen spectral data of the resulting product were as follows:

$^1$H NMR (400 MHz, MeOD) δ 8.27 (d, J = 8.0 Hz, 1H), 7.44 (s, 1H), 7.18 (d, J = 8.4 Hz, 1H), 6.96 (d, J = 12.2 Hz, 1H ), 6.91 (d, J = 8.8 Hz, 1H), 4.82-4.60 (m, 4H), 4.21-4.15 (m, 1H), 3.33-3.23 (m, 1H), 3.08-2.99 (m, 1H), 2.59-2.53 (m, 2H), 2.08-1.86 (m, 4H), 1.08 (t, J = 8.0 Hz, 3H).

**Example 59: InhibitoryActivity of compounds on TRKA, TRKB and TRKC**

**1. Experimental materials and instruments**

| Item | Name | Source/Supplier | Catalogue No. |
|---|---|---|---|
| 1 | MgCl2 | Sigma | M1028 |
| 2 | ADP-Glo Kinase Assay | Promega | V9101 |
| 3 | DTT | Sigma | D0632 |
| 4 | DMSO | Sigma | D4540-1L |
| 5 | TRKA | Carna | 08-186 |
| 6 | TRKB | Carna | 08-187 |
| 7 | TRKC | Carna | 08-196 |
| 8 | ATP | Promega | V915B |
| 9 | Poly (4:1 Glu, Tyr) Peptide | Signal Chem | P61-58 |
| 10 | LOXO101 | MCE | HY-12866A |
| | | | |
| 11 | 384-Well reaction plate (384-well plate, white, low volume, round-bottom) | Greiner | 784075 |
| 12 | 96-well polypropylene plate | Nunc | 249944 |
| 13 | Microplate Low-Speed Centrifuge | Xiang Zhi | TD5B |
| 14 | Biotek Enzyme Label Reader | Biotek | Synergy 4 |

## 2. Experimental methods

### 2.1 Formulation of 1X Kinase reaction buffer

1 volume of 5X kinase reaction buffer and 4 volumes of water were used together with 5 mM $MgCl_2$; and 1 mM DTT.

### 2.2 Preparation of Kinase and Substrate

**Formulation of 2.5X substrate mixture**

| Kinase | ATP concentration [μM] | Poly (4:1 Glu, Tyr) Peptide[μM] |
|---|---|---|
| TRKA | 5 | 0.03 |
| TRKB | 5 | 0.03 |
| TRKC | 5 | 0.03 |

### 2.3 Test procedure on InhibitoryActivity of compounds

1) Dilute the compound by 4 times with DMSO in a dilution plate, in which the initial concentration of the compound was 1000 nM.

2) Dilute the compound by 50 times with a 1X kinase reaction buffer and incubate it on a shaker for 20 minutes.

3) Formulate 2X TRKA with 1X kinase reaction buffer.

4) Add 2 μl of TRKA kinase (prepared in step 3) to each well of a reaction plate.

5) Add 1 μl of the compound diluted in buffer to each well, seal the plate with a sealing film, centrifuge it at 1000g for 30 seconds, and incubate it at room temperature for 10 minutes.

6) Formulate a 4x ATP&sub mixture with 1X kinase reaction buffer, and add 1 μl of the 4x ATP&sub mixture to the reaction plate.

7) Seal the plate with a sealing film, centrifuge it at 1000g for 30 seconds, and incubate it at room temperature for 60 minutes.

8) Transfer 4 μL of ADP-Glo to the 384-well reaction plate, centrifuge it at 1000 rpm/min for 1 minute, and incubate it at 25°C for 40 minutes.

9) Transfer 8 μL of Detection solution to the 384-well reaction plate, centrifuge it at 1000 rpm/min for 1 min, and incubate it at 25°C for 40 min.

10) Use Biotek multi-function plate reader to read RLU (Relative luminescence unit) signal. The signal intensity is used to characterize the degree of kinase activity.

## 3. Data analysis

3.1 Calculate inhibition percentage as follows.

Inhibition percentage of compound (% inh) = 100-(compound-positive control) / (negative control-positive control) * 100%

Negative control: DMSO

Positive control: LOXO101

3.2 Calculate IC50 and plot the inhibition curve for the compound:

IC50 (half inhibitory concentration) of the compound can be obtained using the following nonlinear fitting formula, in which data analysis was conducted using GraphPad 6.0 software.

$$Y=Bottom + (Top-Bottom)/(1+10^((LogIC50-X)*HillSlope))$$

X: log value of the compound concentration

Y: inhibition percentage (% inh)

3.3 Quality Control

After one experimenter has organized the data, another experimenter conducts a re-examination to ensure accuracy of the data analysis.

The experimental data must meet the following criteria: Z-factor > 0.5; S/B > 2; and the IC50 of the positive control within 3 times the average of historical values.

**4. Activity test results of compounds**

| Compounds | IC50(nM) | | |
|---|---|---|---|
| | TRKA | TRKB | TRKC |
| LOXO101(Positive Control) | 0.42 | 1.16 | 0.48 |
| MDI-201 | 5.76 | 6.14 | 2.20 |
| MDI-218 | 9.97 | 32.29 | 5.10 |
| MDI-235 | 0.57 | 1.39 | 0.27 |
| MDI-245 | 0.83 | 3.37 | 0.57 |
| MDI-253 | 11.38 | 98.83 | 13.14 |
| MDI-234 | 1.07 | 1.27 | 0.65 |
| MDI-248 | 12.02 | 3.58 | 3.77 |
| MDI-1233 | 12.17 | 4.16 | 5.91 |
| MDI-1228 | 1.82 | 1.77 | 0.60 |
| MDI-231 | 2.82 | 5.86 | 1.49 |
| MDI-233 | 9.01 | 30.44 | 6.32 |
| MDI-236 | 0.23 | 1.06 | 0.20 |
| MDI-247 | 10.35 | 34.24 | 3.73 |
| MDI-257 | 2.21 | 2.93 | 0.72 |
| MDI-1288 | 0.89 | 1.04 | 0.59 |

**Example 60: InhibitoryActivity of compounds on RET kinse**

**1. Experimental materials and instruments**

| Item | Name | Source/Supplier | Catalogue No. |
|---|---|---|---|
| 1 | HTRF KinEASE-TK kit | Cisbio | 62TK0PEC |
| 2 | RET | Carna | 08-159 |
| 3 | MgCl2 | Sigma | M1028 |
| 4 | ATP | Promega | V910B |
| 5 | DTT | Solarbio | 3483-12-3 |
| 6 | DMSO | Sigma | D4540-1L |
| 7 | Pralsetinib | MCE | HY-112301 |
| | | | |
| 8 | 384-Well reaction plate (384-well plate, white, low volume, round-bottom) | Greiner | 784075 |
| 9 | 96-well polypropylene plate | Nunc | 249944 |
| 10 | Microplate Low-Speed Centrifuge | Xiang Zhi | TD5B |
| 11 | Biotek Enzyme Label Reader | Biotek | Synergy 4 |

**2. Experimental methods**

**2.1 Formulation of 1X Kinase reaction buffer**

1 volume of 5X kinase reaction buffer and 4 volumes of water were used together with 5 mM $MgCl_2$; and 1 mM DTT.

2.2 Reaction condition

| **Kinase** | **ATP concentration [μM]** | **Substrate TK [μM]** |
|---|---|---|
| RET | 16 | 1 |

**2.3 Test procedure on InhibitoryActivity of compounds**

1) Dilute the compound by 4 times with DMSO in a dilution plate, in which the initial concentration of the compound was 1 μM.

2) Dilute the compound by 20 times with a 1X kinase reaction buffer and incubate it on a shaker for 20 minutes.

3) Formulate 2.5X kinase with 1X kinase reaction buffer.

4) Add 2 μl of kinase (prepared in step 3) to each well of a reaction plate.

5) Add 1 μl of the compound diluted in buffer to each well, seal the plate with a sealing film, centrifuge it at 1000g for 30 seconds, and incubate it at room temperature for 10 minutes.

6) Formulate a 2.5x Tk-substrate-biotin&ATP mixture with 1X kinase reaction buffer, and add 2 μl of the Tk-substrate-biotin/ATP mixture to the reaction plate.

7) Seal the plate with a sealing film, centrifuge it at 1000g for 30 seconds, and incubate it at room temperature for 60 minutes.

8) Formulate 5X Sa-XL 665 (250nM) with HTRF dection buffer.

9) Add 5 μl of XL665 and 5 μl of Tk-antibody-Cryptate to each well, centrifuge it at 1000g for 30 seconds, and incubate it at room temperature for 1 hour.

10) Use Biotek to read the fluorescence signals at 615 nm (Cryptate) and 665 nm (XL665).

**3. Data analysis**

3.1 Calculate the ratio (Ratio _665/615 nm) at each well and calculate inhibition percentage as follows.

Inhibition percentage of compound (% inh) = 100-(compound-positive control) / (negative control-positive control) * 100%

Positive control: Pralsetinib 1000 nM

Negative control: 0.5% DMSO

3.2 Calculate IC50 and plot the inhibition curve for the compound:

IC50 (half inhibitory concentration) of the compound can be obtained using the following nonlinear fitting formula, in which data analysis was conducted using GraphPad 6.0 software.

Y=Bottom + (Top-Bottom)/(1+10^((LogIC50-X)*HillSlope))

X: log value of the compound concentration

Y: inhibition percentage (% inh)

3.3 Quality Control

After one experimenter has organized the data, another experimenter conducts a re-examination to ensure accuracy of the data analysis.

The experimental data must meet the following criteria: Z-factor > 0.5; S/B > 2; and the IC50 of the positive control within 3 times the average of historical values.

**3. Activity test results of compounds**

| Compounds | RET IC50(nM) |
|---|---|
| **MDI-235** | 0.10 |
| **MDI-245** | 0.99 |
| **MDI-1233** | 4.21 |
| **MDI-1228** | 0.62 |
| **MDI-247** | 8.36 |

(continued)

| Compounds | RET IC50(nM) |
|---|---|
| **MDI-1288** | 3.43 |
| **Pralsetinib(Positive control)** | 0.14 |

**Example 61: Therapeutic Efficacy of Compounds on Ovalbumin-Induced Asthma Model in mice**

**I. Materials and Methods**

Experimental Materials and Reagents

Ovalbumin (OVA): Sigma- A5243
Aluminum adjuvant: Sigma-239186
Diff Quick: Sinopharm Group, 100092680
Hematoxylin: Hongquan Biology, HQ60002
Eosin: Hongquan Biology, HQ60001
Interleukin 5 (IL-5) Elisa kit: Beyotime-DRE30011
IgE Elisa kit: Beyotime-DRE30653
Phosphate buffered saline (PBS): Solarbio-P1022
TWEEN 80: Solarbio-LA7760

Experimental Instruments

Electronic balance: Wuxin Weighing Apparatus, Model MAX-A3003

Enzyme Label Reader: Finland (Labsystems Multiskan MS), Model 352

Washer: Finland (Thermo Labsystems), Model AC8

Centrifuge: Micro-high speed centrifuge, Model TGL-16

Pipette: Gilson P-type pipette (Pipetman), Model F123601

Incubator: Water-jacketed constant temperature incubator, Model BPN-190RHP

Microscope: OLYMPUS, Model CX41

Constant temperature oven: Shanghai Hengyi Scientific Instruments, Model DHG-9140

Paraffin section machine: Leica, Model SQ2125

Spreading machine: Leica, Model PPTHK-21B

Water bath: Leica, Model HI1210

Nebulizer: Jiangsu Yuwell Medical, Model 403M

Experimental Animals

7-8 week-old female Balb/c mice were purchased from Changzhou Cavens Laboratory Animal Co., Ltd., with animal qualification certificate number: 202005500.

Mice were housed in the animal facility of the Shanghai Institute of Materia Medica, SPF level, with a temperature

of 22-24°C, a humidity of 40%-70%, lighting from 7:00-19:00, and 3 mice per cage. Food and water were provided ad libitum.

Animal Grouping

After 7 days of acclimatization, animals were randomly grouped based on their body weight. The experiment was divided into 8 groups, with 8 mice in each group, as detailed in the table below.

| Group | Compound | quantity (head) | Administration volume | Administration dose | Administration route | Administration frequency and days |
|---|---|---|---|---|---|---|
| | | | ml/head | mg/kg | | |
| 1 | Saline | 6 | NA | NA | NA | NA |
| 2 | OVA+Vehicle | 6 | 0.1 | NA | nasal administration | BID, 5 |
| 3 | OVA + Dexamethasone | 6 | 10 ml/kg | 1 | nasal administration | QD, 5 |
| 4 | OVA +MDI-1228 | 6 | 50 ul | 3 | nasal administration | BID, 5 |
| 5 | OVA +MDI-1288 | 6 | 50 ul | 3 | nasal administration | BID, 5 |
| 6 | OVA +MDI-1233 | 6 | 50 ul | 1 | nasal administration | BID, 5 |
| 7 | OVA +MDI-1233 | 6 | 50 ul | 3 | nasal administration | BID, 5 |
| 8 | OVA +MDI-1233 | 6 | 50 ul | 10 | nasal administration | BID, 5 |
| [a]: 0.2% Tween 80 in normal saline.BID: Twice a day.QD: Once a day. NA: Not applicable or not available. | | | | | | |

Reagent Preparation

- Preparation of Immunogen: Weigh an appropriate amount of OVA, to which an appropriate amount of PBS was added, to achieve a final concentration of 0.1 mg/ml. Then, an equal volume of aluminum adjuvant was added, and the resulting mixture was mixed well, and stored at 4°C, which was prepared before use.
- Preparation of Sensitizer (5% OVA): Weigh an appropriate amount of OVA, to which an appropriate amount of PBS was added, to achieve a final concentration of 5%. The resulting mixture was mixed well, and store it at 4°C for later use, which was prepared before use.
- Preparation of Vehicle: Add 200 μl of Tween 80 to 100 ml of saline, and mix it well. Store it at 4°C for later use.
- Preparation of Compound MDI-1288 solution: Weigh an appropriate amount of MDI-1288 and vehicle, and dissolve MDI-1288 in the vehicle to achieve a final concentration of 1.2 mg/ml. Mix it well, and store it at 4°C for later use. The solution was prepared once every three days.
- Preparation of Compound MDI-1228 solution: Weigh an appropriate amount of MDI-1228 and the vehicle, and dissolve MDI-1228 in the vehicle to achieve a final concentration of 1.2 mg/ml. Mix it well, and store it at 4°C for later use. The solution was prepared once every three days.
- Preparation of Compound MDI-1233 solution: Weigh an appropriate amount of MDI-1233 and vehicle, and dissolve MDI-1233 in the vehicle to achieve a final concentration of 0.4, 1.2 and 4 mg/ml, respectively. Mix it well, and store it at 4°C for later use. The solution was prepared once every three days.

Modeling

- **Immunization:** On days 0, 7, 14, and 21, 200 μl of PBS was injected intraperitoneally for the first group, and 200 μl of (10 μg) OVA/aluminum adjuvant emulsion was injected intraperitoneally for groups 2-8.
- **Sensitization:** From days 28 to 32, the first group of animals was exposed to nebulized PBS in a nebulization box for 20 minutes, and the groups 2-8 were exposed to nebulized 5% OVA in a nebulization box for 20 minutes. And then, they were removed. The sensitization was conducted at 12:30 PM daily.
- **Administration:** The medicine was administrated twice a day at 9 AM and 5 PM via nasal drops. Dexamethasone (Dex) was administered by gavage once a day at 9 AM.

Evaluation Indicators:

**Serum IgE:** Blood was collected from the orbits of animals after carbon dioxide euthanasia, and plasma was separated and stored at -40 degrees for plasma IgE detection.

**Bronchoalveolar Lavage Fluid (BALF) Cell Count:** An appropriate amount of foetal bovine serum was weighted and dissolved in PBS to a final concentration of 1%. After orbital blood collection, the trachea was exposed, and a 1 ml syringe needle was replaced with a mouse gavage needle. 0.8 ml of the lavage fluid was aspirated from the syringe and the lungs were lavaged three times. The lavage fluids were collected, and centrifuged at 1000 rpm for 10 minutes, and the supernatant was collected and stored at -40 degrees Celsius. Cells were resuspended with 1 ml of PBS and counted. 100 ul of centrifuged smear was taken and stained with Diff quick and the number of eosinophils, basophils, lymphocytes and macrophages were recorded.

**Bronchoalveolar Lavage Fluid IL-5:** Bronchoalveolar lavage fluid was collected to detect IL-5.

**Lung histopathology:** Formaldehyde-fixed lung tissue was taken and embedded in sections after 24 hours, followed by HE staining, observation of inflammatory cell infiltration and scoring. Pathological scoring criteria were:

A) 0 points: No lymphocyte infiltration;

B) 1 point: Trace lymphocyte infiltration;

C) 2 points: Mild lymphocyte infiltration;

D) 3 points: Moderate lymphocyte infiltration;

E) 4 points: Severe lymphocyte infiltration;

After scoring, the results were calculated and analyzed.

**II. Experimental Results**

Bronchoalveolar Lavage Fluid IL-5:

The experimental results were shown in FIG. 1. Bronchoalveolar lavage fluid IL-5 expression level was lower in normal group. Compared to the normal group, the bronchoalveolar lavage fluid IL-5 expression level was significantly higher in the vehicle group ($p<0.001$). Compared to the model group (i.e., the vehicle group), the positive control dexamethasone significantly reduced the expression of IL-5 ($p<0.001$); compounds MDI-1228 and MDI-1288 also significantly reduced the expression of IL-5 ($p<0.001$), but were weaker than the positive control. Compared to the model group, compound MDI-1233 could reduce IL-5 expression in all three dosage, and there was a significant difference between the 3 mpk and 10 mpk groups ($p<0.001$). Compared to compounds MDI-1228 and MDI-1288, compound MDI-1233 at 3 mpk had a similar effect on reducing IL-5 expression; compared to the positive control dexamethasone, compound MDI-1233 at 10 mpk had a similar effect on reducing IL-5 expression. The IL-5 assay data of bronchoalveolar lavage fluid showed that MDI-1233 could reduce the expression level of IL-5 in each dosing group with a dose-dependent effect.

Inflammatory Cell Count in Bronchoalveolar Lavage Fluid:

The experimental results were shown in FIG. 2. Inflammatory Cell Count in Bronchoalveolar Lavage Fluid was lower in normal group. Compared to the normal group, the Inflammatory Cell Count in Bronchoalveolar Lavage Fluid was significantly higher in the vehicle group ($p<0.001$). Compared to the model group (i.e., the vehicle group), the positive control dexamethasone significantly reduced the Inflammatory Cell Count ($p<0.001$); compounds MDI-1228 and MDI-1288 also significantly reduced the Inflammatory Cell Count ($p<0.001$), but were weaker than the positive control. Compared to the model group, compound MDI-1233 could reduce the Inflammatory Cell Count in all three dosage, and there was a significant difference between the 3 mpk and 10 mpk groups ($p<0.001$). Compared to compounds MDI-1228 and MDI-1288, compound MDI-1233 at 3 mpk had a similar effect on reducing the Inflammatory Cell Count; compared to the positive control dexamethasone, compound MDI-1233 at 10 mpk had a similar effect on reducing the Inflammatory Cell Count. The Inflammatory Cell Count assay data of bronchoalveolar lavage fluid showed that MDI-1233 could reduce the Inflammatory Cell Count in each dosing group with a dose-dependent effect.

Serum IgE:

The experimental results were shown in FIG. 3. Serum IgE expression level was lower in normal group. Compared to the normal group, the serum IgE expression level was significantly higher in the vehicle group ($p<0.001$). Compared to the model group (i.e., the vehicle group), the positive control dexamethasone significantly reduced the expression of IgE ($p<0.001$); compounds MDI-1228 and MDI-1288 also significantly reduced the expression of IgE ($p<0.001$), but were weaker than the positive control. Compared to the model group, compound MDI-1233 could reduce IgE expression in all three dosage, and there was a significant difference between the 3 mpk and 10 mpk groups ($p<0.001$). Compared to compounds MDI-1228 and MDI-1288, compound MDI-1233 at 3 mpk had a similar effect on reducing IgE expression; compared to the positive control dexamethasone, compound MDI-1233 at 10 mpk had a similar effect on reducing IgE expression. The serum IgE assay data showed that MDI-1233 could reduce the expression level of IgE in each dosing group with a dose-dependent effect.

Pathological Examination:

The experimental results were shown in FIG. 4. There was no abnormality in pathological staining of animals in the normal group. Compared to the normal group, the lung tissue inflammatory cell infiltration was significantly higher in the vehicle group ($p<0.001$). Compared to the model group (i.e., the vehicle group), the positive control dexamethasone significantly reduced the lung tissue inflammatory cell infiltration ($p<0.001$); compounds MDI-1228 and MDI-1288 also significantly reduced the lung tissue inflammatory cell infiltration ($p<0.001$), but were weaker than the positive control. Compared to the model group, compound MDI-1233 could reduce the lung tissue inflammatory cell infiltration in all three dosage, and there was a significant difference between the 3 mpk and 10 mpk groups ($p<0.001$). Compared to compounds MDI-1228 and MDI-1288, compound MDI-1233 at 3 mpk had a similar effect on reducing the lung tissue inflammatory cell infiltration; and compared to the positive control dexamethasone, compound MDI-1233 at 10 mpk had a similar effect on reducing the lung tissue inflammatory cell infiltration. The pathological assay data showed that MDI-1233 could reduce the lung tissue inflammatory cell infiltration in each dosing group with a dose-dependent effect.

The results of lung tissue pathological scoring were shown in FIG. 5. The results indicated that the compound MDI-1233 had a good therapeutic effect on the asthma animal model with a dose-dependent effect; and MDI-1228 and MDI-1288 also had a certain therapeutic effect when compared.

## Example 62: Inhibitory Effect of Compounds to be tested on Allergic Rhinitis in Mice

### I. Materials and Methods

Primary reagents

(1) Preparation of 0.2% Tween 80: Take 1ml of TW80 to which 499 ml saline was added.

(2) Preparation of 0.05 mg/ml ovalbumin suspension (sensitizing):

1). Take 10 mg of OVA to which 10 ml saline was added.

2). To 2 ml of the above 10 mg/10 ml ovalbumin solution, 38 ml saline was added , and shaken well, and then 200 mg aluminum hydroxide was added.

(3) Preparation of MDI-1233:

1) For 1 mg/kg MDI-1233: C = (1 mg/kg × 25 g × $10^{-3}$ kg) ÷ (20 × $10^{-3}$ ml) = 1.25 mg/ml, 0.3 ml of 10 mg/kg MDI-1233 suspension was taken, to which 2.7 ml of 0.2% TW80 was added, and shaken well.

2) For 3 mg/kg MDI-1233: C = (3 mg/kg × 25 g ×$10^{-3}$ kg) ÷ (20 ×$10^{-3}$ ml) = 3.75 mg/ml, 0.9 ml of 10 mg/kg MDI-1233 suspension was taken, to which 2.1 ml of 0.2% TW80 was added, and shaken well.

3) For 10 mg/kg MDI-1233: C = (10 mg/kg × 25 g ×$10^{-3}$ kg) ÷ (20 ×$10^{-3}$ ml) = 12.5 mg/ml, 56.25 mg MDI-1233 was taken, to which 4.5 ml of 0.2% TW80 was added, and shaken well.

(4) Preparation of MDI-1228:

$$C = (3 \text{ mg/kg} \times 25 \text{ g} \times 10^{-3} \text{ kg}) \div (20 \times 10^{-3} \text{ ml}) = 3.75 \text{ mg/ml}.$$

11.25 mg MDI-1228 was taken, to which 3.54 ml saline was added, with shaking well.

(5) Preparation of MDI-1288:

$$C = (3 \text{ mg/kg} \times 25 \text{ g} \times 10^{-3} \text{ kg}) \div (20 \times 10^{-3} \text{ ml}) = 3.75 \text{ mg/ml}.$$

11.2 mg of MDI-1288 was taken, to which 2.98 ml of saline was added, then shaken well.

(6) Preparation of 10% OVA: 12 mg of OVA was taken, to which 1.2 ml of saline was added, and then shaken well.

Instruments

| Experimental instruments | Model | Supplier |
|---|---|---|
| Pipette | 100-1000ul | eppendorf |
| Pipette | 20-200ul | eppendorf |
| Pipette | 5-50ul | eppendorf |
| Automatic Microplate Reader | WD-2102B | Beijing Liuyi Biotech |
| Refrigerator/Freezer | BD/BC-415DKEM | Midea |
| Electric Heated Constant Temperature Incubator | DHP-9054 | Shandong Bok Biological Industry Co., Ltd. |
| Pharmaceutical Refrigerator | BYC-310 | Shandong Bok Biotech |
| Microscope | BX43 | OLYMPUS |
| Slicer | 2235 | Leica |
| Electric Heated Constant Temperature Blast Drying Oven | HGZF-101-1 | Shanghai Yuejin Medical Instrument Co., Ltd. |

Animals

42-49 day-old BALB/C mice (Manufacturer: Liaoning Changsheng Biotechnology Co., Ltd., License Number: SCXK(therapeutic)2015-0001), both male and female, were reared under conventional diet, at a temperature of 20 to 26°C and a humidity of 40% to 70%. Animal grouping and modeling:

1. Blank control group (no treatment)

2. Allergic rhinitis group (vehicle treatment: saline containing 0.2% Tween 80)

3. Allergic rhinitis + compound MDI-1233: 1 mg/kg

4. Allergic rhinitis + compound MDI-1233: 3 mg/kg

5. Allergic rhinitis + compound MDI-1233: 10 mg/kg

6. Allergic rhinitis + compound MDI-1228: 3 mg/kg

7. Allergic rhinitis + compound MDI-1288: 3 mg/kg

8. Allergic rhinitis + positive control group (Fluticasone propionate, GlaxoSmithKline, E88D

The preparation of an AR model in mice involved sensitizing and challenging BALB/c mice with ovalbumin (OVA) to simulate an acute symptoms caused by natural exposure to an allergen. The experimental animals were weighed and separated by gender. Sensitization was performed on days 1, 5, 9, and 12. From day 13, a daily challenge was carried out for 7 consecutive days. After the last challenge, behavior of the mice was observed and scored. The sensitization method involved intraperitoneal injection of an OVA (Sigma, A5503-1G) suspension in 50 μg/ml in which the OVA suspension was prepared by dissolving 500 μg of ovalbumin and 50 mg of AL(OH)3 (CAS 21645-51-2) into 10 ml of normal saline, pH 7.4, with 200 μl injected per mouse. The challenge method involved preparing a normal saline solution of OVA (10 mg/ml) at pH 7.4, then using a microsyringe to administer 10 μl into each nostril of the mice, and observing the number of times the mice scratched their nose, sneezed, and had a runny nose within 30 minutes. On day 12, a single nasal challenge with 10% OVA was performed (PBS was used for the control group). Scoring was conducted according to the table below, using a superposition method to calculate the total score. A total score greater than 5 was considered as successful. Subsequent experiments were conducted after the modeling was deemed successful.

| Score | Sneezing (Times) | Runny Nose | Nasal Itching and Scratching |
|---|---|---|---|
| 1 point (Mild) | 1-3 | Small amount of nasal discharge | Mild forepaw scratching (less than 10 times) |
| 2 points (Moderate) | 4-10 | Runny nose exceeding the front nostrils | Intermediate between mild and severe |
| 3 points (Severe) | ≥11 | Runny nose covering the face | Rubbing all around |

Administration Method

From day 13, compound administration and challenge were conducted. The compound administration method involved calculating the nasal drops volume according to the mouse's body weight and group requirements, and dissolving the compounds MDI-1233, MDI-1228, MDI-1288 into normal saline containing 0.2% Tween 80 at specific concentrations. The administration was given 30 minutes before the challenge, twice a day. The positive control treatment involved administering nasal drop once (10 μl per side) 30 minutes before OVA nasal stimulation, twice a day. Thirty minutes after the second drug administration, a 10% OVA challenge was conducted, and the amount of nasal discharge, sneezing, and nose scratching were observed and recorded within 30 minutes after each challenge and then scored. The control group was treated with normal saline nasal drops.

Sample Collection

After the rhinitis modeling was completed and the scores were stable, mice of each group were anesthetized and sacrificed after a 20-day scoring. Samples were collected, and peripheral serum was obtained by collecting whole blood, and standing it at room temperature for 1 hour and then centrifuging it to collect the supernatant for ELISA detection; nasal mucosa was taken for HE staining (fixed with 10% formalin); and the supernatant of nasal lavage fluid was taken for ELISA detection.

Pathological Examination

(1) **HE Staining:** The tissue was rinsed with running water for several hours, dehydrated through a series of ethanol solutions (70%, 80%, 90%), and then treated with a mixture of pure alcohol and xylene for 15 minutes, followed by xylene I for 15 minutes and xylene II for 15 minutes (until transparent). The tissue was then placed in a mixture of xylene and paraffin at a volume ratio of 1:1 for 15 minutes, followed by immersion with paraffin I and paraffin II each for 50-60 minutes. The tissue was embedded in paraffin and sectioned, and the sections were spreaded, dried, dewaxed and hydrated in distilled water. The resulting sections were then stained in hematoxylin solution for 3 minutes, differentiated in a hydrochloric acid alcohol solution for 15 seconds, slightly washed, blued in a return blue solution for 15 seconds, rinsed with running water, stained with eosin for 3 minutes, rinsed with running water, dehydrated, cleared, and mounted for microscopic examination.

(2) **ELISA detection:** A double-antibody one-step sandwich Enzyme-linked Immunosorbent Assay (ELISA) kit was used. A capture antibody for the mouse target indicators was pre-coated onto microplate wells, to which samples, standards, and HRP-labeled detection antibodies were added sequentially, followed by incubation and thorough washing. TMB substrate was added for color development, which turned into blue in the presence of peroxidase and then into yellow after acidification. The depth of color was positively correlated with the concentration of the target in the samples. The absorbance (OD450 value) was measured at a wavelength of 450 nm using the microplate reader, and the sample concentration was calculated.

**II. Experimental Results**

Animal Modeling Results

The animal modeling was divided into a pilot study and a formal experiment. The pilot study adopted behavioral observations and scoring after OVA nasal challenge to calculate the positive rate and HE staining of pathological sections was used to determine the success of the rhinitis model. A total of 10 mice were used in the pilot study, with a positive rate of only 30% for a 15-day nasal challenge and with a positive rates of 90% and 80% for a 19-day nasal challenge and a 20-day nasal challenge, respectively.

To verify the reliability of the modeling, the nasal mucosa of the positively scored mice was subjected to pathological sectioning and HE staining. The results showed that the nasal mucosa cilia in the blank control group were dense, while in the model group, the nasal mucosal cilia had disappeared, with evident inflammation.

Results of Drug Intervention in the Rhinitis Model

The formal experiment was divided into a total of eight groups. Aapart from the control group, all other groups were subjected to rhinitis modeling. The doses of compound MDI-1233 were 1 mg/kg, 3 mg/kg, and 10 mg/kg, compound MDI-1228 was administered at a dose of 3 mg/kg, compound MDI-1288 was administrated at a dose of 3 mg/kg, and the positive control drug was fluticasone propionate. After a 7-day treatment, the behavior of the animals was scored and statistically analyzed at the planned time points (FIGs. 6 and 7). In male mice, the positive rate of rhinitis scores showed a decreasing trend after intervention with the compounds and the positive rate of rhinitis was significantly lower than that of the model group ($p<0.05$). In female mice, the positive rate of rhinitis also significantly decreased after intervention with compounds ($p<0.05$). Statistical analysis indicated that both the compound intervention groups and the positive control group showed significant effects on inhibiting the rhinitis behavior in the experimental animals. The comprehensive results showed that MDI-1233 at a dose of 10 mg/kg demonstrated inhibitory effects on the male mouse rhinitis model, as evidenced by behavioral scoring analysis; compound MDI-1228 did not significantly inhibit the allergic response in male mice; MDI-1288 at a dose of 3 mg/kg showed inhibitory effects on both male and female mouse rhinitis models, as proven by behavioral scoring analysis.

Pathological Section Analysis

After intervention with compounds, all groups were sampled and sectioned. Following HE staining, the pathological changes in the nasal mucosa were analyzed by observing and recording random fields, with the results shown in FIG. 8. The results indicated that the nasal mucosa structure in both male and female blank control groups was orderly, with dense cilia, prominent goblet cells, and no obvious inflammatory cell infiltration (FIG. 8A, 8H). In the rhinitis group, the cilia disappeared, the nasal mucosa structure was disordered, and the submucosal tissue was accompanied by edema and bleeding symptoms, with a large number of inflammatory cell infiltration (FIG. 8B, 8I). In the MDI-1288 intervention group, the nasal mucosa structure was basically normal, the cilia structure was evident, some of samples still showed nasal submucosal bleeding and cilia shedding, goblet cells were prominent, and the number of inflammatory cells was reduced (FIG. 8C, 8J). In the MDI-1233 treatment groups with concentrations of 1 mg/kg and 3 mg/kg, the nasal

submucosa was still accompanied by bleeding, inflammatory cell infiltration, cilia shedding, and no prominent goblet cells, and edema symptoms were improved (FIG. 8D, 8E, 8K, and 8L). In the MDI-1233 treatment group at10 mg/kg, the nasal mucosa structure was basically clear, some cilia were clustered or inverted, there was still some cilia shedding, edema symptoms were significantly improved, goblet cells were present, accompanied by inflammatory cell infiltration (FIG. 8F, 8M). In the positive control group, the nasal submucosal was accompanied by minimal bleeding, edema symptoms were not obvious, cilia were clustered with minimal loss, goblet cells were prominent, and there was still inflammatory cell infiltration (FIG. 8G, 8N). Overall, the pathological section results indicated that the rhinitis group in both male and female mice met the pathological criteria, mainly manifested by the cilia shedding of nasal mucosal and inflammatory cell infiltration. After intervention with compounds, the pathological symptoms of the nasal mucosa gradually improved. The nasal mucosa structure was basically normal, the cilia structure was evident, some of samples still were accompanied by nasal submucosal bleeding and cilia shedding, goblet cells were prominent, and the number of inflammatory cells was reduced. Fluticasone propionate as a positive control medicine also inhibited the progression of rhinitis and promoted repair of cilia on the nasal mucosa. From the pathological section characteristics, the nasal mucosal cilia repair effect was significant in the MDI-1233 pre-treatment group at 10 mg/kg, with clear cilia, but there was still inflammatory cell infiltration. However, after MDI-1288 intervention in the rhinitis model, its therapeutic effect was similar to that of the positive control drug. The positive control drug fluticasone propionate also inhibited the progression of rhinitis and promoted the repair of cilia on the nasal mucosa.

ELISA Detection

While the nasal mucosa and cilia damage repair in each group of experimental animals were detected by the pathological sectioning, ELISA was further used to test the serum and nasal lavage fluid of the tested animals. The main indicators tested were the inflammatory factors IL-4 and IL-17, type II interferon IFN-$\gamma$, and immunoglobulin IgE. In the serum, the changes in IL-4 expression level showed a consistent trend in both the male and female groups (FIGs. 9, 10). In the rhinitis model group, the IL-4 level in the serum was approximately twice as high as that in the blank control group. After treatment with the positive control drug, the level returned to a level similar to that of the blank group. In the compound treatment groups, there was a significant decrease in the IL-4 cotnent in the serum, showing a drug dose-dependent trend.

## Example 63: Treatment Effect of Compounds to be tested on a chronic obstructive pulmonary disease model in rats

### I. Materials and Methods

Animals

Healthy male SD rats at 6 weeks of age (Manufacturer: Hunan Slake Jingda Co., Ltd., License Number: SCXK(Xiang)2019-0004) half male half female, were reared under conventional diet, at a temperature of 20 to 26°C and a humidity of 40% to 70%.

Animal grouping and modeling:

(1) Blank control group (7 rats)

(2) COPD group + vehicle group (Vehicle: 0.2% Tween 80/saline buffer) (8 rats)

(3) COPD + positive control drug (Tiotropium Bromide) group (8 rats)

(4) COPD + compound MDI-1233 (1mg/kg) dose group (8 rats)

(5) COPD + compound MDI-1233 (3mg/kg) dose group (8 rats)

(6) COPD + compound MDI-1233 (10mg/kg) dose group (8 rats)

**Experimental environment:** Temperature 20°C-26°C, humidity 40%-70%

The rats were acclimated for 7 days, and a chronic obstructive pulmonary disease (COPD) model was created

and treated with different drugs.

**Modeling:** On days 1 and 14, the rats were anesthetized with 10% chloral hydrate via intraperitoneal injection, and lipopolysaccharide (LPS) of 200 μg/200 μL was instilled into the trachea through tracheal intubation. After the procedure, the rats were rotated upright for 10-20 seconds to ensure uniform distribution of LPS in their lungs. From days 2 to 13 and 15 to 28, the rats were exposed to cigarette smoke for 30 minutes daily in a homemade organic glass sealed smoking chamber (80 cm×60 cm×50 cm), with 15 cigarettes per day.

Anmial Administration

After a 29-day smoking exposure, nasal drops were consecutively administered to each group, twice a day, for a total of 30 days.

Method: The blank control group (untreated) received no treatment; the COPD group (saline) received saline nasal drops after modeling; the compound MDI-1233 groups received the corresponding dose of the compound after modeling; the positive control drug group received 0.1 mg/kg/d dose via nasal drops, twice a day.

During this period, the rats were weighted for their body weight once a week, and general conditions such as diet, cough, and activity level were observed.

Animal Sampling

After the treatment, the rats were anesthetized with 10% chloral hydrate via intraperitoneal injection. Their chest was then opened to expose trachea and lungs. Approximately 5 mL of blood was drawn from the heart, and centrifuged at 3500 r/min for 10 minutes, and the serum was collected and stored at -20 °C. The right main bronchus was ligated, and a trocar needle was used to puncture the left lung through the carina. The left lung was lavaged with 2 mL of normal saline, and slowly aspirated back, collecting about 1.5 mL of fluid each time, which lavage process was repeated three times. The lavage fluid was mixed and then centrifuged at 4 °C, 1000 r/min for 10 minutes. The supernatant was collected and stored at -20 °C. The middle lobe of the right lung was immersed in 4% paraformaldehyde solution for fixation for 72 hours, and embedded in conventional paraffin. HE staining was used to observe the pathological conditions of the rat lungs in each group.

HE Staining:

Tissue samples from animal lungs were excised, fixed in 10% neutral formalin buffer solution, dehydrated, embedded in paraffin, and sectioned and HE stained for observation of histopathological morphology changes. The reagents and instruments required for HE staining included: Hematoxylin staining solution (AR11800-1, BOSTER); Eosin staining solution (AR11800-2, BOSTER); Ultra-clean advanced mounting medium (YZB, BASO); Scott's blueing solution (G1865, Solarbio), etc. Medication refrigerator (BYC-310, Shandong Bokai Biotech); Microscope (CX41 OLYMPUS); Microtome (BQ-318D, Berne); Electric heating blast drying oven (DHG-9070A, Hengke Scientific Instrument Co., Ltd.). The taken tissue was rinsed with running water for several hours, dehydrated through a series of ethanol solutions (70%, 80%, 90%), and then treated with a mixture of pure alcohol and xylene at a ratio of 1:1 for 15 minutes, followed by xylene I for 15 minutes and xylene II for 15 minutes (until transparent). The tissue was then placed in a mixture of xylene and paraffin at a ratio of 1:1 for 15 minutes, followed by immersion with paraffin I and paraffin II each for 50-60 minutes. The tissue was embedded in paraffin and sectioned, and the sections were dried, dewaxed and hydrated in distilled water. The resulting sections were then stained in hematoxylin solution for 3 minutes, differentiated in a hydrochloric acid alcohol solution for 15 seconds, slightly washed, blued in a return blue solution for 15 seconds, rinsed with running water, stained with eosin for 3 minutes, rinsed with running water, dehydrated, cleared, and mounted for microscopic examination.

ELISA Detection

ELISA was used to detect the levels of inflammatory factors TNF-α, IL-1β, IL-6, and IL-8 in serum and alveolar lavage fluid.

Main Instruments

| Experimental instruments | Model | Supplier |
|---|---|---|
| Pipette | 100-1000ul | eppendorf |

(continued)

| Experimental instruments | Model | Supplier |
|---|---|---|
| Pipette | 20-200ul | eppendorf |
| Pipette | 5-50ul | eppendorf |
| Automatic Microplate Reader | WD-2102B | Beijing Liuyi Biotech |
| Refrigerator/Freezer | BD/BC-415DKEM | Midea |
| Electric Heated Constant Temperature Incubator | DHP-9054 | Shandong Bok Biological Industry Co., Ltd. |
| Pharmaceutical Refrigerator | BYC-310 | Shandong Bok Biotech |
| Microscope | BX43 | OLYMPUS |
| Slicer | 2235 | Leica |
| Electric Heated Constant Temperature Blast Drying Oven | HGZF-101-1 | Shanghai Yuejin Medical Instrument Co., Ltd. |

## II. Experimental Results

Chronic Obstructive Pulmonary Disease (COPD) Modeling in rats

The chronic obstructive pulmonary disease modeling was conducted by injecting LPS into rats' trachea via through tracheal intubation on days 1 and 14, and by exposing them to cigarette smoke for 30 minutes daily in a self-made organic glass sealed smoking chamber on days 2-13 and days 15-28. The lung tissue staining revealed that in the model group a large number of inflammatory cells were attached to the bronchial wall, with necrosis, shedding, and disorganization of the tracheal mucosal epithelium, as well as significant hyperplasia of goblet cells and glands, indicating the model was successfully established.

HE Staining

After 30 days of administration, the morphology and inflammatory cell infiltration of rat lung tissue in each group were observed under a light microscope (as shown in FIG. 11). In the normal control group, no thickening of the bronchial wall was observed, and no significant inflammatory cell infiltration was seen. The alveolar structure was intact, and no cilia shedding was observed. In the COPD model group, there was a large number and variety of inflammatory cell infiltration around the bronchial wall and blood vessels, with a significant shedding of epithelial cells, cilia lying down, obvious tracheal wall fracture and thickening, narrowing oflumens, and fracture and fusion of alveoli. In the positive control group, local mild deformation of rats' tracheal lumen was observed, with slight wall thickening, minimal shedding of epithelial cells, partial cilia lying down, and insignificant shedding. Some alveoli were fused, and the inflammatory cell infiltration was significantly reduced. In the MDI-1233 drug administration group, visible deformation and inflammatory cells infiltration in the rat tracheal lumen were observed to be reduced compared to the COPD model group. As the drug concentration increased, the lumen deformation was mitigated, and the number of inflammatory cells gradually decreased. Among them, the high-dose MDI-1233 drug group showed the best effects, although it was slightly less effective than the positive control drug group.

ELISA Detection

Based on the oxidative stress response and inflammatory response during the onset of COPD, the assay primarily selected indicators such as inflammatory chemokines during COPD lesions to evaluate the effect of MDI-1233 on the level of lung inflammation and oxidative stress in COPD model mice. COPD is a chronic inflammatory disease, and the stimulation of inflammation activates macrophages, neutrophils, etc., increasing the secretion of IL-1$\beta$. As shown in FIG. 12, compared to the blank control group, the model group treated with vehicle still had increased levels of IL-1$\beta$ in serum and alveolar lavage fluid. The positive control drug treatment could down-regulate its expression. Different doses of MDI-1233 could also down-regulate its expression, with a certain dose-dependent effect (FIG. 12), which was consistent with the staining observation results.

## III. Conclusion

The above results indicated that MDI-1233 has a dose-dependent therapeutic effect on the COPD model in rats.

## Example 64: Alleviating and Therapeutic Effects of Compounds to be tested on External Hemorrhoids in Rats

### I. Materials and Methods

Animals

Healthy male SD rats at 6 weeks of age (Manufacturer: Hunan Slake Jingda Co., Ltd., License Number: SCXK (Xiang)2019-0004) male, were reared under conventional diet, at a temperature of 20 to 26°C and a humidity of 40% to 70%.

Animal grouping and modeling:

(1) Blank control group (7 rats)

(2) Hemorrhoid model group (10 rats)

(3) Hemorrhoid model group + positive control drug (Mayinglong Hemorrhoid Ointment) group (8 rats)

(4) Hemorrhoid model group + vehicle treatment (Gel excipient) (8 rats)

(5) Hemorrhoids model group + 0.1% MDI-1228 gel preparation group (8 rats)

(6) Hemorrhoids model group + 0.3% MDI-1228 gel preparation group (8 rats)

(7) Hemorrhoids model group + 1% MDI-1228 gel preparation group (8 rats)

(8) Hemorrhoids model group + 3% MDI-1228 gel preparation group (8 rats)

**Experimental environment:** Temperature 20°C-26°C, humidity 40%-70%

Apart from the normal control group, all groups were treated by first disinfecting the perianal skin. A 75% acetic acid solution was prepared and injected subcutaneously in a scattered pattern around the anus of each rat at a dose of 0.05 ml. Observation 24 hours later revealed white ulcerative surfaces, perianal swelling, and inflammatory exudate, indicating that the modeling was successful.

Preparation of MDI-1228 Gel

The recipe and preparation method for 1% MDI-1228 gel were as follows:

| Ingredient | Mass Percentage |
|---|---|
| MDI-1228 mesylate | 1.2% |
| Propylene glycol | 83.3% |
| Water | 12.5% |
| Carbopol ETD2020 | 1.5 |
| Triethanolamine to adjust pH to approximately 6.5 | 1.5 |

Step 1: the prescribed amount of water was weighted, to which 10% of the propylene glycol was added, and stirred evenly (for 5-15 minutes).

Step 2: MDI-1228 mesylate was weighted and added to the solution obtained from step 1 together with the remaining amount of propylene glycol, and stirred evenly (for 30-60 minutes).

Step 3: Carbopol was added to the solution from step 2, and stirred for 12-18 hours for full swelling.

Step 4: Triethanolamine was added (after adding about 1.4% of the prescription amount, which was required to finely add), with stirring, so as to adjust the pH to approximately 6.5.

The recipes for 0.5% and 3% MDI-1228 gels were modified based on the amount of MDI-1228 and prepared using the same process.

Animal Administration

The control group and model group received no treatment (no therapy). Administration begined 24 hours after the model induction was successful. Group 3) was administrated with Mayinglong Hemorrhoid Ointment (7.5 g/Kg), three times, 10 min each time; Group 4) was administrated with a blank gel forming agent (1 g/Kg) around the anus; Groups 5)-8) were administrated with different concentrations of MDI-1228 compound gel preparation (1 g/Kg) around the anus, twice daily, 10 min each time, followed by covering with plastic wrap and fixing with tape to prevent the drug from falling off and affecting efficacy. After 6 hours, the dressing was removed, and the application site was cleaned with normal saline. The administration was continued for 11 days.

Perianal Ulcer Scoring

Observe the healing of the ulcer site on days 3, 5, 7, 9, and 11 of administration, and assess the ulcer score (scoring criteria: ulcer exudate present = 1 point; minimal ulcer exudate = 2 points; scab present, nearly healed = 3 points; completely healed = 4 points).

Animal Sampling

On an 11-day administration, animals were euthanized by inhalation of excess carbon dioxide, and perianal rectal tissue was taken for HE staining, and serum was taken for ELISA detection.

HE Staining:

Tissue samples from animal Rectal were excised, fixed in 10% neutral formalin buffer solution, dehydrated, embedded in paraffin, and sectioned and HE stained for observation of rectal histopathological morphology changes. The reagents and instruments required for HE staining included: Hematoxylin staining solution (AR11800-1, BOSTER); Eosin staining solution (AR11800-2, BOSTER); Ultra-clean advanced mounting medium (YZB, BASO); Scott's blueing solution (G1865, Solarbio), etc. Medication refrigerator (BYC-310, Shandong Bokai Biotech); Microscope (CX41 OLYMPUS); Microtome (BQ-318D, Berne); Electric heating blast drying oven (DHG-9070A, Hengke Scientific Instrument Co., Ltd.). The taken tissue was rinsed with running water for several hours, dehydrated through a series of ethanol solutions (70%, 80%, 90%), and then treated with a mixture of pure alcohol and xylene at a ratio of 1:1 for 15 minutes, followed by xylene I for 15 minutes and xylene II for 15 minutes (until transparent). The tissue was then placed in a mixture of xylene and paraffin at a ratio of 1:1 for 15 minutes, followed by immersion with paraffin I and paraffin II each for 50-60 minutes. The tissue was embedded in paraffin and sectioned, and the sections were dried, dewaxed and hydrated in distilled water. The resulting sections were then stained in hematoxylin solution for 3 minutes, differentiated in a hydrochloric acid alcohol solution for 15 seconds, slightly washed, blued in a return blue solution for 15 seconds, rinsed with running water, stained with eosin for 3 minutes, rinsed with running water, dehydrated, cleared, and mounted for microscopic examination.

ELISA Detection

A double-antibody one-step sandwich Enzyme-linked Immunosorbent Assay (ELISA) kit was used. A capture antibody for the rat target indicators was pre-applied to microplate wells, to which samples, standards, and HRP-labeled detection antibodies were added sequentially, followed by incubation and thorough washing. TMB substrate was added for color development, which turned into blue in the presence of peroxidase and then into yellow after acidification. The depth of color was positively correlated with the concentration of the target in the samples. The absorbance (OD450 value) was measured at a wavelength of 450 nm using the microplate reader, and the sample concentration was calculated.

Histopathological Staining Observation

The pathological changes of rat rectal tissue were graded as follows: "-" indicating neat arrangement of the rectal mucosal glandular epithelium with no other pathological changes, scored as to be 0; "+" indicating neat arrangement of the rectal mucosal glandular epithelium with occasional inflammatory cell infiltration, scored as to be 1; "++" indicating hyperplasia and disordered arrangement of the rectal mucosal epithelium with a large number of inflammatory cells infiltration under the mucosa, scored as to be 3; and "+++" indicating rectal mucosal defects, necrosis, with inflammatory exudate on the surface, inflammatory cell infiltration, a large number of neutrophil infiltration, and connective tissue

hyperplasia, scored as to be 5.

**II. Experimental Results**

Confirmation of the External Hemorrhoid Modeling in Rats and the Positive Control Drug Effectiveness

Using acetic acid injection, an external hemorrhoid model in rats was successfully established, with ulcers appearing around the anus, and severe cases had fluid leakage. After treatment with the positive control drug, Mayinglong Hemorrhoid Ointment, pathological section examination revealed that after hemorrhoid modeling, tissue structural damage appeared in the rectal ulcer area, with blood clots and accompanied by inflammation; and after treatment with the positive control drug, the ulcer area healed and inflammation was reduced (FIG. 13). This proves that the operations for external hemorrhoid modeling and positive control drug treatment were correct. Therapeutic Efficacy of the Drugs to be tested

After successful modeling of external hemorrhoids in rats, the treatment was conducted with a gel excipient and gels containing different concentrations of MDI-1228 and observed for healing, which was then scored. The results showed that the blank group had no spontaneous external hemorrhoids, scoring full marks. The model group has a lower healing rate than the other treatment groups from the third day. The positive control drug group showed a significant difference in therapeutic effect after a 7-day administration compared to the model group. This indicates that the external hemorrhoid modeling and positive control drug treatment test system were normal. The blank gel excipient for preparing the gel preparation of MDI-1228 showed a promoting effect on ulcer healing after a 9-day administration compared to the model group, but its effect was significantly lower than that of the gel preparation groups containing various doses of MDI-1228. Among the gel preparation groups with different doses of MDI-1228, the 1% concentration group showed a significant therapeutic effect afater a 7-days administration. The 3% concentration group had a similar therapeutic effect after a 9-days administration and an 11-days administration.

HE Staining

After modeling and drug administration, the perianal rectal tissue of the animals was taken for pathological sectioning and HE staining, and the pathological characteristics of each group were scored. The results showed that the model group had significant pathological characteristics, which were significantly relieved after treatment with the positive control drug. The gel excipient had a certain effect on promoting the healing of external hemorrhoid ulcers. The therapeutic effect of MDI-1228 at concentrations of 0.3% or more was more significant, with the 3% concentration of MDI-1228 showing the best effect (FIG. 14) (*: compared to the control group, $p<0.05$; #: compared to the excipient group, $p<0.05$).

ELISA Assay

In addition to the analysis of pathological samples, the levels of inflammation-related factors IL-1$\beta$ and IL-6, tumor necrosis factor TNF-$\alpha$, and the content of NO in the peripheral blood of each group were measured. The results indicated that after external hemorrhoid modeling, the expression of TNF-$\alpha$ in rat blood was upregulated, and it significantly decreased after treatment with the positive control drug. After application of the excipient in the external hemorrhoid group, the expression of TNF-$\alpha$ did not significantly reduce compared to the model group. Different concentrations of MDI-1228 could all downregulate the expression of TNF-$\alpha$, with the degree of downregulation being linearly related to the drug dose (FIG. 15, *: compared to the control group, $p<0.05$; #: compared to the excipient, $p<0.05$).

**III. Conclusion**

The comprehensive results of the experiment showed that MDI-1228 gel preparations at different concentrations can effectively alleviate and treat external hemorrhoids in rats.

**Example 65: Therapeutic Efficacy of Compound MDI-1228 on Phenol-Induced Cervicitis Model in Rats**

**I. Materials and Methods**

Experimental Animals

6-8-week-old SD rats (180-220g) (Supplier: Hunan Slake Jingda Co., Ltd., License No.: SCXK(Xiang) 2019-0004), female, were reared under conventional diet, at a temperature of 20 to 26°C and a humidity of 40% to 70%.

Preparation of Primary Reagents

25% Phenol Slurry: Take 5 ml of 50°C liquid phenol + 15 ml of distilled water + 8 g of acacia gum and stir thoroughly in a water bath.
Vehicle Preparation (20% DMA + 65% PEG400 + 15% H2O): Take 10 ml of DMA + 32.5 ml of PEG400 + 7.5 ml of H2O.
MDI-1228 (4.5 mg/ml): For the first time, MDI-1228 57.7 mg was weighted and dissolved in 6.1 ml DMA, to which 19.7 ml PEG400 was added, followed by 4.5 ml of water to obtain a colorless clear solution; For the second time, MDI-1228 50.9 mg was weighted and dissolved in 2.3 ml DMA, to which 7.4 ml PEG400 was added, followed by 1.7 ml of water to obtain a colorless clear solution.
MDI-1228 (15 mg/ml): For the first time, MDI-1228 186.5 mg was weighted and dissolved in 2.5 ml DMA, to which 8.1 ml PEG400 was added, followed by 1.8 ml of water to obtain a colorless clear solution; For the second time, MDI-1228 157.2 mg was weighted and dissolved in 2.1 ml DMA, to which 6.8 ml PEG400 was added, followed by 1.6 ml of water to obtain a colorless clear solution.
MDI-1228 (30 mg/kg, 45 mg/ml): 0.67 ml/kg For the first time, MDI-1228 362.4 mg was weighted and dissolved in 1.6 ml DMA, to which 5.2 ml PEG400 was added, followed by 1.2 ml of water to obtain a pale yellow clear solution; For the second time, MDI-1228 368.0 mg was weighted and dissolved in 1.6 ml DMA, to which 5.3 ml PEG400 was added, followed by 1.2 ml of water to obtain a pale yellow clear solution.
Preparation of Positive Control Drug Jieeryin (Enwei Pharmaceutical, National Drug Approval Number: Z10930008) (1:5 dilution) 0.67 ml/kg. For the first time, take 4.0 ml of Jieeryin + 16.0 ml of water, for the second time, take 4.0 ml of Jieeryin + 16.0 ml of water.

Animal Grouping and Modeling

Blank Control Group (untreated) (N=5+2 rats)

Cervicitis Model Group (vehicle treatment: 20% DMA + 65% PEG400 + 15% H2O) (N=8+2 rats)

Cervicitis Model + Positive Control Drug Group (Jieeryin lotion (1:5 dilution), 2 times/day, for 7 consecutive days) (N=8 rats)

Cervicitis Model + Low-Dose MDI-1228 Compound Lotion Group (dose: 3 mg/kg/time, 2 times/day, for 7 consecutive days) (N=8 rats)

Cervicitis Model + Medium-Dose MDI-1228 Compound Lotion Group (dose: 10 mg/kg/time, 2 times/day, for 7 consecutive days) (N=8 rats)

Cervicitis Model + High-Dose MDI-1228 Compound Lotion Group (dose: 30 mg/kg/time, 2 times/day, for 7 consecutive days) (N=8 animals)

**Experimental Environment:** Temperature 20°C-26°C, humidity 40%-70%.

**Modeling:**

SD rats were acclimated for one week. After anesthesia with isoflurane, the rats were fixed in a supine position on the operation table with their limbs secured. A 1 mL syringe with a blunt needle was inserted approximately 1 cm into the rat's vagina, and 0.1 mL of 25% phenol slurry was slowly injected. The vaginal opening was immediately plugged with a sterile cotton ball, and the rat was kept in a head-down and tail-high position for 20 minutes. The injection was performed every 2 days, for a total of 3 times. The vaginal opening of the rats was observed, and the model was considered successful if there was white secretion, vaginal congestion, and swelling.

Animal Administration

The successfully modelled rats were randomly divided into 6 groups. Animals in each group were treated according to their group assignment. The blank group (untreated) received no treatment. The positive control group was administrated with Jieeryin lotion (1:5 dilution) for vaginal irrigation twice a day, for 7 consecutive days. The low, medium,

and high-dose MDI-1228 compound lotions were used for vaginal irrigation twice a day, for 7 consecutive days. The model group was given an equal amount of vehicle treatment, with the same way and duration as the test drug groups.

Detection Indicators

**Clinical Symptoms Observation:** The general condition of the rats (activity, diet, mental state) was continuously observed, and the rats were weighted before and after administration. Daily observations and recordings were made of changes in the local area and secretions of the rat's vulva, whether the vaginal opening is dry, and whether there is congestion, swelling in the vulva and vaginal mucosa, and whether there is vaginal fluid blood. Scoring was conducted according to the following table.

| Erythema | score | Edema | score | Secretion | score |
|---|---|---|---|---|---|
| No erythema | 0 | No edema | 0 | No secretion | 0 |
| Very mild erythema | 1 | Very mild edema | 1 | Very small amount of secretion | 1 |
| Mild erythema (pale red) | 2 | Mild edema | 2 | Small amount of secretion | 2 |
| Moderate erythema (obvious red)) | 3 | Severe edema (protruding about 1 mm) | 3 | Moderate amount of secretion | 3 |
| Severe erythema (beet red or purple red) | 4 | Very severe edema (protruding about 1 mm with exposed area) | 4 | Large amount of secretion (wetting most of the area around the vagina) | 4 |

**Organ Weight and Organ Index Calculation:** After the last administration, the rats were sacrificed the next day, and the uterus was removed, with fat tissue and mesentery dissected away, and the weight of the cervix (from the vaginal opening to the bifurcation of the double uterus) and the liver and spleen were measured to calculate the organ index.

**Pathological Detection:** ELISA detection for serum markers IL-4, IL-6, IL-1β, TNF-α; ELISA detection for plasma CP; biochemical detection for MDA and T-SOD in cervical tissue.

Histopathological Detection Method

**HE Staining:** Tissue samples from animal vaginal, cervical, and uterine were excised, fixed in 4% paraformaldehyde, dehydrated, embedded in paraffin, and sectioned and HE stained for observation of histopathological morphology changes. The reagents and instruments required for HE staining included: Hematoxylin staining solution (AR11800-1, BOSTER); Eosin staining solution (AR11800-2, BOSTER); Ultra-clean advanced mounting medium (YZB, BASO); Scott's blueing solution (G1865, Solarbio), etc. Medication refrigerator (BYC-310, Shandong Bokai Biotech); Microscope (CX41 OLYMPUS); Microtome (BQ-318D, Berne); Electric heating blast drying oven (DHG-9070A, Hengke Scientific Instrument Co., Ltd.). The taken tissue was rinsed with running water for several hours, dehydrated through a series of ethanol solutions (70%, 80%, 90%), and then treated with a mixture of pure alcohol and xylene at a ratio of 1:1 for 15 minutes, followed by xylene I for 15 minutes and xylene II for 15 minutes (until transparent). The tissue was then placed in a mixture of xylene and paraffin at a ratio of 1:1 for 15 minutes, followed by immersion with paraffin I and paraffin II each for 50-60 minutes. The tissue was embedded in paraffin and sectioned, and the sections were dried, dewaxed and hydrated in distilled water. The resulting sections were then stained in hematoxylin solution for 3 minutes, differentiated in a hydrochloric acid alcohol solution for 15 seconds, slightly washed, blued in a return blue solution for 15 seconds, rinsed with running water, stained with eosin for 3 minutes, rinsed with running water, dehydrated, cleared, and mounted for microscopic examination.

**ELISA Detection:** The information of the kits used was as follows: Rat Tumor Necrosis Factor-α (TNF-α) kit (MM-0180R1, enzyme immunoassay), Rat Interleukin-1β (IL-1β) kit (MM-0047R1, enzyme immunoassay), Rat Interleukin-4 (IL-4) kit (MM-0191R1, enzyme immunoassay), Rat Interleukin-6 (IL-6) kit (MM-0190R1, enzyme immunoassay), and Rat Ceruloplasmin (CP) kit (MM-21238R1, enzyme immunoassay).

**Biochemical Detection:** The information of the kits used was as follows: MDA Detection Kit (MDA, E-BC-K025-M, Elabscience), and Total Superoxide Dismutase Detection Kit (T-SOD, E-BC-K019-M, Elabscience).

Main Instruments

| Experimental instruments | Model | Supplier |
|---|---|---|
| Pipette | 100-1000ul | eppendorf |
| Pipette | 20-200ul | eppendorf |
| Pipette | 5-50ul | eppendorf |
| Automatic Microplate Reader | WD-2102B | Beijing Liuyi Biotech |
| Refrigerator/Freezer | BD/BC-415DKEM | Midea |
| Electric Heated Constant Temperature Incubator | DHP-9054 | Shandong Bok Biological Industry Co., Ltd. |
| Pharmaceutical Refrigerator | BYC-310 | Shandong Bok Biotech |
| Microscope | BX43 | OLYMPUS |
| Slicer | 2235 | Leica |
| Electric Heated Constant Temperature Blast Drying Oven | HGZF-101-1 | Shanghai Yuejin Medical Instrument Co., Ltd. |

**II. Experimental Results**

Observation of General Symptoms in Rats

During the process of cervical inflammation modeling, the rats showed irritability and piloerection. Obvious erythema and edema were visible at the external vaginal opening, accompanied by a large amount of yellow or white, and even blood-streaked sticky secretions. After administration, the symptoms in each treatment group were improved to varying extends compared to the model group, with MDI-1228 (30 mg/kg) treatment significantly reducing the comprehensive score of cervicitis. The area under the continuous scoring curve also indicated that MDI-1228 (30 mg/kg) significantly reduced the score for vulvar inflammation, erythema, edema, and secretion by 23.42%, 29.99%, 19.61%, and 21.05%, respectively (FIGs. 16-19).

Effect of Drugs on Organ Index

After phenol challenging and modeling, the mean cervical index in the cervicitis model in animals slightly increased. After drug intervention, the cervical index in the MDI-1228 (30 mg/kg) group was significantly reduced (FIG. 20).

HE Staining Results

The observation results were shown in FIG. 21. In the model (solvent) group, there was a large number of inflammatory cell infiltrates in the vaginal, cervical, and uterine tissue sections, with varying degrees of thinning or thickening of the vaginal mucosa. Some samples showed mucosal layer necrosis and exfoliation, cervical vascular congestion and interstitial hemorrhage, fibrosis, endometrial shedding, and structural disorder. In the positive control drug group, the number of inflammatory cells was significantly reduced, and the structure of the vaginal mucosa and endometrium tended to be intact, with some samples still showing significant inflammatory cell infiltration and minimal fibrosis in the cervix. In the MDI-1228 treatment groups, compared to the model (vehicle) group, all drug concentrations showed some therapeutic effect, with the MDI-1228 (30 mg/kg) group showing the best effect. The MDI-1228 (3 mg/kg) group still showed significant inflammatory cell infiltration in the entire tissue structure, but there was some improvement in the structure of each part. The therapeutic effect of the MDI-1228 (10 mg/kg) and MDI-1228 (30 mg/kg) groups was not significantly different, with a marked reduction in inflammatory cells in both groups. However, observing the overall tissue structure, some samples in the MDI-1228 (10 mg/kg) group still had damage to the mucosal structure and fibrosis in the vagina and cervix, while the MDI-1228 (30 mg/kg) high-dose group showed overall better recovery symptoms.

**III. Conclusion**

MDI-1228 at various doses can improve the conditions of erythema, edema, and secretion in rats with cervicitis, reduce the appearance inflammation score, and decrease the levels of inflammatory factors IL-1$\beta$, IL-4, IL-6, and TNF-$\alpha$, thereby treating cervicitis.

**Example 66: Therapeutic Effect of Compound Gel on Atopic Dermatitis/Eczema in Mice**

**I. Materials and Methods**

Experimental Animals

Healthy C57BL/6 mice, 6-8 weeks old, weighing about 20g (Manufacturer: Hunan Slake Jingda Co., Ltd., License No.: SCXK(xiang)2019-0004), with an equal number of males and females, were reared under conventional diet, at a temperature of 20-26°C and a humidity of 40%-70%.

Preparation of Main Reagents

1) Dust mite preparation (Derf, Lot: 361863, GREER): one bottle of dust mite (containing 4.51 mg of protein) was taken to which 4.51 ml of PBS was added and then mixed well until the dust mite was completely dissolved, to prepare a 1 mg/ml stock solution. Each time, the dust mite protein solution was diluted with PBS to 100 μg/ml.

2) SEB preparation (Lot: 92815B, Toxin Technology): one bottle of SEB was taken, to which 10 ml of PBS was added, to prepare a 100 μg/ml stock solution. Each time, it was diluted with PBS to 10 μg/ml.

3) Tofacitinib preparation: 10 mg of tofacitinib (CAS# 477600-75-2, lot# 79614, MCE) was taken, to which 40 μl of dimethylacetamide (CAS#127-19-5, Source Leaf Biotech) was added to dissolve, and then 200 μl of normal saline was added to dilute to 41.67 mg/ml.

4) Blank gel, 0.5%, 1%, and 3% MDI-1228 gels were prepared according to the process described in Example 64.

Animal Grouping and Modeling

Blank control group (no treatment) (N=5+3)

Blank dermatitis model group (no treatment) (N=8+3)

Dermatitis model + blank excipient group (vehicle treatment: blank gel formulation, dose: 1 g/kg, twice a day per mouse) (N=8)

Dermatitis model + positive control drug group (Tofacitinib, 10 mg/kg) (N=8)

Dermatitis model + MDI-1228 (0.5%) (dose: 0.7 g gel /kg per mouse, twice a day per mouse) (N=8)

Dermatitis model + MDI-1228 (1%) (dose: 1 g gel /kg per mouse, twice a day per mouse) (N=8)

Dermatitis model + MDI-1228 (3%) (dose: 1 g gel /kg per mouse, twice a day per mouse) (N=8)

**Experimental environment:** Temperature 20°C-26°C, humidity 40%-70%.

**Modeling:**

Mice were acclimated for 7 days and anesthetized with isoflurane, and their back hair was removed to form an approximately 3 $cm^2$ hairless area. The skin was lightly scraped to cause damage after hair removal. The mouse's back was cleaned with normal saline and gently dried with a sterile cotton swab. Sterile cotton pads were cut into 1.5×1.5 cm squares, and 100 μL of Derf and 50 μL of SEB were dropped onto the cotton pads, which were then fixed to the hairless area of the mouse with tape and wrapped around the mouse's waist and abdomen with tape. Four days after the first allergen challenge, the application device was removed, and after a 1-2 day rest, the medication was repeated once (if the skin damage was not severe or hair regrowth occurred, the depilatory cream was used again to cause skin damage before administration). The first two administrations were the first round of stimulation. The second round of medication was administered 9 day later (twice in total), following the same steps as before. A total of four rounds of allergen challenges were used (8 times in total). On the 40th day of modeling, mice with a skin lesion score of 5 or higher were considered successfully modeled.

**Skin Lesion Scoring and Scratching Experiment:** Four parameters of the modeled area on the mouse's back

were evaluated: Skin Erythema, Hemorrhage, Eruption, and Desquamation, and were rated according to the following severity levels: 0-no symptoms, 1-mild, 2-moderate, 3-severe. On the 40th day of modeling, a mixture of derf and SEB was sprayed on a specific area of the mouse's back (self-determined and marked), with the same dose as the modeling dose, and behavior was observed for 30-60 minutes. The primary observation indicators were the number of scratching instances at the modeling and application sites in mice. The criteria for effectiveness were defined as valid when the mice scratched areas other than the head and face. One scratching instance was counted when the mouse started scratching until its paws touched the ground again or it licked the scratching paws. Finally, the total duration of each scratching episode was statistically combined. Additionally, the time interval from the onset of allergen stimulation to the start of scratching was recorded and analyzed as the scratching latency indicator.

**Model Validation:** On the 40th day of modeling, mice with a score of 5 or higher were considered successfully modeled. Three mice each from the normal group and the model group are selected for HE staining and toluidine blue staining to judge the pathological characteristics and validate the success of the modeling. The blood of three normal group mice was used for ELISA detection in Experiment 2.

Anmial Administration

The specific procedure was as follows: On the 40th day of modeling (Day 0 of administration), the mice were treated with topical applications according to the above groups for 14 days, with two applications per day (at 9 am and 5 pm, with an interval of at least 6 hours). Skin lesion severity scoring weree conducted on the mice on Days 1, 4, 7, and 14 (using the same method as above). Scratch tests and behavioral changes were recorded before 5 pm (using the same method as above).

Note: To prevent animals from licking the medication off each other, after the gel was applied, the animals were placed individually in a pad-free independent cage to move around for a period of time to allow the gel to air dry for 1 hour before being returned to the normal feeding cage. Single housing is discouraged as it can lead to depression; wrapping the application area with gauze is also prohibited as it can absorb the medication.

Detection Indicators

On the 14th day of treatment, immediately after the behavioral changes had been recorded, the mice were subjected to cardiac blood collection and skin tissue removal:

**ELISA Detection of Inflammatory Indicators:** Serum was collected, and the level of IL-4, IL-13, TNF-$\alpha$, and IFN-$\gamma$ were measured using ELISA. The detection method was strictly followed according to the instructions provided with the ELISA kit.

**Histopathological Examination:** Skin tissue was taken for HE staining and toluidine blue staining to observe differences in epidermal thickness, the number of mast cells, capillary permeability, edema, and the exudation of inflammatory components. Skin tissue was also taken for immunohistochemical detection of TSLP expression.

Histopathological Examination:

**HE staining:** The reagents and instruments required for HE staining included: Hematoxylin staining solution (AR11800-1, BOSTER); Eosin staining solution (AR11800-2, BOSTER); Ultra-clean advanced mounting medium (YZB, BASO); Scott's blueing solution (G1865, Solarbio), etc. Medication refrigerator (BYC-310, Shandong Bokai Biotech); Microscope (CX41 OLYMPUS); Microtome (BQ-318D, Berne); Electric heating blast drying oven (DHG-9070A, Hengke Scientific Instrument Co., Ltd.). Tissue samples from animal skin were excised, fixed in 4% paraformaldehyde, dehydrated, embedded in paraffin, and sectioned and HE stained for observation of histopathological morphology changes. The sample was fixed in 4% paraformaldehyde for 4 hours at normal temperature, and the taken tissue was rinsed with running water for several hours, dehydrated through a series of ethanol solutions (70%, 80%, 90%), and then treated with a mixture of pure alcohol and xylene at a ratio of 1:1 for 15 minutes, followed by xylene twice, each for 15 minutes until transparent. The tissue was then placed in a mixture of xylene and paraffin at a ratio of 1:1 for 15 minutes, followed by immersion with paraffin I and paraffin II each for 50-60 minutes. The tissue was embedded in paraffin and sectioned at a predetermined direction. The paraffin sections were baked, dewaxed and hydrated in distilled water. The resulting sections were then stained in hematoxylin solution for 3 minutes, differentiated in a hydrochloric acid alcohol solution for 15 seconds, slightly washed, blued in a return blue solution for 15 seconds, rinsed with running water, stained with eosin for 3 minutes, rinsed with running water, dehydrated, cleared, and mounted for microscopic examination.

Main Instruments

| Experimental instruments | Model | Supplier |
|---|---|---|
| Pipette | 100-1000ul | eppendorf |
| Pipette | 20-200ul | eppendorf |
| Pipette | 5-50ul | eppendorf |
| Automatic Microplate Reader | WD-2102B | Beijing Liuyi Biotech |
| Refrigerator/Freezer | BD/BC-415DKEM | Midea |
| Electric Heated Constant Temperature Incubator | DHP-9054 | Shandong Bok Biological Industry Co., Ltd. |
| Pharmaceutical Refrigerator | BYC-310 | Shandong Bok Biotech |
| Microscope | BX43 | OLYMPUS |
| Slicer | 2235 | Leica |
| Electric Heated Constant Temperature Blast Drying Oven | HGZF-101-1 | Shanghai Yuejin Medical Instrument Co., Ltd. |

**II. Experimental Results**

Effect of the Drug on Mouse Skin Erythema, Hemorrhage, Eruption, and Desquamation (Lesion Scoring)

Throughout the experiment, the skin of the mice in the model group exhibited erythea, hemorrhage, rash, and scales. The lesion scores in the model group were significantly higher than those in the normal group, indicating that the modeling was successful (Table 1). After successful modeling, the lesion scores of the model group without any intervention showed a gradual downward trend on days 0, 4, 7, and 14, suggesting that the mice's skin has a certain ability to heal itself. The score of the blank gel group on day 14 was lower than that of the model group, indicating that the blank gel intervention also has a certain promoting effect on skin repair. However, in terms of clinical scoring, the therapeutic effect of the positive control drug was significantly better than that of the blank gel. In addition, gels containing different concentrations of MDI-1228 also showed therapeutic effects similar to the positive control drug. As shown by the area under the curve (AUC) of the dermatitis scores, compared to the model group, all groups showed a decrease in AUC, with the MDI-1228 (3%) dose group showing the most significant reduction (23.75%) (Table 1, FIGs. 22 and 23).

Table 1. Effect of the Drug on Mouse Skin Erythema, Hemorrhage, Eruption, and Desquamation (Lesion Scoring) (Mean ± SE)

| Groups | N | Day 1 | Day 4 | Day 7 | Day 14 |
|---|---|---|---|---|---|
| Normal group | 6 | 0 | 0 | 0 | 0 |
| Model group | 8 | 3.88±0.69 | 3.00±0.76 | 3.00±0.63 | 2.25±0.41 |
| Excipient group | 8 | 3.63±0.56 | 2.63±0.23 | 2.38±0.26 | 2.00±0.19 |
| Tofacitinib group | 8 | 4.38±0.46 | 2.63±0.26 | 2.00±0.33 | 1.50±0.19 |
| MDI-1228 (0.5%) | 8 | 4.13±0.55 | 2.88±0.64 | 2.13±0.58 | 1.25±0.36 |
| MDI-1228 (1%) | 8 | 4.75±0.75 | 3.25±0.49 | 2.13±0.30 | 1.38±0.18 |
| MDI-1228 (3%) | 8 | 3.38±0.42 | 2.63±0.46 | 1.75±0.31 | 1.63±0.26 |

Effect of the Drug on the Number of Scratch Incidents and Scratch Duration in Mice

Additionally, the behavior of the mice during the treatment process was recorded and analyzed. The evaluation was based on two parameters: the number of scratch incidents and the total scratch duration. Regarding the number of scratch incidents, comparing the data on the first day with the data on the $14^{th}$ day, the MDI-1228 high-dose group showed little to no change, while the other groups all showed an increase, indicating that the tested animals exhibited an active stress response to skin irritation. On day 14, the number of scratch incidents in the MDI-1228 high-dose group was also lower than that in the model group and other treatment groups. Regarding the total scratch duration, except for the blank gel group, the total scratch time on day 14 was generally lower than that on day 1, with the MDI-1228 high-dose group showing a greater reduction in scratch duration. These data suggested that the MDI-1228 high-dose group showed a certain therapeutic effect in the behavioral analysis, while the positive control drug tofacitinib group did not show significant effects in the behavioral analysis (Table 2).

Table 2. Effect of the Drug on the Number of Scratch Incidents and Scratch Duration in Mice ($\bar{x} \pm SE$)

| Groups | N | Day 1 | | Day 14 | |
|---|---|---|---|---|---|
| | | Number of Scratch Incidents | Scratch Duratio | Number of Scratch Incidents | Scratch Duratio |
| Normal group | 6 | 13.33±2.54 | 143.67±34.20 | 22.67±2.62 | 150.83±31.48 |
| Model group | 8 | 22.25±2.32 | 291.63±60.65 | 28.13±3.56 | 193.5±36.41 |
| Excipient group | 8 | 20.13±1.99 | 203.00±30.01 | 28.00±3.64 | 236.13±33.22 |
| Tofacitinib group | 8 | 17.75±0.86 | 278.25±50.09 | 26.7±1.96 | 217.5±47.85 |
| MDI-1228 (0.5%) | 8 | 24.0±3.60 | 241.00±48.16 | 30.38±3.79 | 165.63±27.56 |
| MDI-1228 (1%) | 8 | 20.38±2.08 | 211.50±22.88 | 28.13±2.05 | 182.0±25.39 |
| MDI-1228 (3%) | 8 | 23.00±2.46 | 288.00±19.68 | 23.75±2.62 | 173.13±19.26 |

HE Staining and Toluidine Blue Staining

As shown in FIGs. 24 and 25, compared to the normal group, the model group exhibited various degrees of hyperkeratosis or incomplete keratinization, thickening of the dermis, disordered structure, significant infiltration of inflammatory cells, damaged and reduced hair follicles, and marked increase in mast cells (Toluidine Blue staining). The blank excipient group and the positive control drug group both showed slight therapeutic effects on these pathological phenomena, with relatively reduced damage of epidermal layer compared to the model group, and a decrease in mast cells in some samples, but the infiltration of inflammatory cells remained evident. The 0.5% MDI-1228 drug group had a smoother epidermal structure compared to the model group, still with thickened stratum corneum and accompanied by inflammatory cell infiltration, but with a decrase in mast cells and inflammatory cells compared to the model group. The 1% MDI-1228 drug group and the 3% MDI-1228 drug group showed relatively significant therapeutic effects, with a marked reduction in inflammatory cells and mast cells compared to the model group, intact and clear tissue structure, and no obvious damage to the epidermis, but no significant difference was observed between the two groups.

Effect of the Drug on the levels of IL-4, IL-13, TNF-$\alpha$, and IFN-$\gamma$ in Mouse Serum

Atopic dermatitis is a biphasic inflammatory skin disease caused by Th1/Th2 immune responses, and there is an antagonistic relationship between Th1/Th2 cytokines. IFN-$\gamma$ is primarily secreted by Th1 cells, while IL-4 and IL-13 are mainly secreted by Th2 cells. Th1/Th2 imbalance can lead to allergic diseases, and the balance of Th1/Th2 is important for maintaining the normal immunity of the body. The results (FIGs. 26-28) showed that, compared to the normal group, the model group had increased levels of IL-4 and IL-13 and decreased level of IFN-$\gamma$. Compared to the model group, the MDI-1228 drug group had significantly reduced levels of IL-4 and IL-13 and significantly increased level of IFN-$\gamma$, suggesting that MDI-1228 can reduce the body's allergic response and regulate the balance of the Th1 and Th2 cell systems.

After the body was stimulated by an allergen, it could secrete large amounts of IL-1$\beta$, leading to an increase in TNF-$\alpha$ secretion and participating in the occurrence and development of AD. TNF-$\alpha$ is a multifunctional pro-inflammatory factor that promotes the occurrence of inflammatory responses. The results (FIG. 29) showed that, compared to the normal group, TNF-$\alpha$ levels were increased in all groups, with the model group showing the highest increase. Compared to the model group, the high, medium, and low dose MDI-1228 drug groups all showed a reduction in TNF-$\alpha$ levels.

**III. Conclusion**

The MDI-1228 gel preparation has shown significant inhibitory effects on inflammation in the treatment of atopic dermatitis/eczema induced by dust mite extract and Staphylococcus aureus enterotoxin B in mice, and it has also shown improvement in skin appearance and pathology. In terms of scratching behavior, a trend of improvement was also observed.

**Example 67: Therapeutic Effect of the Compound on a Vitiligo Model in Mice**

**I. Materials and Methods**

Experimental Animals

Healthy C57BL/6 mice, with an equal number of males and females, (Manufacturer: Hunan Slake Jingda Co., Ltd., License No.: SCXK (xiang)2019-0004), were reared under conventional diet, at a temperature of 20 to 26°C and a humidity of 40% to 70%.

Animal Grouping and Modeling

(1) Normal control group (5 mice, no treatment)

(2) Vitiligo model group (8 mice, no treatment)

(3) Vitiligo model + vehicle treatment group (8 mice, blank gel preparation, dose: 1.5 g/kg, twice daily)

(4) Vitiligo model + positive control drug treatment group (8 mice, 0.03% Tacrolimus, twice daily, 50 mg per dose)

(5) Vitiligo model + MDI-1228 (0.5%) compound gel preparation group (8 mice, 1 g/kg, twice daily)

(6) Vitiligo model + MDI-1228 (1%) compound gel preparation group (8 mice, 1.5 g/kg, twice daily)

(7) Vitiligo model + MDI-1228 (3%) compound gel preparation group (8 mice, 1.5 g/kg, twice daily)

**Experimental environment:** Temperature 20°C - 26°C, humidity 40% - 70%

**Main reagents:**

Blank gel, 0.5%, 1%, and 3% MDI-1228 gels were prepared according to the process described in Example 64.

Monobenzone depigmentation cream (Manufacturer: Nanjing Xinzhuangyan);
Tacrolimus ointment (Manufacturer: Astellas Pharma, Approval Number: National Medicine Permission J20140147).

**Modeling:** Mice were acclimated for 7 days and subjected to vitiligo modeling by removing the black hair from a $2cm^2$ area on the back of the mice and applying the medication with a spatula using moderate force. The normal control group was administrated with Vaseline 50 mg once daily; and the model group was administrated with 40% monobenzone cream 50 mg once daily for 93 consecutive days.

**Model validation:** One mouse from the blank group and one from the model group were selected for model validation. The criteria for hair depigmentation assessment: Observe the skin&hair depigmentation daily and record the scores. Divide the depigmented area into the treated area and the non-treated area, with a total score of 5 for each area, making a total of 10 points for each mouse. The scoring criteria for mouse skin&hair depigmentation: 0 points for no depigmentation, 1 point for depigmentation area of 0-10%, 2 points for depigmentation area of 11%-25%, 3 points for depigmentation area of 26%-50%, 4 points for depigmentation area of 51%-75%, and 5 points for depigmentation area of 76%-100%.

Note: To prevent animals from licking the medication off each other, after the gel was applied, the animals were placed individually in a pad-free independent cage to move around for a period of time to allow the gel to air dry for 1 hour before being returned to the normal feeding cage. Single housing is discouraged as it can lead to depression; wrapping the application area with gauze is also prohibited as it can absorb the medication.

Observation of the Therapeutic Efficacy of Vitiligo Mice after Drug Administration

After a 35-days treatment administration, a 15-day visual observation of the therapeutic efficacy for vitiligo in the treated mice began, with observations conducted every 3 days (for a total of 5 times) and photographs taken during each observation. The criteria for evaluating efficacy were as follows: The depigmented areas were divided into treated and non-treated areas, with a total score of 5 for each area, making a total of 10 points for each mouse. The scoring criteria for mouse skin&hair depigmentation are: 0 points for no depigmentation, 1 point for a depigmentation area of 0-10%, 2 points for a depigmentation area of 11%-25%, 3 points for a depigmentation area of 26%-50%, 4 points for a depigmentation area of 51%-75%, and 5 points for a depigmentation area of 76%-100%.

Pathological Examination:

(1) **HE Staining:** The removed tissue was rinsed with running water for several hours, dehydrated through a series of ethanol solutions (70%, 80%, 90%), and then treated with a mixture of pure alcohol and xylene at a ratio of 1:1 for 15 minutes, followed by xylene I for 15 minutes and xylene II for 15 minutes (until transparent). The tissue was then placed in a mixture of xylene and paraffin at a ratio of 1:1 for 15 minutes, followed by immersion with paraffin I and paraffin II each for 50-60 minutes. The tissue was embedded in paraffin and sectioned. The sections were mounted, baked, dewaxed and hydrated in distilled water. The resulting sections were then stained in hematoxylin solution for 3 minutes, differentiated in a hydrochloric acid alcohol solution for 15 seconds, slightly washed, blued in a return blue solution for 15 seconds, rinsed with running water, stained with eosin for 3 minutes, rinsed with running water, dehydrated, cleared, and mounted for microscopic examination.

(2) **ELISA detection:** A double-antibody one-step sandwich Enzyme-linked Immunosorbent Assay (ELISA) kit was used. A capture antibody for the mouse target indicators was pre-coated onto microplate wells, to which samples, standards, and HRP-labeled detection antibodies were added sequentially, followed by incubation and thorough washing. TMB substrate was added for color development, which turned into blue in the presence of peroxidase and then into yellow after acidification. The depth of color was positively correlated with the concentration of the target in the samples. The absorbance (OD450 value) was measured at a wavelength of 450 nm using the microplate reader, and the sample concentration was calculated. The information of the kit used was as follows: Mouse Interleukin-6 (IL-6) Kit (MM-0163M1, Enzyme Immunoassay), Mouse Tumor Necrosis Factor-$\alpha$ (TNF-$\alpha$) Kit (MM-0132M1, Enzyme Immunoassay).

Main Instruments

| Experimental instruments | Model | Supplier |
|---|---|---|
| Pipette | 100-1000ul | eppendorf |
| Pipette | 20-200ul | eppendorf |
| Pipette | 5-50ul | eppendorf |
| Automatic Microplate Reader | WD-2102B | Beijing Liuyi Biotech |
| Refrigerator/Freezer | BD/BC-415DKEM | Midea |
| Electric Heated Constant Temperature Incubator | DHP-9054 | Shandong Bok Biological Industry Co., Ltd. |
| Pharmaceutical Refrigerator | BYC-310 | Shandong Bok Biotech |
| Microscope | BX43 | OLYMPUS |
| Slicer | 2235 | Leica |
| Electric Heated Constant Temperature Blast Drying Oven | HGZF-101-1 | Shanghai Yuejin Medical Instrument Co., Ltd. |

**II. Experimental Results**

Observation of the Therapeutic Efficacy of Vitiligo Mice after Drug Administration

After the administration of Monobenzone cream, spotted depigmentation first appeared at the administration site, and then depigmentation appeared in non- administration areas such as ears and tail. This is primarily due to Monobenzone's ability to activate T lymphocyte autoimmune responses, leading to depigmentation in pigmented areas and non-exposed regions. The depigmentation area scores for the administration and non-administration areas in the model group were 3.43$\pm$0.20 and 1.57$\pm$0.20, respectively. Compared to the model group, the MDI-1228 administration groups showed a reduction in depigmentation area scores, indicating an improvement in the depigmentation of skin&hair of vitiligo mice, with a significant difference (p<0.01). By comparing the area under the curve (AUC) for depigmentation scores, the MDI-1228 drug groups were comparable to the positive control drug group. Compared to the model group, the MDI-1228 (3%) gel preparation group showed the greatest reduction in AUC scores, indicating the best effect (FIGs. 30, 31).

HE Staining:

The results were shown in FIG. 32. Compared to the normal group, the model group showed a decrease in the number of hair follicles and in melanin granule aggregation in the hair follicles, with some samples showing significant epidermal thinning, vacuolar degeneration of prickle cells, and marked inflammatory cell infiltration, which was more pronounced in females. The positive control drug group showed an increase in the number of hair follicles and melanin granules, thickened epidermis, and a decrease in inflammatory cells. Among the treatment groups, MDI-1228 (1%) and MDI-1228 (3%) showed the most significant effects, with a noticeable increase in the number of hair follicles and a noticeable melanin granule aggregation, with no significant difference in overall therapeutic efficacy. However, the number of inflammatory cells was generally higher in female mice than in male mice, with the MDI-1228 (1%) group showing the most significant improvement in inflammatory cell infiltration in male mice.

ELISA Detection:

Compared to the normal blank control, the model group showed an increase in TNF-$\alpha$ level. Compared to the model group, both the positive control drug group and the MDI-1228 drug groups showed a reduced TNF-$\alpha$ level, with a dose-dependent effect. The MDI-1228 (3%) drug group showed the most significant reduction in TNF-$\alpha$ level ($p<0.01$), as shown in FIG. 33.

Compared to the normal blank control, serum IL-6 levels were increased in all groups, with the model group and blank gel group showing the most significant increase ($p<0.01$). Compared to the model group, the positive control drug group and the MDI-1228 drug groups showed a reduced IL-6 level, with a dose-dependent effect. The MDI-1228 (3%) drug group showed the most significant reduction in IL-6 level ($p<0.01$), as shown in FIG. 34.

III. Conclusion:

In a drug-induced vitiligo model in mice, after administrated with different concentrations of MDI-1228, the skin&hair depigmentation area scores were reduced in all treatment groups, the number of hair follicles significantly increased, melanin granule aggregation was apparent, and serum TNF-$\alpha$ and IL-6 levels were reduced, indicating that MDI-1228 can improve the skin&hair depigmentation phenomenon in vitiligo mice and has a significant therapeutic effect.

## Example 68: Therapeutic Effect of the MDI-1228 Compound Gel Preparation and MDI-1228 Compound on Alopecia Areata in mice

### I. Materials and Methods

Experimental Animals

Seventy-two healthy C3H/HeJ mice (6 weeks old), half male and half female (Vendor: Beijing Charles River Laboratory Animal Co., Ltd., License No.: SCXK(Jing)2016-0011), were raised under conditions of normal feed, temperature of 20-26°C, and humidity of 40%-70%.

Animal Grouping and Modeling

Blank control group (no treatment) (N=5)

Blank alopecia areata model group (no treatment) (N=8)

Alopecia areata model + blank excipient group (vehicle treatment: blank gel preparation, dose: 1.5 g/kg per mouse, twice daily per mouse) (N=8)

Alopecia areata model + positive control drug group (compound Betamethasone cream (corticosteroid), twice daily, 50 mg each time per mouse) (N=8)

Alopecia areata model + 0.5% MDI-1228 compound gel preparation group (dose: 1.0 g/kg per mouse, twice daily per mouse) (N=8)

Alopecia areata model + 1% MDI-1228 compound gel preparation group (dose: 1.5 g/kg per mouse, twice daily per mouse) (N=8)

Alopecia areata model + 3% MDI-1228 compound gel preparation group (dose: 1.5 g/kg per mouse, twice daily) (N=8)

Blank alopecia areata model (vehicle treatment: 5% DMSO + 75% PEG300 + 20% EtOH) (N=8)

**Modeling:** Imiquimod cream was applied to the nape of the animals (no hair removal required), three times a week for three consecutive weeks. The control group was treated with a blank excipient without imiquimod. Each time, a clean medical cotton swab was used to apply approximately 0.03 g of imiquimod evenly over an area of about 1cm×1cm, and the application site was cleaned with normal saline before each application. The operation was performed gently to avoid skin abrasions or mechanical hair loss due to friction. After about 20 days, a bald patch larger than 1cm×1cm formed near the area where the imiquimod cream was applied, indicating that the alopecia areata pathologic model was successfully prepared. During the modeling, the hair loss of the mice was recorded.

Main Reagents and Preparation

The blank gel, 0.5%, 1%, and 3% MDI-1228 gels were prepared according to the process described in Example 64.

Imiquimod (Manufacturer: Sichuan Mingxin Pharmaceutical Co., Ltd., Approval No.: National Medicine H20030129)

Compound Betamethasone Neomycin Cream (Manufacturer: United Laboratories, Approval No.: National Medicine 1120040492)

Animal Administration

The blank control group (untreated) received no treatment; the drug groups were treated with the corresponding doses of compound betamethasone cream, blank gel preparation, and MDI-1228 compound gel preparation applied to the skin, twice daily (at 9 AM and 5 PM, with an interval of at least 6 hours). Note: To prevent animals from licking the medication off each other, after the gel was applied, the animals were placed individually in a pad-free independent cage to move around for a period of time to allow the gel to air dry for 1 hour before being returned to the normal feeding cage. Single housing is discouraged as it can lead to depression; wrapping the application area with gauze is also prohibited as it can absorb the medication.

Clinical Symptom Scoring

During the treatment period, the local changes in the hair loss area of the mice were observed, and the hair growth and adverse reactions were recorded in detail, and scores were assigned according to the following table.

| Score | Scoring Standard |
|---|---|
| 0 points | No hair growth |
| 1 point | Sparse hair growth in the hair loss area |
| 2 points | New hair length and density are about half of the non-hair loss area |
| 3 points | No difference in hair growth between the alopecia areata area and the non-alopecia area |

HE Staining

The reagents and instruments required for HE staining included: Hematoxylin staining solution (AR11800-1, BOSTER); Eosin staining solution (AR11800-2, BOSTER); Ultra-clean advanced mounting medium (YZB, BASO); Scott's blueing solution (G1865, Solarbio), etc. Medication refrigerator (BYC-310, Shandong Bokai Biotech); Microscope (CX41 OLYMPUS); Microtome (BQ-318D, Berne); Electric heating blast drying oven (DHG-9070A, Hengke Scientific Instrument Co., Ltd.). The taken tissue was rinsed with running water for several hours, dehydrated through a series of ethanol solutions (70%, 80%, 90%), and then treated with a mixture of pure alcohol and xylene at a ratio of 1:1 for 15 minutes, followed by xylene I for 15 minutes and xylene II for 15 minutes until transparent. The tissue was then placed in a mixture of xylene and paraffin at a ratio of 1:1 for 15 minutes, followed by immersion with paraffin I and paraffin II each for 50-60

minutes. The tissue was embedded in paraffin and sectioned.

The paraffin sections were dried, dewaxed and hydrated in distilled water. The resulting sections were then stained in hematoxylin solution for 3 minutes, differentiated in a hydrochloric acid alcohol solution for 15 seconds, slightly washed, blued in a return blue solution for 15 seconds, rinsed with running water, stained with eosin for 3 minutes, rinsed with running water, dehydrated, cleared, and mounted for microscopic examination.

**II. Experimental Results**

Clinical Symptom Scoring

After administrating the drug imiquimod for modeling, the mice's hair became messy and disordered, forming obvious alopecia areata -like lesions. In the linear graph of hair growth scores at different time points, the MDI-1228 gels with different doses continuously promoted hair growth in the tested animals over time. The positive control drug, compound betamethasone and neomycin cream, promoted hair growth during the first two weeks of treatment, but from the third week, significant side effects gradually appeared, including thinning and atrophy of the skin, telangiectasia, erythema, and a decrease in hair growth rate. By calculating the area under the curve (AUC) of the hair scores for each group and comparing them with the blank gel group, the results showed that MDI-1228 gels at different concentrations significantly promoted hair growth in alopecia areata animals (FIG. 35). After 21 days of drug treatment, the hair growth of the model group mice was slow, and the scores were low. The hair growth scores of all MDI-1228 treatment groups increased to varying degrees, and were significantly higher than the model group, with MDI-1228 (3%) showing the best effect (FIG. 36).

HE Staining

After HE staining, the mouse skin samples were subjected to pathological testing and analysis. The hair follicle structure of normal mice was well developed. In the alopecia areata model group, the epidermis of the mouse skin showed hyperplasia, the dermis had an increased infiltration of fibroblasts, and the hair follicles appeared in clusters, with a large number of keratin plugs extending into some follicle openings. The hair follicles showed atrophy and vacuolation, and the hair papillae moved upward. No significant changes were observed in the blank gel group and the vehicle group, with most hair follicles in the same regression or resting phase as the model group. In the MDI-1228 treatment groups, all concentrations of the drug showed some therapeutic effect, with intact anagen hair follicles in the three concentration groups, notably in the female MDI-1228 (1%) group and the male MDI-1228 (3%) group. However, in terms of the total number of hair follicles and the recovery phase of the hair follicles, the female MDI-1228 (3%) group and the male MDI-1228 (1%) group had a higher number of hair follicles (FIGs. 37 and 38).

**III. Experimental Conclusion**

The above experimental results indicate that MDI-1228 can improve the symptoms of alopecia areata in mice to a certain extent and help promote hair growth.

**Example 69: In vivo Efficacy of the Compound in an Imiquimod-Induced Psoriasis Model**

**I. Experimental Materials and Equipment**

Experimental Materials

5% Imiquimod cream, Aldara, 3M Pharmaceuticals

**Test Compound:**

The test compound was MDI-1228 mesylate, and its preparation method was as follows:

1) Compound MDI-1228 was added to a three-neck flask, to which 100 mL of methanol was added, and then was heated to 50-55°C with stirring until the system becomes viscous. Then, another 50 mL of methanol was added with stirring for 5 hours. The resulting mixture was filtered, and the resulting filter cake was washed with 20 mL of methanol, and vacuum dried at 45°C for 8 hours to obtain 3.7 g of crystals, which was hydrate of MDI-1228.

2) Approximately 200 mg of MDI-1228 hydrate crystals was weighted and charged into a 20 mL glass vial;

3) 54 mg of methanesulfonic acid was added to the 20 mL glass vial, to which 10 mL of EtOAc was added;

4) Suspension stirring was carried out at 25°C for about 3 days to obtain a milky suspension, which was filtered at room temperature under reduced pressure to obtain the solid. The solid was transferred to room temperature vacuum drying for 3 hours to obtain a white powder sample, which was MDI-1228 mesylate with a purity of greater than 99%.

**Vehicle:** 5% DMSO: 75% PEG300: 20% EtOH

**Test Compound Solution Preparation Method:** the compound was weighted and dissolved in DMSO, to which a solution of (PEG300: ethanol = 3.75:1) was added to formulate the highest dose (30 MPK, stock solution). When used, the stock solution was diluted with the vehicle to prepare a low-concentration compound solution.
**Control Compound:** VecticalTM (Calcitriol, i.e., calcipotriol ointment), Ointment, Galderma

Experimental Instruments

Hair clipper: Codos CP-5000.

Compound weighing balance: Sartorius, CPA225D

Animal weighing balance: Changzhou Tianping Electronic Balance, YH2000

Experimental Animals and Housing Conditions

| | |
|---|---|
| Animal strain: | C57BL/6J mice |
| Supplier: | Beijing Charles River Laboratory Animal Technology Co., Ltd. |
| Qualification certificate number: | 20170011006702 |
| Gender and weight: | Female, 8-9 weeks |
| Acclimation period: | 7 days |
| Temperature: | 20-26°C |
| Humidity: | 40-70% |
| Lighting: | Fluorescent light, light (08:00~20:00), dark (20:00~08:00) each for 12 hours |
| Breeding density | 5 animals per cage |
| Food | Free feeding (radiation-sterilized feed, provided by Shanghai Slake Laboratory Animal Co., Ltd.) |
| Water | Free access to water (prepared Water with Moles (ultra) pure water system) |

Experimental Method

**Reagent Preparation**

Imiquimod cream: For all groups except for the normal group, 62.5mg was weighed for each mouse daily.
Vehicle: 5% DMSO was measured to which 75% PEG300 was added, and finally 20% EtOH was added, and then mixed well and stored at 4°C for later use.
MDI-1228 methanesulfonate: the compound was weighted and dissolved in DMSO, and then to which a solution of (PEG300: ethanol = 3.75:1) was added to formulate the highest dose (30MPK, stock solution). When used, the stock solution was diluted with the vehicle to prepare a low-concentration compound solution. The compound was formulated every 3 days and stored at 4°C.

**Mouse Hair Removal**

Before the experiment begins, the back of the mice was shaved using a mold to ensure a consistent area of approximately 2cm*3cm.

**Grouping and Administration Schedule**

One day before the experiment begins, 80 mice were randomly divided into 6 groups based on weight to ensure the average weights each group were consistent. Mice with excessive or insufficient weight within the group, as well as those with dark or excessively wrinkled back skin after shaving, were excluded and not included in this experiment. A total of 55 mice were finally included in the study. The detailed grouping information was shown in the table below:

| **G** | **N** | **Treatment groups** | **dose (mg/kg)** | **Administration route** | **Administration volume** | **Administration Interval** |
|---|---|---|---|---|---|---|
| 1 | 5 | Normal Animal Group | - | - | - | - |
| 2 | 10 | vehicle | - | Topical Application | 5ml/kg | BID × 7d |
| 3 | 10 | Calcitriol | 75 mg/mice | Topical Application | - | QD × 7d |
| 4 | 10 | MDI-1228 Methanesulfonate | 3 | Topical Application | 5ml/kg | BID × 7d |
| 5 | 10 | MDI-1228 Methanesulfonate | 10 | Topical Application | 5ml/kg | BID × 7d |
| 6 | 10 | MDI-1228 Methanesulfonate | 30 | Topical Application | 5ml/kg | BID × 7d |

Imiquimod sensitization and test drug administration were topical applied to the shaved skin on the backs of mice in groups 2 to 6 at 9:00 AM.

Topical Application were carried out on the shaved skin on the backs of mice in groups 2, 4, 5, and 6 at 4:30 PM.

To induce psoriasis-like symptoms in mice from groups 2 to 6, imiquimod cream was applied to the shaved skin on the backs of the mice at 1:00 PM for 7 consecutive days. The normal group mice were shaved but not treated with any substance.

Model Scoring

The body weight of the mice was recorded every morning, and the severity of back skin inflammation was scored. The clinical scoring included three indicators: erythema, crusting, and thickening, each with a score ranging from 0 to 3.

Pathological Analysis

Mouse skin samples were collected in a fixative solution, then were subjected to paraffin embeding, sectioning, and HE staining. A pathologist scored the staining results. The specific scoring criteria for psoriasis skin pathology were shown in the table below.

**Scoring Criteria for Psoriasis Skin Pathology**

| | Item | Score | Description |
|---|---|---|---|
| Corneous layer | Munro abscesses | 1.5 | Munro's microabscess refers to the microabscesses formed by the aggregation of neutrophils within the stratum corneum. |
| | Parakeratosis | 0.5-1.5 | Incomplete keratinization |
| | Hyperkeratosis | 0.5 | Hyperkeratosis |
| Epidermis | length of rete ridges | 0.5-1.5 | The length of the epithelial ridges. In psoriasis, the epithelial ridges often show a pronounced and regular elongation. |
| | Acanthosis | 1 | Acanthosis |
| | lack of granular layer | 1 | Reduction in the granular layer |

(continued)

| | Item | Score | Description |
|---|---|---|---|
| Dermis | dermis lymphocytic infiltrate | 0.5-1 | Lymphocytic infiltration in the dermis |
| | papillary papillae congestion | 1 | Vasodilation within the dermal papillae |
| | thinning above papillae | 0.5 | Thinning above the papillary layer |

Cytokine Detection

The back skin of the animals was frozen rapidly, and homogenized, and the supernatant was collected for subsequent cytokine detection (TNF-$\alpha$) using a hypersensitive assay kit.

Statistical Analysis

Experimental data were expressed as mean $\pm$ standard error of the mean (Mean $\pm$ SEM). Clinical scores and body weight were analyzed using two-way ANOVA, while AUC, cytokine levels, and pathological results were analyzed using one-way ANOVA. A p-value of less than 0.05 was considered statistically significant.

**II. Experimental Results and Discussion**

Clinical Scoring

This experiment evaluated the improvement in clinical scoring by the compound MDI-1228 methanesulfonate in the imiquimod-induced psoriasis model in mice. The average clinical score of the vehicle control group gradually increased, reaching 7.8 points by the fourth day (with a skin thickness score of 2.4 points), indicating the successful establishment of the imiquimod-induced psoriasis model in mice (FIGs. 39-42, Tables 3 and 4). MDI-1228 methanesulfonate at doses of 3, 10, and 30 mg/kg all showed inhibitory effects on the clinical scores of psoriasis in mice, with the 30 mg/kg MDI-1228 methanesulfonate group showing the most significant effect. Regarding skin thickness, the 30 mg/kg MDI-1228 methanesulfonate group showed a significant difference from the solvent group starting from the third day until the end of the experiment, with an effect similar to that of the positive control drug (p<0.0001).

By analyzing the skin thickness score curves of each animal in each group, the area under the curve (AUC) was calculated. Based on the average AUC between groups, the inhibition percentage of each treatment group relative to the vehicle control group was calculated, as shown in FIG. 39. The inhibition percentages of MDI-1228 methanesulfonate at 3, 10, and 30 mg/kg were 30.2%, 25.1%, and 58.1%, respectively. Compared to the vehicle control group, the p-value for the 3 mg/kg dose group was less than 0.05; for the 10 mg/kg dose group, less than 0.01; and for the 30 mg/kg dose group, less than 0.0001. The inhibition percentage of the positive control drug, calcitriol, was 64.2%.

By analyzing the clinical score curves of each animal in each group, the AUC was calculated. Based on the average AUC between groups, the inhibition percentage of each treatment group relative to the vehicle control group was calculated, as shown in FIG. 40. The inhibition percentages of MDI-1228 methanesulfonate at 3, 10, and 30 mg/kg were 10.7%, 12.0%, and 29.8%, respectively, showing a dose-dependent effect. Compared to the vehicle control group, the p-values for the 3 and 10 mg/kg dose groups were less than 0.05; for the 30 mg/kg dose group, less than 0.0001. The inhibition percentage of the positive control drug, calcitriol, was 47.3%.

At the end of the experiment, the spleens of the mice were collected and weighed. In this model, spleen weight was positively correlated with the severity of the disease. As shown in FIG. 43, the 30 mg/kg MDI-1228 methanesulfonate group significantly inhibited the increase in spleen weight (p<0.05).

Table 3. Statistical Analysis of Skin Thickness Scores in Imiquimod-Induced Psoriasis Mice

| Day | G1 normal animal | G2 vehicle tropical application | G3 Calcitriol tropical application | G4 MDI-1228_mesylate 3mg/kg tropical application | G5 MDI-1228_mesylate 10mg/kg tropical application | G6 MDI-1228_mesylate 30mg/kg tropical application |
|---|---|---|---|---|---|---|
| | mean ± standard error | mean ± standard error | mean ± standard error | mean ± standard error | mean ± standard error | mean ± standard error |
| 0 | 0.0±0.00 | 0.0±0.00 | 0.0±0.00 | 0.0±0.00 | 0.0±0.00 | 0.0±0.00 |
| 1 | 0.0±0.00 | 0.0±0.00 | 0.0±0.00 | 0.0±0.00 | 0.0±0.00 | 0.0±0.00 |
| 2 | 0.0±0.00 | 0.1±0.10 | 0.0±0.00 | 0.0±0.00 | 0.0±0.00 | 0.0±0.00 |
| 3 | 0.0±0.00 **** | 1.4±0.16 | 0.7±0.15 *** | 1.4±0.16 | 1.2±0.13 | 0.6±0.16 **** |
| 4 | 0.0±0.00 **** | 2.4+0.16 | 1.2+0.20 **** | 1.7±0.15 *** | 2.0±0.15 | 1.4±0.27 **** |
| 5 | 0.0±0.00 **** | 2.4±0.16 | 0.8±0.20 **** | 1.4±0.16 **** | 1.8±0.13 ** | 0.9±0.18 **** |
| 6 | 0.0±0.00 **** | 1.9±0.18 | 0.5±0.17 **** | 1.4±0.16 * | 1.2±0.13 *** | 0.8±0.13 **** |
| 7 | 0.0±0.00 **** | 1.5±0.22 | 0.0±0.00 **** | 0.7±0.15 **** | 1.0±0.21 * | 0.1±0.10 **** |
| *p<0.05, **p<0.01, ***p<0.001, ****p<0.0001 vs. vehicle control group, Two-way ANOVA | | | | | | |

Table 4. Statistical Analysis of Clinical Scores in Imiquimod-Induced Psoriasis Mice

| Day | G1 normal animal | G2 vehicle tropical application | G3 Calcitriol tropical application | G4 MDI-1228_mesylate 3mg/kg tropical application | G5 MDI-1228_mesylate 10mg/kg tropical application | G6 MDI-1228_mesylate 30mg/kg tropical application |
|---|---|---|---|---|---|---|
| | mean ± standard error | mean ± standard error | mean ± standard error | mean ± standard error | mean ± standard error | mean ± standard error |
| 0 | 0.0±0.00 | 0.0±0.00 | 0.0±0.00 | 0.0±0.00 | 0.0±0.00 | 0.0±0.00 |
| 1 | 0.0±0.00 | 0.3±0.15 | 0.2±0.13 | 0.3±0.15 | 0.2±0.13 | 0.3±0.15 |
| 2 | 0.0±0.00 **** | 1.7±0.21 | 1.2±0.13 | 1.4±0.16 | 1.3±0.15 | 1.2±0.13 |
| 3 | 0.0±0.00 **** | 5.2±0.20 | 3.1±0.28 **** | 5.2±0.20 | 5.1±0.18 | 3.7±0.33 **** |
| 4 | 0.0±0.00 **** | 7.8±0.29 | 4.3±0.26 **** | 7.1±0.23 | 7.1±0.18 | 5.8±0.51 **** |
| 5 | 0.0±0.00 **** | 7.1±0.23 | 2.9±0.18 **** | 6.3±0.21 * | 6.4±0.27 | 5.3±0.26 **** |
| 6 | 0.0±0.00 **** | 5.0±0.21 | 2.5±0.17 **** | 4.1±0.28 ** | 3.8±0.20 **** | 2.9±0.18 **** |
| 7 | 0.0±0.00 **** | 3.5±0.22 | 2.0±0.00 **** | 2.7±0.15 * | 3.0±0.21 | 2.1±0.10 **** |
| *p<0.05, **p<0.01, ****p<0.0001 vs. vehicle control group, Two-way ANOVA | | | | | | |

Pathological Results

The mouse skin samples were collected in fixative solution, then were subjected to paraffin embedding, sectioning, and HE staining. A pathologist scored the staining results. The pathological scoring results for psoriasis skin were shown in FIG. 44. MDI-1228 methanesulfonate at doses of 3, 10, and 30 mg/kg significantly inhibited the phenomena of incomplete keratinization, epidermal spine length, acanthosis, and lymphocyte infiltration in psoriasis-like dermatitis, with inhibition percentages of the total pathological score of 59.7%, 51.6%, and 50.8%, respectively. Histopathologically, calcitriol showed a trend of mitigation.

TNF-α Detection Results

The back skin of the animals was frozen rapidly and homogenized, and the supernatant was collected for subsequent TNF-α cytokine detection using a hypersensitive assay kit. The cytokine detection results were shown in FIG. 45. Corresponding to the pathological results, MDI-1228 methanesulfonate at doses of 3, 10, and 30 mg/kg significantly inhibited the concentration of TNF-α in the skin samples (*p<0.05).

**Experimental Conclusions**

The test compound MDI-1228 methanesulfonate at doses of 3, 10, and 30 mg/kg all showed significant improvement in psoriasis; the test compound MDI-1228 methanesulfonate at doses of 3, 10, and 30 mg/kg had a significant inhibitory effect on the cytokine TNF-α of skin samples in psoriasis model.

**Example 70: Therapeutic Efficacy of the Compound on Acne Model in Rabbits**

**I. Materials and Methods**

Experimental Animals

New Zealand white rabbits weighing 1.7-2.2 kg (Supplier: Ganzhou Animal Husbandry Research Institute, License No.: SCXK(Gan)2018-0009), male, were housed under conditions of normal diet, temperature of 20-26°C, and humidity of 40%-70%.

Main Reagents and Drug Preparation

**1) Preparation of 2% coal tar solution:**

49 ml of corn oil was taken, to which n1 ml of coal tar was added, and shaken well to mix thoroughly.

**2) Columbia blood agar plate:**

3.9 g of Columbia blood agar base medium powder was dissolved in 100 ml of distilled water, sterilized at 121°C for 20 min, and cooled to 50-55°C, to which 5 ml of sterile defibrinated sheep blood was added. The resulting mixture was mixed well, and poured into plates for later use. BHI liquid medium: 3.85 g of BHI was dissolved in 100 ml of distilled water, sterilized at 121°C for 20 min, cooled, and stored in the refrigerator for later use.

**3) Propionibacterium acnes (P. acnes ATCC11827):**

The bacterial strain was activated on Columbia blood agar plates, then inoculated into BHI medium and cultured under anaerobic conditions (10%H2, 10%CO2, and 80%N2) at 37°C for 48h. The cultured bacteria were collected using centrifugation, suspended in PBS, and the resulting suspension will be 6×108 CFU/ml.

**4) Vhechile preparation (5% DMSO + 75% PEG300 + 20% EtOH):**

Mix 1 ml of DMSO + 4 ml of EtOH + 15 ml of PEG300 well to obtain a colorless clear solution.

**5) Preparation of 20 mg/ml MDI-1228 (1 mg/kg):**

0.0817 g of MDI-1228 was weighted and dissolved in 0.21 ml of DMSO, to which 0.82 ml of EtOH was added, then 3.08 ml of PEG300 was added to obtain a pale white opalescent solution.

**6) Preparation of 60 mg/ml MDI-1228 (3 mg/kg):**

0.2634 g of MDI-1228 was weighted and dissolved in 0.22 ml of DMSO, to which 0.88 ml of EtOH was added, then 3.29 ml of PEG300 was added to obtain a white opalescent solution.

**7) Preparation of 180 mg/ml MDI-1228 (9 mg/kg):**

0.5788 g of MDI-1228 was weighted and dissolved in 0.16 ml of DMSO, to which 0.64 ml of EtOH was added, then 2.41 ml of PEG300 was added to obtain a white opalescent solution.

**8) Preparation of 60 mg/ml MDI-1288 (3 mg/kg):**

0.2595 g of MDI-1288 was weighted and dissolved in 0.22 ml of DMSO, to which 0.86 ml of EtOH was added, then 3.24 ml of PEG300 was added to obtain a yellow clear solution.

Animal Grouping and Modeling

Blank Control Group (no treatment) (N=5+2)

Model Group (vehicle treatment: 5% DMSO + 75% PEG300 + 20% EtoH, twice per day per animal, topical application, for 2 weeks) (N=8+2)

Model + Positive Control Group (1% Clindamycin gel, 0.5 g/kg, once per day per animal, topical application, for 2 weeks) (N=8)

Model + Low-Dose MDI-1228 Compound Group (dose: 1 mg/kg each time, twice per day per animal, topical application, for 2 weeks) (N=8)

Model + Medium-Dose MDI-1228 Compound Group (dose: 3 mg/kg each time, twice per day per animal, topical application, for 2 weeks) (N=8)

Model + High-Dose MDI-1228 Compound Group (dose: 9 mg/kg each time, 2 twice per day per animal, topical application, for 2 weeks) (N=8)

Model + MDI-1288 Compound Group (dose: 3 mg/kg each time, twice per day per animal, topical application, for 2 weeks) (N=8)

**Modeling:** Rabbits were acclimated for 7 days. On the right inner ear canal exit of the rabbits, a 2 cm×2 cm area was treated with 2% coal tar solution once a day, 0.5-1 mL each time, for 26 consecutive days. Propionibacterium acnes were directly inoculated into a spore-forming bacteria culture medium, placed in an anaerobic bag, and cultured at 37°C for 48 hours. The Propionibacterium acnes were adjusted to 6×108 CFU/ml with normal saline and reserved. On the 8th day, Propionibacterium acnes was injected intradermally at 3-4 sites on the rabbit ears, 50 μl per rabbit, injected every other day for a total of 5 times. Treatment was started randomly on the 9th day after the last injection.

Animal Administration

Rabbits were treated according to their groups. The blank group received no treatment. The positive control group was given Clindamycin gel, applied once a day for 14 consecutive days. The low, medium, and high doses of MDI-1228 compounds and MDI-1288 were applied to the ears twice a day for 14 consecutive days. The model group received an equal amount of vehicle treatment, with the same application method and duration as the test drug groups.

Detection Indicators

Twenty-four hours after the last administration, a 2.8 cm punch was used to prepare ear samples at the application site, and their weight was measured. The ears from the modeling site were fixed with 4% paraformaldehyde, and consecutive sections (3 slices) were taken from the same location for HE staining and histopathological analysis. At the same time, the area of 3 hair follicles and the diameter of 3 sebaceous glands were measured to calculate the average values. Blood was drawn from the heart and centrifuged to obtain serum, and the levels of interleukin-1α (IL-1α), interleukin-6 (IL-6), and dihydrotestosterone (DHT) in serum were determined according to the ELISA kit instructions.

Pathological Examination

**HE Staining:**

The reagents and instruments required for HE staining included: Hematoxylin staining solution (AR11800-1, BOSTER); Eosin staining solution (AR11800-2, BOSTER); Ultra-clean advanced mounting medium (YZB, BASO); Scott's blueing solution (G1865, Solarbio), etc.

The apparatus included: Medication refrigerator (BYC-310, Shandong Bokai Biotech); Microscope (CX41 OLYMPUS); Microtome (BQ-318D, Berne); Electric heating blast drying oven (DHG-9070A, Hengke Scientific Instrument Co., Ltd.).

Tissue samples from animal ears were excised, fixed in 4% paraformaldehyde, dehydrated, embedded in paraffin, and sectioned and HE stained for observation of histopathological morphology changes. The tissue samples were fixed in 4% paraformaldehyde at normal temperature and the taken ears tissue was rinsed with running water for several hours, dehydrated through a series of ethanol solutions (70%, 80%, 90%), and then treated with a mixture of pure alcohol and xylene for 15 minutes, followed by xylene I for 15 minutes and xylene II for 15 minutes (until transparent). The tissue was then placed in a mixture of xylene and paraffin at a volume ratio of 1:1 for 15 minutes, followed by immersion with paraffin I and paraffin II each for 50-60 minutes. The tissue was embedded in paraffin and sectioned, and the sections were baked, dewaxed and hydrated in distilled water. The resulting sections were then stained in hematoxylin solution for 3 minutes, differentiated in a hydrochloric acid alcohol solution for 15 seconds, slightly washed, blued in a return blue solution for 15 seconds, rinsed with running water, stained with eosin for 3 minutes, rinsed with running water, dehydrated, cleared, and mounted for microscopic examination.

**ELISA detection:**

A double-antibody one-step sandwich Enzyme-linked Immunosorbent Assay (ELISA) ket was used. A capture antibody for the mouse target indicators was pre-coated onto microplate wells, to which samples, standards, and HRP-labeled detection antibodies were added sequentially, followed by incubation and thorough washing. TMB substrate was added for color development, which turned into blue in the presence of peroxidase and then into yellow after acidification. The depth of color was positively correlated with the concentration of the target in the samples. The absorbance (OD450 value) was measured at a wavelength of 450 nm using the microplate reader, and the sample concentration was calculated.

The information of the kits used was as follows: rabbit interleukin-1$\alpha$ (IL-1$\alpha$) kit (MM-8267601, enzyme immunoassay), rabbit interleukin-6 (IL-6) kit (MM-030201, enzyme immunoassay), and rabbit dihydrotestosterone (DHT) kit (MM-8267901, enzyme immunoassay).

Instruments

| Experimental instruments | Model | Supplier |
|---|---|---|
| Pipette | 100-1000ul | eppendorf |
| Pipette | 20-200ul | eppendorf |
| Pipette | 5-50ul | eppendorf |
| Automatic Microplate Reader | WD-2102B | Beijing Liuyi Biotech |
| Refrigerator/Freezer | BD/BC-415DKEM | Midea |
| Electric Heated Constant Temperature Incubator | DHP-9054 | Shandong Bok Biological Industry Co., Ltd. |
| Pharmaceutical Refrigerator | BYC-310 | Shandong Bok Biotech |
| Microscope | BX43 | OLYMPUS |
| Slicer | 2235 | Leica |
| Electric Heated Constant Temperature Blast Drying Oven | HGZF-101-1 | Shanghai Yuejin Medical Instrument Co., Ltd. |

**II. Experimental Results**

1. Observation of General Symptoms in Rabbits and the Effect of Drugs on Rabbit Ear Weights

In general observations, the ears of rabbits in the normal group were thin, soft, with clear capillaries, and visible

distinct cell hair follicles with fine hair. In the model group, the ears were significantly thickened, with enlarged and rough pores. In the measurement of rabbit ear weights, compared to the blank control group, the ear weight in the model group significantly increased. The positive control drug, clindamycin, did not reduce ear weight, while the 3 mg/kg MDI-1228, 9 mg/kg MDI-1228, and 3 mg/kg MDI-1288 groups reduced ear weight and alleviated the thickening of rabbit ears (FIG. 46).

2. HE Staining Results

In the model (vehicle) group, there was thickening of corneous layer, uneven epidermal layer, disordered structure, enlarged pores, increased sebaceous gland acinar cells, and dermal hemorrhage and inflammatory cell infiltration. In the Clindamycin positive control group, there was still a significant amount of keratin hyperplasia, with reduced acne lesions on the epidermis, but the enlarged pore issue was not improved. Among the other treatment groups, data analysis showed that the 3 mg/kg MDI-1228 medium-dose group had the best treatment effect. Compared to the other groups, the medium-dose group showed more significant pore constriction, higher healing of acne lesions on the epidermis, and a relatively lower degree of keratinization in the stratum corneum (FIG. 47). By measuring and calculating the sebaceous gland diameter and hair follicle area of each section, the results showed that there was no significant change in sebaceous gland diameter across groups (FIG. 48), but there was a significant change in hair follicle area. The hair follicle area in the model group significantly increased, which was not improved by Clindamycin treatment, but both 3 mg/kg MDI-1228 and 9 mg/kg MDI-1228 significantly reduced the enlargement of hair follicles (FIG. 49).

3. The Effect of Drugs on Rabbit Serum IL-1$\alpha$, IL-6, and DHT Levels

DHT can bind to receptors inside sebaceous gland cells, stimulating the proliferation and secretion of sebaceous gland cells. The hyperkeratosis of hair follicle sebaceous glands causes the narrowing of the channel for sebum excretion, leading to an anaerobic environment that promotes the reproduction of Propionibacterium acnes, forming the acne pathogenesis axis of sebaceous hyperplasia, blocked sebum excretion, and bacterial infection. The binding of Propionibacterium acnes to toll-like receptors triggers a series of inflammatory responses, inducing the production of IL-1$\alpha$ and IL-6, which further cause inflammatory damage to the hair follicles. In this model, there was an abnormal increase in testosterone, leading to an increase in DHT synthesis due to the substantial increase in testosterone.

The detection of IL-1$\alpha$, IL-6, and DHT in rabbit each group showed that, compared to the blank control group, the model (vehicle) group had significantly increased levels of IL-1$\alpha$, IL-6, and DHT. The 3 mg/kg MDI-1228, 9 mg/kg MDI-1228, and 3 mg/kg MDI-1288 groups significantly reduced IL-1$\alpha$ and DHT levels compared to the model (vehicle) group (FIGs. 50 and 52). The 1 mg/kg MDI-1228 low-dose group showed a trend of reduction in all three indicators (FIG. 51).

**III. Conclusion**

The above experiments indicate that in the coal tar combined with Propionibacterium acnes-induced acne model in rabbits, MDI-1288 and MDI-1228 have the effects of reducing the expression of inflammatory factors and improving acne.

**Example 72: Effect of the Compound on Lipolysis in Vitro in Adipocytes**

Newly extracted neonatal C57BL/6 mouse skin fibroblasts (specific operational steps can be found in the literature: Cell Reports (2023)/doi: 10.1016/j.celrep.2023.112647) were plated and cultured in petri dishes until the cell density reached the highest and contact inhibition. Differentiation medium (DMEM + 10% FBS + 1x antibiotic; specific formula can be found in the literature: Cell Reports (2023)/doi: 10.1016/j.celrep.2023.112647) was then added to induce the generation of new adipocytes. Subsequently, insulin was added to promote adipocyte maturation, followed by the addition of experimental drugs (MDI-1228 or Tofacitinib). The control group received an equal volume of DMSO. The transformation of adipocytes was observed using a Leica inverted phase contrast microscope. The experimental process and schedule were shown in FIG. 53A. From the phase contrast images of the cells (FIG. 53B), it can be seen that the addition of MDI-1228 (5 $\mu$M) to the already differentiated mature adipocytes promotes the lipolysis of adipocytes into smaller lipid droplets, while the addition of another JAK inhibitor, Tofacitinib (5 $\mu$M), does not promote lipolysis.

**Example 73: Therapeutic Effect of the Compound on a Diabetic Foot Animal Model**

**1. Establishment of a High-Fat-Induced Obesity Model**

Twenty 2-month-old normal wild-type C57BL/6 male mice were ordered from Jicai Pharmaceutical and divided

into two groups of 10 each. One group was fed a standard diet (SD), and the other group was fed a 60% high-fat diet (HFD) to induce obesity. Both groups were fed under the same conditions for about 6 months.

Additionally, five 2-month-old young male mice were ordered to perform subsequent wound modeling and comparison with the SD group (8 months old, 10 mice) and the HFD group (8 months old, 10 mice).

### 2. Mouse Wound Modeling + Drug Administration

#### 2.1. Mouse Wound Modeling:

Mice were anesthetized with isoflurane, and a razor was used to remove the back hair, exposing the back skin. A marker pen and ruler were used to draw a 1.0 cm x 0.5 cm rectangular area on each side of the back. A sterilized surgical scissor was used to cut away the skin in this area without damaging the subcutaneous tissue. After the mice regained consciousness, they were carefully placed back in their cages. The animals were euthanized at specified time intervals after the injury, and the wound tissue was collected for subsequent experiments and analysis.

#### 2.2. Drug Administration:

After creating wounds on the left and right sides of the mouse's back, the mice were grouped and administered drugs as indicated in the table below (where Control represents a blank drug control with DMSO + vehicle). The 1228 gel (0.5% or 1%) was administered every 12 hours, with a dose of 50mg each time. The wounds were photographed and weighed daily (every 24 hours) for 7 days, and then samples were collected at the 7$^{th}$ day. Specific operational details are described in the section "3. Sample Collection and Analysis" below.

| Group | Mice | Number | Administration | |
|---|---|---|---|---|
| **1** | | **5** | Left | **control** |
| | | | Right | **0.5%**MDI-1228 |
| **2** | **SD** | **3** | Left | **control** |
| | | | Right | **0.5%**MDI-1228 |
| | | **2** | Left | **0.5%**MDI-1228 |
| | | | Right | **0.5%**MDI-1228 |
| **3** | **SD** | **3** | Left | **control** |
| | | | Right | **1%**MDI-1228 |
| | | **2** | Left | **1%**MDI-1228 |
| | | | Right | **1%**MDI-1228 |
| **4** | **HFD** | **3** | Left | **control** |
| | | | Right | **0.5%**MDI-1228 |
| | | **2** | Left | **0.5%**MDI-1228 |
| | | | Right | **0.5%**MDI-1228 |
| **5** | **HFD** | **3** | Left | **control** |
| | | | Right | **1%**MDI-1228 |
| | | **2** | Left | **1%**MDI-1228 |
| | | | Right | **1%**MDI-1228 |

#### 2.3. Observation (Recording Wound Area Changes Daily)

After anesthesia, the mice were placed on an operation table with their backs extended, and high-definition photographs of the entire back and the local wound area of the mice were taken (including a ruler). These data was used for later display of wound images and quantitative comparison of wound size. The body weight of the mice was also measured to analyze the impact of the drug on systemic metabolism.

## Sample Collection and Analysis

### 3.1. Sample Collection Procedure

On the seventh day of wound repair after modeling on the mouse's back, the mice were euthanized, and the wound tissue was collected for subsequent experiments and analysis, including tissue from the center of the wound and distal non-wounded control skin. The tissue was embedded in OCTand subjected to cryosectioning for subsequent HE or multiple staining analysis.

### 3.2. Freezing Tissue and Sectioning

Mouse skin tissue was placed in an embedding box filled with OCT (Tissue-Tek® O.C.T. Compound, Catalog#4583, Sakura Finetek USA Inc) and laid flat on dry ice to quick-freeze. Once the OCT was fully frozen, the embedding box was transferred to a -20°C freezer for storage. Before sectioning, the cryostat was pre-cooled to -25°C. The frozen tissue block was fixed on the sample holder, and the section thickness was first set to 50um to level the tissue, then adjusted to 16um for formal sectioning. The flattened tissue sections were attached to glass slides and checked under a microscope for completeness. The sections were left to air-dry at room temperature for 2-3 hours before being stored in a -20°C freezer.

### 3.3. HE Staining

The sections were taken out of the -20°C freezer and allowed to thaw at room temperature, and the condensation water was dried for 10-20 minutes until completely dry. The sections were then fixed with 500-1000 μL of frozen 4% paraformaldehyde (4% paraformaldehyde prepared by diluting 10 ml of 16% PFA- paraformaldehyde in 30 ml of frozen PBS and stored at 4°C) for 10 minutes at room temperature. The sections were washed once with dH2O (single distilled water) (incubation <1 minute). After washing, hematoxylin solution was added, and the sections were incubated for 55-60 seconds, then quickly washed three times with running tap water until no blue dye was visible. Eosin solution was added next, and the sections were incubated for 25-30 seconds, then quickly washed three times with running tap water, followed by washing with 70% ethanol once. The sections were dehydrated in 100% ethanol for 5 minutes and then in xylene for 5 minutes. Finally, the sections were coverslipped with a neutral resin, taking care not to trap air bubbles when placing the coverslip with forceps. After allowing time to set in a fume hood, the results could be observed under a microscope and recorded with photographs.

The experimental results were shown in FIG. 54.

FIG. 54A: The non-wounded skin and wound tissue from each group of mice were excised, embedded in OCT, and subjected to cryosectioning. The tissue was fixed with PFA and used for hematoxylin and eosin (HE) staining. The image showed the overall view of the stained tissue, with the area to be enlarged outlined by a yellow dashed line, and the epidermal migration tongue structure at the wound edge was outlined in yellow.

FIG. 54B: Enlarged view of the area within the yellow frame in FIG. 54A, with the white hollow structures preliminarily identified as adipose tissue, and the scale bar represents 400 micrometers.

From FIG. 54A, it can be seen that the epidermal migration tongues above the wound in the HFD group were the farthest apart, the wound in the 2MSD group (i.e., SD-2M group) was the smallest, and it contaied the most substantial cells. In the MDI-1228 treatment group, compared to the control group, there was less blank area (preliminarily judged as adipocytes) and more fibroblast-like cells in the wound. After further magnifying the local area at the center of the wound (FIG. 54B), it can be observed that the size of the adipocytes in the wound center was significantly reduced after MDI-1228 treatment, over the control group suggesting that the inhibitor MDI-1228 may have promoted the lipolysis of adipocytes and their transdifferentiation into pAd or myoFB.

Additionally, through Bodipy (green for lipid droplets) / PHA (red for actin) and nuclear (DAPI staining, blue) staining experiments, it was further discovered that in the MDI-1228 treatment group, the area of green lipid droplets in the wound center was significantly reduced, and the number of small lipid droplets increased; which further confirmed that MDI-1228 may have promoted the lipolysis of adipocytes in the wound and their transdifferentiation into pAd/myoFB.

### 3.4. DAPI Staining

To further clarify the effect of the drug on the migration of epithelial cells towards the wound center, the wound tissue was stained with Bodipy (green for lipid droplets) / PHA (red for actin), and the cell nuclei were stained with DAPI (blue), which clearly distinguishes the tissue structures of different skin layers. Bodipy was purchased from ThermoFisher

Scientific (Catalog D3922); PHA was purchased from Cytoskeleton Inc (Catalog PHDH1-A); and the mounting medium containing DAPI was purchased from ThermoFisher Scientific (Catalog P36931). The specific staining method is referenced in the literature Cell Reports (2023)/doi: 10.1016/j.celrep.2023.112647.

The Bodipy/PHA staining process was as follows: The sections were taken out of -20°C and thawed and dried at room temperature for 10 minutes. The top and bottom of the slides were marked with an Immunopen slide marker pen. The slides were then fixed with 1 ml of 4% paraformaldehyde solution at room temperature for 10 minutes, washed with PBS solution for 5 minutes, which process was repeated twice. The tissue area on the slide was circled with the marking pen. The tissue slides were then incubated in diluted Bodipy (lipid droplet dye) and Rhodamine Phalloidin (actin dye) solutions in PBS buffer at room temperature in a suitable humidity and dark environment for 1 hour, followed by washing with PBS solution for 10 minutes, which process was repeated three times. Finally, the stained sections were mounted with ProLong™ Gold Antifade Mountant with DAPI, which stains the nuclei blue with DAPI, and the mounted sections were stored at 4°C. The sections were then observed and photographed using a confocal microscope.

The experimental results were shown in FIG. 55. In FIG. 55, the migrating epithelium tongues towards the wound center from both sides were marked with yellow dotted lines, and the distance between the centers of the epithelium on both sides was marked with red double arrows, with the length quantified.

FIG. 55 (A): The wound skin of mice in various groups (SD, standard diet; HFD, high fat diet; ctrl = blank gel; 1228 = 1% MDI-1228 gel) was stained with Bodipy+PHA+DAPI. The migrating epithelium tongues towards the center from both sides of the wound was marked with yellow dotted lines, and the distance without epithelial migration tongue at the wound center (wound non-healing distance) was marked with red double arrow lines, with quantification.

FIG. 55 (B): A bar graph quantifying the wound non-healing distance from FIG. 55 (A), with the y-axis in millimeters (mm). The p-value for significant differences between groups (One way Anova) was noted on the graph, with each group consisting of 3-4 wounds.

During the wound healing process, the epithelium on both sides was activated to form migrating tongues, which move towards the wound center. When the two migrating tongues meet at the wound center, re-epithelialization was complete (Adv Wound Care (New Rochelle). 2014 Jul 1;3(7):445-464.). Therefore, by measuring the distance of the migrating tongue, the process of wound re-epithelialization can be quantified more accurately. The inventor quantitatively measured the distance of the migrating tongue between different groups and found that the epithelial healing in high-fat diet (HFD) mice was slower than that in the normal diet (SD) group and that administrating 1% MDI-1228 to the HFD group could effectively promote epithelial healing (FIG. 55).

## Conclusion

The above experiments demonstrate that in the diabetic foot model in mice, MDI-1288 can significantly promote lipolysis, significantly increase the proportion of myofibroblasts, and effectively promote wound healing.

Although specific embodiments of the present disclosure have been illustrated and described, it does not mean that these embodiments illustrate and describe all possible implementation forms of the present disclosure. More precisely, the language used in this specification are only descriptive words and not restrictive. It will be obvious to those skilled in the art that various kinds of changes and modifications can be made without departing from the general scope of the present disclosure. Therefore, the appended claims are intended to include all these changes and modifications within the scope of the present disclosure.

## Claims

**1.** Use of a compound of Formula (G),

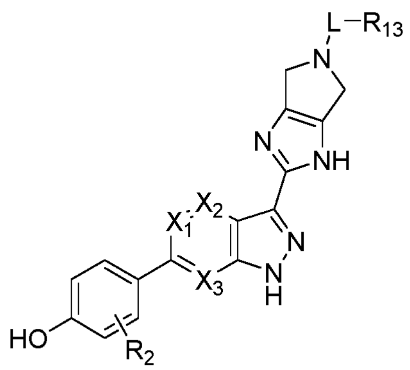

(G)

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, in the preparation of a TRK-inhibiting medicine and/or a RET-inhibiting medicine,

in which

L is C=O, O=S=O, $CH_2$ or a bond; and

$X_1$ is N or $CR_{14}$; and

$X_2$ is N or $CR_{15}$; and

$X_3$ is N or $CR_{16}$; and

$R_{14}$, $R_{15}$, $R_{16}$ are each independently selected from H, -OH, -SH, -CN, halogen, $-NO_2$, - $SF_5$, -S-$C_{1-4}$ alkyl, $C_{1-6}$ alkyl, $C_{2-8}$ alkenyl, $C_{2-8}$ alkynyl, $C_{1-6}$ alkoxy, $C_{3-7}$ cycloalkyl, 3-7 membered heterocycloalkyl, $C_{5-7}$ aryl, 5-7 membered heteroaryl, $-N(R_9)(R_{10})$, $-N(R_{11})(C(=O)R_{12})$, $-C(=O)-N(R_9)(R_{10})$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the -S-$C_{1-4}$ alkyl, $C_{1-6}$ alkyl, $C_{1-6}$ alkoxy, $C_{3-7}$ cycloalkyl, and 3-7 membered heterocycloalkyl are optionally substituted with 1, 2 or 3 substitutes selected from halogen, -OH, $-NH_2$, $-NH(CH_3)$, $-N(CH_3)_2$, -CN, $C_{1-4}$ alkyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, $-C(=O)-NH_2$, -C(=O)-N($C_{1-4}$ alkyl)$_2$, -N($C_{1-4}$ alkyl)(C(=O) $C_{1-4}$ alkyl), $C_{1-4}$ haloalkyl, $C_{1-4}$ alkoxy and $C_{1-4}$ haloalkoxy; and

$R_{13}$ is H, $-N(R_{17})(R_{18})$, $C_{1-6}$ alkoxy, $-SR_{12}$, $-OR_{12}$, -CN, halogen, $-NO_2$, $-SF_5$, -S-$C_{1-4}$ alkyl, $C_{1-6}$ alkyl or $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$bicycloalkyl, or 5-11 membered bicyclic heteroalkyl, and $R_{13}$ is substituted with 0, 1, 2, 3 or 4 $R_1$(s), in which $R_{17}$ and $R_{18}$ are each independently selected from H, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, $C_{3-7}$ heterocycloalkyl, $C_{5-7}$ aryl, 5-7 membered heteroaryl, $C_{7-11}$ bicyclic aryl, 7-11 membered bicyclic heteroaryl, 11-15membered tricyclyl, $C_{5-11}$ bicycloalkyl, and 5-11 membered bicyclic heteroalkyl and are optionally substituted with one or more substitutes each independently selected from -OH, - CN, -SH, halogen, $-NO_2$, $-SF_5$, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, $-N(R_9)(R_{10})$, - $N(R_{11})(C(=O)R_{12})$, $-C(=O)-N(R_9)(R_{10})$, $-C(=O)-R_{12}$, $-C(=O)-OR_{12}$, $-OC(=O)R_{12}$, - $N(R_{11})(S(=O)_2R_{12})$, $-S(=O)_2-N(R_9)(R_{10})$, $-SR_{12}$ and $-OR_{12}$, wherein the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2 or 3 substitutes each independently selected from halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, $-C(=O)-OR_{12}$, -C(=O)H, $-C(=O)R_{12}$, $-C(=O)-N(R_9)(R_{10})$, $-N(R_{11})(S(=O)_2R_{12})$, $-S(=O)_2-N(R_9)(R_{10})$, $-SR_{12}$ and $-OR_{12}$; or $R_{17}$, $R_{18}$ and the N atom connected thereto together form a 3-14 membered ring; and

0, 1, 2, 3 or 4 $R_2$(s) are present in formula (G), and $R_2$ is selected from H, halogen, -OH, $-NO_2$, -CN, $-SF_5$, -SH, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, $-N(R_9)(R_{10})$, $-N(R_{11})(C(=O)R_{12})$, $-C(=O)-N(R_9)(R_{10})$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, - $C(=O)-OR_{12}$, -C(=O)H, $-C(=O)R_{12}$, $-C(=O)-N(R_9)(R_{10})$, $-N(R_{11})(S(=O)_2R_{12})$, $-S(=O)_2-N(R_9)(R_{10})$, $-SR_{12}$ and $-OR_{12}$; and

$R_1$ is selected from H, halogen, -OH, $-NO_2$, -CN, $-SF_5$, -SH, -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$bicycloalkyl, 5-11 membered bicyclic heteroalkyl, $-N(R_9)(R_{10})$, - $N(R_{11})(C(=O)R_{12})$, $-C(=O)-N(R_9)(R_{10})$, $-C(=O)-R_{12}$, $-C(=O)-OR_{12}$, $-OC(=O)R_{12}$, - $N(R_{11})(S(=O)_2R_{12})$, $-S(=O)_2-N(R_9)(R_{10})$, $-SR_{12}$ and $-OR_{12}$, in which the -S-$C_{1-4}$ alkyl, $C_{1-8}$ alkyl, $C_{2-8}$ alkenyl, $C_{2-8}$ alkynyl, and $C_{1-8}$ alkoxy are optionally substituted with 1, 2, 3, or 4 $R_3$(s), and in which the $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2, 3, or 4 $R_4$(s); and

$R_3$ and $R_4$ are each independently selected from H, halogen, -OH, $-NO_2$, -CN, $-SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, $-N(R_5)(R_6)$, $-N(R_{11})(C(=O)R_{12})$, $-CON(R_7)(R_8)$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2, 3 or 4 substituent(s)

each independently selected from the group consisting of halogen, -CN, -OH, $C_{1-4}$ alkyl, $C_{1-6}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), -C(=O)-O$R_{12}$, -C(=O)H, -C(=O)$R_{12}$, -C(= O)-N($R_9$)($R_{10}$), -N($R_{11}$)(S(=O)$_2$$R_{12}$), -S(=O)$_2$-N($R_9$)($R_{10}$), -S$R_{12}$ and -O$R_{12}$; and
$R_5$, $R_6$, $R_7$, $R_8$, $R_9$, $R_{10}$, $R_{11}$, and $R_{12}$ are each independently H or selected from the group consisting of $C_{1-6}$ alkyl, $C_{1-4}$ haloalkyl, $C_{3-7}$ cycloalkyl, 4-14 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein the options included in the above group are each optionally substituted with 1, 2, 3 or 4 substituent(s) each independently selected from the group consisting of halogen, -$CF_3$, -OH, -$NH_2$, -NH($CH_3$), -N($CH_3$)$_2$, -CN, oxo, $C_{1-4}$ alkyl, $C_{2-6}$ alkenyl, $C_{2-6}$ alkynyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, -C(=O)-$NH_2$, -C(=O)-N($C_{1-4}$ alkyl)$_2$, $C_{1-4}$ haloalkyl, $C_{1-4}$ alkoxy and $C_{1-4}$ haloalkoxy.

**2.** The use according to claim 1, wherein all Hs are each independently optionally substituted with D.

**3.** The use according to claim 1 or 2, wherein only one of $X_1$, $X_2$, and $X_3$ is N.

**4.** The use according to claim 1 or 2, wherein only two of $X_1$, $X_2$, and $X_3$ are N.

**5.** The use according to claim 1 or 2, wherein $X_1$, $X_2$ and $X_3$ are the same.

**6.** The use according to claim 5, wherein $X_1$ is $CR_{14}$, $X_2$ is $CR_{15}$, $X_3$ is $CR_{16}$ and $R_{14}$, $R_{15}$ and $R_{16}$ are the same.

**7.** The use according to claim 6, wherein $R_{14}$, $R_{15}$, and $R_{16}$ are selected from H, -OH, - SH, -CN, halogen, -$NO_2$, and $C_{1-6}$ alkyl.

**8.** The use according to claim 7, wherein $R_{14}$, $R_{15}$, and $R_{16}$ are selected from H, -OH, and $C_{1-6}$ alkyl.

**9.** The use according to claim 7, wherein $X_1$, $X_2$ and $X_3$ are CH.

**10.** The use according to claim 5, wherein $X_1$, $X_2$ and $X_3$ are N.

**11.** The use according to any one of claims 1 to 10, wherein L is C=O, O=S=O or $CH_2$.

**12.** The use according to any one of claims 1 to 10, wherein $R_{13}$ is H, -N($R_{17}$)($R_{18}$), $C_{1-6}$ alkoxy, -OH, -SH, -CN, halogen, -$NO_2$, -$SF_5$, -S-$C_{1-4}$ alkyl, $C_{1-6}$ alkyl, or $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$bicycloalkyl, or 5-11 membered bicyclic heteroalkyl, and $R_{13}$ is substituted with 0, 1, 2, 3 or 4 $R_1$(s), in which $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of -OH, -CN, -SH, halogen, -$NO_2$,-and $SF_5$.

**13.** The use according to any one of claims 1 to 10, wherein $R_{13}$ is H, -N($R_{17}$)($R_{18}$), $C_{1-6}$ alkoxy, -OH, -SH, -CN, halogen, -$NO_2$, -$SF_5$, -S-$C_{1-4}$ alkyl, $C_{1-6}$ alkyl, $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, or 11-15 membered tricyclyl, and $R_{13}$ is substituted with 0, 1, 2, 3 or 4 $R_1$(s).

**14.** The use according to any one of claims 1 to 10, wherein $R_{13}$ is H, -N($R_{17}$)($R_{18}$), $C_{1-6}$ alkoxy, $C_{1-6}$ alkyl, $C_{3-7}$ cycloalkyl, 3-7 membered heterocycloalkyl, $C_{5-7}$ aryl, or 5-7 membered heteroaryl, and $R_{13}$ is substituted with 0, 1, 2, 3, or 4 $R_1$(s).

**15.** The use according to any one of claims 1 to 10, wherein $R_{13}$ is -N($R_{17}$)($R_{18}$), $C_{1-6}$ alkoxy, $C_{1-6}$ alkyl, $C_{3-7}$ cycloalkyl, 4-6 membered heterocycloalkyl, phenyl, or 5-6 membered heteroaryl, and $R_{13}$ is substituted with 0, 1, 2, or 3 $R_1$(s).

**16.** The use according to any one of claims 1 to 10, wherein $R_{17}$ and $R_{18}$ are each independently selected from H, $C_{1-6}$ alkyl, $C_{3-7}$ cycloalkyl, and $C_{3-7}$ heterocycloalkyl, and are optionally substituted with one or more of -OH, -CN, -SH, halogen, -$NO_2$,-and -$SF_5$.

**17.** The use according to any one of claims 1 to 10, wherein $R_{17}$, $R_{18}$ and the N atom connected thereto together form a 4-10 membered ring.

**18.** The use according to any one of claims 1 to 10, wherein L is C=O, and $R_{13}$ is - $N(R_{17})(R_{18})$, $C_{1-6}$ alkoxy, -OH, -SH, -CN, halogen, $-NO_2$, $-SF_5$, or -S-$C_{1-4}$ alkyl, and $R_{13}$ is substituted with 0, 1, 2, 3 or 4 $R_1$(s) in which $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of -OH, -CN, -SH, halogen, $-NO_2$,-and $-SF_5$, or $R_{17}$, $R_{18}$ and the N atom connected thereto together form a 3-14 membered ring.

**19.** The use according to any one of claims 1 to 10, wherein 1, 2 or 3 $R_2$(s) are present and $R_2$ is selected from H, halogen, -OH, $-NO_2$, -CN, $-SF_5$, -SH, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, and 4-10 membered heterocycloalkyl, in which the -S-$C_{1-4}$ alkyl, $C_{1-6}$ alkyl, $C_{3-7}$ cycloalkyl, and 4-10 membered heterocycloalkyl are each optionally substituted with 1, 2 or 3 substituent(s) each independently selected from the group consisting of halogen, -OH, $-NH_2$, $-NH(CH_3)$, - $N(CH_3)_2$, -CN, $C_{1-4}$ alkyl, $C_{1-4}$ haloalkyl, $C_{1-4}$ alkoxy, and $C_{1-4}$ haloalkoxy.

**20.** The use according to any one of claims 1 to 10, wherein 1, 2 or 3 $R_2$(s) are present, and $R_2$ is selected from halogen, $C_{1-6}$ alkyl, and $C_{3-6}$ cycloalkyl in which the $C_{1-6}$ alkyl and $C_{3-6}$ cycloalkyl are each optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy.

**21.** The use according to claim 15, wherein 1 or 2 $R_2$(s) are present, and $R_2$ is selected from halogen, and $C_{1-6}$ alkyl.

**22.** The use according to any one of claims 1 to 10, wherein $R_{13}$ is substituted with 0 or 1 $R_1$, and $R_1$ is selected from halogen, -OH, CN, $C_{1-6}$ alkyl, 5-7 membered heterocycloalkyl, and $C_{3-7}$ cycloalkyl, in which the $C_{1-6}$ alkyl is optionally substituted with 1, 2, or 3 $R_3$(s) and in which the 5-7 membered heterocycloalkyl, and $C_{3-7}$ cycloalkyl is optionally substituted with 1, 2, 3 or 4 $C_{1-3}$ alkyl(s).

**23.** The use according to claim 1, wherein the compound is a compound of Formula (I),

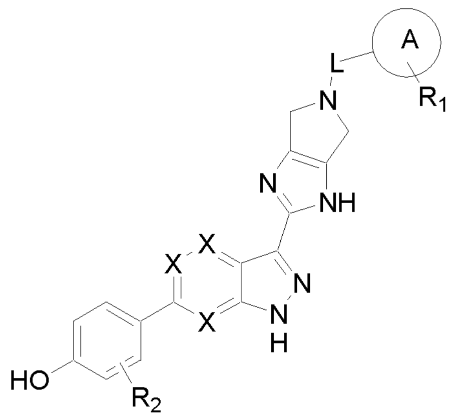

(I)

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof,
in which
L is C=O, O=S=O, $CH_2$ or a bond; and
X is CH or N;
the ring A is $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, or 11 -15 membered tricyclyl;
0, 1, 2, 3 or 4 $R_1$(s) are present in formula (I), and $R_1$ is selected from H, halogen, $C_{1-8}$ alkyl, $C_{2-8}$ alkenyl, $C_{2-8}$ alkynyl, $C_{1-8}$ alkoxy, $C_{3-7}$ cycloalkyl, 3-7 membered heterocycloalkyl, $C_{5-7}$ aryl, 5-7 membered heteroaryl, $C_{7-11}$ bicyclic aryl, and 7-11 membered bicyclic heteroaryl, in which the $C_{1-8}$ alkyl, $C_{2-8}$ alkenyl, $C_{2-8}$ alkynyl, and $C_{1-8}$ alkoxy are optionally substituted with 1, 2, 3 or 4 $R_3$(s), and in which the $C_{3-7}$ cycloalkyl, 3-7 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2, 3 or 4 $R_4$(s),
0, 1, 2, 3 or 4 $R_2$(s) are present in formula (I), and $R_2$ is selected from H, halogen, -OH, $-NO_2$, -CN, $-SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $-N(R_9)$

$(R_{10})$, $-N(R_{11})(C(=O)R_{12})$, $-C(=O)-N(R_9)(R_{10})$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl and 4-10 membered heterocycloalkyl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, $-C(=O)-OR_{12}$, $-C(=O)H$, $-C(=O)R_{12}$, $-C(=O)-N(R_9)(R_{10})$, $-N(R_{11})(S(=O)_2R_{12})$, $-S(=O)_2-N(R_9)(R_{10})$, $-SR_{12}$ and $-OR_{12}$;

$R_3$ is selected from halogen, cyano, $C_{1-3}$ alkyl, hydroxy, $C_{1-6}$ alkoxy, $-N(R_5)(R_6)$, $-CON(R_7)(R_8)$ or 3-7 membered heterocycloalkyl, in which the 3-7 membered heterocycloalkyl is optionally substituted with 1, 2, 3 or 4 $R_4$(s);

$R_4$ is selected from halogen, $C_{1-3}$ alkyl, hydroxyl, $C_{1-6}$ alkoxy, $-NH_2$, $-NHCH_3$ or $-N(CH_3)_2$;

$R_5$, $R_6$, $R_7$, $R_8$ are each independently hydrogen or $C_{1-4}$ alkyl;

$R_9$ is selected from H, $C_{1-4}$ alkyl, $C_{1-4}$ haloalkyl or $C_{3-7}$ cycloalkyl;

$R_{10}$ is H or selected from the group consisting of $C_{1-4}$ alkyl, $C_{1-4}$ haloalkyl, $C_{3-7}$ cycloalkyl, 4-10 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each option included in the above group is optionally substituted with 1, 2, 3 or 4 substituent(s) each independently selected from the group consisting of -OH, $-NH_2$, $-NH(CH_3)$, $-N(CH_3)_2$, -CN, $C_{1-4}$ alkyl, $C_{3-7}$ cycloalkyl, $C_{1-4}$ hydroxyalkyl, $-S-C_{1-4}$ alkyl, $-C(=O)H$, $-C(=O)-C_{1-4}$ alkyl, $-C(=O)-O-C_{1-4}$ alkyl, $-C(=O)-NH_2$, $-C(=O)-N(C_{1-4}$ alkyl$)_2$, $C_{1-4}$ haloalkyl, $C_{1-4}$ alkoxy and $C_{1-4}$ haloalkoxy;

$R_{11}$ is selected from H, $C_{1-4}$ alkyl and $C_{3-7}$ cycloalkyl; and

$R_{12}$ is selected from the group consisting of $C_{1-6}$ alkyl, $C_{3-7}$ cycloalkyl, 4- to 14-membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each option included in the above group is optionally substituted with 1, 2 or 3 substituent(s) each independently selected from the group consisting of halogen, $-CF_3$, -CN, -OH, $-NH_2$, $-NH(CH_3)$, $-N(CH_3)_2$, oxo, $-S-C_{1-4}$ alkyl, $C_{1-4}$ alkyl, $C_{1-4}$ haloalkyl, $C_{2-6}$ alkenyl, $C_{2-6}$ alkynyl, $C_{3-7}$ cycloalkyl, $C_{1-4}$ alkoxy and $C_{1-4}$ haloalkoxy.

**24.** The use according to claim 23, wherein L is C=O, O=S=O or $CH_2$.

**25.** The use according to claim 23, wherein X is CH.

**26.** The use according to any one of claims 23 to 25, wherein the ring A is $C_{3-7}$ cycloalkyl, 3-7 membered heterocycloalkyl, $C_{5-7}$ aryl, or 5-7 membered heteroaryl.

**27.** The use according to any one of claims 23 to 25, wherein the ring A is 5-6 membered heteroaryl, or phenyl.

**28.** The use according to any one of claims 23 to 25, wherein 0, or 1 $R_1$ is present, and $R_1$ is selected from $C_{1-6}$ alkyl, and 5-7 membered heterocycloalkyl in which the $C_{1-6}$ alkyl is optionally substituted with 1 or 2 $R_3$(s), and in which the 5-7 membered heterocycloalkyl is optionally substituted with 1, 2, 3 or 4 $C_{1-3}$ alkyl(s).

**29.** The use according to any one of claims 23 to 25, wherein 1 or 2 $R_2$(s) are present, and $R_2$ is selected from halogen, $C_{1-6}$ alkyl and $C_{3-6}$ cycloalkyl, in which the $C_{1-6}$ alkyl and $C_{3-6}$ cycloalkyl are each optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy.

**30.** The use according to any one of claims 23 to 25, wherein

L is C=O, and O=S=O;

X is CH;

the ring A is 5-7 membered heteroaryl or $C_{5-7}$ aryl;

0, 1, 2, 3 or 4 $R_1$(s) are present in formula (I), and $R_1$ is selected from $C_{1-8}$ alkyl, and 3-7 membered heterocycloalkyl, in which the $C_{1-8}$ alkyl is optionally substituted with 1, 2, 3 or 4 $R_3$(s), and in which the 3-7 membered heterocycloalkyl is optionally substituted with 1, 2, 3 or 4 $R_4$(s),

1, 2, or 3 $R_2$(s) are present in formula (I), and $R_2$ is selected from H, halogen, -OH, $-NO_2$, -CN, $-SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $-N(R_9)(R_{10})$, $-N(R_{11})(C(=O)R_{12})$, $-C(=O)-N(R_9)(R_{10})$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl and 4-10 membered heterocycloalkyl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, $-N(R_9)$

($R_{10}$), -N($R_{11}$)(C(=O)$R_{12}$), -C(=O)-O$R_{12}$, -C(=O)H, -C(=O)$R_{12}$, - C(=O)-N($R_9$)($R_{10}$), -N($R_{11}$)(S(=O)$_2R_{12}$), -S(=O)$_2$-N($R_9$)($R_{10}$), -S$R_{12}$ and -O$R_{12}$;
$R_3$ is selected from halogen, cyano, $C_{1-3}$ alkyl, hydroxy, $C_{1-6}$ alkoxy, -N($R_5$)($R_6$), - CON($R_7$)($R_8$) or 3-7 membered heterocycloalkyl, in which the 3-7 membered heterocycloalkyl is optionally substituted with 1, 2, 3 or 4 $R_4$(s);
$R_4$ is selected from halogen, $C_{1-3}$ alkyl, hydroxyl, $C_{1-6}$ alkoxy, -$NH_2$, -$NHCH_3$ or - N($CH_3$)$_2$;
$R_5$, $R_6$, $R_7$, $R_8$ are each independently hydrogen or $C_{1-4}$ alkyl;
$R_9$ is selected from H, $C_{1-4}$ alkyl, $C_{1-4}$ haloalkyl or $C_{3-7}$ cycloalkyl;
$R_{10}$ is H or selected from the group consisting of $C_{1-4}$ alkyl, $C_{1-4}$ haloalkyl, $C_{3-7}$ cycloalkyl, 4-10 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each option included in the above group is optionally substituted with 1, 2, 3 or 4 substituent(s) each independently selected from the group consisting of -OH, -$NH_2$, -NH($CH_3$), -N($CH_3$)$_2$, -CN, $C_{1-4}$ alkyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, -C(=O)-$NH_2$, -C( =O)-N($C_{1-4}$ alkyl)$_2$, $C_{1-4}$ haloalkyl, $C_{1-4}$ alkoxy and $C_{1-4}$ haloalkoxy;
$R_{11}$ is selected from H, $C_{1-4}$ alkyl and $C_{3-7}$ cycloalkyl; and
$R_{12}$ is selected from the group consisting of $C_{1-6}$ alkyl, $C_{3-7}$ cycloalkyl, 4- to 14-membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each option included in the above group is optionally substituted with 1, 2 or 3 substituent(s) each independently selected from the group consisting of halogen, -$CF_3$, -CN, -OH, -$NH_2$, -NH($CH_3$), -N($CH_3$)$_2$, oxo, -S-$C_{1-4}$ alkyl, $C_{1-4}$ alkyl, $C_{1-4}$ haloalkyl, $C_{2-6}$ alkenyl, $C_{2-6}$ alkynyl, $C_{3-7}$ cycloalkyl, $C_{1-4}$ alkoxy and $C_{1-4}$ haloalkoxy.

**31.** The use according to claim 30, wherein the ring A is 5-6 membered heteroaryl, or phenyl.

**32.** The use according to claim 30, wherein 0 or 1 $R_1$ is present, and $R_1$ is selected from $C_{1-6}$ alkyl, and 5-7 membered heterocycloalkyl, wherein the $C_{1-6}$ alkyl is optionally substituted by 1 or 2 $R_3$(s), and wherein the 5-7 membered heterocycloalkyl group is optionally substituted with 1, 2, 3, or 4 $C_{1-3}$ alkyl(s).

**33.** The use according to claim 30, wherein 1 or 2 $R_2$(s) are present, and $R_2$ is selected from halogen, $C_{1-6}$ alkyl and $C_{3-6}$ cycloalkyl, wherein the $C_{1-6}$ alkyl and $C_{3-6}$ cycloalkyl are each optionally substituted with 1, 2 or 3 substituent(s) each independently selected from the group consisting of halogen, -OH, -$NH_2$, -NH($CH_3$), -N($CH_3$)$_2$, -CN, $C_{1-4}$ alkyl, $C_{1-4}$ haloalkyl, $C_{1-4}$ alkoxy and $C_{1-4}$ haloalkoxy.

**34.** The use according to claim 1, wherein the compound is selected from a group consisting of:

(2-(6-(2-ethyl-5-fluoro-4-hydroxyphenyl)-1H-indazol-3-yl)-4,6-dihydropyrrolo[3,4-d]imidazol-5(1H)-yl)(5-(piperidin-1-yl)pyrazin-2-yl)ketone (MDI-2);
(2-(6-(2-ethyl-5-fluoro-4-hydroxyphenyl)-1H-indazol-3-yl)-4,6-dihydropyrrolo[3,4-d]imidazol-5(1H)-yl)(5-morpholinylpyrazin-2-yl)ketone (MDI-201);
(2-(6-(2-ethyl-5-fluoro-4-hydroxyphenyl)-1H-indazol-3-yl)-4,6-dihydropyrrolo[3,4-d]imidazol-5(1H)-yl)(1-methyl-1H-pyrazol-4-yl)ketone (MDI-202);
(2-(6-(2-ethyl-5-fluoro-4-hydroxyphenyl)-1H-indazol-3-yl)pyrrolo[3,4-d]imidazol-5(1H, 4H,6H)-yl)(1-methylpiperidin-4-yl)ketone (MDI-203);
(2-(6-(2-ethyl-5-fluoro-4-hydroxyphenyl)-1H-indazol-3-yl)pyrrolo[3,4-d]imidazol-5(1H,4H,6H)-yl)(5-(4-methylpiperzin-1-yl)pyrazin-2-yl)ketone (MDI-204);
(2-(6-(2-ethyl-4-hydroxyphenyl)-1H-indazol-3-yl)pyrrolo[3,4-d]imidazol-5(1H,4H,6H)-yl)(5-(4-methylpiperzin-1-yl)pyrazin-2-yl)ketone (MDI-205);
5-ethyl-2-fluoro-4-(3-(5-(benzenesulfonyl)-1,4,5,6-tetrahydropyrrolo[3,4-d]imidazol-2-yl)-1H-indazol-6-yl)phenol (MDI-206);
5-ethyl-2-fluoro-4-(3-(5-(pyrazin-2ylmethyl)-1,4,5,6-tetrahydropyrrolo[3,4-d]imidazol-2-yl)-1H-indazol-6-yl) phenol (MDI-207);
4-(3-(5-(cyclopropylmethyl)-1,4,5,6-tetrahydropyrrolo[3,4-d]imidazol-2-yl)-1H-indazol-6-yl)-5-ethyl-2-fluorophenol (MDI-208);
Cyclopropyl (2-(6-(2-ethyl-5-fluoro-4-hydroxyphenyl)-1H-indazol-3-yl)pyrrolo[3,4-d]imidazol-5(1H,4H,6H)-yl) ketone (MDI-1233);
4-(3-(5-(cyclobutylmethyl)-1,4,5,6-tetrahydropyrrolo[3,4-d]imidazol-2-yl)-1H-indazol-6-yl)-5-ethyl-2-fluorophenol (MDI-210);
Cyclobutyl (2-(6-(2-ethyl-5-fluoro-4-hydroxyphenyl)-1H-indazol-3-yl)pyrrolo[3,4-d]imidazol-5(1H,4H,6H)-yl)ketone (MDI-211);
(2-(6-(2-ethyl-5-fluoro-4-hydroxyphenyl)-1H-indazol-3-yl)-pyrrolo[3,4-d]imidazol-5-(1H,4H,6H)-yl)(3-hydroxycyclobutyl)ketone (MDI-213);
(2-(6-(2-ethyl-5-fluoro-4-hydroxyphenyl)-1H-indazol-3-yl)-pyrrolo[3,4-d]imidazol-5-(1H,4H,6H)-yl)(pyridazin-4-yl)ketone (MDI-214);
(2-(6-(2-ethyl-5-fluoro-4-hydroxyphenyl)-1H-indazol-3-yl)-pyrrolo[3,4-d]imidazol-5-(1H,4H,6H)-yl)(pyridazin-3-yl)ketone (MDI-215);
(S)-(2-(6-(2-ethyl-5-fluoro-4-hydroxyphenyl)-1H-indazol-3-yl)-4,6-dihydropyrrolo[3,4-d]imidazol-5(1H)-yl)(3-hydroxypyrrolidin-1-yl)keone (MDI-1228);
5-ethyl-2-fluoro-4-(3-(5-(4-hydroxycyclohexyl)-1,4,5,6-tetrahydropyrrolo[3,4-d]imidazol-2-yl)-1H-indazol-6-yl) phenol (MDI-217);
4-(3-(5-(cyclopropanesulfonyl)-1,4,5,6-tetrahydropyrrolo[3,4-d]imidazol-2-yl)-1H-indazol-6-yl)-5-ethyl-2-fluorophenol (MDI-218);
4-(3-(5-(cyclobutylsulfonyl)-1,4,5,6-tetrahydropyrrolo[3,4-d]imidazol-2-yl)-1H-indazol-6-yl)-5-ethyl-2-fluorophenol (MDI-219);
4-(3-(5-(cyclopentylsulfonyl)-1,4,5,6-tetrahydropyrrolo[3,4-d]imidazol-2-yl)-1H-indazol-6-yl)-5-ethyl-2-fluorophenol (MDI-220);
5-ethyl-2-fluoro-4-(3-(5-((1-methyl-1H-pyrazol-4-yl)methyl)-1,4,5,6-tetrahydropyrrolo[3,4-d]imidazol-2-yl)-1H-indazol-6-yl)phenol (MDI-221);
4-(3-(5-(cyclopentyl-1,4,5,6-tetrahydropyrrolo[3,4-d]imidazol-2-yl)-1H-indazol-6-yl)-5-ethyl-2-fluorophenol (MDI-224);
5-ethyl-2-fluoro-4-(3-(5-(tetrahydro-2H-pyran-4-yl)-1,4,5,6-tetrahydropyrrolo[3,4-d]imidazol-2-yl)-1H-indazol-6-yl)phenol (MDI-225);
1-(2-(6-(2-ethyl-5-fluoro-4-hydroxyphenyl)-1H-indazol-3-yl)-4,6-dihydropyrrolo[3,4-d]imidazol-5(1H)-yl) ethan-1-one (MDI-226);
1-(2-(6-(2-ethyl-5-fluoro-4-hydroxyphenyl)-1H-indazol-3-yl)-4,6-dihydropyrrolo[3,4-d]imidazol-5(1H)-yl)propan-1-one (MDI-227);
(1-(2-(6-(2-ethyl-5-fluoro-4-hydroxyphenyl)-1H-indazol-3-yl)-4,6-dihydropyrrolo[3,4-d]imidazol-5(1H)-yl)-2-methylpropan-1-one) (MDI-228);
2-cyclopropyl-1-(2-(6-(2-ethyl-5-fluoro-4-hydroxyphenyl)-1H-indazol-3-yl)-4,6-dihydropyrrolo[3,4-d]imidazol -5(1H)-yl)ethan-1-one (MDI-229);
1-(2-(6-(2-ethyl-5-fluoro-4-hydroxyphenyl)-1H-indazol-3-yl)-4,6-dihydropyrrolo[3,4-d]imidazol -5(1H)-yl)-3-methylbutan-1-one (MDI-230);
(2-(6-(2-ethyl-5-fluoro-4-hydroxyphenyl)-1H-indazol-3-yl)-4,6-dihydropyrrolo[3,4-d]imidazol-5-(1H)-yl)(pyrrolidin-1-yl)ketone (MDI-231);
N-(3-Chloro-2-hydroxypropyl)-2-(6-(2-ethyl-5-fluoro-4-hydroxyphenyl)-1H-indazol-3-yl)-4,6-dihydropyrrolo [3,4-d]pyrimidin-5(1H)-carboxamide (MDI-1288);
(2-(6-(2-ethyl-5-fluoro-4-hydroxyphenyl)-1H-indazol-3-yl)-4,6-dihydropyrrolo[3,4-d]imidazol-5-(1H)-yl)(piperidin-1-yl)ketone (MDI-233);
(2-(6-(2-ethyl-5-fluoro-4-hydroxyphenyl)-1H-indazol-3-yl)-4,6-dihydropyrrolo[3,4-d]imidazol-5-(1H)-yl)(morpholino)ketone (MDI-234);
(2-(6-(2-ethyl-5-fluoro-4-hydroxyphenyl)-1H-indazol-3-yl)-4,6-dihydropyrrolo[3,4-d]imidazol-5-(1H)-yl)(4-methylpiperzin-1-yl)ketone (MDI-235);
(2-(6-(2-ethyl-5-fluoro-4-hydroxyphenyl)-1H-indazol-3-yl)-4,6-dihydropyrrolo[3,4-d]imidazol-5-(1H)-yl)(4-ethylpiperzin-1-yl)ketone (MDI-236);
Cyclopropyl(2-(6-(2-ethyl-5-fluoro-4-hydroxyphenyl)-1H-pyrazolo [4,3-b]pyridin-3-yl)-4,6-dihydropyrrolo[3,4-d] imidazol-5(1H)-yl)ketone (MDI-237);
Cyclopropyl(2-(6-(2-ethyl-5-fluoro-4-hydroxyphenyl)-4-methyl-1H-indazol-3-yl)-4,6-dihydropyrrolo[3,4-d]imidazol-5(1H)-yl)ketone (MDI-239);
(S)-(2-(6-(2-ethyl-5-fluoro-4-hydroxyphenyl)-4-methyl-1H-indazol-3-yl)-4,6-dihydropyrrolo[3,4-d]imidazol-5(1H)-y1)(3-hydroxylpyrrolidin-1-yl)ketone (MDI-240);
Cyclopropyl(2-(6-(2-ethyl-5-fluoro-4-hydroxyphenyl)-1H-pyrazolo[4,3-c]pyridin-3-yl)-4,6-dihydropyrrolo[3,4-d] imidazol-5(1H)-yl)ketone (MDI-242);
(R)-(2-(6-(2-ethyl-5-fluoro-4-hydroxyphenyl)-1H-indazol-3-yl)-4,6-dihydropyrrolo[3,4-d]imidazol-5(1H )-yl)(3-hydroxylpyrrolidin-1-yl)ketone (MDI-243);
(2-(6-(2-ethyl-5-fluoro-4-hydroxyphenyl)-1H-indazol-3-yl)-4,6-dihydropyrrolo[3,4-d]imidazol-5(1H)-yl)(4-hydroxylpiperidin-1-yl)ketone (MDI-245);
2-(6-(2-ethyl-5-fluoro-4-hydroxyphenyl)-1H-indazol-3-yl)-N-methyl-4, 6-dihydropyrrolo[3,4-d]imidazol-5-(1H)-

carboxamide (MDI-246);
2-(6-(2-ethyl-5-fluoro-4-hydroxyphenyl)-1H-indazol-3-yl)-N-ethyl-4, 6-dihydropyrrolo[3,4-d]imidazol-5-(1H)-carboxamide (MDI-247);
2-(6-(2-ethyl-5-fluoro-4-hydroxyphenyl)-1H-indazol-3-yl)-N-(2-hydroxyethyl)-4, 6-dihydropyrrolo[3,4-d]imidazol-5-(1H)- carboxamide (MDI-248);
1-(2-(6-(2-ethyl-5-fluoro-4-hydroxyphenyl)-1H-indazol-3-yl)-1,4,5,6-tetrahydropyrrolo[3,4-d]imidazol-5-carbonyl)pyrrolidin-3-nitrile (MDI-250);
2-(6-(2-ethyl-5-fluoro-4-hydroxyphenyl)-1H-indazol-3-yl)-N-(tetrahydrofuran-3-yl)-4,6-dihydropyrrolo[3,4-d]imidazol-5(1H)-carboxamide (MDI-251);
Methyl 2-(6-(2-ethyl-5-fluoro-4-hydroxyphenyl)-1H-indazol-3-yl)-4,6-dihydropyrrolo[3,4-d]imidazol-5(1H)-carboxylate (MDI-252);
Ethyl 2-(6-(2-ethyl-5-fluoro-4-hydroxyphenyl)-1H-indazol-3-yl)-4,6-dihydropyrrolo[3,4-d]imidazol-5(1H)-carboxylate (MDI-253);
(S)-(2-(6-(2-ethyl-5-fluoro-4-hydroxyphenyl)-1H-pyrazolo[3,4-b]pyridin-3-yl)-4,6-dihydropyrrolo[3,4-d]imidazol-5(1H)-yl)(3-hydroxylpyrrolidin-1-yl)ketone (MDI-255);
3-(2-(6-(2-ethyl-5-fluoro-4-hydroxyphenyl)-1H-indazol-3-yl)-4,6-dihydropyrrolo[3,4-d]imidazol-5(1H)-yl)-3-oxopropanenitrile (MDI-256);
2-(6-(2-ethyl-5-fluoro-4-hydroxyphenyl)-1H-indazol-3-yl)-N,N-dimethyl-4,6-dihydropyrrolo[3,4-d]imidazol-5(1H)-carboxamide (MDI-257);
N-(2-cyanoethyl)-2-(6-(2-ethyl-5-fluoro-4-hydroxyphenyl)-1H-indazol-3-yl)-4,6-dihydropyrrolo[3,4-d]imidazol-5(1H)-carboxamide (MDI-258);
N-cyclopropyl-2-(6-(2-ethyl-5-fluoro-4-hydroxyphenyl)-1H-indazol-3-yl)-4,6-dihydropyrrolo[3,4-d]imidazol-5(1H)-carboxamide (MDI-259);
N-cyclobutyl-2-(6-(2-ethyl-5-fluoro-4-hydroxyphenyl)-1H-indazol-3-yl)-4,6-dihydropyrrolo[3,4-d]imidazol-5(1H)-carboxamide (MDI-260);
(S)-6-(2-ethyl-5-fluoro-4-hydroxyphenyl)-3-(5-prolyl-1,4,5,6-tetrahydropyrrolo[3,4-d]imidazol-2-yl)- 1H-indazol (MDI-262); and
(R)-6-(2-ethyl-5-fluoro-4-hydroxyphenyl)-3-(5-prolyl-1,4,5,6-tetrahydropyrrolo[3,4-d]imidazol-2-yl)- 1H-indazol (MDI-263),
or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof.

**35.** The use according to claim 1, wherien the compound is selected from

(2-(6-(2-ethyl-5-fluoro-4-hydroxyphenyl)-1H-indazol-3-yl)-4,6-dihydropyrrolo[3,4-d]imidazol-5(1H)-yl)(5-morpholinylpyrazin-2-yl)ketone (MDI-201)

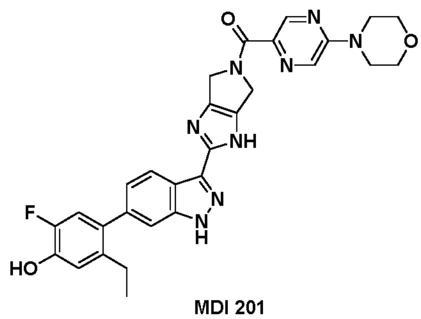

MDI 201

;

Cyclopropyl (2-(6-(2-ethyl-5-fluoro-4-hydroxyphenyl)-1H-indazol-3-yl)pyrrolo[3,4-d]imidazol-5(1H,4H,6H)-yl) ketone (MDI-1233)

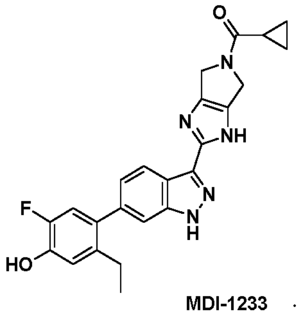

MDI-1233 ;

(S)-(2-(6-(2-ethyl-5-fluoro-4-hydroxyphenyl)-1H-indazol-3-yl)-4,6-dihydropyrrolo[3,4-d]imidazol-5(1H)-yl)(3-hydroxypyrrolidin-1-yl)keone (MDI-1228)

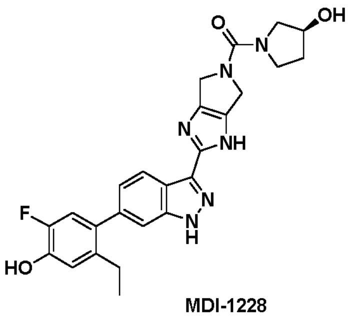

MDI-1228 ;

4-(3-(5-(cyclopropanesulfonyl)-1,4,5,6-tetrahydropyrrolo[3,4-d]imidazol-2-yl)-1H-indazol-6-yl)-5-ethyl-2-fluorophenol (MDI-218)

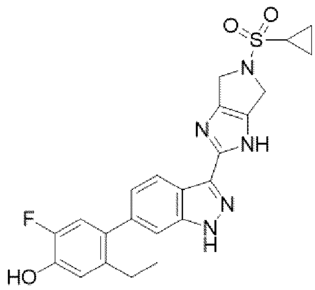

MDI-218 ;

N-(3-Chloro-2-hydroxypropyl)-2-(6-(2-ethyl-5-fluoro-4-hydroxyphenyl)-1H-indazol-3-yl)-4,6-dihydropyrrolo[3,4-d]pyrimidin-5(1H)-carboxamide (MDI-1288)

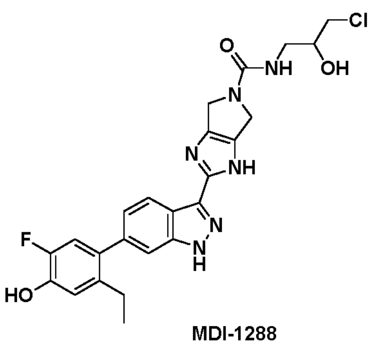

MDI-1288 ;

(2-(6-(2-ethyl-5-fluoro-4-hydroxyphenyl)-1H-indazol-3-yl)-4,6-dihydropyrrolo[3,4-d]imidazol-5-(1H)-yl)(pyrrolidin-1-yl)ketone (MDI-231)

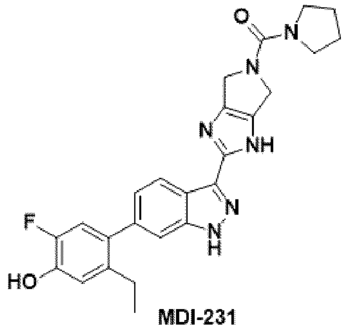

MDI-231

;

(2-(6-(2-ethyl-5-fluoro-4-hydroxyphenyl)-1H-indazol-3-yl)-4,6-dihydropyrrolo[3,4-d]imidazol-5-(1H)-yl)(piperidin-1-yl)ketone (MDI-233)

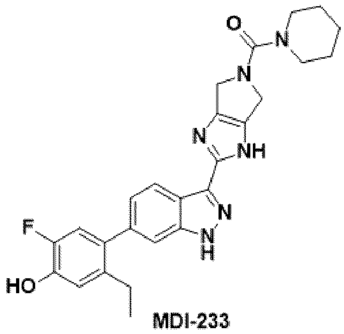

MDI-233

;

(2-(6-(2-ethyl-5-fluoro-4-hydroxyphenyl)-1H-indazol-3-yl)-4,6-dihydropyrrolo[3,4-d]imidazol-5-(1H)-yl)(morpholino)ketone (MDI-234)

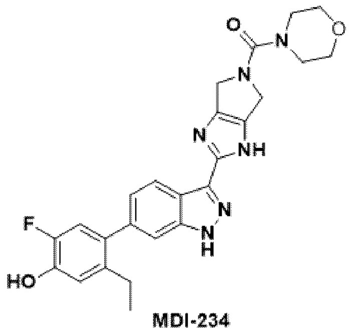

MDI-234

;

(2-(6-(2-ethyl-5-fluoro-4-hydroxyphenyl)-1H-indazol-3-yl)-4,6-dihydropyrrolo[3,4-d]imidazol-5-(1H)-yl)(4-methylpiperzin-1-yl)ketone (MDI-235)

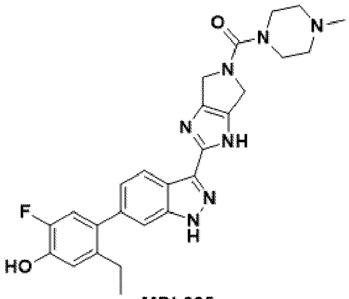

MDI-235

;

(2-(6-(2-ethyl-5-fluoro-4-hydroxyphenyl)-1H-indazol-3-yl)-4,6-dihydropyrrolo[3,4-d]imidazol-5-(1H)-yl)(4-ethylpiperzin-1-yl)ketone (MDI-236)

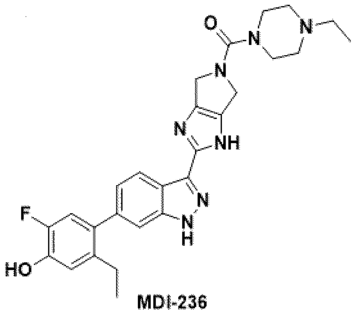

MDI-236

;

(2-(6-(2-ethyl-5-fluoro-4-hydroxyphenyl)-1H-indazol-3-yl)-4,6-dihydropyrrolo[3,4-d]imidazol-5(1H)-yl)(4-hydroxylpiperidin-1-yl)ketone (MDI-245)

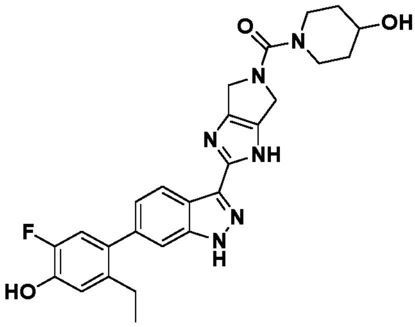

MDI-245

;

2-(6-(2-ethyl-5-fluoro-4-hydroxyphenyl)-1H-indazol-3-yl)-N-ethyl-4, 6-dihydropyrrolo[3,4-d]imidazol-5-(1H)-carboxamide (MDI-247)

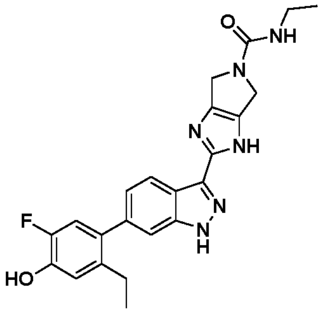

MDI-247

;

2-(6-(2-ethyl-5-fluoro-4-hydroxyphenyl)-1H-indazol-3-yl)-N-(2-hydroxyethyl)-4, 6-dihydropyrrolo[3,4-d]imidazol-5-(1H)- carboxamide (MDI-248)

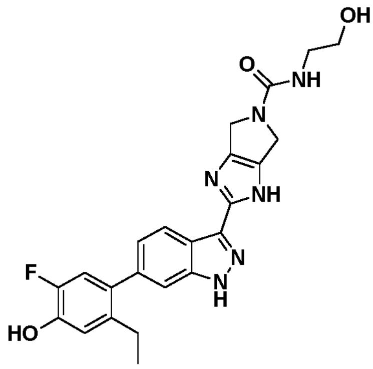

MDI-248 ;

Ethyl 2-(6-(2-ethyl-5-fluoro-4-hydroxyphenyl)-1H-indazol-3-yl)-4,6-dihydropyrrolo[3,4-d]imidazol-5(1H)-carboxylate (MDI-253)

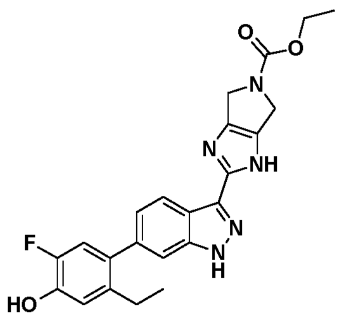

MDI-253 ;

2-(6-(2-ethyl-5-fluoro-4-hydroxyphenyl)-1H-indazol-3-yl)-N,N-dimethyl-4,6-dihydropyrrolo[3,4-d]imidazol-5(1H)-carboxamide (MDI-257)

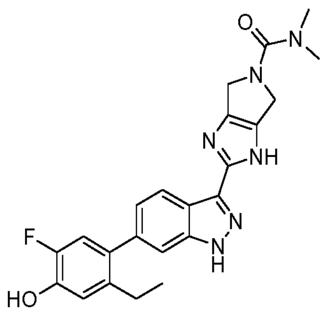

MDI-257 ,

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof.

**36.** The use according to any one of claims 1 to 35, wherein the compound is used as a JAK/TRK dual inhibitor (preferably used as a pan-JAK/pan-TRK dual inhibitor) or as a JAK/TRK/RET multiple inhibitor (preferably used as a pan-JAK/pan-TRK/RET multiple inhibitor).

**37.** A pharmaceutical composition for use as a TRK inhibitor and/or RET inhibitor, comprising the compound as defined in

any one of claims 1-35, or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, and one or more pharmaceutically acceptable carriers, adjuvants or excipients.

**38.** The pharmaceutical composition according to claim 37, which is formulated as an oral dosage form or as a topical dosage form suitable for external application.

**39.** The pharmaceutical composition according to claim 37, which is formulated as a JAK/TRK dual inhibiting medicine (preferably used as a pan-JAK/pan-TRK dual inhibiting medicine) or as a JAK/TRK/RET multiple inhibiting medicine (preferably formulated as a pan-JAK/pan-TRK/RET multiple inhibiting medicine).

**40.** Use of the compound, or isotopically labeled compound thereof, or optical isomer thereof, geometric isomer thereof, a tautomer thereof or a mixture of various isomers, or a pharmaceutically acceptable salt thereof, or a prodrug thereof, or a metabolite thereof as defined in any one of claims 1-35 or the pharmaceutical composition of claim 37, 38 or 39 in the manufacture of a medicament for the treatment and/or prevention of a TRK and/or RET related disease or disorder.

**41.** The use according to claim 40, wherein the TRK and/or RET related disease or disorder is selected from the group consisting of arthritis, autoimmune diseases or disorders, cancer or tumor, diabetes and its complications, (diabetes-induced) delayed wound healing, eye diseases, disorders or conditions, intestinal inflammation, allergies or conditions, neurodegenerative diseases, skin diseases, conditions or disorders, allergies, asthma and other obstructive airway diseases, and transplant rejection.

**42.** The use according to claim 40, wherein the TRK and/or RET related disease or disorder is selected from the group consisting of itching, psoriasis, atopic dermatitis, skin side effects caused by EGFR inhibitors, acne, vitiligo, alopecia areata, asthma, rhinitis, hemorrhoids, cervicitis, pneumonia, delayed wound healing caused by diabetes, diabetic foot, diabetic retinopathy, cancer (tumor), and bedsores.

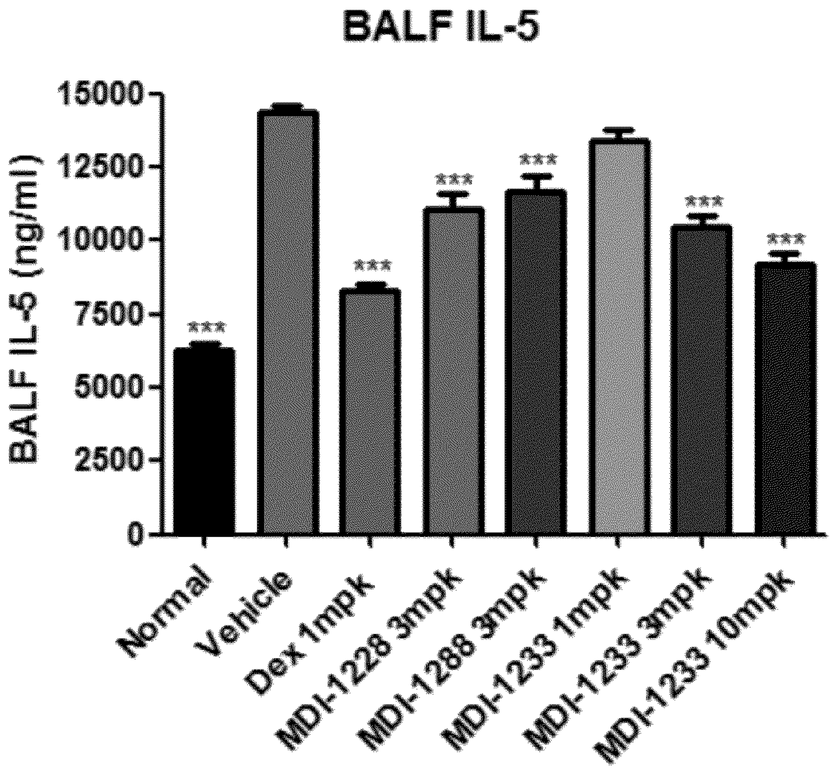
BALF IL-5
BALF IL-5 (ng/ml)
15000
12500
10000
7500
5000
2500
0
***
Normal
Vehicle
Dex 1mpk
MDI-1228 3mpk
MDI-1288 3mpk
MDI-1233 1mpk
MDI-1233 3mpk
MDI-1233 10mpk

FIG. 1

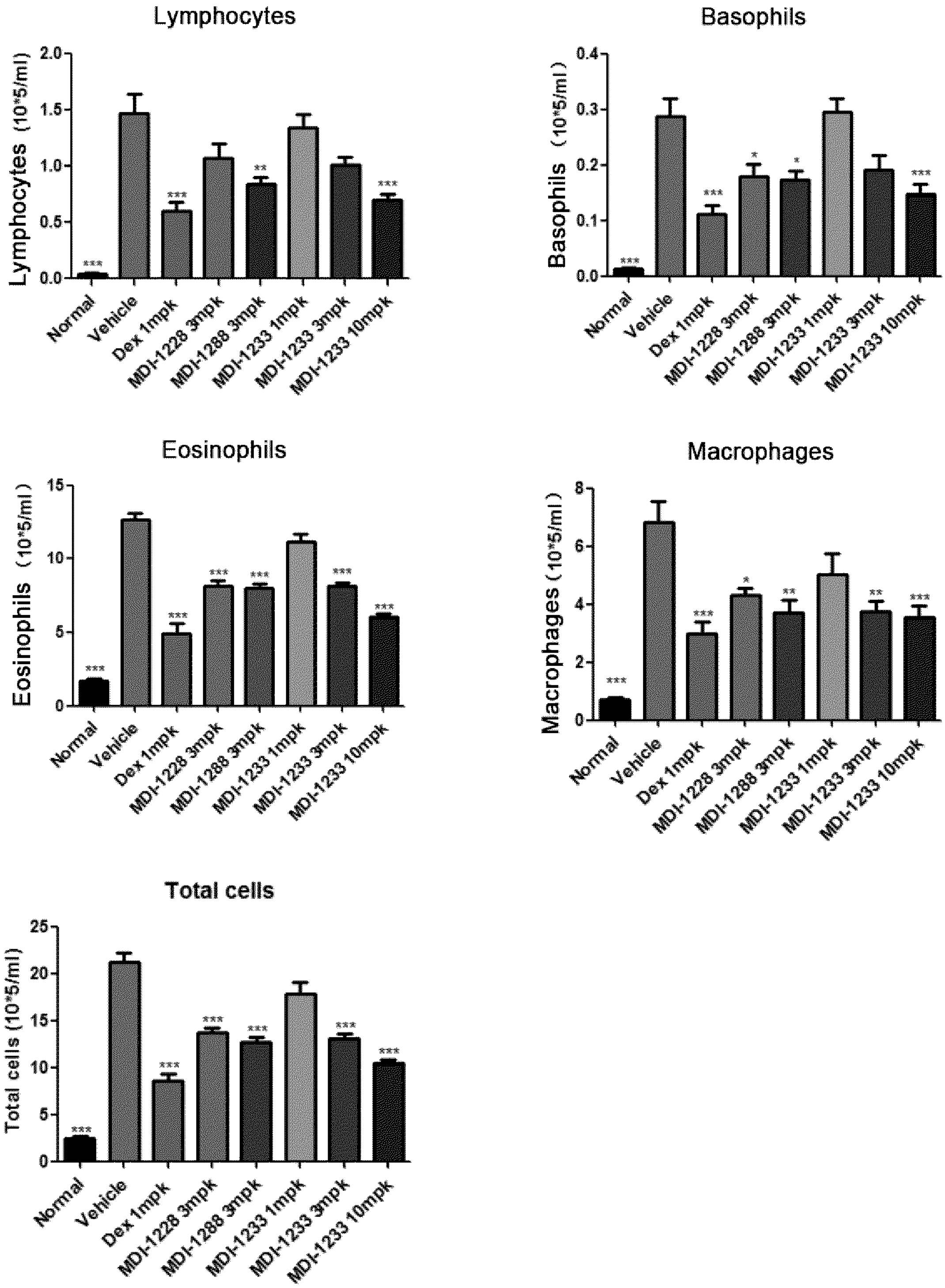
Lymphocytes
Lymphocytes (10*5/ml)
2.0
1.5
1.0
0.5
0.0
***
***
**
***
Basophils
Basophils (10*5/ml)
0.4
0.3
0.2
0.1
0.0
***
***
*
*
***
Eosinophils
Eosinophils (10*5/ml)
15
10
5
0
***
***
***
***
***
***
Macrophages
Macrophages (10*5/ml)
8
6
4
2
0
***
***
*
**
**
***
Total cells
Total cells (10*5/ml)
25
20
15
10
5
0
***
***
***
***
***
***
Normal
Vehicle
Dex 1mpk
MDI-1228 3mpk
MDI-1288 3mpk
MDI-1233 1mpk
MDI-1233 3mpk
MDI-1233 10mpk

FIG. 2

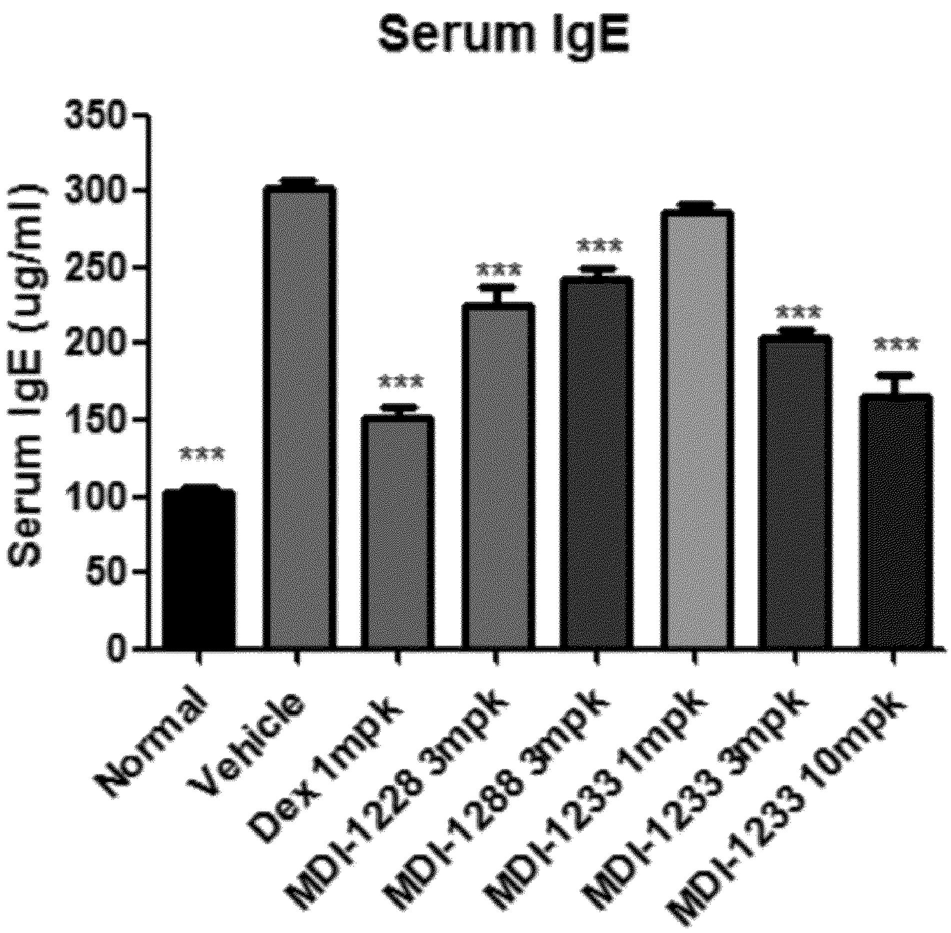
Serum IgE
Serum IgE (ug/ml)
350
300
250
200
150
100
50
0
***
***
***
***
***
***
Normal
Vehicle
Dex 1mpk
MDI-1228 3mpk
MDI-1288 3mpk
MDI-1233 1mpk
MDI-1233 3mpk
MDI-1233 10mpk

FIG. 3

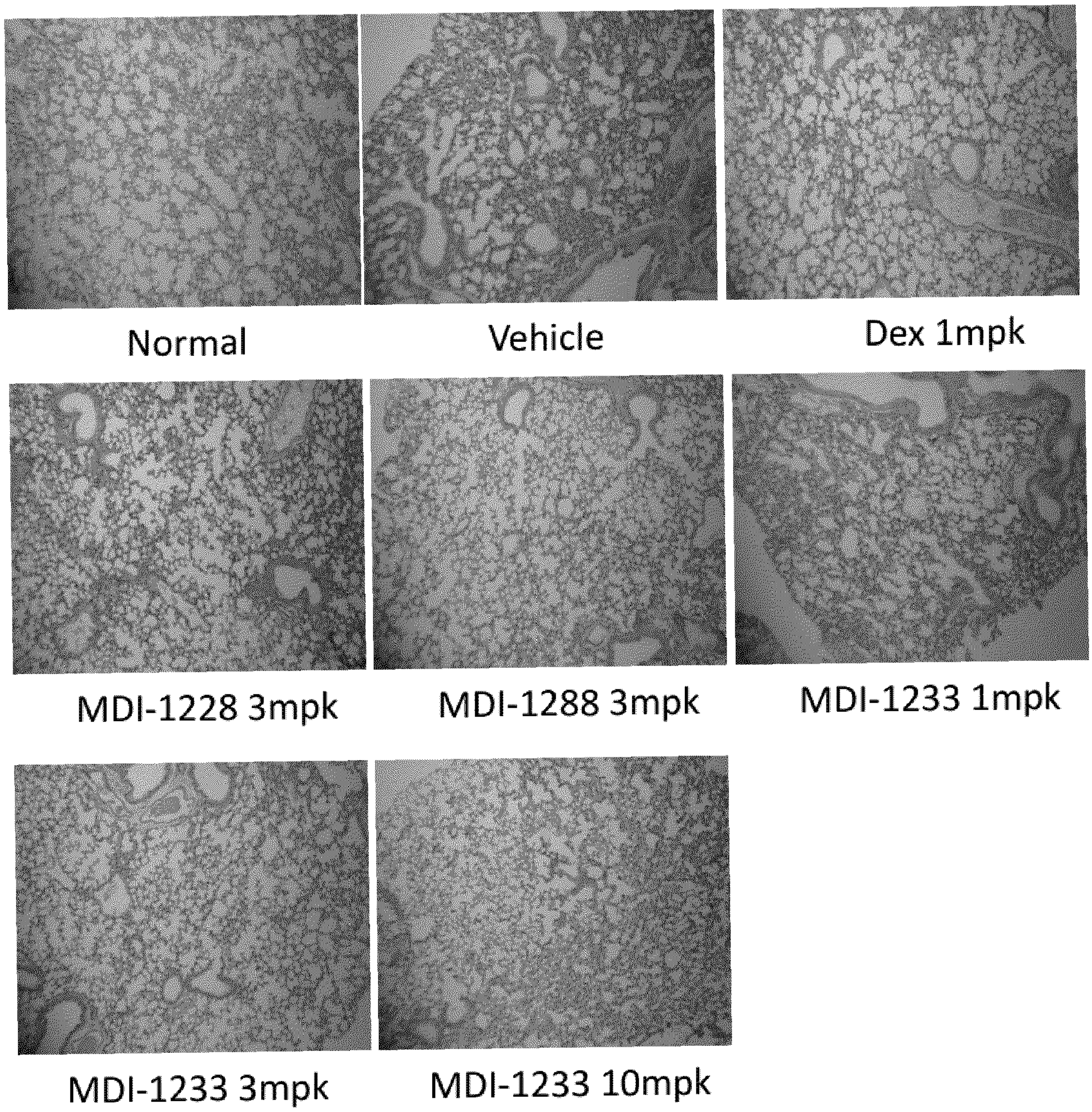
Normal
Vehicle
Dex 1mpk
MDI-1228 3mpk
MDI-1288 3mpk
MDI-1233 1mpk
MDI-1233 3mpk
MDI-1233 10mpk

FIG. 4

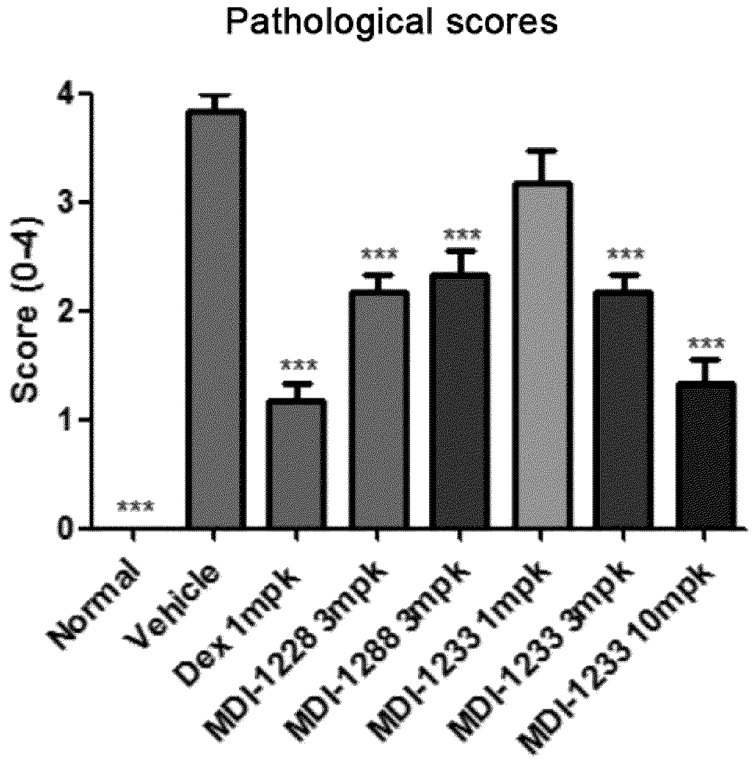
Pathological scores
Score (0-4)
0
1
2
3
4
***
Normal
Vehicle
Dex 1mpk
MDI-1228 3mpk
MDI-1288 3mpk
MDI-1233 1mpk
MDI-1233 3mpk
MDI-1233 10mpk

FIG. 5

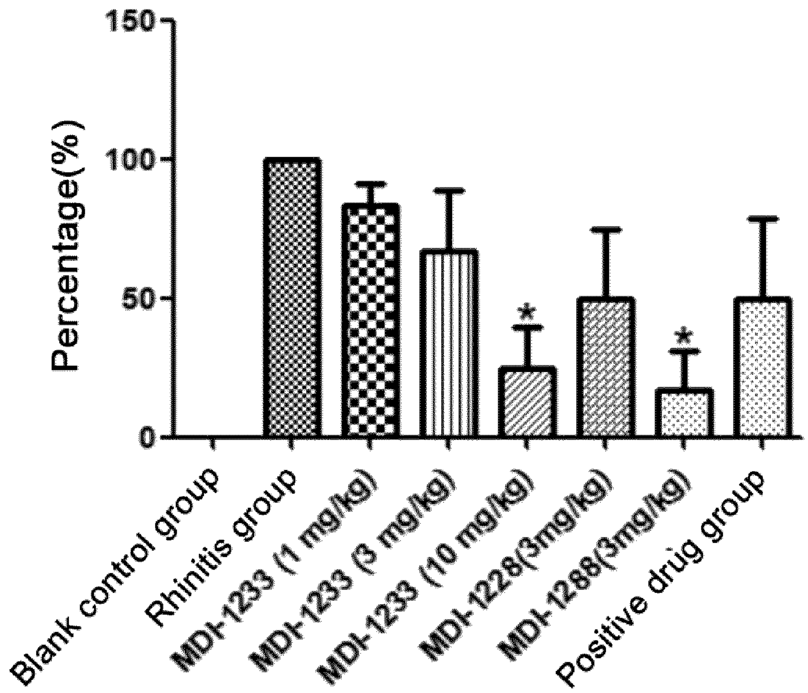
Percentage(%)
0
50
100
150
*
Blank control group
Rhinitis group
MDI-1233 (1 mg/kg)
MDI-1233 (3 mg/kg)
MDI-1233 (10 mg/kg)
MDI-1228(3mg/kg)
MDI-1288(3mg/kg)
Positive drug group

FIG. 6

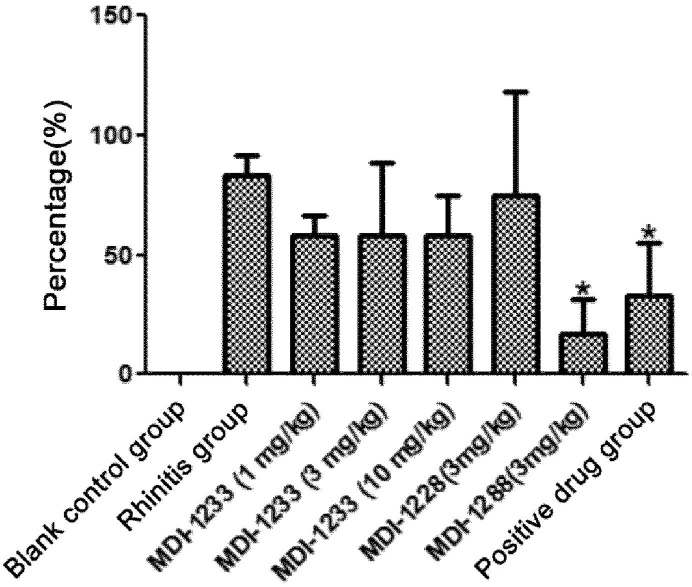
150
100
50
0
Percentage(%)
*
*
Blank control group
Rhinitis group
MDI-1233 (1 mg/kg)
MDI-1233 (3 mg/kg)
MDI-1233 (10 mg/kg)
MDI-1228(3mg/kg)
MDI-1288(3mg/kg)
Positive drug group

FIG. 7

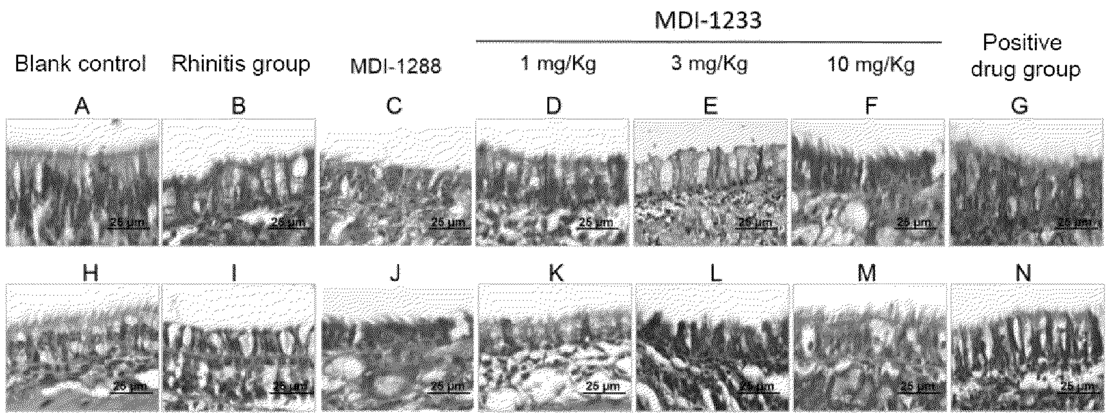
MDI-1233
Blank control
Rhinitis group
MDI-1288
1 mg/Kg
3 mg/Kg
10 mg/Kg
Positive drug group
A
B
C
D
E
F
G
H
I
J
K
L
M
N

FIG. 8

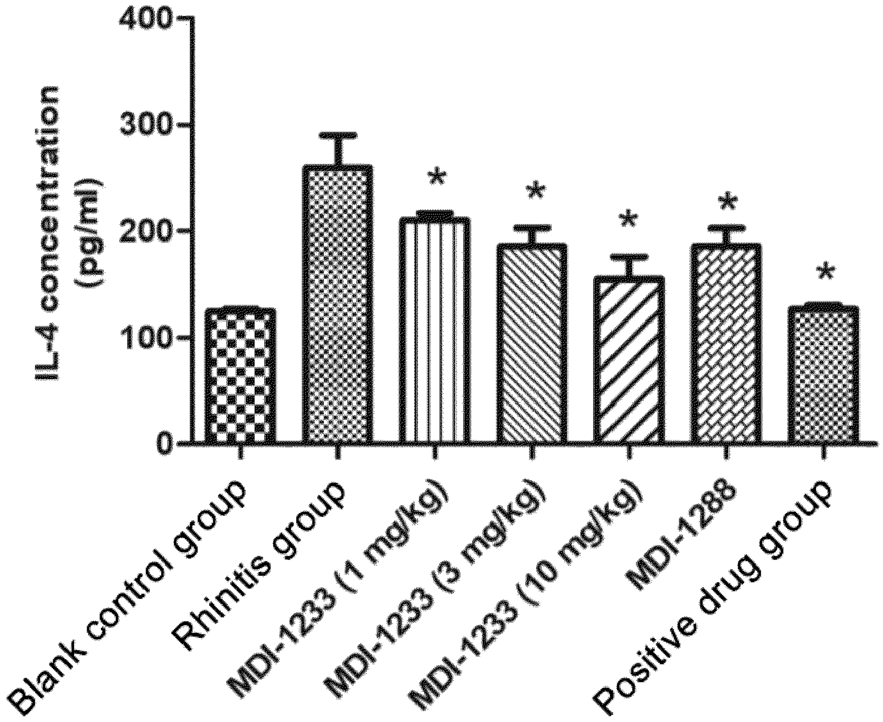
400
300
200
100
0
IL-4 concentration (pg/ml)
*
*
*
*
*
Blank control group
Rhinitis group
MDI-1233 (1 mg/kg)
MDI-1233 (3 mg/kg)
MDI-1233 (10 mg/kg)
MDI-1288
Positive drug group

FIG. 9

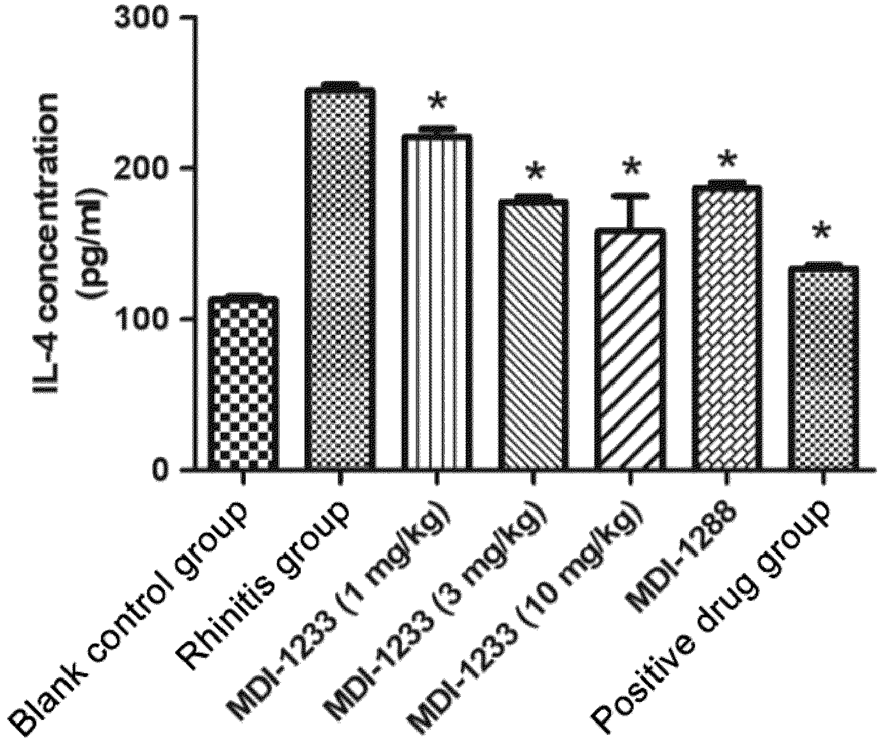
300
200
100
0
IL-4 concentration (pg/ml)
*
*
*
*
*
Blank control group
Rhinitis group
MDI-1233 (1 mg/kg)
MDI-1233 (3 mg/kg)
MDI-1233 (10 mg/kg)
MDI-1288
Positive drug group

FIG. 10

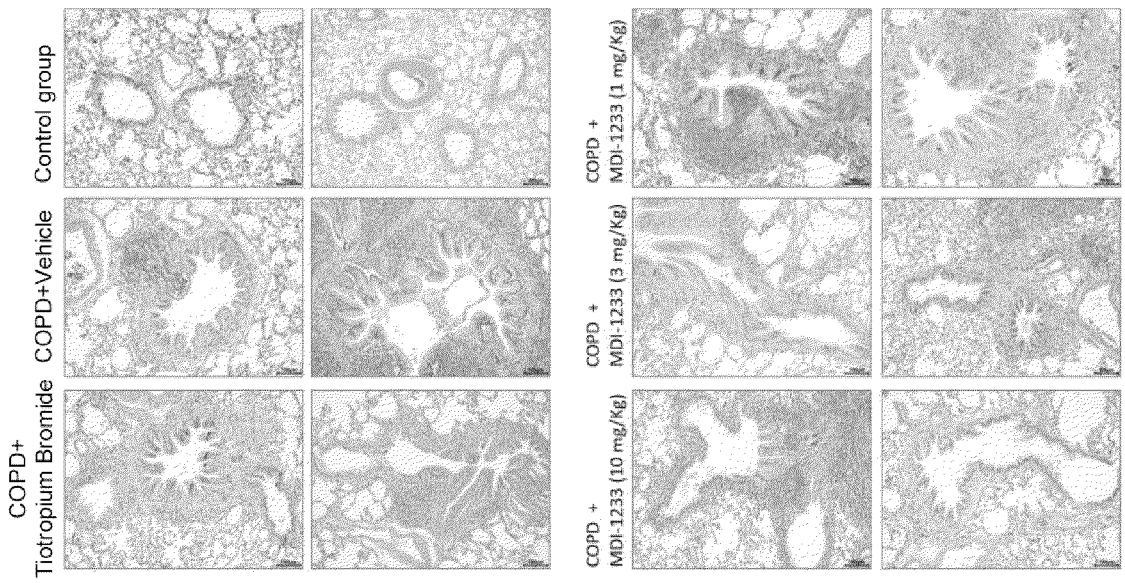
Female
Male
Female
Male
Control group
COPD+Vehicle
COPD+
Tiotropium Bromide
COPD +
MDI-1233 (1 mg/Kg)
COPD +
MDI-1233 (3 mg/Kg)
COPD +
MDI-1233 (10 mg/Kg)

FIG. 11

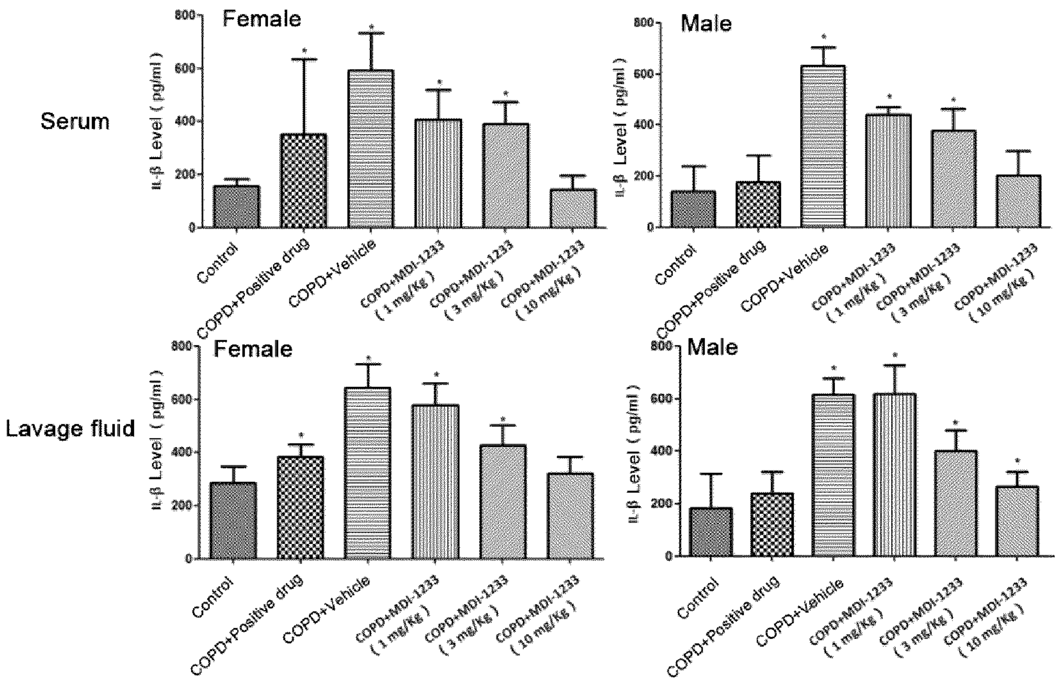
Serum
Lavage fluid
Female
Male
IL-β Level ( pg/ml )
Control
COPD+Positive drug
COPD+Vehicle
COPD+MDI-1233 ( 1 mg/Kg )
COPD+MDI-1233 ( 3 mg/Kg )
COPD+MDI-1233 ( 10 mg/Kg )

FIG. 12

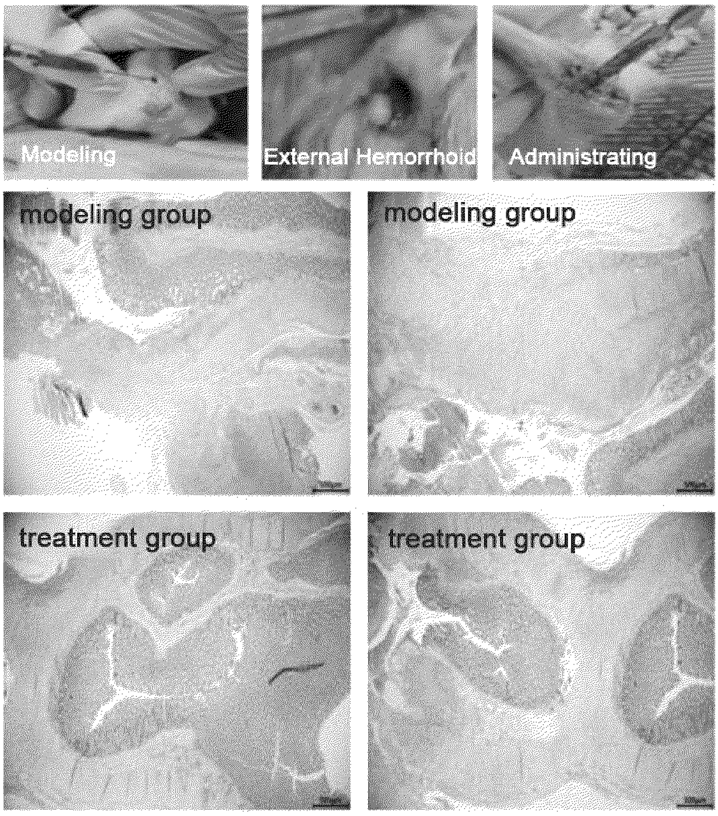
Modeling
External Hemorrhoid
Administrating
modeling group
modeling group
treatment group
treatment group

FIG. 13

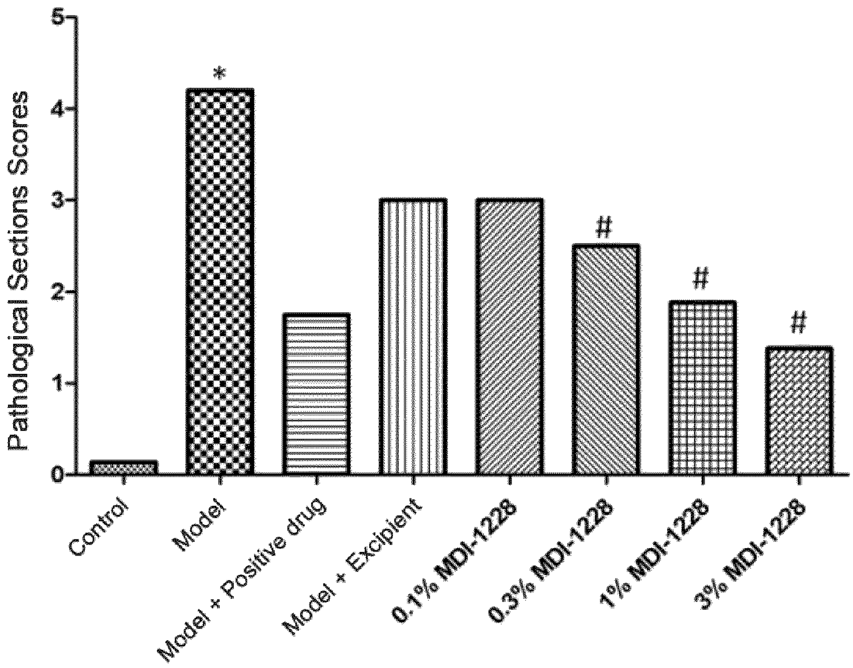
Pathological Sections Scores
0
1
2
3
4
5
*
#
#
#
Control
Model
Model + Positive drug
Model + Excipient
0.1% MDI-1228
0.3% MDI-1228
1% MDI-1228
3% MDI-1228

FIG. 14

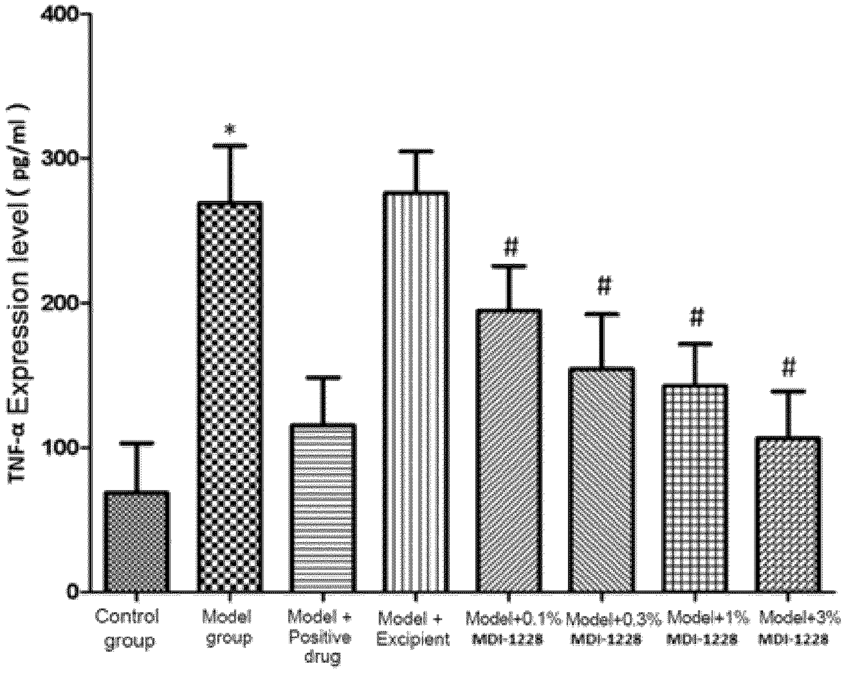
400
300
200
100
0
TNF-α Expression level ( pg/ml )
*
#
#
#
#
Control group
Model group
Model + Positive drug
Model + Excipient
Model+0.1% MDI-1228
Model+0.3% MDI-1228
Model+1% MDI-1228
Model+3% MDI-1228

FIG. 15

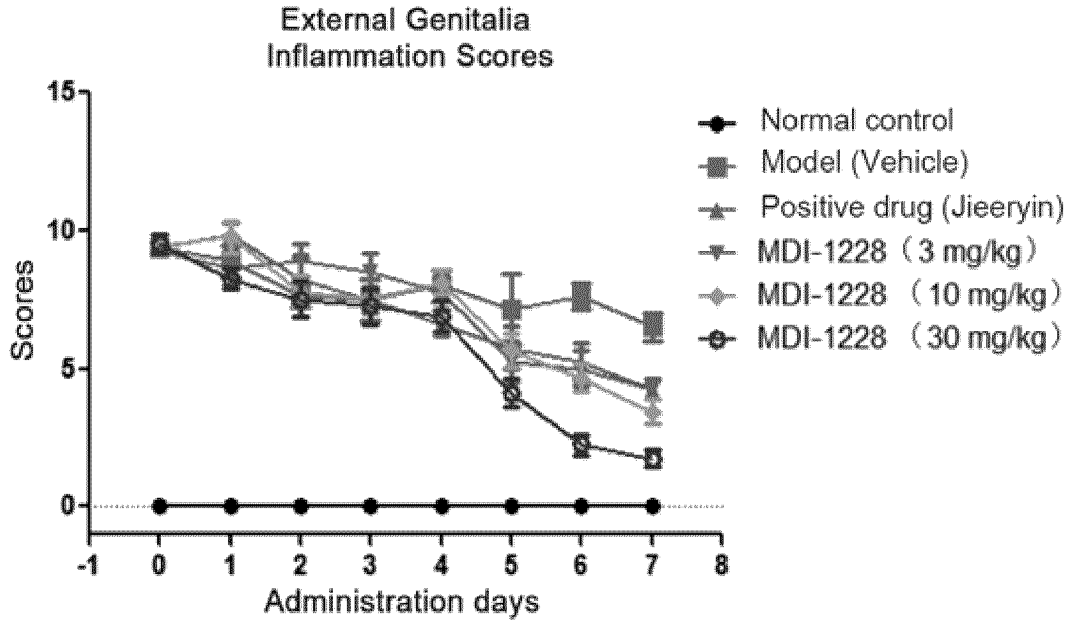
External Genitalia
Inflammation Scores
Scores
Administration days
15
10
5
0
-1
0
1
2
3
4
5
6
7
8
Normal control
Model (Vehicle)
Positive drug (Jieeryin)
MDI-1228（3 mg/kg）
MDI-1228 （10 mg/kg）
MDI-1228 （30 mg/kg）

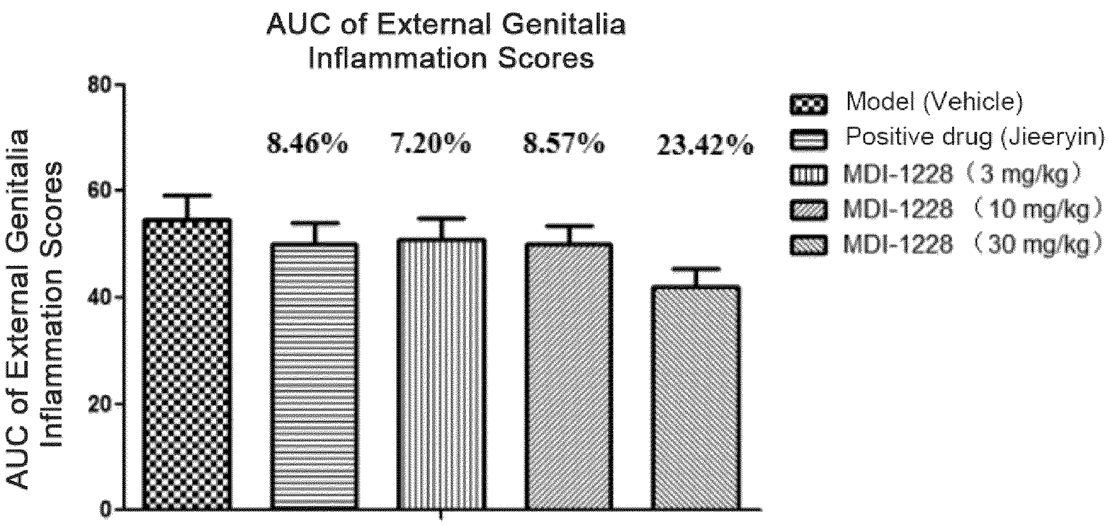
AUC of External Genitalia
Inflammation Scores
AUC of External Genitalia
Inflammation Scores
80
60
40
20
0
8.46%
7.20%
8.57%
23.42%
Model (Vehicle)
Positive drug (Jieeryin)
MDI-1228（3 mg/kg）
MDI-1228 （10 mg/kg）
MDI-1228 （30 mg/kg）

FIG. 16

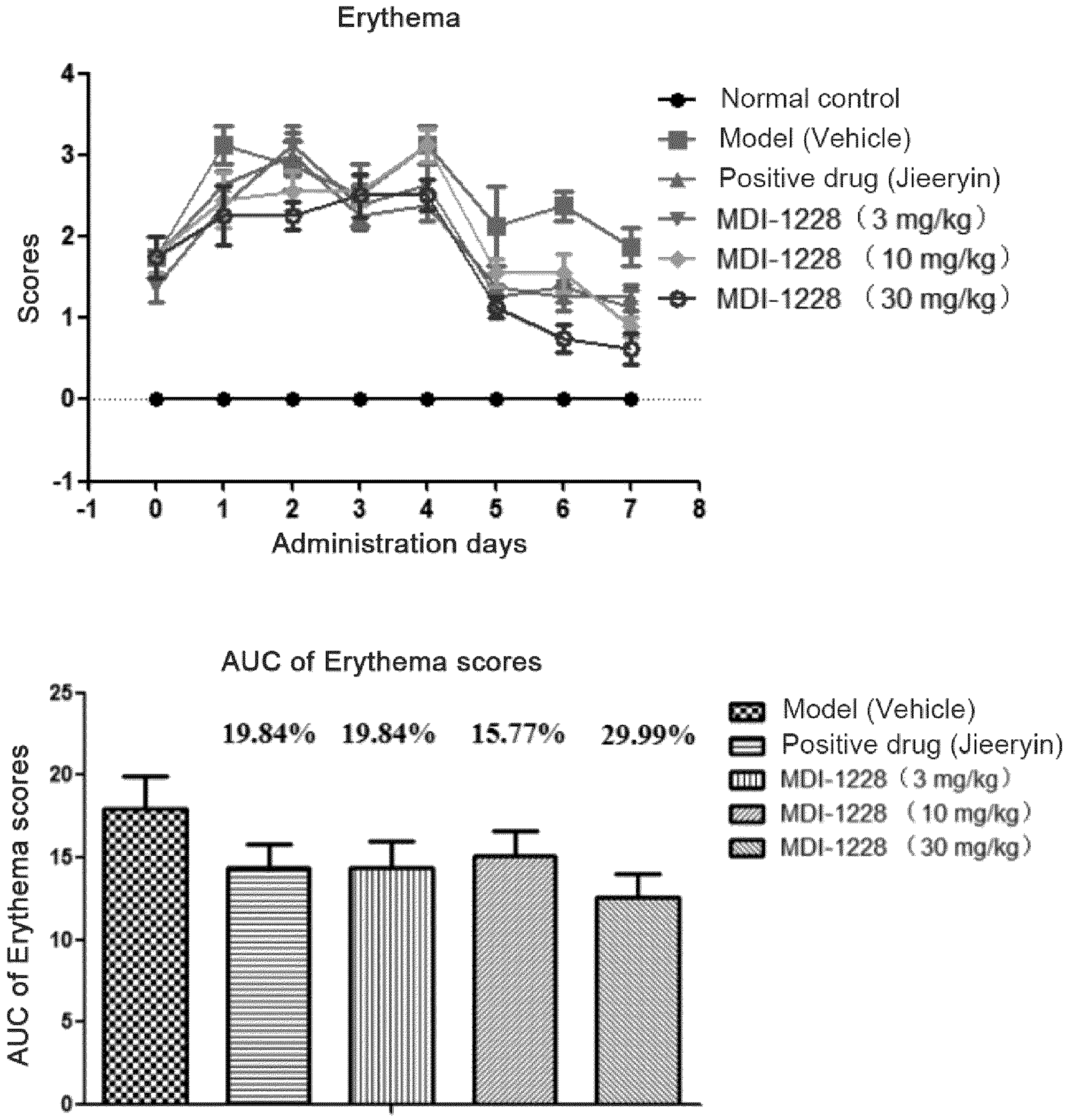
Erythema
Scores
Administration days
Normal control
Model (Vehicle)
Positive drug (Jieeryin)
MDI-1228（3 mg/kg）
MDI-1228 （10 mg/kg）
MDI-1228 （30 mg/kg）
AUC of Erythema scores
AUC of Erythema scores
19.84%
19.84%
15.77%
29.99%
Model (Vehicle)
Positive drug (Jieeryin)
MDI-1228（3 mg/kg）
MDI-1228 （10 mg/kg）
MDI-1228 （30 mg/kg）

FIG. 17

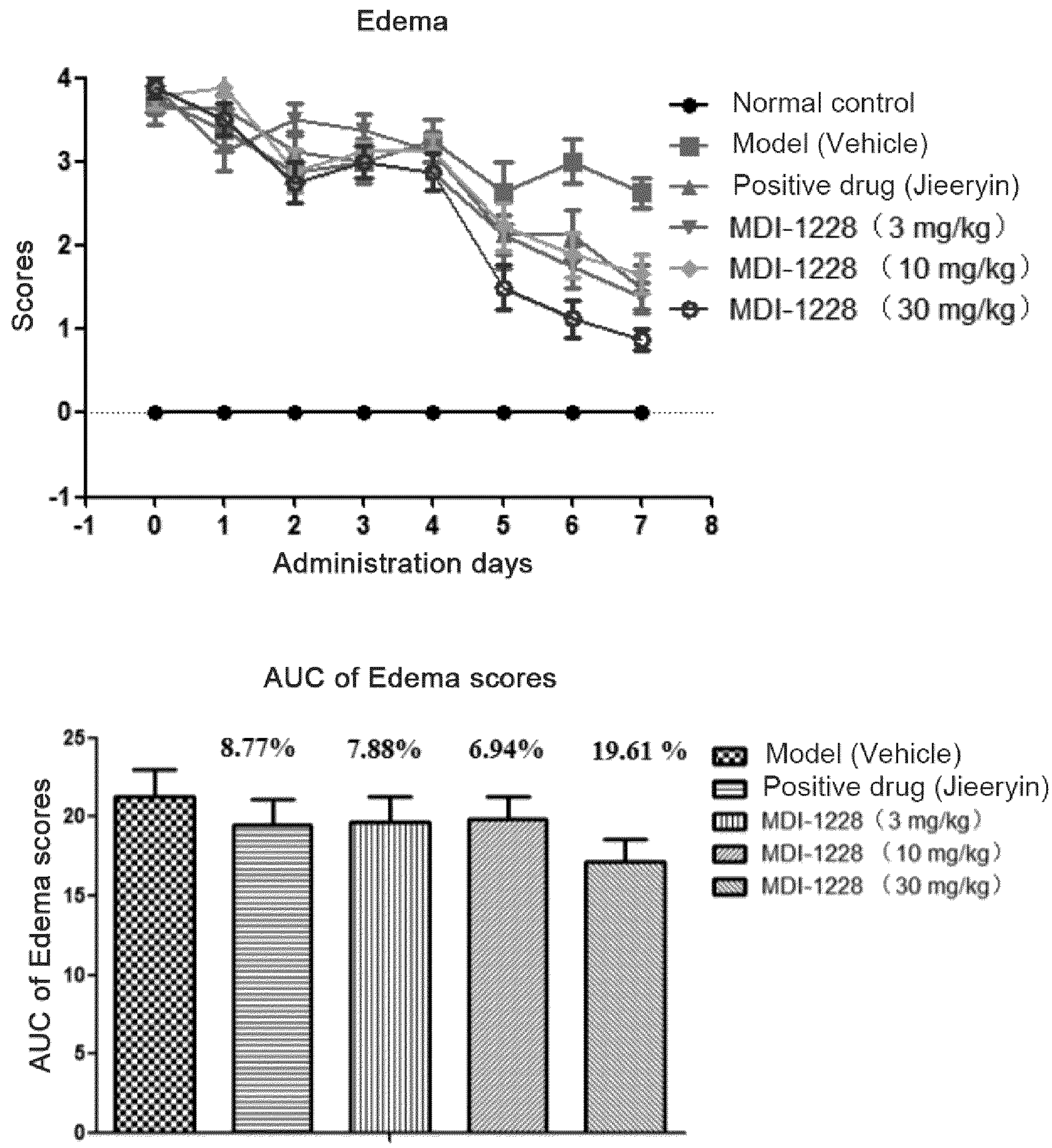
Edema
Scores
Administration days
Normal control
Model (Vehicle)
Positive drug (Jieeryin)
MDI-1228（3 mg/kg）
MDI-1228 （10 mg/kg）
MDI-1228 （30 mg/kg）
AUC of Edema scores
AUC of Edema scores
8.77%
7.88%
6.94%
19.61 %
Model (Vehicle)
Positive drug (Jieeryin)
MDI-1228（3 mg/kg）
MDI-1228 （10 mg/kg）
MDI-1228 （30 mg/kg）

FIG. 18

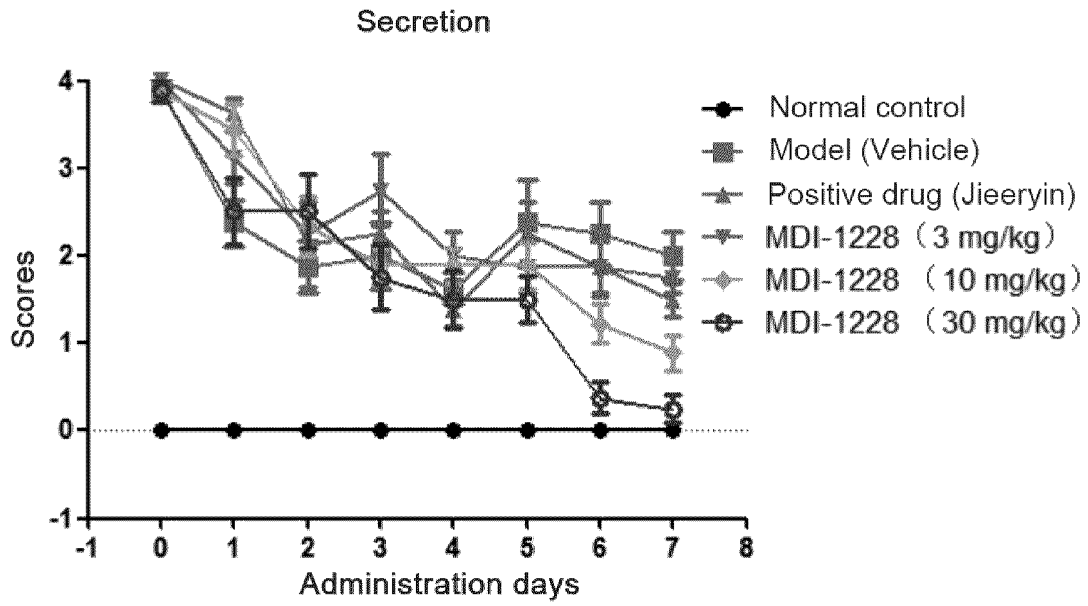
Secretion
Scores
Administration days
Normal control
Model (Vehicle)
Positive drug (Jieeryin)
MDI-1228（3 mg/kg）
MDI-1228 （10 mg/kg）
MDI-1228 （30 mg/kg）

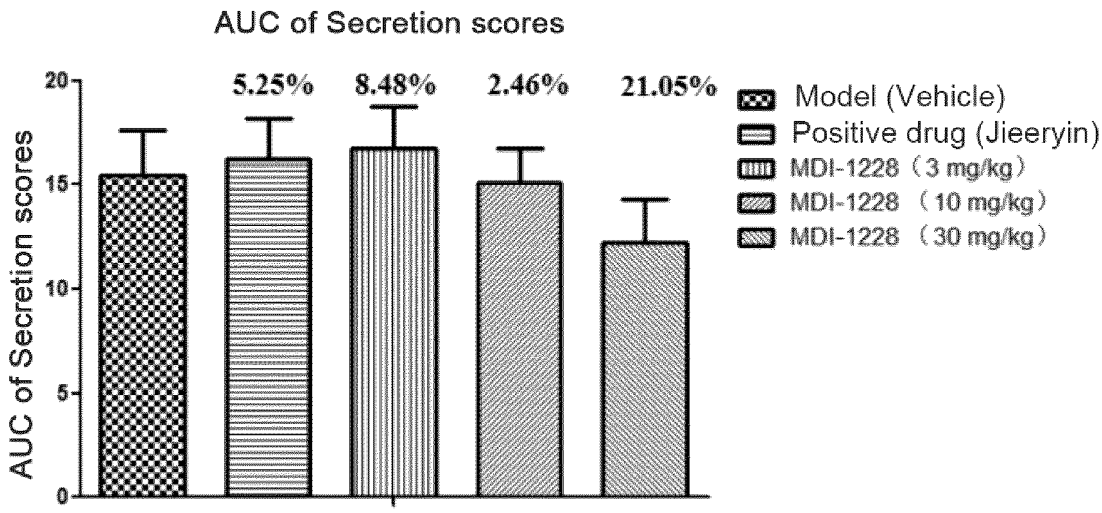
AUC of Secretion scores
AUC of Secretion scores
5.25%
8.48%
2.46%
21.05%
Model (Vehicle)
Positive drug (Jieeryin)
MDI-1228（3 mg/kg）
MDI-1228 （10 mg/kg）
MDI-1228 （30 mg/kg）

FIG. 19

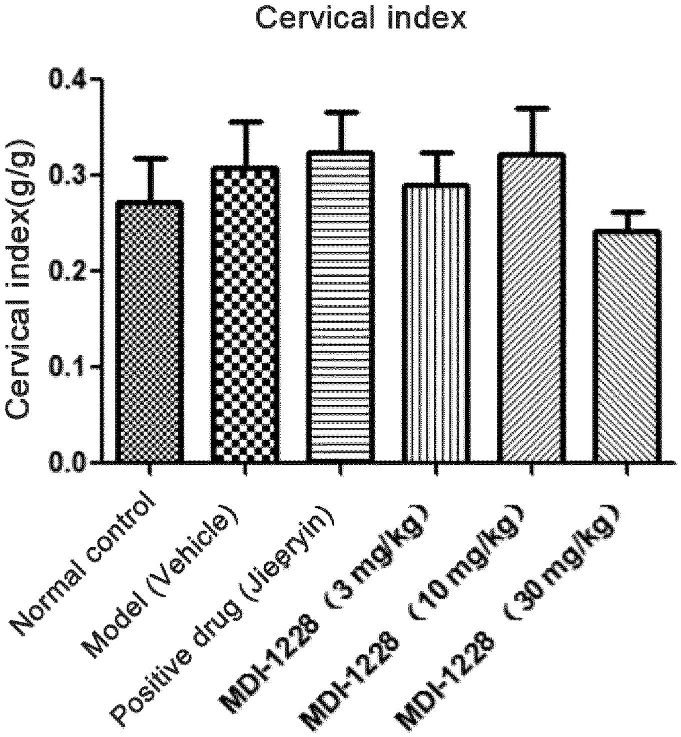
Cervical index
Cervical index(g/g)
0.4
0.3
0.2
0.1
0.0
Normal control
Model (Vehicle)
Positive drug (Jieeryin)
MDI-1228 （3 mg/kg）
MDI-1228 （10 mg/kg）
MDI-1228 （30 mg/kg）

FIG. 20

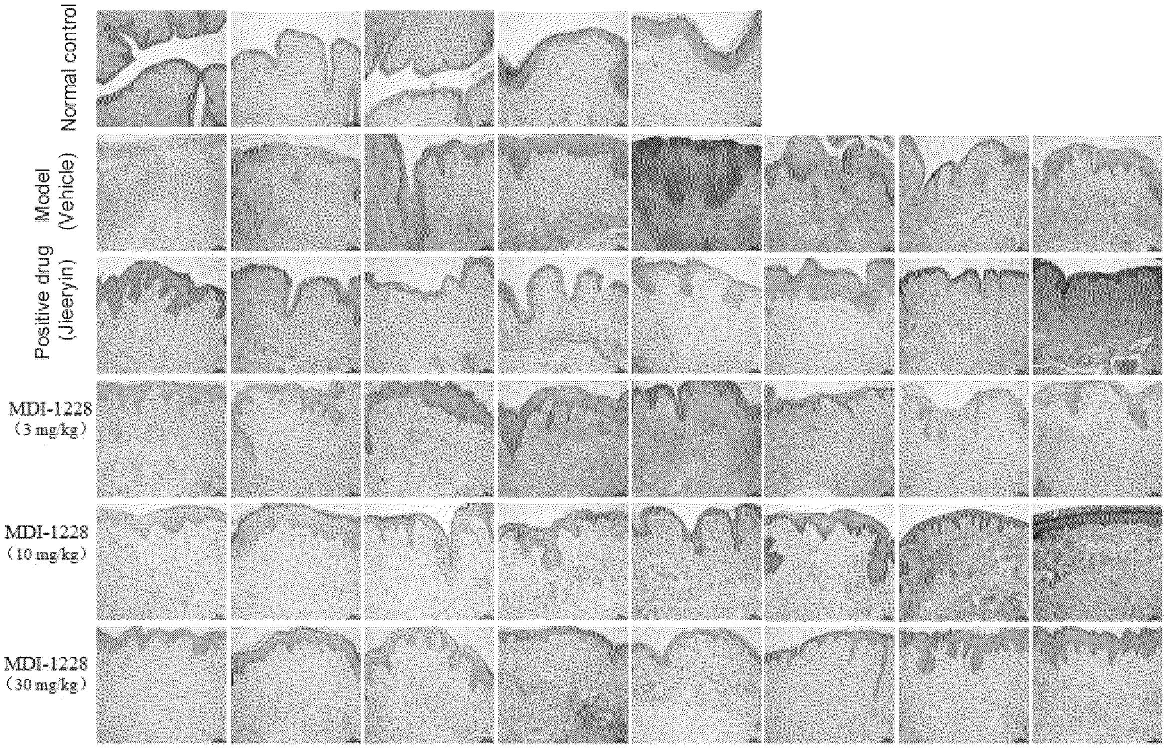
Normal control
Model (Vehicle)
Positive drug (Jieeryin)
MDI-1228 （3 mg/kg）
MDI-1228 （10 mg/kg）
MDI-1228 （30 mg/kg）

FIG. 21

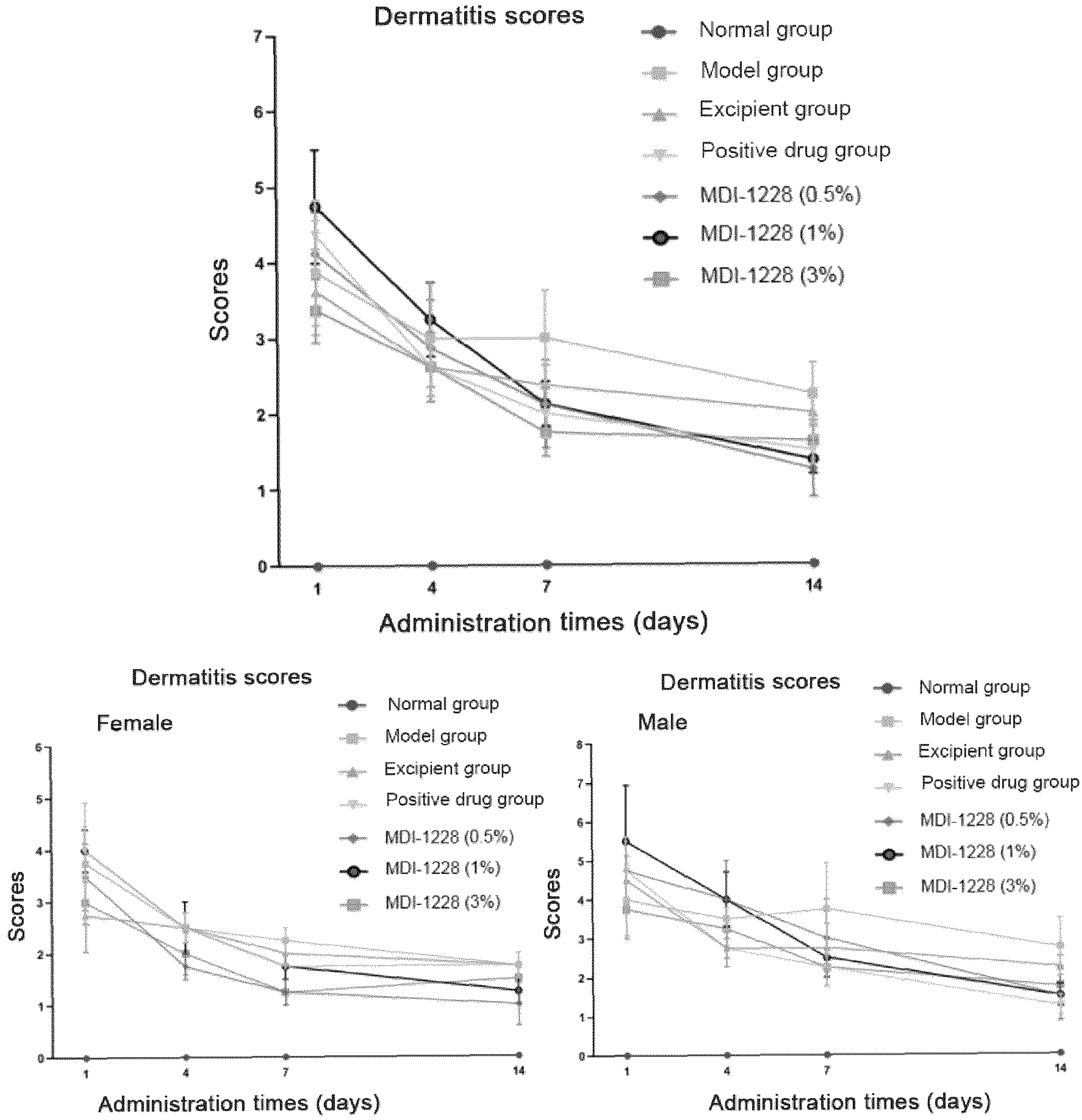
Dermatitis scores
Normal group
Model group
Excipient group
Positive drug group
MDI-1228 (0.5%)
MDI-1228 (1%)
MDI-1228 (3%)
Scores
Administration times (days)
Dermatitis scores
Female
Dermatitis scores
Male

FIG. 22

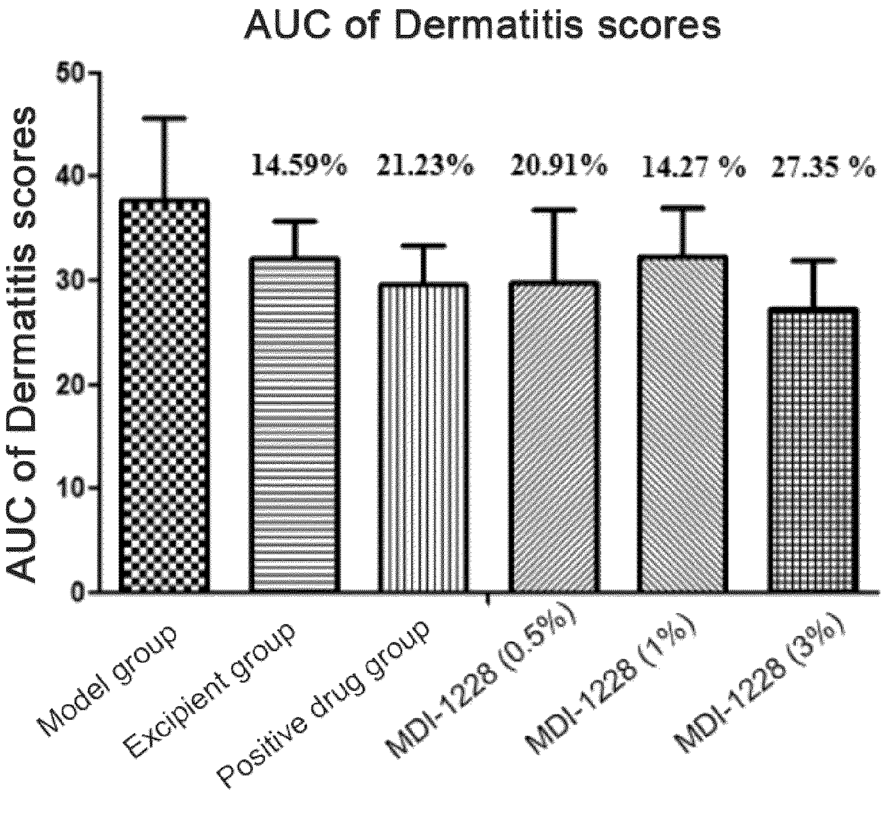
AUC of Dermatitis scores
AUC of Dermatitis scores
0
10
20
30
40
50
14.59%
21.23%
20.91%
14.27 %
27.35 %
Model group
Excipient group
Positive drug group
MDI-1228 (0.5%)
MDI-1228 (1%)
MDI-1228 (3%)

FIG. 23

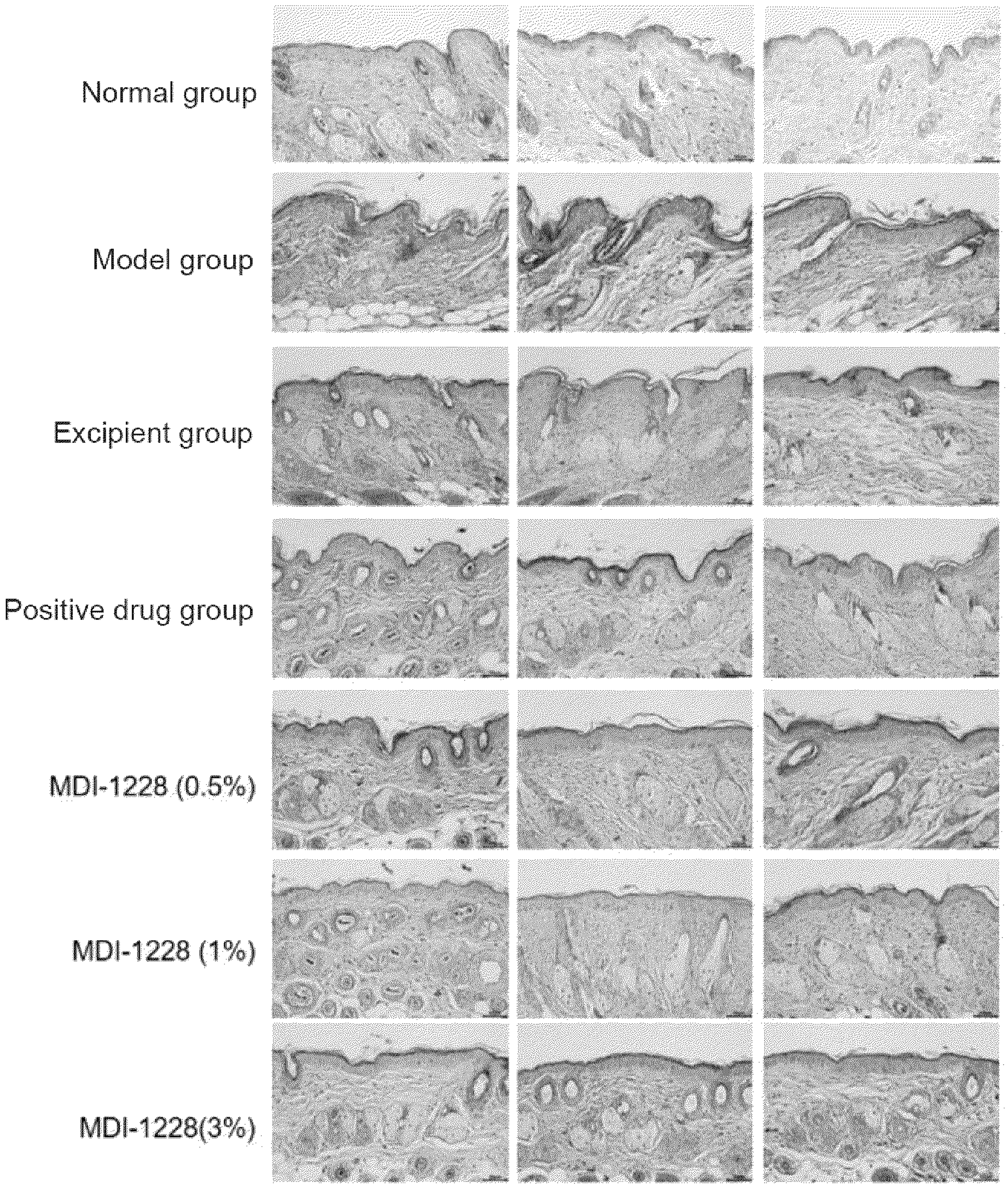
Normal group
Model group
Excipient group
Positive drug group
MDI-1228 (0.5%)
MDI-1228 (1%)
MDI-1228(3%)

FIG. 24

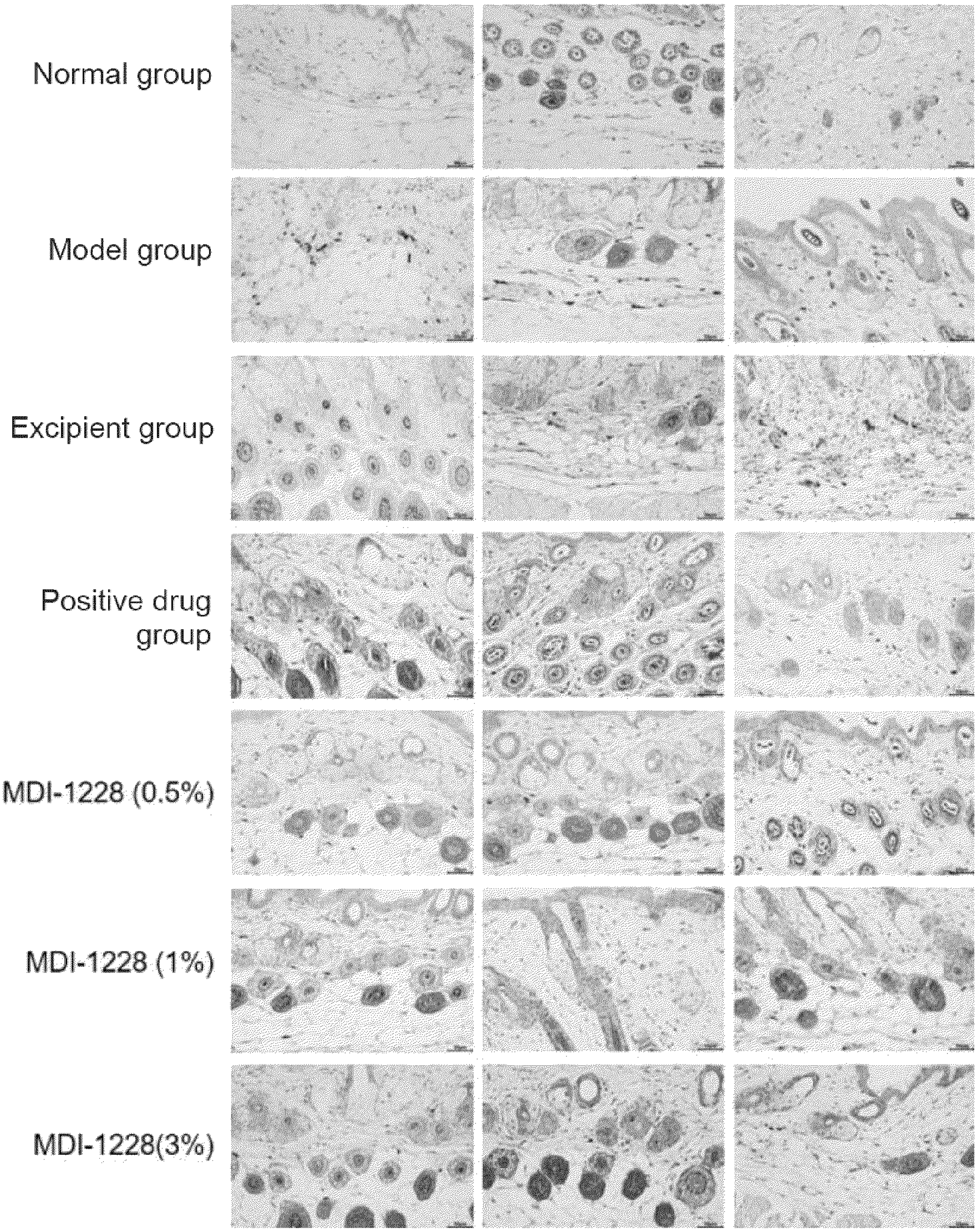
Normal group
Model group
Excipient group
Positive drug group
MDI-1228 (0.5%)
MDI-1228 (1%)
MDI-1228(3%)

FIG. 25

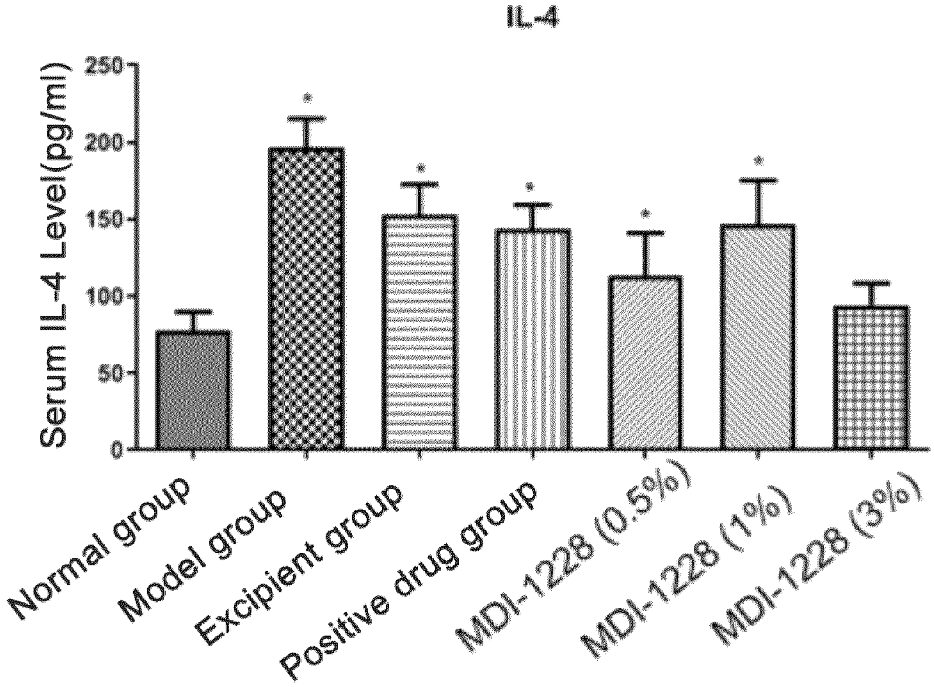
IL-4
Serum IL-4 Level(pg/ml)
250
200
150
100
50
0
*
Normal group
Model group
Excipient group
Positive drug group
MDI-1228 (0.5%)
MDI-1228 (1%)
MDI-1228 (3%)

FIG. 26

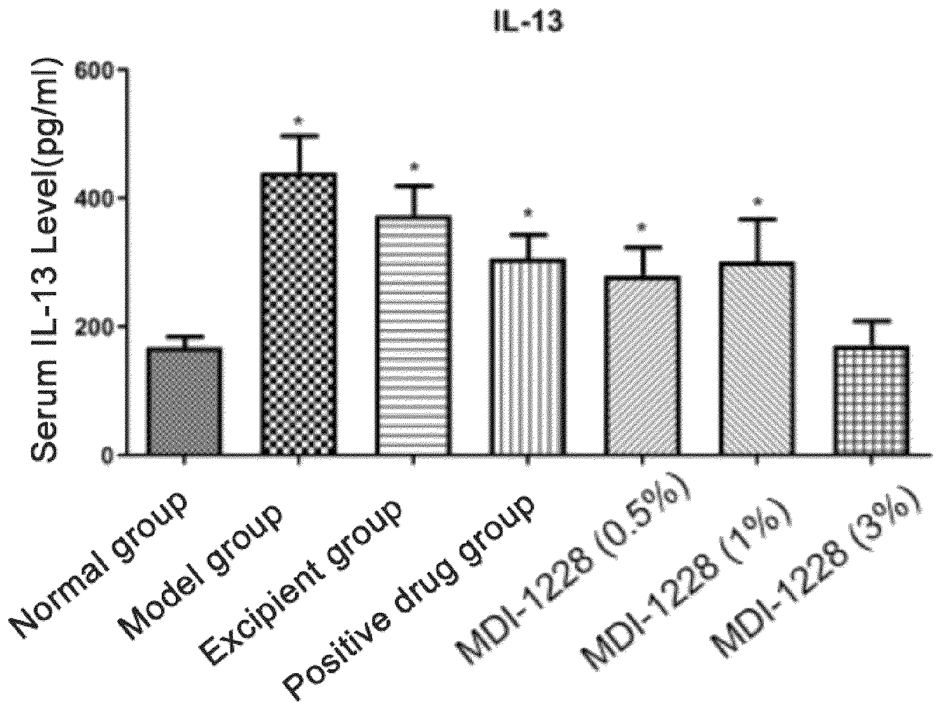
IL-13
Serum IL-13 Level(pg/ml)
600
400
200
0
*
Normal group
Model group
Excipient group
Positive drug group
MDI-1228 (0.5%)
MDI-1228 (1%)
MDI-1228 (3%)

FIG. 27

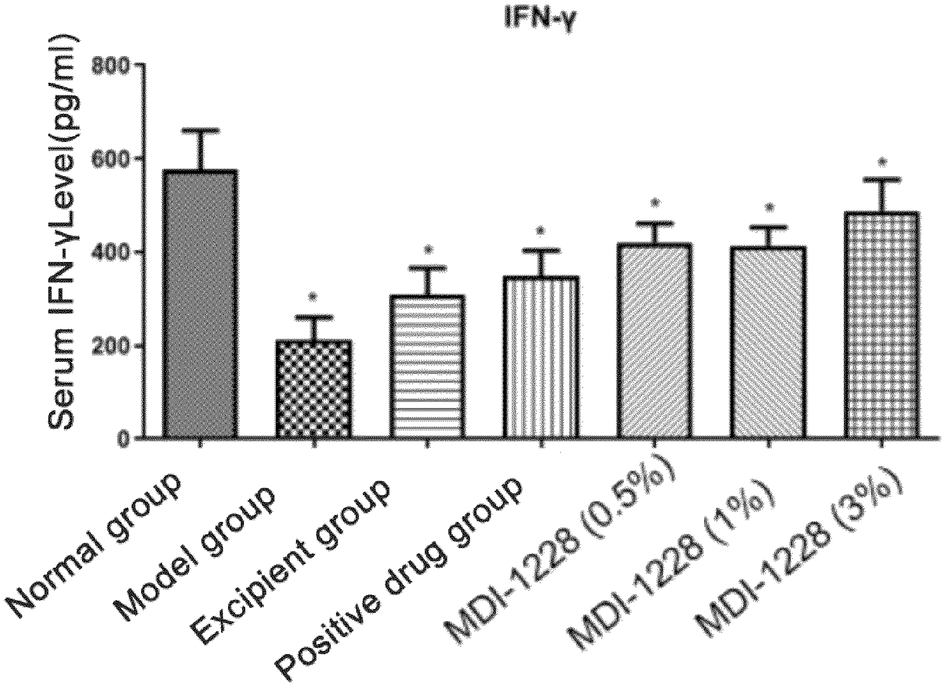
IFN-γ
Serum IFN-γLevel(pg/ml)
800
600
400
200
0
*
Normal group
Model group
Excipient group
Positive drug group
MDI-1228 (0.5%)
MDI-1228 (1%)
MDI-1228 (3%)

FIG. 28

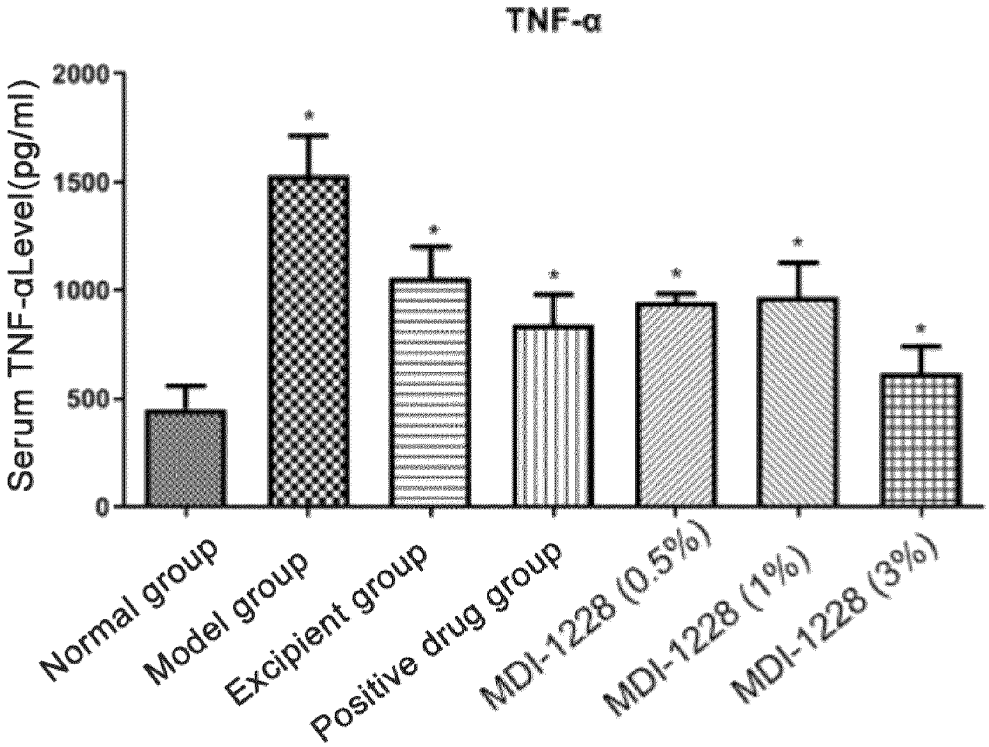
TNF-α
Serum TNF-αLevel(pg/ml)
2000
1500
1000
500
0
*
Normal group
Model group
Excipient group
Positive drug group
MDI-1228 (0.5%)
MDI-1228 (1%)
MDI-1228 (3%)

FIG. 29

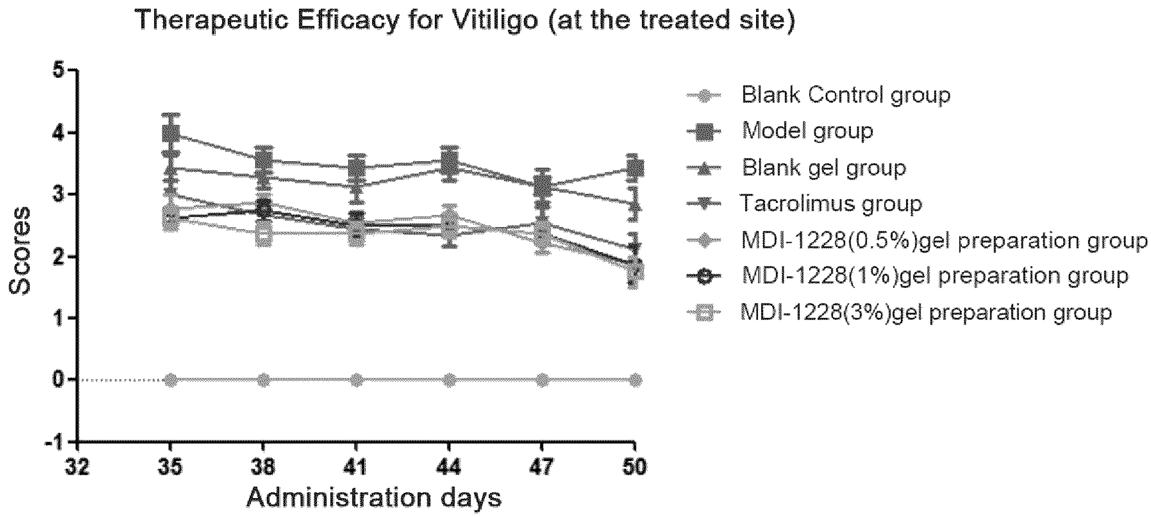
Therapeutic Efficacy for Vitiligo (at the treated site)
Scores
Administration days
Blank Control group
Model group
Blank gel group
Tacrolimus group
MDI-1228(0.5%)gel preparation group
MDI-1228(1%)gel preparation group
MDI-1228(3%)gel preparation group

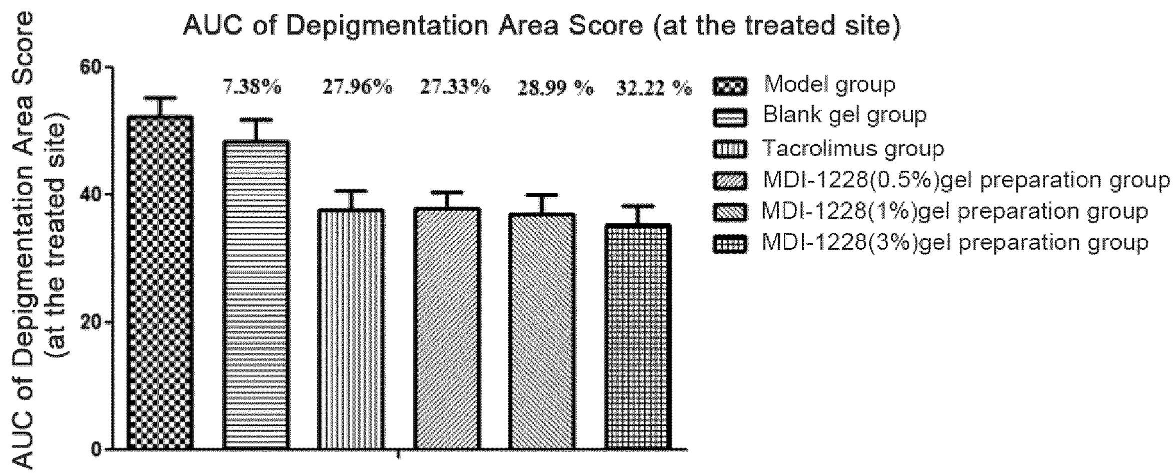
AUC of Depigmentation Area Score (at the treated site)
AUC of Depigmentation Area Score (at the treated site)
7.38%
27.96%
27.33%
28.99 %
32.22 %
Model group
Blank gel group
Tacrolimus group
MDI-1228(0.5%)gel preparation group
MDI-1228(1%)gel preparation group
MDI-1228(3%)gel preparation group

FIG. 30

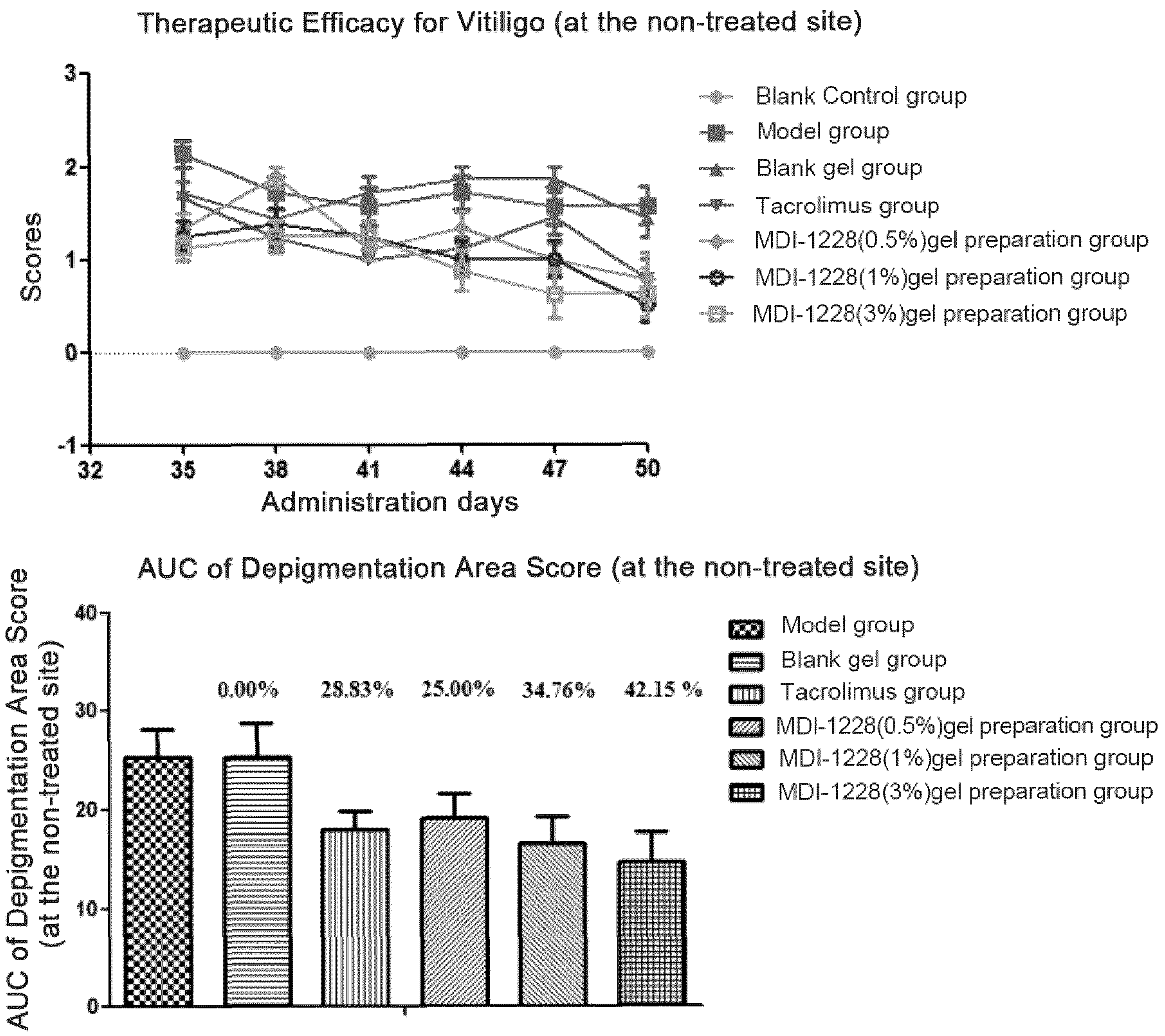
Therapeutic Efficacy for Vitiligo (at the non-treated site)
Scores
3
2
1
0
-1
32
35
38
41
44
47
50
Administration days
Blank Control group
Model group
Blank gel group
Tacrolimus group
MDI-1228(0.5%)gel preparation group
MDI-1228(1%)gel preparation group
MDI-1228(3%)gel preparation group
AUC of Depigmentation Area Score (at the non-treated site)
AUC of Depigmentation Area Score (at the non-treated site)
40
30
20
10
0
0.00%
28.83%
25.00%
34.76%
42.15 %
Model group
Blank gel group
Tacrolimus group
MDI-1228(0.5%)gel preparation group
MDI-1228(1%)gel preparation group
MDI-1228(3%)gel preparation group

FIG. 31

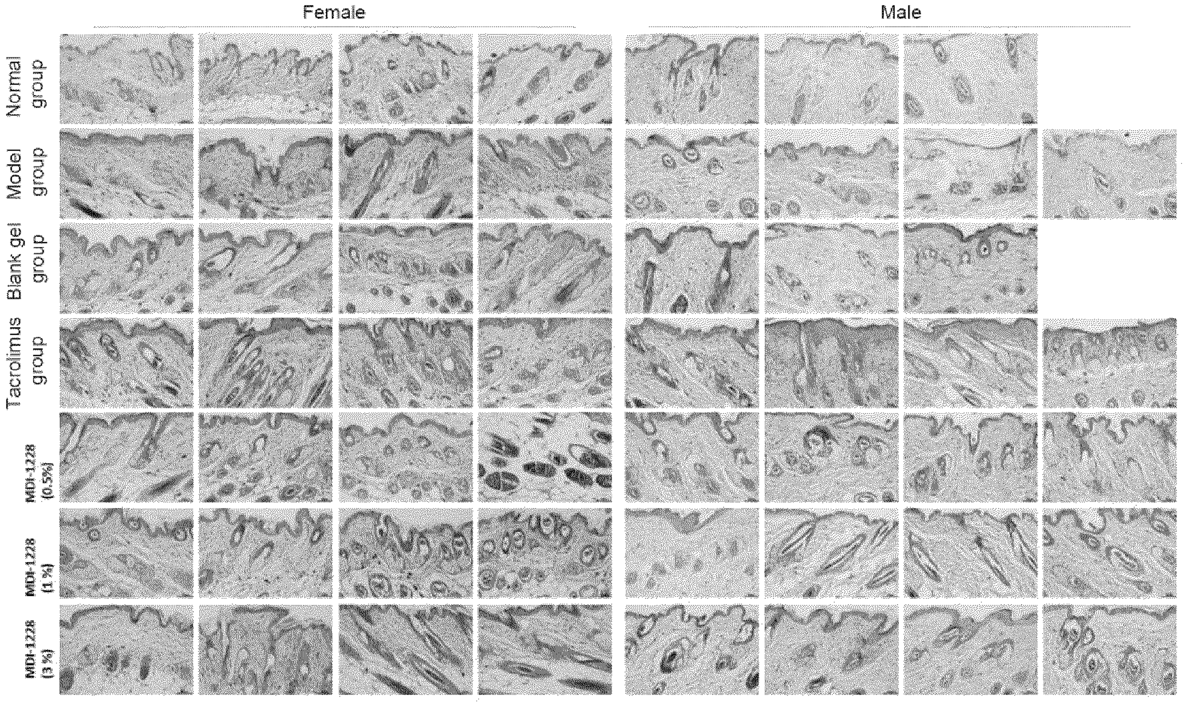
Female
Male
Normal group
Model group
Blank gel group
Tacrolimus group
MDI-1228 (0.5%)
MDI-1228 (1%)
MDI-1228 (3%)

FIG. 32

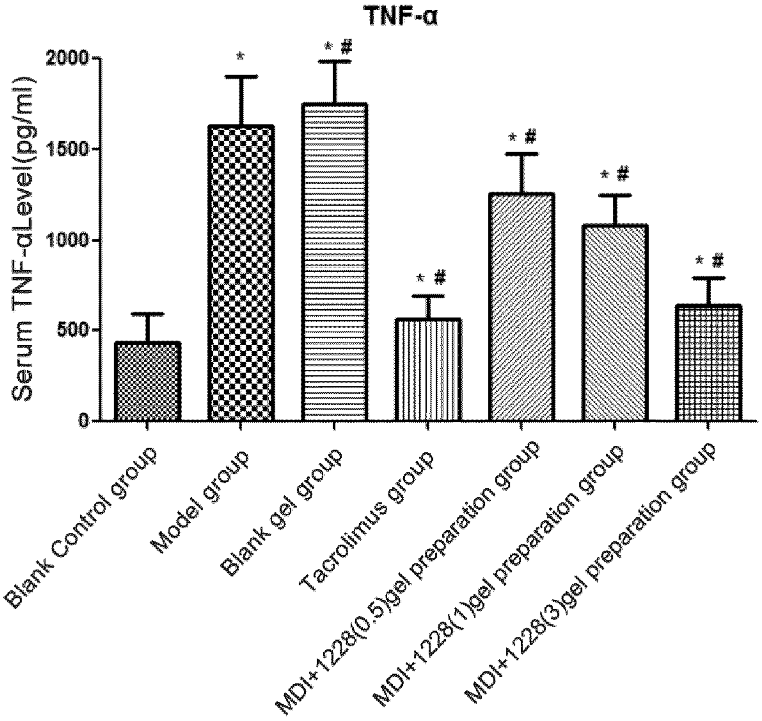
TNF-α
Serum TNF-αLevel(pg/ml)
2000
1500
1000
500
0
*
* #
* #
* #
* #
* #
Blank Control group
Model group
Blank gel group
Tacrolimus group
MDI+1228(0.5)gel preparation group
MDI+1228(1)gel preparation group
MDI+1228(3)gel preparation group

FIG. 33

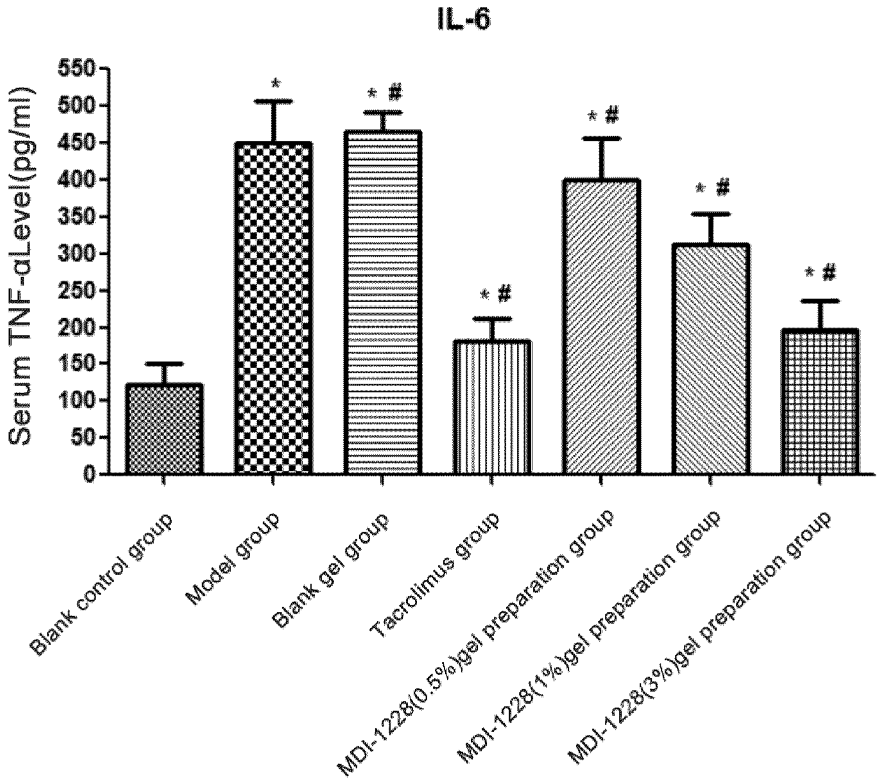
IL-6
Serum TNF-αLevel(pg/ml)
550
500
450
400
350
300
250
200
150
100
50
0
*
* #
* #
* #
* #
* #
Blank control group
Model group
Blank gel group
Tacrolimus group
MDI-1228(0.5%)gel preparation group
MDI-1228(1%)gel preparation group
MDI-1228(3%)gel preparation group

FIG. 34

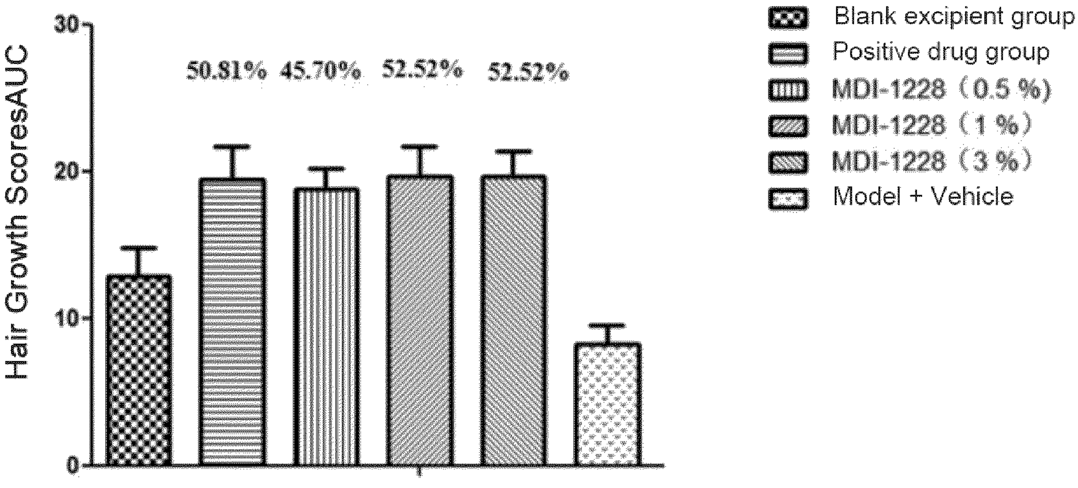
Hair Growth ScoresAUC
30
20
10
0
50.81% 45.70% 52.52% 52.52%
Blank excipient group
Positive drug group
MDI-1228（0.5 %)
MDI-1228（1 %）
MDI-1228（3 %）
Model + Vehicle

FIG. 35

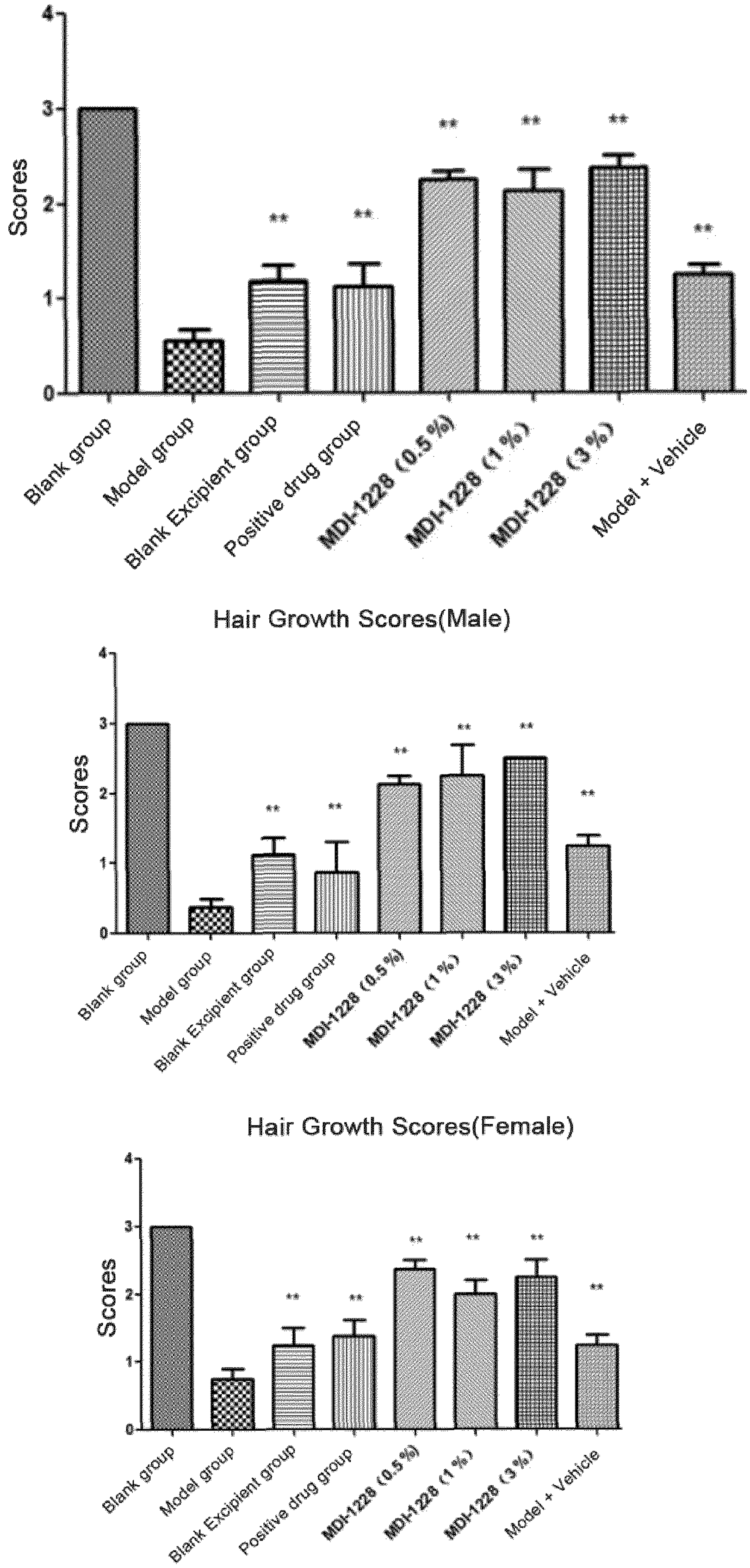
Scores
0
1
2
3
4
**
Blank group
Model group
Blank Excipient group
Positive drug group
MDI-1228 (0.5%)
MDI-1228 (1%)
MDI-1228 (3%)
Model + Vehicle
Hair Growth Scores(Male)
Hair Growth Scores(Female)

FIG. 36

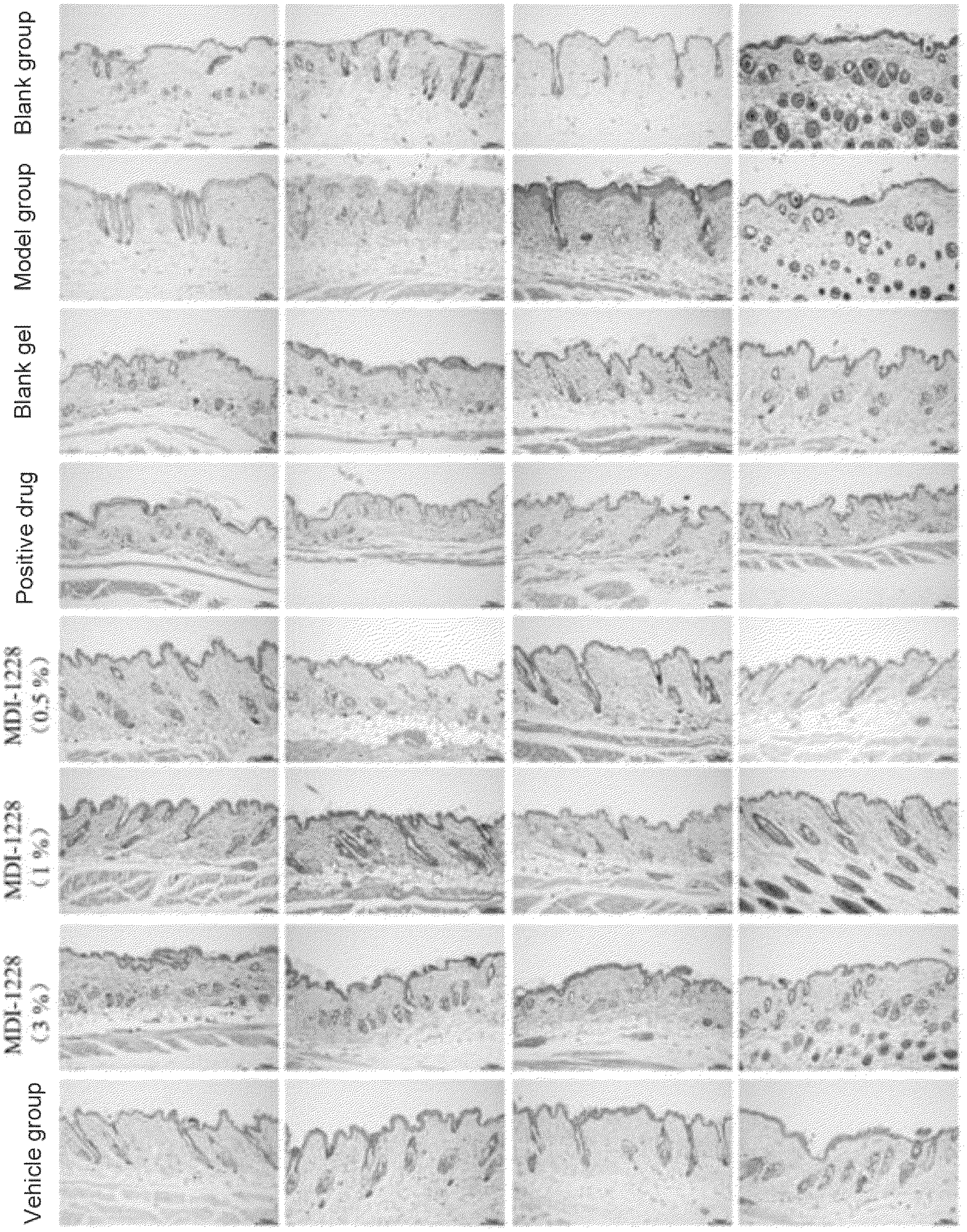
Blank group
Model group
Blank gel
Positive drug
MDI-1228 (0.5 %)
MDI-1228 (1 %)
MDI-1228 (3 %)
Vehicle group

FIG. 37

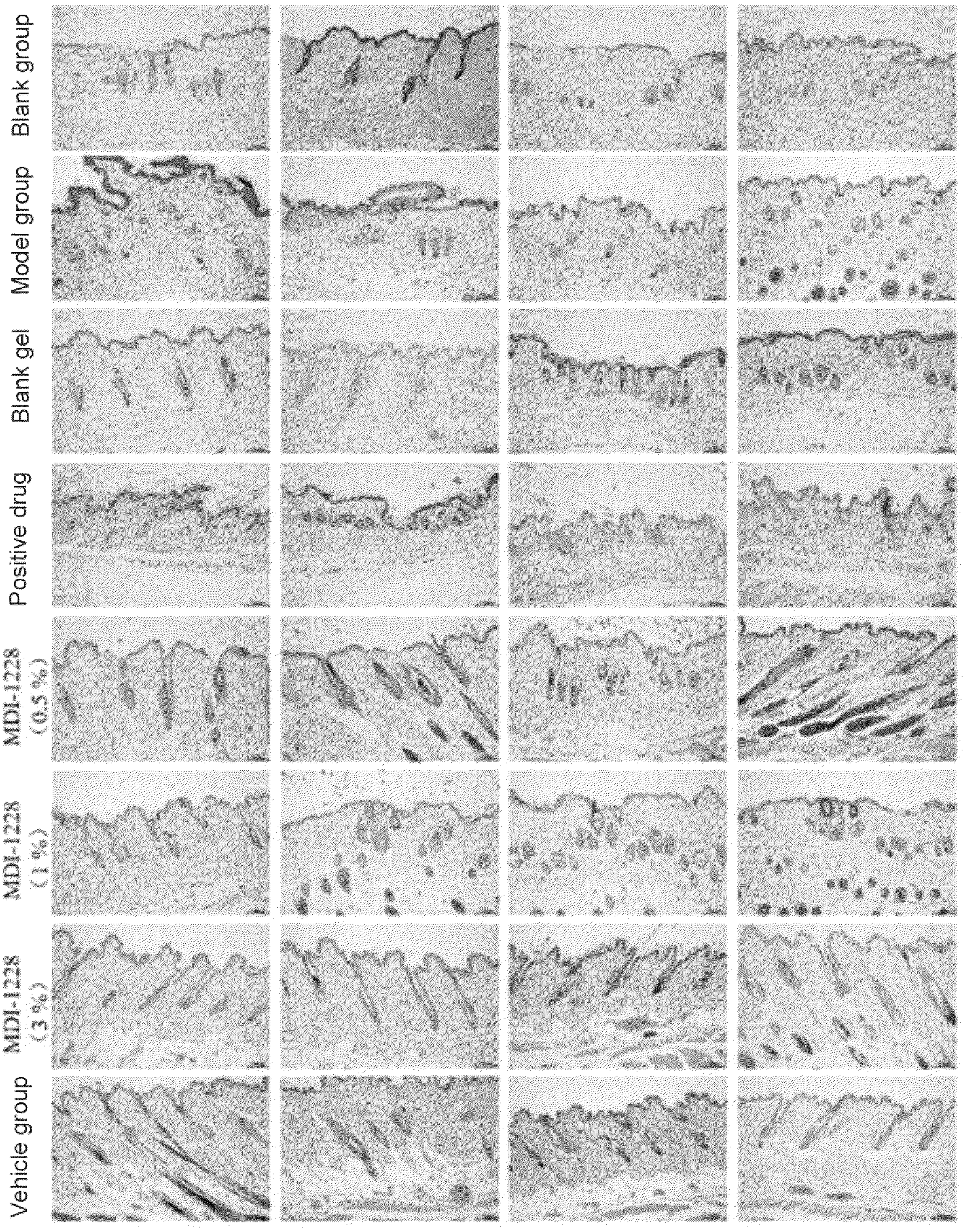
Blank group
Model group
Blank gel
Positive drug
MDI-1228 (0.5 %)
MDI-1228 (1 %)
MDI-1228 (3 %)
Vehicle group

FIG. 38

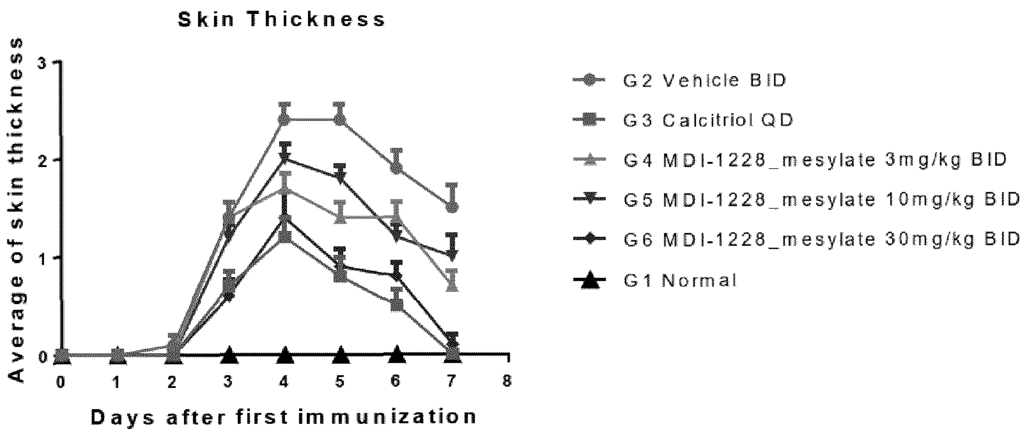
Skin Thickness
Average of skin thickness
3
2
1
0
0
1
2
3
4
5
6
7
8
Days after first immunization
G2 Vehicle BID
G3 Calcitriol QD
G4 MDI-1228_mesylate 3mg/kg BID
G5 MDI-1228_mesylate 10mg/kg BID
G6 MDI-1228_mesylate 30mg/kg BID
G1 Normal

FIG. 39

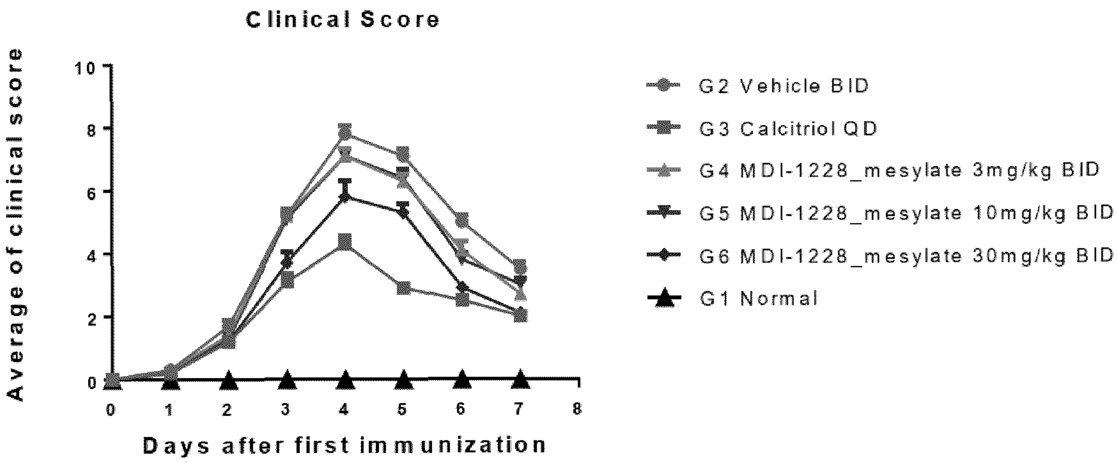
Clinical Score
Average of clinical score
10
8
6
4
2
0
0
1
2
3
4
5
6
7
8
Days after first immunization
G2 Vehicle BID
G3 Calcitriol QD
G4 MDI-1228_mesylate 3mg/kg BID
G5 MDI-1228_mesylate 10mg/kg BID
G6 MDI-1228_mesylate 30mg/kg BID
G1 Normal

FIG. 40

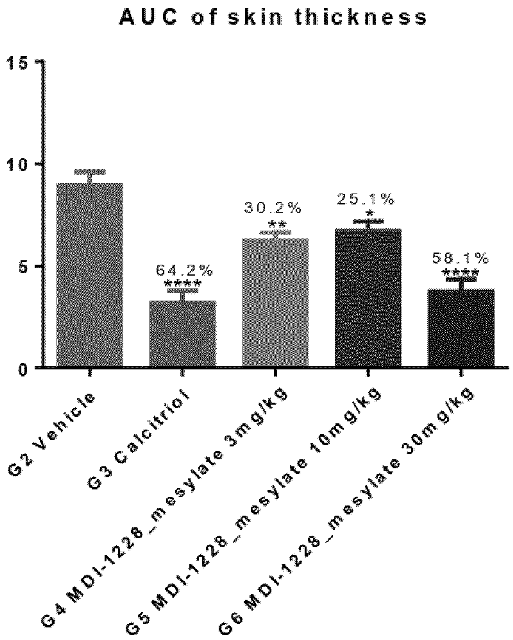
AUC of skin thickness
15
10
5
0
64.2%
****
30.2%
**
25.1%
*
58.1%
****
G2 Vehicle
G3 Calcitriol
G4 MDI-1228_mesylate 3mg/kg
G5 MDI-1228_mesylate 10mg/kg
G6 MDI-1228_mesylate 30mg/kg

FIG. 41

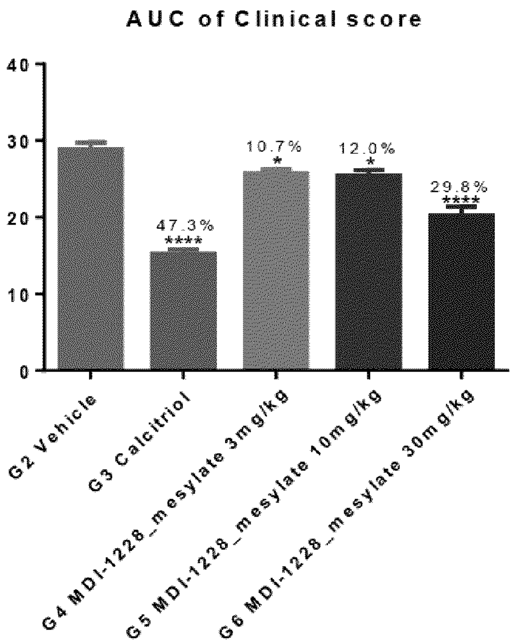
AUC of Clinical score
40
30
20
10
0
47.3%
****
10.7%
*
12.0%
*
29.8%
****
G2 Vehicle
G3 Calcitriol
G4 MDI-1228_mesylate 3mg/kg
G5 MDI-1228_mesylate 10mg/kg
G6 MDI-1228_mesylate 30mg/kg

FIG. 42

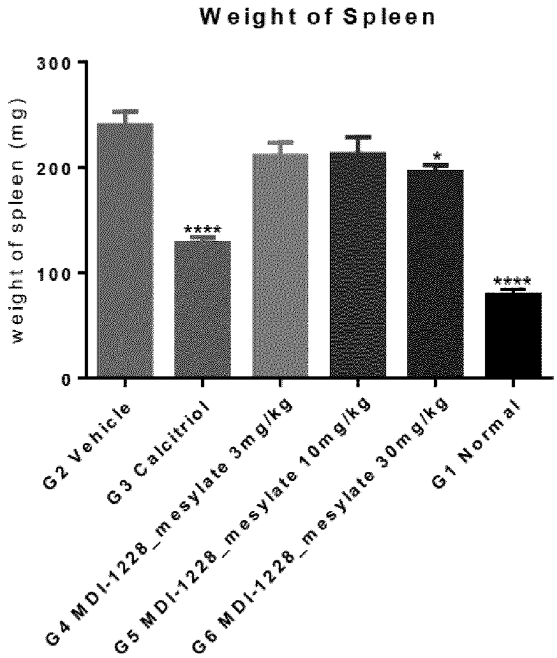
Weight of Spleen
weight of spleen (mg)
300
200
100
0
****
*
****
G2 Vehicle
G3 Calcitriol
G4 MDI-1228_mesylate 3mg/kg
G5 MDI-1228_mesylate 10mg/kg
G6 MDI-1228_mesylate 30mg/kg
G1 Normal

FIG. 43

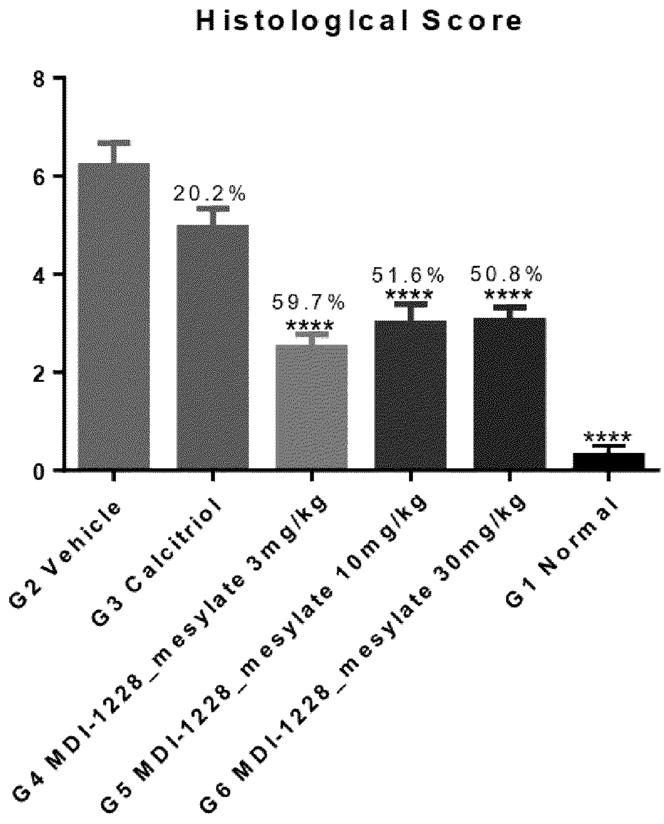
Histological Score
8
6
4
2
0
20.2%
59.7%
****
51.6%
****
50.8%
****
****
G2 Vehicle
G3 Calcitriol
G4 MDI-1228_mesylate 3mg/kg
G5 MDI-1228_mesylate 10mg/kg
G6 MDI-1228_mesylate 30mg/kg
G1 Normal

FIG. 44

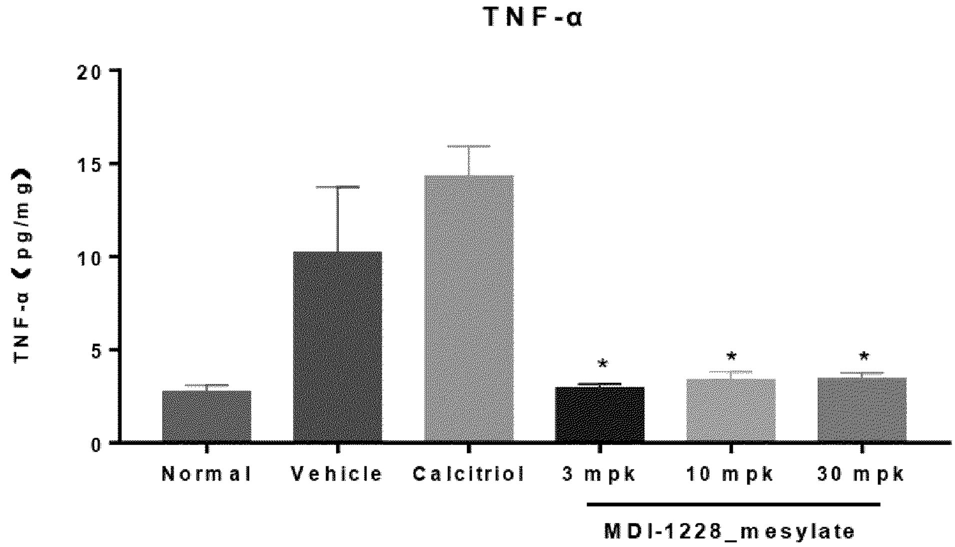
TNF-α
TNF-α（pg/mg）
20
15
10
5
0
Normal
Vehicle
Calcitriol
3 mpk
10 mpk
30 mpk
MDI-1228_mesylate
*
*
*

FIG. 45

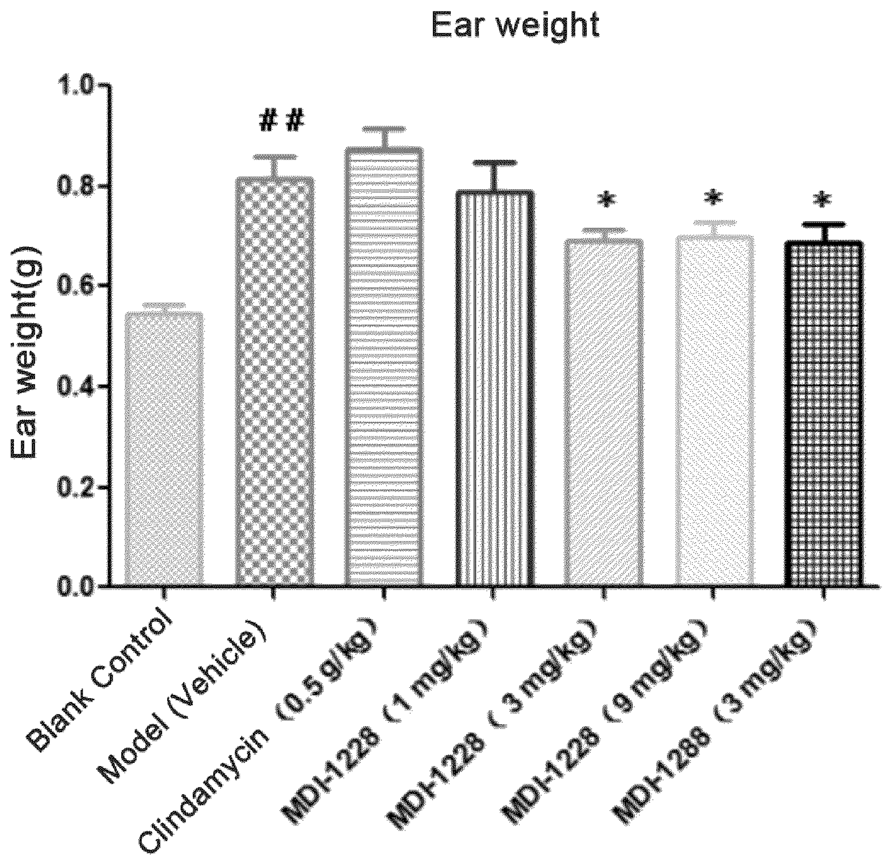
Ear weight
Ear weight(g)
1.0
0.8
0.6
0.4
0.2
0.0
##
*
*
*
Blank Control
Model (Vehicle)
Clindamycin （0.5 g/kg）
MDI-1228 （1 mg/kg）
MDI-1228 （ 3 mg/kg）
MDI-1228 （9 mg/kg）
MDI-1288 （3 mg/kg）

FIG. 46

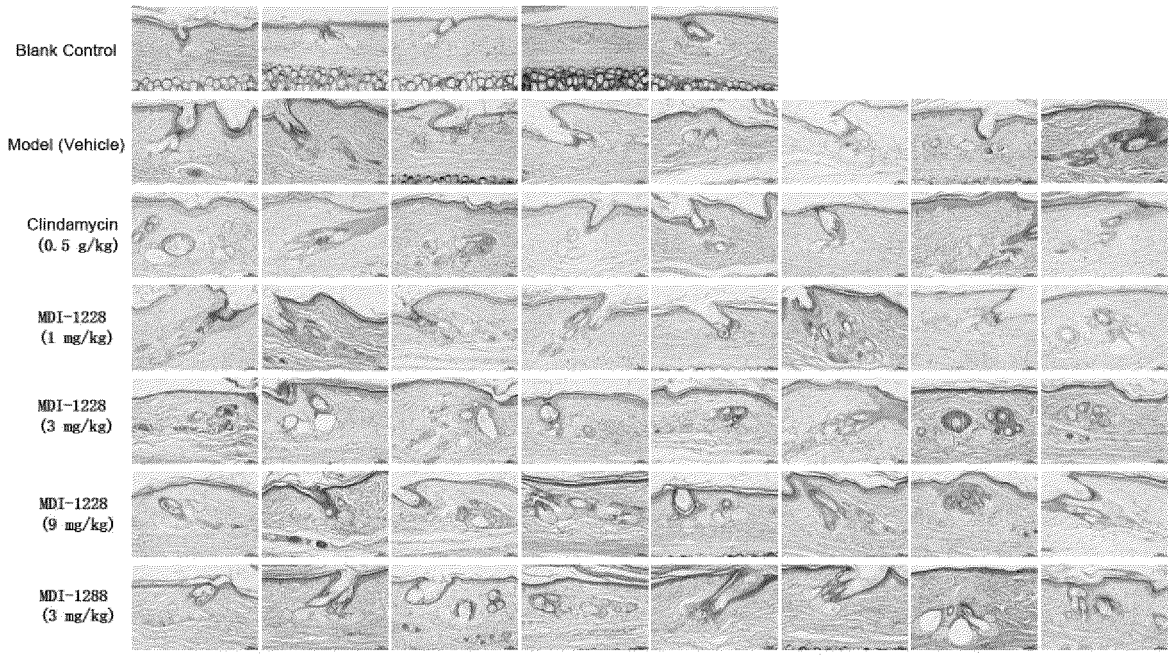
Blank Control
Model (Vehicle)
Clindamycin
(0.5 g/kg)
MDI-1228
(1 mg/kg)
MDI-1228
(3 mg/kg)
MDI-1228
(9 mg/kg)
MDI-1288
(3 mg/kg)

FIG. 47

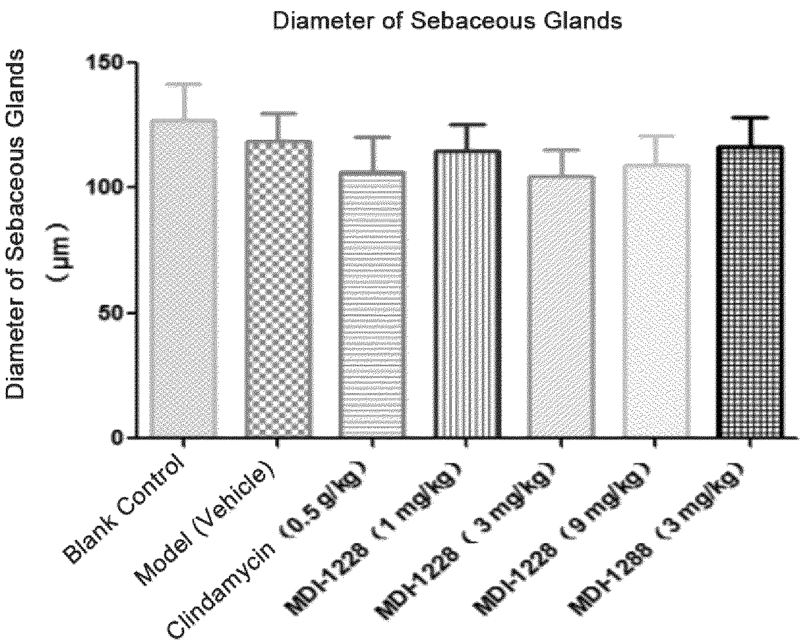
Diameter of Sebaceous Glands
Diameter of Sebaceous Glands
(μm)
150
100
50
0
Blank Control
Model (Vehicle)
Clindamycin (0.5 g/kg)
MDI-1228 (1 mg/kg)
MDI-1228 (3 mg/kg)
MDI-1228 (9 mg/kg)
MDI-1288 (3 mg/kg)

FIG. 48

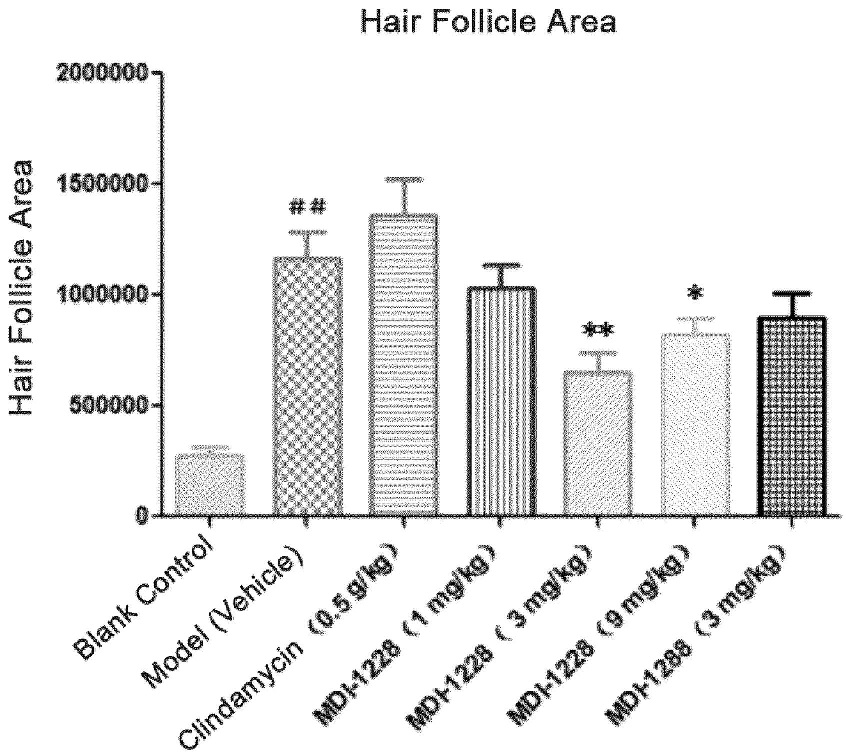
Hair Follicle Area
Hair Follicle Area
2000000
1500000
1000000
500000
0
##
**
*
Blank Control
Model (Vehicle)
Clindamycin (0.5 g/kg)
MDI-1228 (1 mg/kg)
MDI-1228 (3 mg/kg)
MDI-1228 (9 mg/kg)
MDI-1288 (3 mg/kg)

FIG. 49

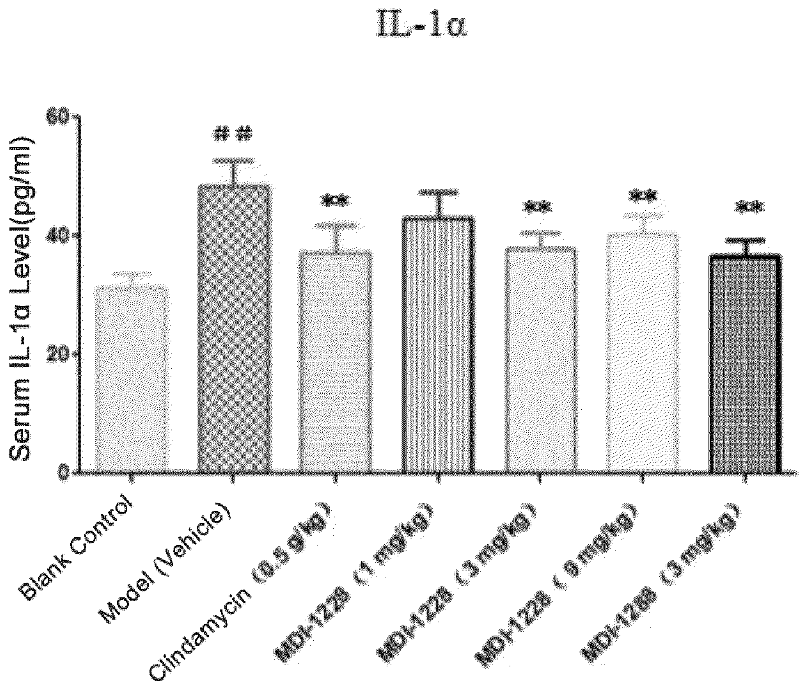
IL-1α
Serum IL-1α Level(pg/ml)
60
40
20
0
##
**
**
**
**
Blank Control
Model (Vehicle)
Clindamycin (0.5 g/kg)
MDI-1228 (1 mg/kg)
MDI-1228 (3 mg/kg)
MDI-1228 (9 mg/kg)
MDI-1288 (3 mg/kg)

FIG. 50

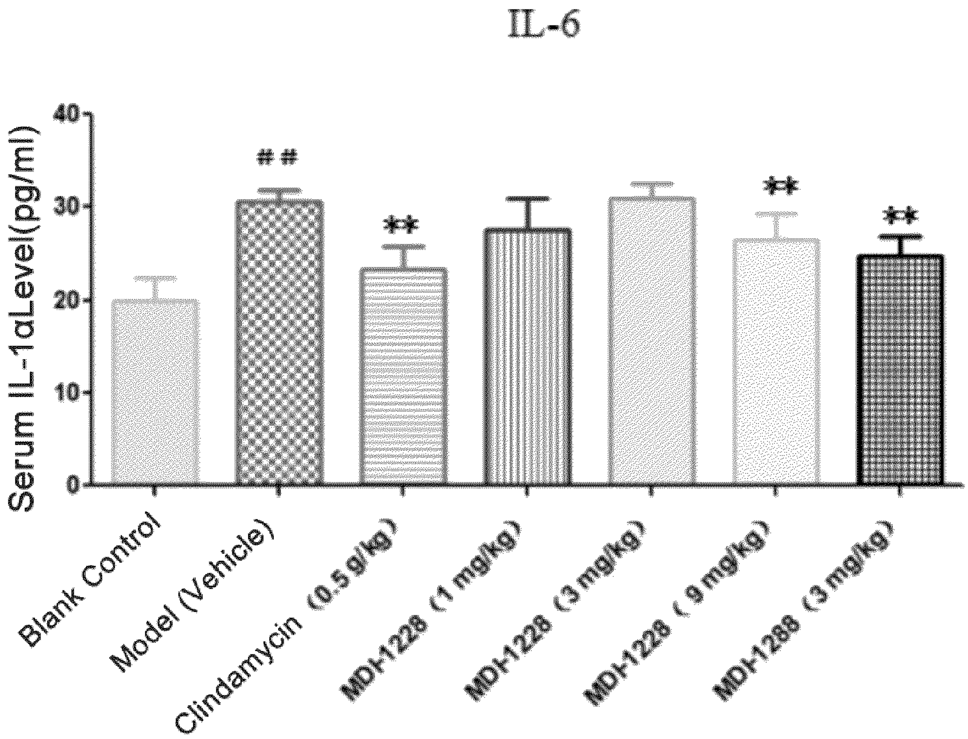
IL-6
Serum IL-1αLevel(pg/ml)
40
30
20
10
0
##
**
**
**
Blank Control
Model (Vehicle)
Clindamycin（0.5 g/kg）
MDI-1228（1 mg/kg）
MDI-1228（3 mg/kg）
MDI-1228（9 mg/kg）
MDI-1288（3 mg/kg）

FIG. 51

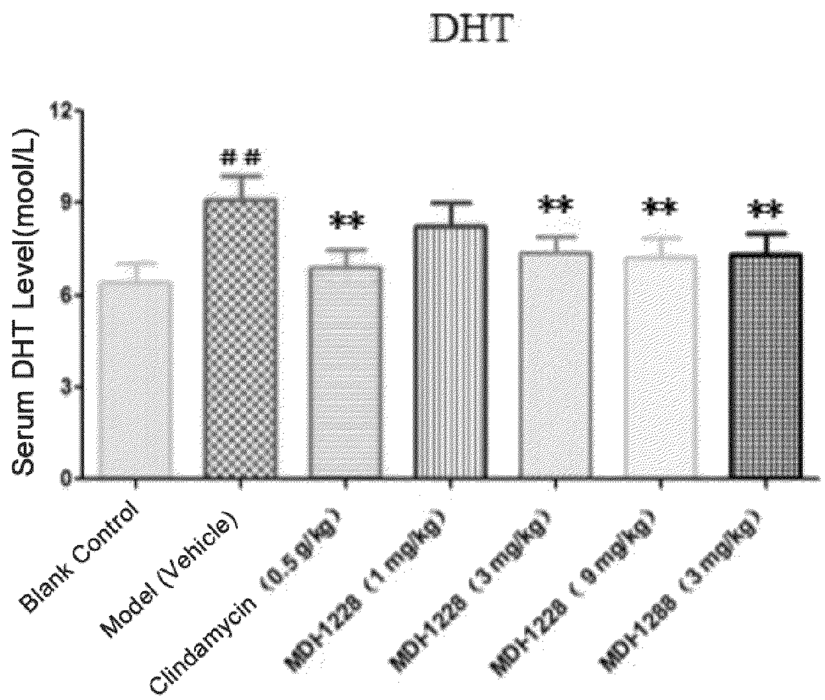
DHT
Serum DHT Level(mool/L)
12
9
6
3
0
##
**
**
**
**
Blank Control
Model (Vehicle)
Clindamycin（0.5 g/kg）
MDI-1228（1 mg/kg）
MDI-1228（3 mg/kg）
MDI-1228（9 mg/kg）
MDI-1288（3 mg/kg）

FIG. 52

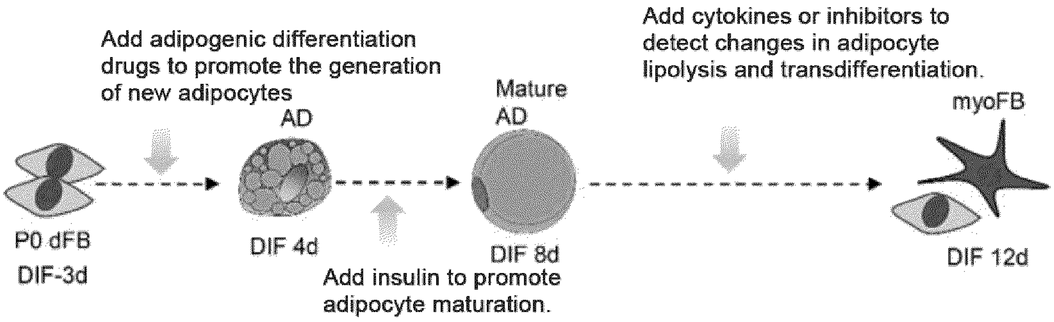


FIG. 53A

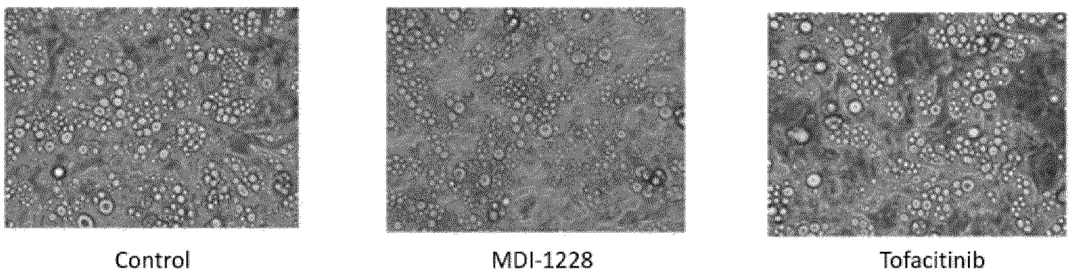


FIG. 53B

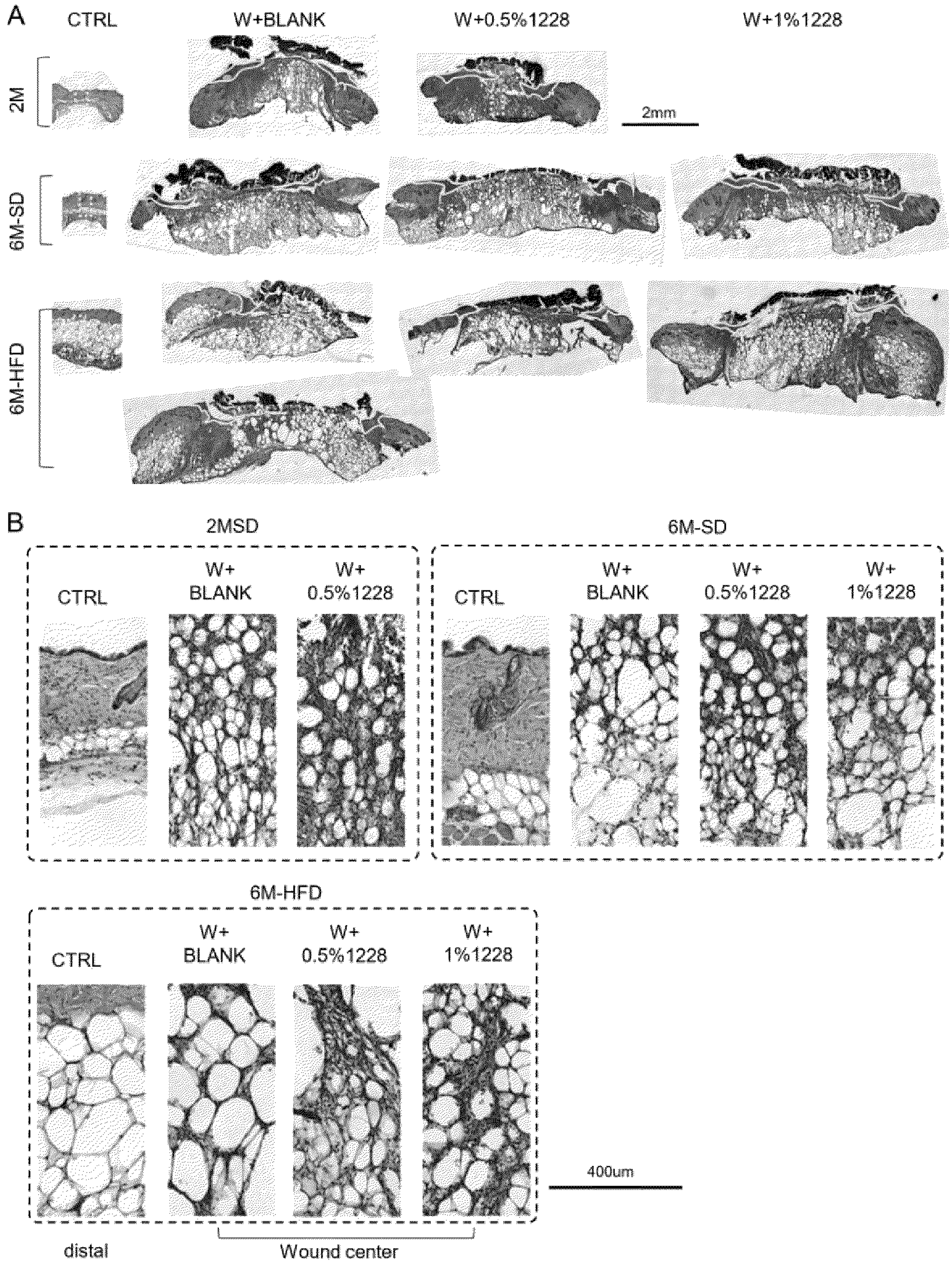
A
CTRL
W+BLANK
W+0.5%1228
W+1%1228
2M
6M-SD
6M-HFD
2mm
B
2MSD
6M-SD
CTRL
W+ BLANK
W+ 0.5%1228
W+ 1%1228
6M-HFD
400um
distal
Wound center

FIG. 54

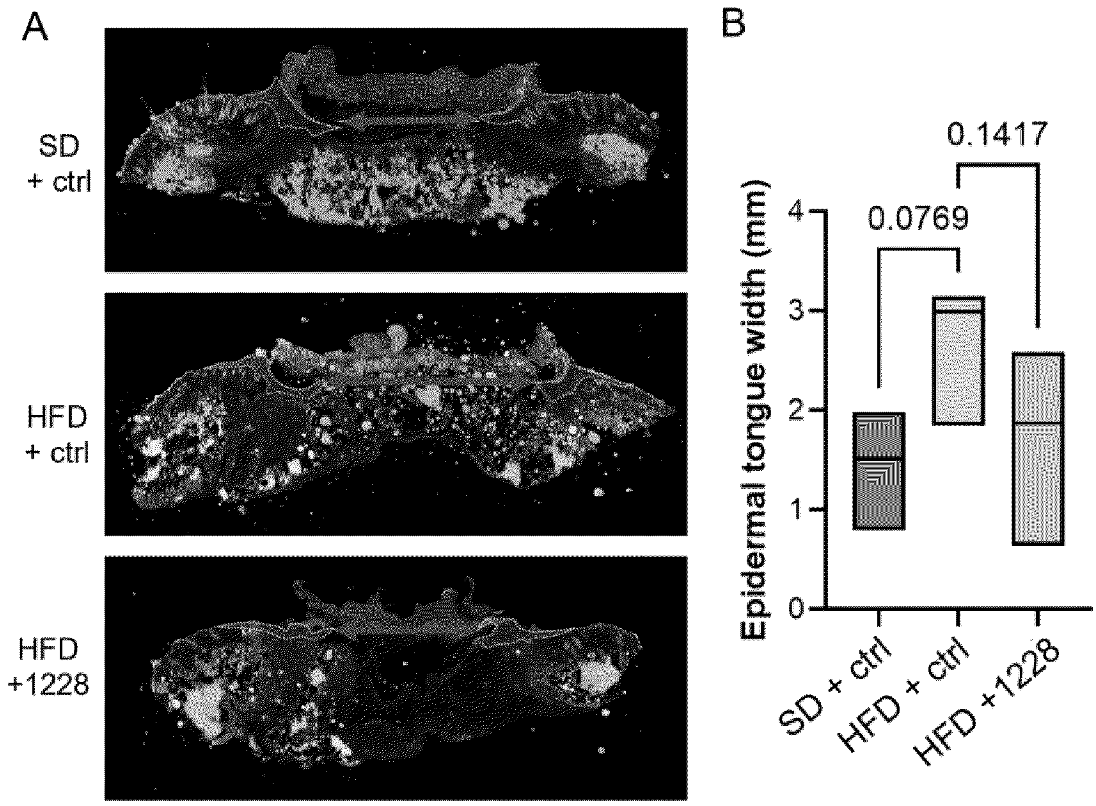
A
SD
+ ctrl
HFD
+ ctrl
HFD
+1228
B
0.1417
0.0769
Epidermal tongue width (mm)
0
1
2
3
4
SD + ctrl
HFD + ctrl
HFD +1228

FIG. 55

**INTERNATIONAL SEARCH REPORT**

International application No. **PCT/CN2023/105745**

**A. CLASSIFICATION OF SUBJECT MATTER**

C07D487/04(2006.01)i； C07D519/00(2006.01)i； A61K31/497(2006.01)i； A61K31/5377(2006.01)i； A61K31/4188(2006.01)i； A61K31/454(2006.01)i； A61K31/501(2006.01)i； A61K31/437(2006.01)i； A61K31/496(2006.01)i； A61P19/02(2006.01)i； A61P37/06(2006.01)i； A61P37/00(2006.01)i； A61P35/00(2006.01)i； A61P17/04(2006.01)i； A61P17/10(2006.01)i； A61P17/14(2006.01)i； A61P17/00(2006.01)i； A61P17/00(2006.01)i； A61P25/28(2006.01)i； A61P27/02(2006.01)i； A61P3/10(2006.01)i； A61P9/10(2006.01)i； A61P11/06(2006.01)i； A61P11/02(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

DWPI, CNTXT, STN(REGISTRY, CAPLUS): 河南迈英诺, 酪氨酸激酶, 抑制剂, +吡咯并, 咪唑, 吲唑, 苯酚, HENAN MEDINNO, tyrosine kinase, inhibitor, JAK, TRK, RET, +pyrrolo, imidazol, indazol, phenol, hydroxyphenyl, STN中结构式检索, structural formula search in STN

**C. DOCUMENTS CONSIDERED TO BE RELEVANT**

| Category* | Citation of document, with indication, where appropriate, of the relevant passages | Relevant to claim No. |
|---|---|---|
| X | CN 111606908 A (HENAN MAIYINGNUO PHARMACEUTICAL TECHNOLOGY CO., LTD.) 01 September 2020 (2020-09-01) description, paragraphs [0352]-[0447] and [0337]-[0349], embodiments 1-60 | 1-42 |
| X | CN 114075200 A (HENAN MAIYINGNUO PHARMACEUTICAL TECHNOLOGY CO., LTD.) 22 February 2022 (2022-02-22) description, paragraphs [0323]-[0415] and [0300]-[0319], embodiments 1-57 | 1-42 |
| A | CN 103717599 A (PFIZER INC.) 09 April 2014 (2014-04-09) entire document | 1-42 |
| A | WO 2017077283 A1 (TOPIVERT PHARMA LIMITED) 11 May 2017 (2017-05-11) entire document | 1-42 |
| A | WO 2017077288 A1 (TOPIVERT PHARMA LIMITED) 11 May 2017 (2017-05-11) entire document | 1-42 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 October 2023** | **13 October 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/105745**

**C. DOCUMENTS CONSIDERED TO BE RELEVANT**

| Category* | Citation of document, with indication, where appropriate, of the relevant passages | Relevant to claim No. |
|---|---|---|
| A | CN 113121509 A (LU LIANG) 16 July 2021 (2021-07-16)<br>entire document | 1-42 |
| A | CN 114524818 A (SHENZHEN KANGSU PHARMACEUTICAL TECHNOLOGY CO., LTD.) 24 May 2022 (2022-05-24)<br>entire document | 1-42 |
| A | 房良华 (FANG, Lianghua). "第三节, 多靶点小分子靶向药物 (Non-official translation: Section 3, Multi-Target Small Molecule Targeted Medicine)"<br>*现代肿瘤免疫靶向治疗 (Modern Tumor Immuno-Targeted Therapy),*<br>30 November 2010 (2010-11-30),<br>pp. 316-329 | 1-42 |

Form PCT/ISA/210 (second sheet) (July 2022)

**INTERNATIONAL SEARCH REPORT**
**Information on patent family members**

International application No.
**PCT/CN2023/105745**

| Patent document cited in search report | | | Publication date (day/month/year) | Patent family member(s) | | | Publication date (day/month/year) |
|---|---|---|---|---|---|---|---|
| CN | 111606908 | A | 01 September 2020 | KR | 20210132666 | A | 04 November 2021 |
| | | | | AU | 2020228799 | A1 | 07 October 2021 |
| | | | | IL | 285783 | A | 31 October 2021 |
| | | | | MX | 2021010242 | A | 21 September 2021 |
| | | | | EP | 3932919 | A1 | 05 January 2022 |
| | | | | EP | 3932919 | A4 | 14 December 2022 |
| | | | | US | 2022073524 | A1 | 10 March 2022 |
| | | | | US | 11629148 | B2 | 18 April 2023 |
| | | | | WO | 2020173400 | A1 | 03 September 2020 |
| | | | | CL | 2021002244 | A1 | 18 March 2022 |
| | | | | CA | 3131293 | A1 | 03 September 2020 |
| | | | | PE | 20212069 | A1 | 26 October 2021 |
| | | | | JP | 2022523274 | A | 21 April 2022 |
| | | | | ZA | 202106511 | B | 31 August 2022 |
| | | | | US | 2023192711 | A1 | 22 June 2023 |
| CN | 114075200 | A | 22 February 2022 | TW | 202206434 | A | 16 February 2022 |
| | | | | WO | 2022033562 | A1 | 17 February 2022 |
| | | | | EP | 4186907 | A1 | 31 May 2023 |
| CN | 103717599 | A | 09 April 2014 | DOP | 2014000016 | A | 15 July 2014 |
| | | | | CU | 20140009 | A7 | 26 March 2014 |
| | | | | WO | 2013014567 | A1 | 31 January 2013 |
| | | | | MX | 2014001004 | A | 13 May 2014 |
| | | | | NI | 201400005 | A | 23 April 2014 |
| | | | | BR | 112014001801 | A2 | 14 February 2017 |
| | | | | EP | 2736907 | A1 | 04 June 2014 |
| | | | | EP | 2736907 | B1 | 07 October 2015 |
| | | | | KR | 20140026627 | A | 05 March 2014 |
| | | | | AR | 087348 | A1 | 19 March 2014 |
| | | | | CO | 6862160 | A2 | 10 February 2014 |
| | | | | AP | 201407372 | D0 | 31 January 2014 |
| | | | | TW | 201313712 | A | 01 April 2013 |
| | | | | TWI | 462922 | B | 01 December 2014 |
| | | | | JP | 5579351 | B1 | 27 August 2014 |
| | | | | JP | 2014522865 | A | 08 September 2014 |
| | | | | ECSP | 14013222 | A | 31 March 2014 |
| | | | | UY | 34220 | A | 28 February 2013 |
| | | | | AU | 2012288491 | A1 | 30 January 2014 |
| | | | | CA | 2841882 | A1 | 31 January 2013 |
| | | | | CR | 20140042 | A | 19 March 2014 |
| | | | | GT | 201400016 | A | 19 February 2015 |
| | | | | UA | 108442 | C2 | 27 April 2015 |
| | | | | EA | 201490357 | A1 | 30 May 2014 |
| | | | | IL | 230662 | A0 | 31 March 2014 |
| | | | | US | 2013029968 | A1 | 31 January 2013 |
| | | | | US | 8575336 | B2 | 05 November 2013 |
| | | | | MA | 35285 | B1 | 03 July 2014 |
| | | | | CL | 2014000122 | A1 | 18 August 2014 |
| | | | | US | 2014024634 | A1 | 23 January 2014 |
| | | | | US | 8895544 | B2 | 25 November 2014 |
| | | | | AP | 2014007372 | A0 | 31 January 2014 |

Form PCT/ISA/210 (patent family annex) (July 2022)

## INTERNATIONAL SEARCH REPORT
**Information on patent family members**

International application No.
**PCT/CN2023/105745**

| Patent document cited in search report | Publication date (day/month/year) | Patent family member(s) | Publication date (day/month/year) |
|---|---|---|---|
| | | MD 20140002 A2 | 30 June 2014 |
| | | PE 20141059 A1 | 30 August 2014 |
| | | TN 2014000033 A1 | 01 July 2015 |
| WO 2017077283 A1 | 11 May 2017 | EP 3371184 A1 | 12 September 2018 |
| | | US 2018319791 A1 | 08 November 2018 |
| | | US 10851097 B2 | 01 December 2020 |
| | | DK 3712152 T3 | 22 March 2021 |
| | | EP 3712152 A1 | 23 September 2020 |
| | | EP 3712152 B1 | 13 January 2021 |
| | | ES 2864035 T3 | 13 October 2021 |
| | | CA 3037243 A1 | 11 May 2017 |
| | | PT 3712152 T | 15 April 2021 |
| | | EP 3865481 A1 | 18 August 2021 |
| | | PL 3712152 T3 | 02 August 2021 |
| WO 2017077288 A1 | 11 May 2017 | CA 3037248 A1 | 11 May 2017 |
| | | US 2020223843 A1 | 16 July 2020 |
| | | US 10882859 B2 | 05 January 2021 |
| | | PT 3371185 T | 28 December 2020 |
| | | PL 3371185 T3 | 06 April 2021 |
| | | DK 3371185 T3 | 30 November 2020 |
| | | EP 3371185 A1 | 12 September 2018 |
| | | EP 3371185 B1 | 30 September 2020 |
| | | ES 2840650 T3 | 07 July 2021 |
| | | EP 3831830 A1 | 09 June 2021 |
| | | US 2018370963 A1 | 27 December 2018 |
| | | US 10556901 B2 | 11 February 2020 |
| CN 113121509 A | 16 July 2021 | EP 4071145 A1 | 12 October 2022 |
| | | EP 4071145 A4 | 19 April 2023 |
| | | US 2022388992 A1 | 08 December 2022 |
| | | WO 2021136345 A1 | 08 July 2021 |
| | | JP 2023508772 A | 03 March 2023 |
| CN 114524818 A | 24 May 2022 | WO 2022152274 A1 | 21 July 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 111606908 B
- US 20220073524 A1
- CN 112933095 A


### Non-patent literature cited in the description


- *Int. J. Dermatol.*, 22 August 2021
- *Cell Stem Cell*, 04 June 2020, vol. 26, 1-16
- **STAHL** ; **WERMUTH**. Handbook of Pharmaceutical Salts: Properties, Selection and Use. Wiley-VCH, 2002
- *CHEMICAL ABSTRACTS*, 21645-51-2
- *CHEMICAL ABSTRACTS*, 477600-75-2
- *CHEMICAL ABSTRACTS*, 127-19-5
- *Cell Reports*, 2023
- *Adv Wound Care (New Rochelle)*, 01 July 2014, vol. 3 (7), 445-464